(19) Europäisches Patentamt / European Patent Office / Office européen des brevets

(11) **EP 3 585 777 B1**

(12) # EUROPÄISCHE PATENTSCHRIFT

(45) Veröffentlichungstag und Bekanntmachung des Hinweises auf die Patenterteilung:
**10.05.2023 Patentblatt 2023/19**

(21) Anmeldenummer: **17787881.6**

(22) Anmeldetag: **26.09.2017**

(51) Internationale Patentklassifikation (IPC):
***C07D 403/10** (2006.01)* ***C07D 403/14** (2006.01)*
***H01L 51/00** (2006.01)*

(52) Gemeinsame Patentklassifikation (CPC):
**C07D 403/10; C07D 403/14;** Y02E 10/549;
Y02P 70/50

(86) Internationale Anmeldenummer:
**PCT/EP2017/074409**

(87) Internationale Veröffentlichungsnummer:
**WO 2018/153510 (30.08.2018 Gazette 2018/35)**

(54) **ORGANISCHE MOLEKÜLE, INSBESONDERE ZUR VERWENDUNG IN ORGANISCHEN OPTOELEKTRONISCHEN VORRICHTUNGEN**

ORGANIC MOLECULES, ESPECIALLY FOR USE IN ORGANIC OPTOELECTRONIC DEVICES

MOLÉCULES ORGANIQUES, EN PARTICULIER DESTINÉES À ÊTRE UTILISÉES DANS DES DISPOSITIFS OPTOÉLECTRONIQUES ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.02.2017 DE 102017103542**

(43) Veröffentlichungstag der Anmeldung:
**01.01.2020 Patentblatt 2020/01**

(73) Patentinhaber: **Samsung Display Co., Ltd.**
**Gyeonggi-do 17113 (KR)**

(72) Erfinder: **SEIFERMANN, Stefan**
**77815 Bühl (DE)**

(74) Vertreter: **Dr. Weitzel & Partner**
**Patent- und Rechtsanwälte mbB**
**Friedenstrasse 10**
**89522 Heidenheim (DE)**

(56) Entgegenhaltungen:
**WO-A1-2015/199303** **WO-A1-2016/116497**
**WO-A1-2016/116529** **US-A1- 2016 126 474**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 585 777 B1

**Beschreibung**

Die Erfindung betrifft rein organische Moleküle und deren Verwendung in organischen lichtemittierenden Dioden (OLEDs) und in anderen organischen optoelektronischen Vorrichtungen. Die US 2016/126474 A1 beschreibt Carbazol-basierte Verbindungen und organische lichtemittierende Vorrichtungen mit derartigen Verbindungen.

Der vorliegenden Erfindung lag die Aufgabe zu Grunde, Moleküle bereitzustellen, die sich zur Verwendung in optoelektronischen Vorrichtungen eignen.

Diese Aufgabe wird durch die Erfindung gelöst, die eine neue Klasse von organischen Molekülen bereitstellt.

Die erfindungsgemäßen organischen Moleküle sind rein organische Moleküle, weisen also keine Metallionen auf und grenzen sich so von den zur Verwendung in organischen optoelektronischen Vorrichtungen bekannten Metallkomplexverbindungen ab.

Die erfindungsgemäßen organischen Moleküle zeichnen sich durch Emissionen im blauen, himmelblauen oder grünen Spektralbereich aus. Die Photolumineszenzquantenausbeuten der erfindungsgemäßen organischen Moleküle betragen insbesondere 20 % und mehr. Die erfindungsgemäßen Moleküle zeigen insbesondere thermisch aktivierte verzögerte Fluoreszenz (TADF). Die Verwendung der erfindungsgemäßen Moleküle in einer optoelektronischen Vorrichtung, beispielsweise einer organischen lichtemittierenden Diode (OLED), führt zu höheren Effizienzen der Vorrichtung. Entsprechende OLEDs weisen eine höhere Stabilität auf als OLEDs mit bekannten Emittermaterialien und vergleichbarer Farbe.

Unter dem blauen Spektralbereich wird hier der sichtbare Bereich von 420 nm bis 470 nm verstanden. Unter dem himmelblauen Spektralbereich wird hier der Bereich von 470 nm bis 499 nm verstanden. Unter dem grünen Spektralbereich wird hier der Bereich von 500 nm bis 599 nm verstanden. Dabei liegt das Emissionsmaximum im jeweiligen Bereich.

Die organischen Moleküle enthalten eine erste chemische Einheit, die eine Struktur gemäß Formel I aufweisen oder aus einer Struktur gemäß Formel I bestehen,

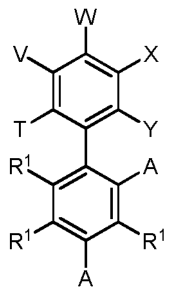

Formel I

und

- zwei zweite chemische Einheiten D jeweils bei jedem Auftreten gleich oder verschieden aufweisend eine oder bestehend aus einer Struktur gemäß Formel II,

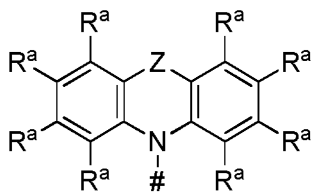

Formel II

Hierbei ist die erste chemische Einheit jeweils über eine Einfachbindung mit den zwei zweiten chemischen Einheiten D verknüpft.

T ist Anknüpfungspunkt der Einfachbindung zwischen der chemischen Einheit gemäß Formel I und einer chemischen Einheit D oder ist H.

V ist Anknüpfungspunkt der Einfachbindung zwischen der chemischen Einheit gemäß Formel I und einer chemischen Einheit D oder ist H.

W ist Anknüpfungspunkt der Einfachbindung zwischen der chemischen Einheit gemäß Formel I und einer

chemischen Einheit D oder ist ausgewählt aus der Gruppe bestehend aus H, CN und $CF_3$.

X ist Anknüpfungspunkt der Einfachbindung zwischen der chemischen Einheit gemäß Formel I und einer chemischen Einheit D oder ist ausgewählt aus der Gruppe bestehend aus H, CN und $CF_3$.

Y ist Anknüpfungspunkt der Einfachbindung zwischen der chemischen Einheit gemäß Formel I und einer chemischen Einheit D oder ist ausgewählt aus der Gruppe bestehend aus H, CN und $CF_3$;
# ist Anknüpfungspunkt der Einfachbindung zwischen der jeweiligen chemischen Einheit D und der chemischen Einheit gemäß Formel I.

Z ist bei jedem Auftreten gleich oder verschieden eine direkte Bindung oder ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$.

A ist bei jedem Auftreten ausgewählt aus der Gruppe bestehend aus CN und $CF_3$, wobei eines der beiden A gleich $CF_3$ und das andere A gleich CN ist. A kann somit weder in beiden Fällen gleich CN sein, noch in beiden Fällen gleich $CF_3$ sein.

$R^1$ ist bei jedem Auftreten gleich oder verschieden H, Deuterium, eine lineare Alkylgruppe mit 1 bis 5 C-Atomen, wobei jeweils ein oder mehrere H-Atome durch Deuterium ersetzt sein können; eine lineare Alkenyl- oder Alkinylgruppe mit 2 bis 8 C-Atomen, wobei jeweils ein oder mehrere H-Atome durch Deuterium ersetzt sein können; eine verzweigte oder cyclische Alkyl-, Alkenyl- oder Alkinylgruppe mit 3 bis 10 C-Atomen, wobei jeweils ein oder mehrere H-Atome durch Deuterium ersetzt sein können, oder ein aromatisches oder heteroaromatisches Ringsystem mit 5 bis 15 aromatischen Ringatomen, das jeweils mit einem oder mehreren Resten $R^6$ substituiert sein kann.

$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;

oder eine lineare Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
oder ein aromatisches oder heteroaromatisches Ringsystem mit 5 bis 60 aromatischen Ringatomen, das jeweils mit einem oder mehreren Resten $R^5$ substituiert sein kann; oder eine Aryloxy- oder Heteroaryloxygruppe mit 5 bis 60 aromatischen Ringatomen, die mit einem oder mehreren Resten $R^5$ substituiert sein kann;
oder eine Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann.

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können und wobei ein oder mehrere H-Atome durch Deuterium, CN, $CF_3$ oder $NO_2$ ersetzt sein können;

oder eine lineare Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können und wobei ein oder mehrere H-Atome durch Deuterium, CN, $CF_3$ oder $NO_2$ ersetzt sein können; oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können;
oder ein aromatisches oder heteroaromatisches Ringsystem mit 5 bis 60 aromatischen Ringatomen, das jeweils

mit einem oder mehreren Resten $R^6$ substituiert sein kann; oder eine Aryloxy- oder Heteroaryloxygruppe mit 5 bis 60 aromatischen Ringatomen, die mit einem oder mehreren Resten $R^6$ substituiert sein kann;
oder eine Diarylaminogruppe, Diheteroarylaminogruppe oder Arylheteroarylaminogruppe mit 10 bis 40 aromatischen Ringatomen, welche mit einem oder mehreren Reste $R^6$ substituiert sein kann.

$R^6$ ist bei jedem Auftreten gleich oder verschieden H, Deuterium, OH, $CF_3$, CN, F, eine lineare Alkyl-, Alkoxy- oder Thioalkoxygruppe mit 1 bis 5 C-Atomen, wobei jeweils ein oder mehrere H-Atome durch Deuterium, CN, $CF_3$ oder $NO_2$ ersetzt sein können;

oder eine lineare Alkenyl- oder Alkinylgruppe mit 2 bis 5 C-Atomen, wobei jeweils ein oder mehrere H-Atome durch Deuterium, CN, $CF_3$ oder $NO_2$ ersetzt sein können;
oder eine verzweigte oder cyclische Alkyl-, Alkenyl-, Alkinyl-, Alkoxy- oder Thioalkoxygruppe mit 3 bis 5 C-Atomen, wobei jeweils ein oder mehrere H-Atome durch Deuterium, CN, $CF_3$ oder $NO_2$ ersetzt sein können;
oder ein aromatisches oder heteroaromatisches Ringsystem mit 5 bis 60 aromatischen Ringatomen;
oder eine Aryloxy- oder Heteroaryloxygruppe mit 5 bis 60 aromatischen Ringatomen;
oder eine Diarylaminogruppe, Diheteroarylaminogruppe oder Arylheteroarylaminogruppe mit 10 bis 40 aromatischen Ringatomen.

Erfindungsgemäß kann jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden.
Erfindungsgemäß ist genau ein Rest ausgewählt aus W, X und Y gleich CN oder $CF_3$ und genau zwei Reste ausgewählt aus der Gruppe bestehend aus T, V, W, X und Y sind gleich einem Anknüpfungspunkt einer Einfachbindung zwischen der chemischen Einheit gemäß Formel I und einer chemischen Einheit D.
In einer weiteren Ausführungsform weisen die erfindungsgemäßen organischen Moleküle eine Struktur der Formel Ia auf bzw. bestehen aus einer Struktur der Formel Ia:

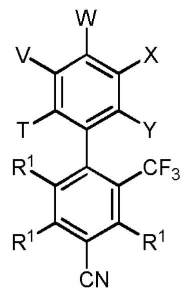

Formel Ia

wobei für $R^1$, T, V, W, X, und Y die für Formel ! und II genannten Definitionen gelten.
In einer Ausführungsform ist $R^1$ bei jedem Auftreten gleich oder verschieden H, Methyl oder Phenyl.
In einer Ausführungsform ist W gleich CN.
In einer weiteren Ausführungsform der organischen Moleküle weist die chemische Gruppe D bei jedem Auftreten gleich oder verschieden eine Struktur der Formel IIa auf bzw. besteht aus einer Struktur der Formel IIa:

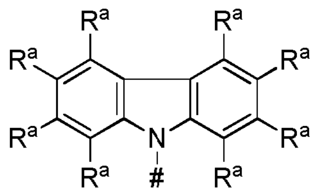

Formel IIa

wobei für # und $R^a$ die für Formel I und II genannten Definitionen gelten.
In einer weiteren Ausführungsform der erfindungsgemäßen organischen Moleküle weist die chemische Einheit D bei jedem Auftreten gleich oder verschieden eine Struktur der Formel IIb, der Formel IIb-2, der Formel IIb-3 oder der der Formel IIb-4 auf oder besteht daraus:

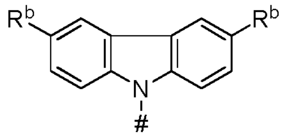

Formel IIb

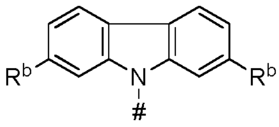

Formel IIb-2

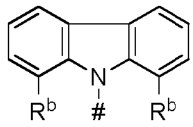

Formel IIb-3

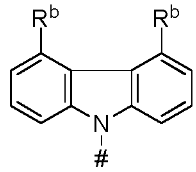

Formel IIb-4

wobei gilt

$R^b$ ist bei jedem Auftreten gleich oder verschieden $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
oder eine lineare Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können;
oder ein aromatisches oder heteroaromatisches Ringsystem mit 5 bis 60 aromatischen Ringatomen, das jeweils mit einem oder mehreren Resten $R^5$ substituiert sein kann;
oder eine Aryloxy- oder Heteroaryloxygruppe mit 5 bis 60 aromatischen Ringatomen, die mit einem oder mehreren Resten $R^5$ substituiert sein kann;
oder eine Diarylaminogruppe, Diheteroarylaminogruppe oder Arylheteroarylaminogruppe mit 10 bis 40 aromatischen Ringatomen, welche mit einem oder mehreren Resten $R^5$ substituiert sein kann. Ansonsten gelten die oben genannten Definitionen.

In einer weiteren Ausführungsform der erfindungsgemäßen organischen Moleküle weist die chemische Einheit D bei jedem Auftreten gleich oder verschieden eine Struktur der Formel IIc, der Formel IIc-2, der Formel IIc-3 oder der Formel IIc-4 auf oder besteht daraus:

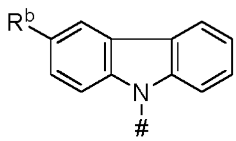

Formel IIc

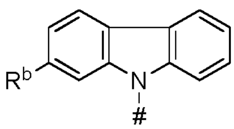

Formel IIc-2

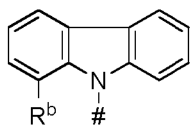

Formel IIc-3

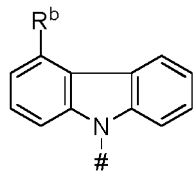

Formel IIc-4

wobei die oben genannten Definitionen gelten.

In einer weiteren Ausführungsform der erfindungsgemäßen organischen Moleküle ist $R^b$ bei jedem Auftreten unabhängig voneinander ausgewählt aus der Gruppe bestehend aus Me, $^iPr$, $^tBu$, CN, $CF_3$, Ph, das jeweils mit einem oder mehreren Resten ausgewählt aus Me, $^iPr$, $^tBu$, CN, $CF_3$ oder Ph substituiert sein kann,

Pyridinyl, das jeweils mit einem oder mehreren Resten ausgewählt aus Me, $^iPr$, $^tBu$, CN, $CF_3$ oder Ph substituiert sein kann,
Pyrimidinyl, das jeweils mit einem oder mehreren Resten ausgewählt aus Me, $^iPr$, $^tBu$, CN, $CF_3$ oder Ph substituiert sein kann,
Carbazolyl, das jeweils mit einem oder mehreren Resten ausgewählt aus Me, $^iPr$, $^tBu$, CN, $CF_3$ oder Ph substituiert sein kann,
Triazinyl, das jeweils mit einem oder mehreren Resten ausgewählt aus Me, $^iPr$, $^tBu$, CN, $CF_3$ oder Ph substituiert

sein kann, und $N(Ph)_2$.

Im Folgenden sind beispielhaft Ausführungsformen der chemischen Gruppe D gezeigt:

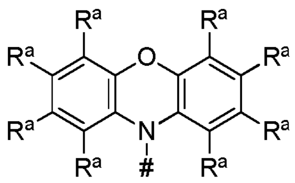
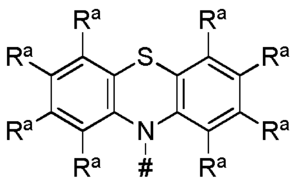
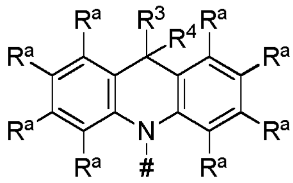
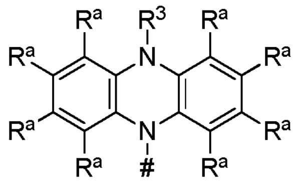
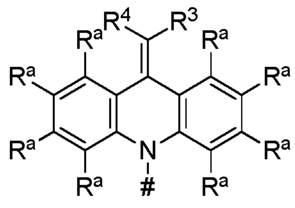
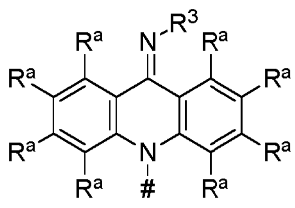
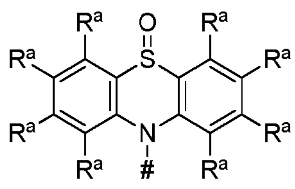
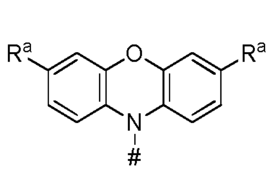
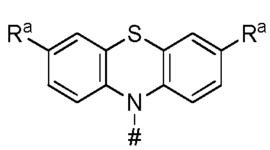
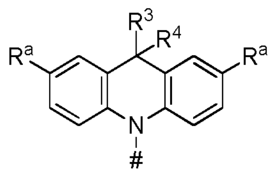
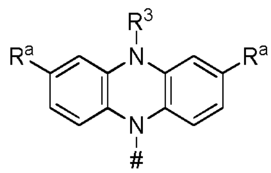
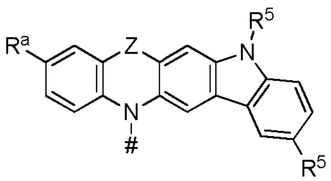
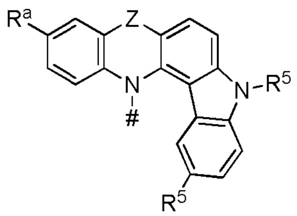
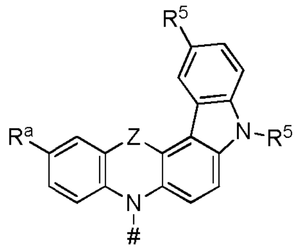
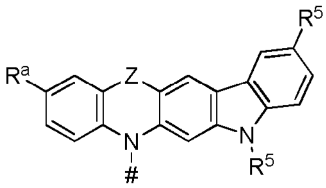
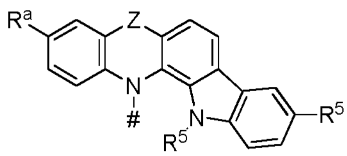
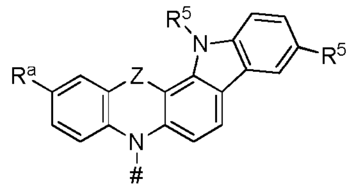
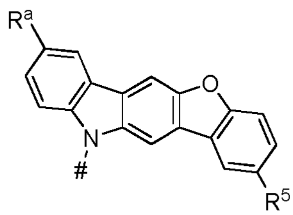
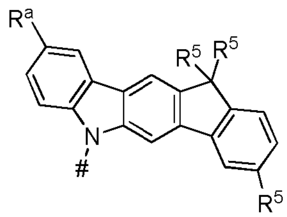
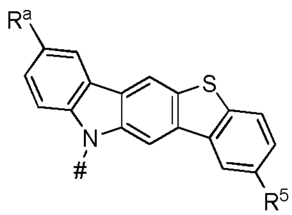
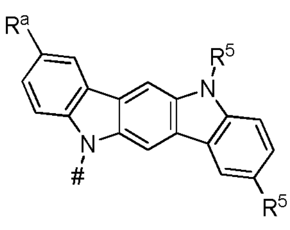

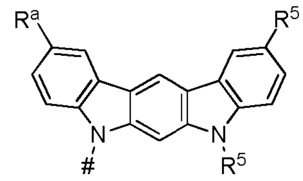
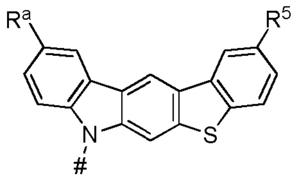
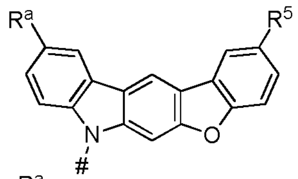

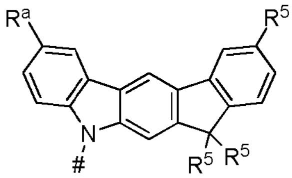
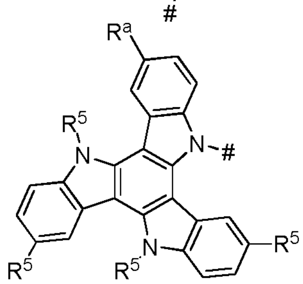

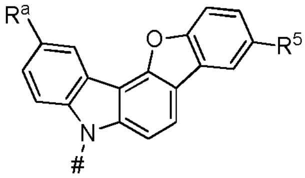
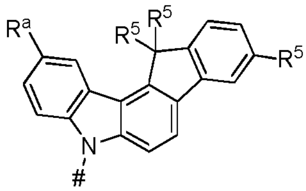
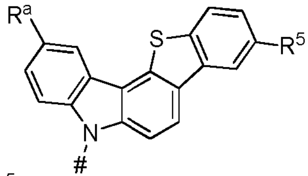

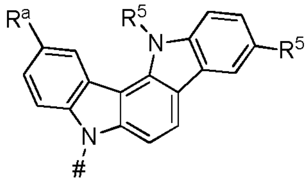
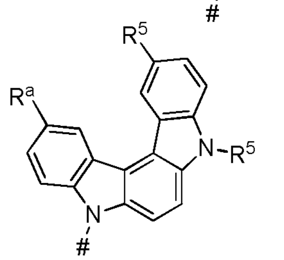

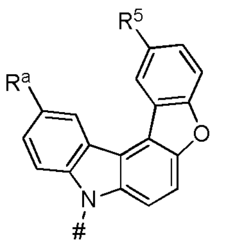
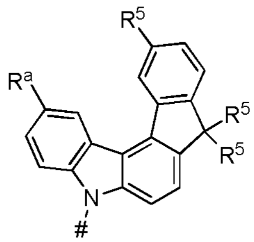
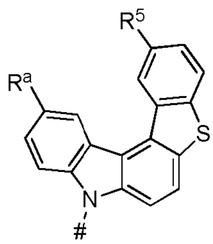

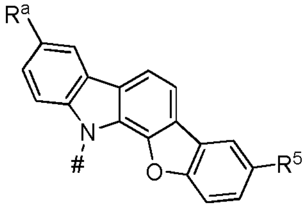
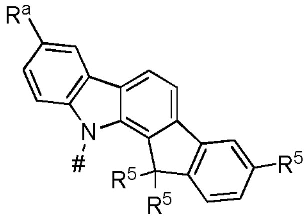
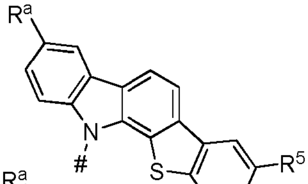

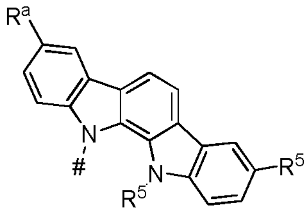
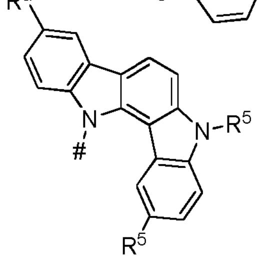

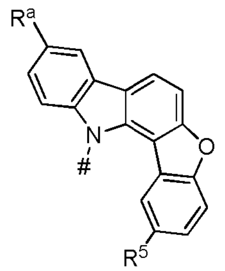

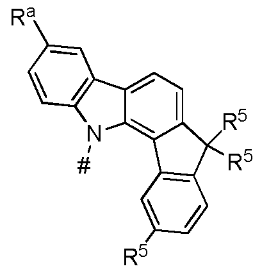

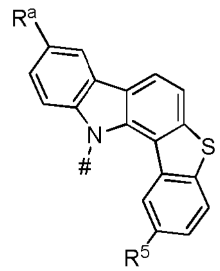

wobei für #, Z, $R^a$, $R^3$, $R^4$ und $R^5$ die oben genannten Definitionen gelten. In einer Ausführungsform ist der Rest $R^5$ bei jedem Auftreten gleich oder verschieden ausgewählt aus der Gruppe bestehend aus H, Methyl, Ethyl, Phenyl und Mesityl. In einer Ausführungsform ist $R^a$ bei jedem Auftreten gleich oder verschieden ausgewählt aus der Gruppe bestehend aus H, Methyl (Me), i-Propyl ($CH(CH_3)_2$)($^i$Pr), t-Butyl ($^t$Bu), Phenyl (Ph), CN, $CF_3$ und Diphenylamin ($NPh_2$).

In einer Ausführungsform weisen die erfindungsgemäßen organischen Moleküle eine Struktur der Formel III-1 oder Formel III-2 auf:

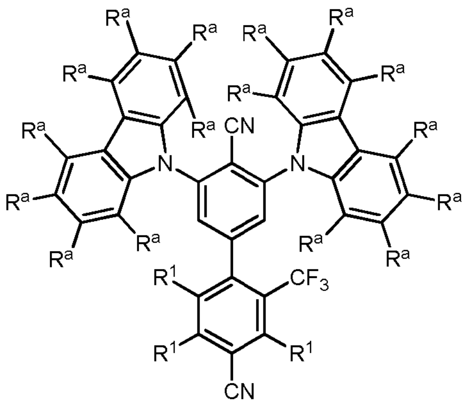

Formel III-1

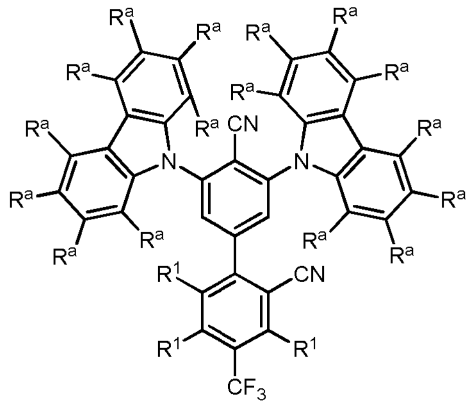

Formel III-2

wobei die oben genannten Definitionen gelten.

In einer weiteren Ausführungsform weisen die erfindungsgemäßen organischen Moleküle eine Struktur der Formel III-1 auf, wobei die für die Formeln III-1 bis III-2 genannten Definitionen gelten.

In einer weiteren Ausführungsform weisen die erfindungsgemäßen organischen Moleküle eine Struktur der Formel IIIa-1 oder Formel IIIa-2 auf:

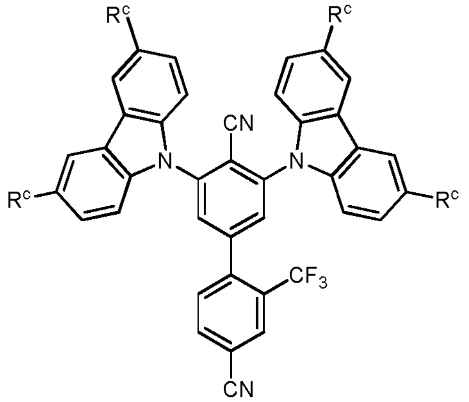

Formel IIIa-1

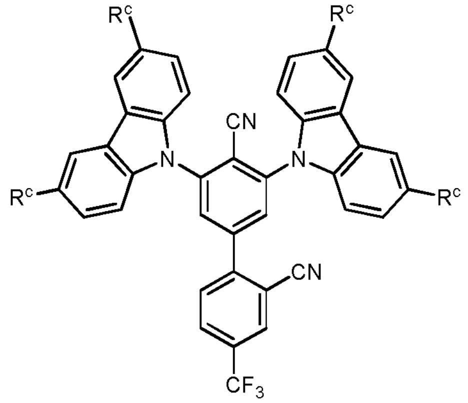

Formel IIIa-2

wobei

$R^c$ bei jedem Auftreten unabhängig voneinander ausgewählt aus der Gruppe bestehend aus Me, $^i$Pr, $^t$Bu, CN, $CF_3$,

Ph, das jeweils mit einem oder mehreren Resten ausgewählt aus Me, $^{i}$Pr, $^{t}$Bu, CN, $CF_3$ oder Ph substituiert sein kann,
Pyridinyl, das jeweils mit einem oder mehreren Resten ausgewählt aus Me, $^{i}$Pr, $^{t}$Bu, CN, $CF_3$ oder Ph substituiert sein kann,
Pyrimidinyl, das jeweils mit einem oder mehreren Resten ausgewählt aus Me, $^{i}$Pr, $^{t}$Bu, CN, $CF_3$ oder Ph substituiert sein kann,
Carbazolyl, das jeweils mit einem oder mehreren Resten ausgewählt aus Me, $^{i}$Pr, $^{t}$Bu, CN, $CF_3$ oder Ph substituiert sein kann,
Triazinyl, das jeweils mit einem oder mehreren Resten ausgewählt aus Me, $^{i}$Pr, $^{t}$Bu, CN, $CF_3$ oder Ph substituiert sein kann,
und $N(Ph)_2$ ist.

In einer weiteren Ausführungsform weisen die erfindungsgemäßen organischen Moleküle eine Struktur der Formel IIIa-1 auf, wobei die oben genannten Definitionen gelten.

In einer weiteren Ausführungsform weisen die erfindungsgemäßen organischen Moleküle eine Struktur der Formel IIIb-1 oder Formel IIIb-2 auf:

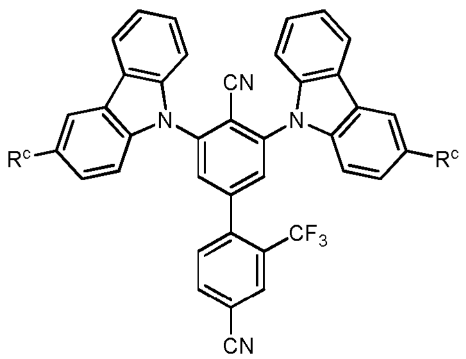

Formel IIIb-1

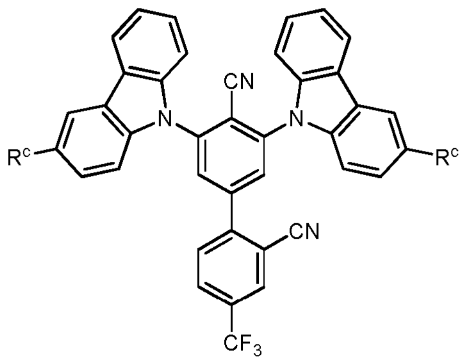

Formel IIIb-2

wobei die oben genannten Definitionen gelten.

In einer weiteren Ausführungsform weisen die erfindungsgemäßen organischen Moleküle eine Struktur der Formel IIIb-1 auf, wobei die oben genannten Definitionen gelten.

In einer weiteren Ausführungsform weisen die erfindungsgemäßen organischen Moleküle eine Struktur der Formel IIIc-1 oder Formel IIIc-2 auf:

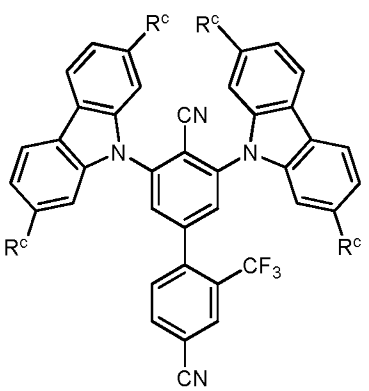

Formel IIIc-1

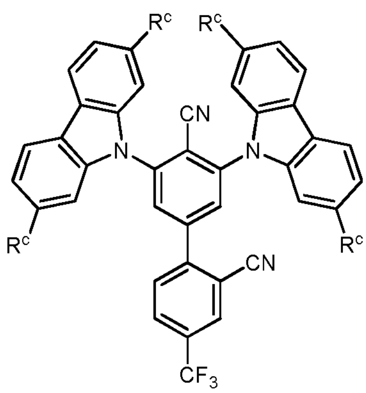

Formel IIIc-2

wobei die oben genannten Definitionen gelten.

In einer weiteren Ausführungsform weisen die erfindungsgemäßen organischen Moleküle eine Struktur der Formel IIIc-1 auf, wobei die oben genannten Definitionen gelten.

In einer weiteren Ausführungsform weisen die erfindungsgemäßen organischen Moleküle eine Struktur der Formel IIId-1 oder Formel IIId-2 auf:

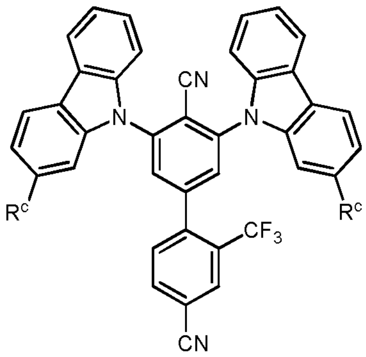

Formel IIId-1

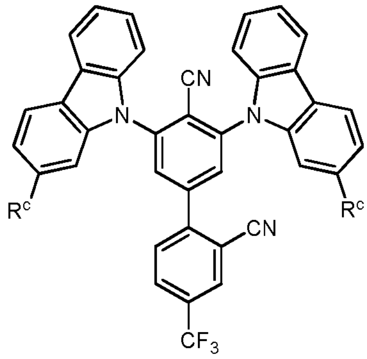

Formel IIId-2

wobei die oben genannten Definitionen gelten.

In einer weiteren Ausführungsform weisen die erfindungsgemäßen organischen Moleküle eine Struktur der Formel IIId-1 auf, wobei die oben genannten Definitionen gelten.

In einer weiteren Ausführungsform weisen die erfindungsgemäßen organischen Moleküle eine Struktur der Formel IIIe-1 oder Formel IIIe-2 auf:

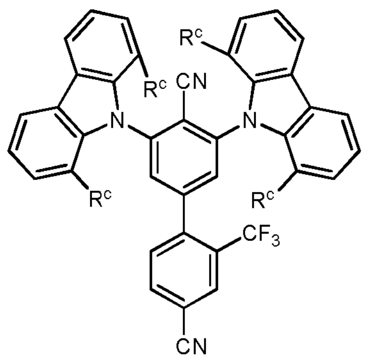

Formel IIIe-1

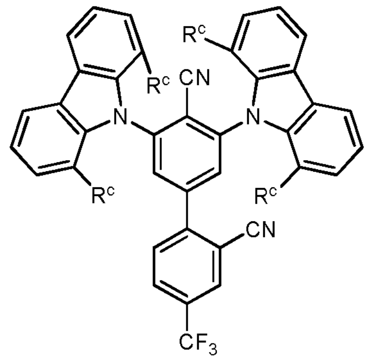

Formel IIIe-2

wobei die oben genannten Definitionen gelten.

In einer weiteren Ausführungsform weisen die erfindungsgemäßen organischen Moleküle eine Struktur der Formel IIIe-1 auf, wobei die oben genannten Definitionen gelten.

In einer weiteren Ausführungsform weisen die erfindungsgemäßen organischen Moleküle eine Struktur der Formel IIIf-1 oder Formel IIIf-2 auf:

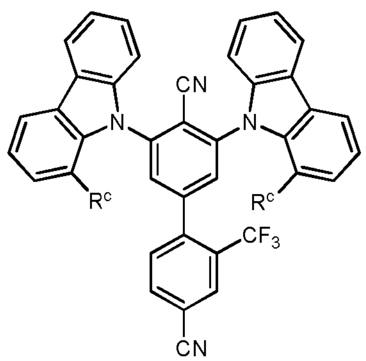

Formel IIIf-1

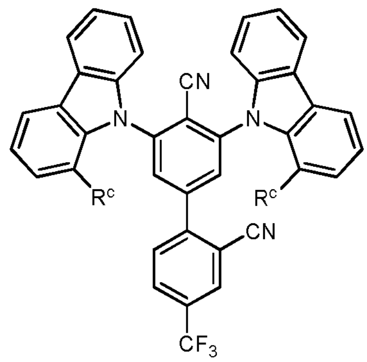

Formel IIIf-2

wobei die oben genannten Definitionen gelten.

In einer weiteren Ausführungsform weisen die erfindungsgemäßen organischen Moleküle eine Struktur der Formel IIIf-1 auf, wobei die oben genannten Definitionen gelten.

In einer weiteren Ausführungsform weisen die erfindungsgemäßen organischen Moleküle eine Struktur der

Formel IIIg-1 oder Formel IIIg-2 auf:

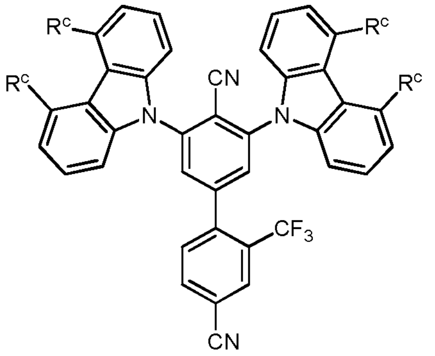

Formel IIIg-1

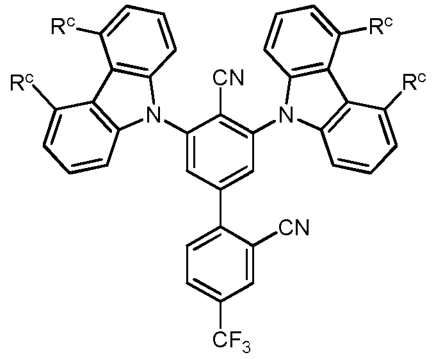

Formel IIIg-2

wobei die oben genannten Definitionen gelten.

In einer weiteren Ausführungsform weisen die erfindungsgemäßen organischen Moleküle eine Struktur der Formel IIIg-1 auf, wobei die oben genannten Definitionen gelten.

In einer weiteren Ausführungsform weisen die erfindungsgemäßen organischen Moleküle eine Struktur der Formel IIIh-1 oder Formel IIIh-2 auf:

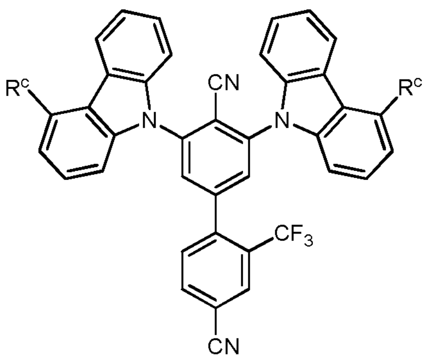

Formel IIIh-1

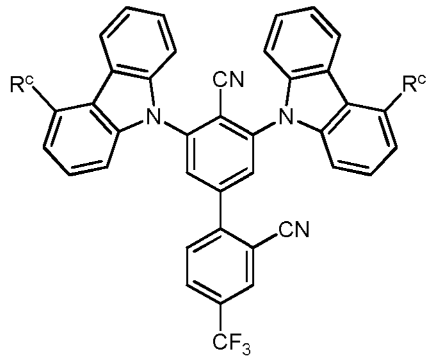

Formel IIIh-2

wobei die oben genannten Definitionen gelten.

In einer weiteren Ausführungsform weisen die erfindungsgemäßen organischen Moleküle eine Struktur der Formel IIIh-1 auf, wobei die oben genannten Definitionen gelten.

In einer Ausführungsform weisen die erfindungsgemäßen organischen Moleküle eine Struktur der Formel IV-1 oder Formel IV-2 auf:

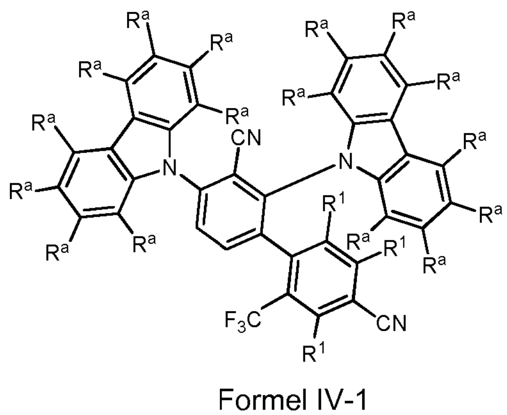

Formel IV-1

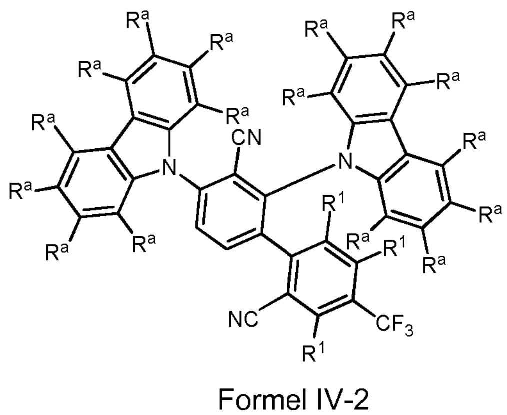

Formel IV-2

wobei die oben genannten Definitionen gelten.

In einer Ausführungsform weisen die erfindungsgemäßen organischen Moleküle eine Struktur der Formel IV-1 auf, wobei die oben genannten Definitionen gelten.

In einer weiteren Ausführungsform weisen die erfindungsgemäßen organischen Moleküle eine Struktur der Formel IVa-1 oder Formel IVa-2 auf:

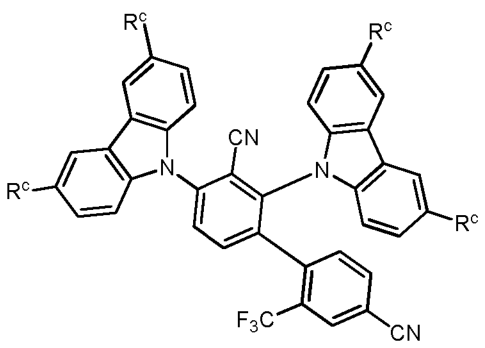

Formel IVa-1

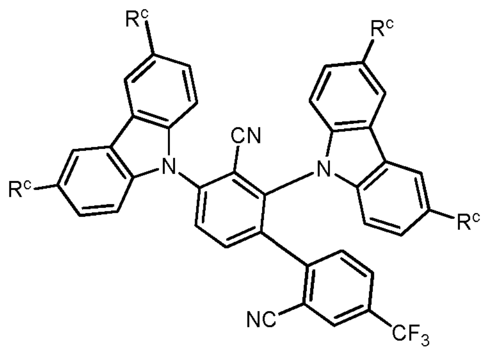

Formel IVa-2

wobei die oben genannten Definitionen gelten.

In einer weiteren Ausführungsform weisen die erfindungsgemäßen organischen Moleküle eine Struktur der Formel IVa-1 auf, wobei die oben genannten Definitionen gelten.

In einer weiteren Ausführungsform weisen die erfindungsgemäßen organischen Moleküle eine Struktur der Formel IVb-1 oder Formel IVb-2 auf:

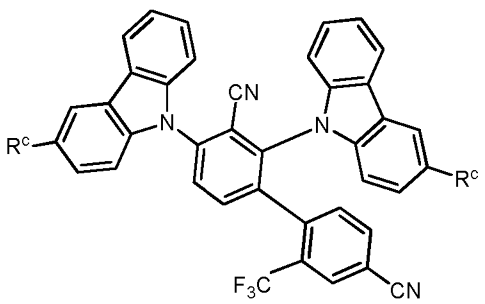

Formel IVb-1

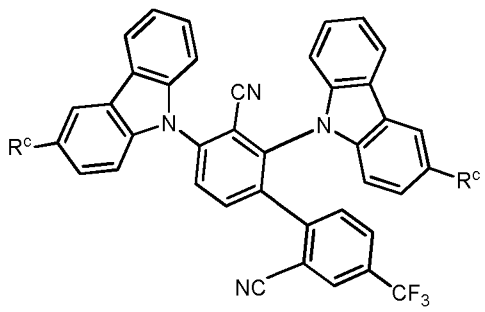

Formel IVb-2

wobei die oben genannten Definitionen gelten.

In einer weiteren Ausführungsform weisen die erfindungsgemäßen organischen Moleküle eine Struktur der Formel IVb-1 auf, wobei die oben genannten Definitionen gelten.

In einer weiteren Ausführungsform weisen die erfindungsgemäßen organischen Moleküle eine Struktur der Formel IVc-1 oder Formel IVc-2 auf:

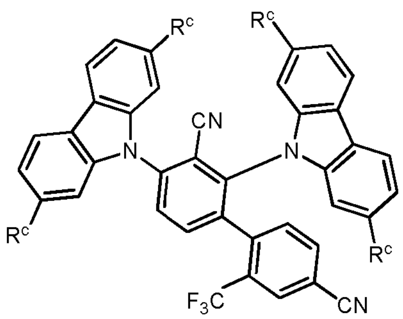

Formel IVc-1

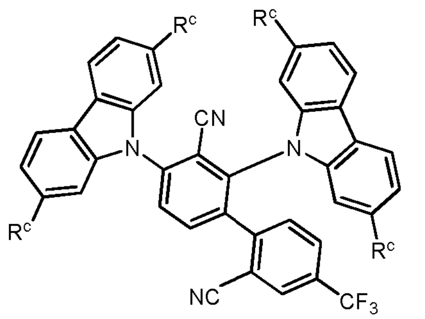

Formel IVc-2

wobei die oben genannten Definitionen gelten.

In einer weiteren Ausführungsform weisen die erfindungsgemäßen organischen Moleküle eine Struktur der Formel IVc-1 auf, wobei die oben genannten Definitionen gelten.

In einer weiteren Ausführungsform weisen die erfindungsgemäßen organischen Moleküle eine Struktur der Formel IVd-1 oder Formel IVd-2 auf:

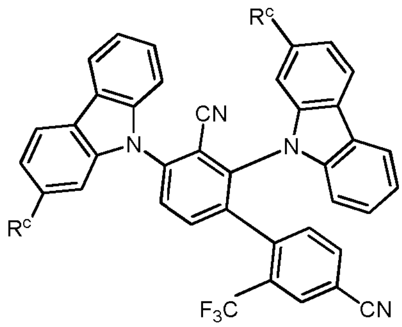

Formel IVd-1

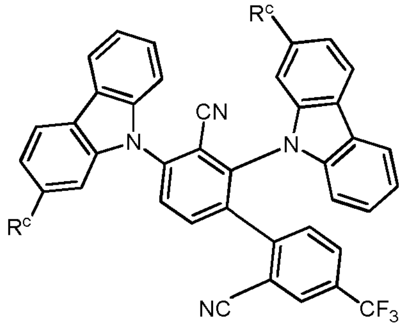

Formel IVd-2

wobei die oben genannten Definitionen gelten.

In einer weiteren Ausführungsform weisen die erfindungsgemäßen organischen Moleküle eine Struktur der Formel IVd-1 auf, wobei die oben genannten Definitionen gelten.

In einer weiteren Ausführungsform weisen die erfindungsgemäßen organischen Moleküle eine Struktur der Formel IVe-1 oder Formel IVe-2 auf:

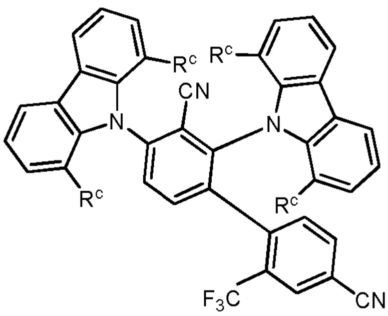

Formel IVe-1

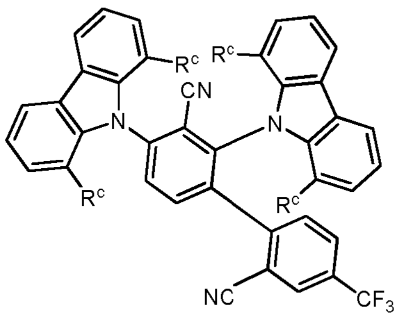

Formel IVe-2

wobei die oben genannten Definitionen gelten.

In einer weiteren Ausführungsform weisen die erfindungsgemäßen organischen Moleküle eine Struktur der Formel IVe-1 auf, wobei die oben genannten Definitionen gelten.

In einer weiteren Ausführungsform weisen die erfindungsgemäßen organischen Moleküle eine Struktur der Formel IVf-1 oder Formel IVf-2 auf:

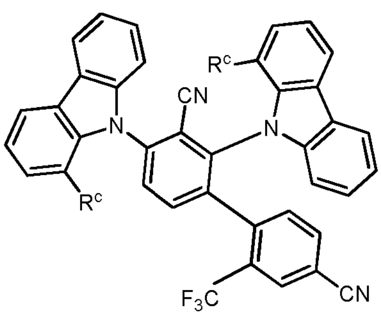

Formel IVf-1

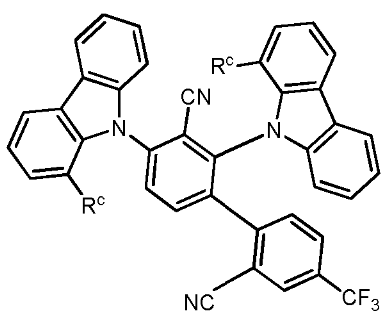

Formel IVf-2

wobei die oben genannten Definitionen gelten.

In einer weiteren Ausführungsform weisen die erfindungsgemäßen organischen Moleküle eine Struktur der Formel IVf-1 auf, wobei die oben genannten Definitionen gelten.

In einer weiteren Ausführungsform weisen die erfindungsgemäßen organischen Moleküle eine Struktur der Formel IVg-1 oder Formel IVg-2 auf:

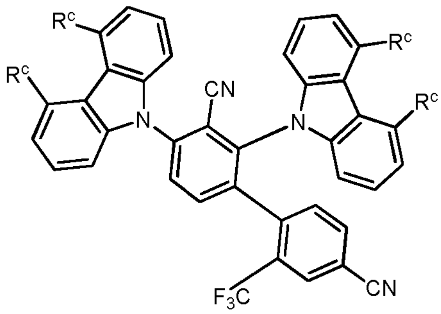

Formel IVg-1

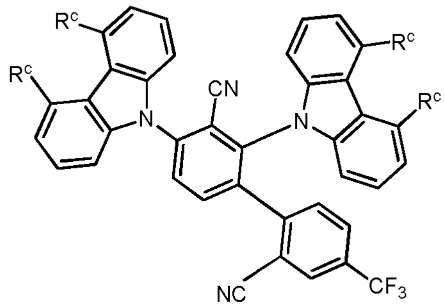

Formel IVg-2

wobei die oben genannten Definitionen gelten.

In einer weiteren Ausführungsform weisen die erfindungsgemäßen organischen Moleküle eine Struktur der Formel IVg-1 auf, wobei die oben genannten Definitionen gelten.

In einer weiteren Ausführungsform weisen die erfindungsgemäßen organischen Moleküle eine Struktur der Formel IVh-1 oder Formel IVh-2 auf:

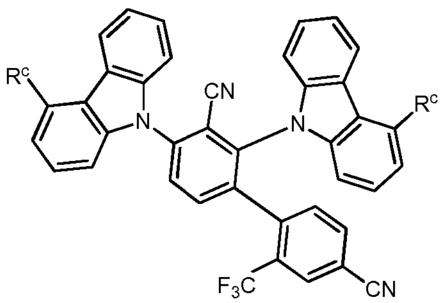

Formel IVh-1

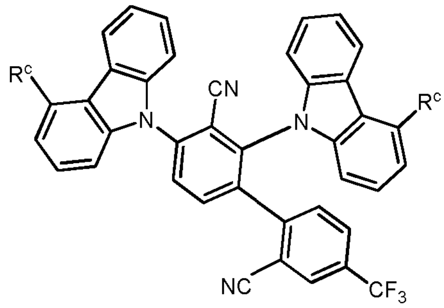

Formel IVh-2

wobei die oben genannten Definitionen gelten.

In einer weiteren Ausführungsform weisen die erfindungsgemäßen organischen Moleküle eine Struktur der Formel IVh-1 auf, wobei die oben genannten Definitionen gelten.

In einer weiteren Ausführungsform weisen die erfindungsgemäßen organischen Moleküle eine Struktur der Formel V-1 oder Formel V-2 auf:

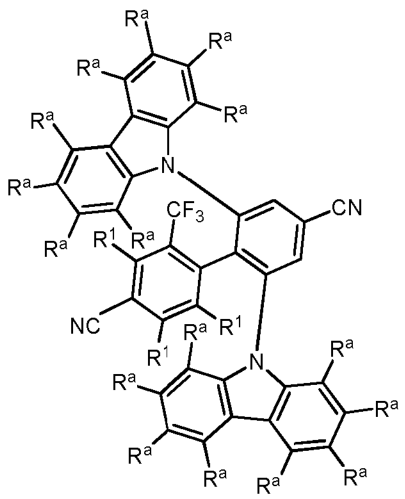

Formel V-1

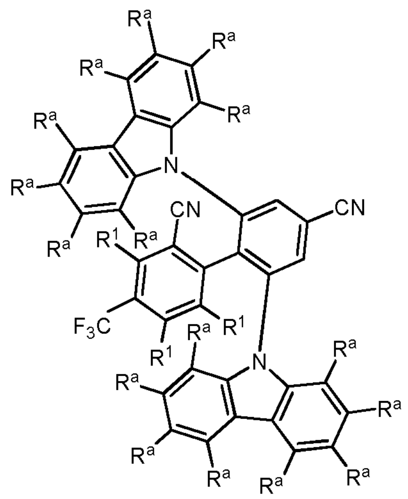

Formel V-2

wobei die oben genannten Definitionen gelten.

In einer weiteren Ausführungsform weisen die erfindungsgemäßen organischen Moleküle eine Struktur der Formel V-1 auf, wobei die oben genannten Definitionen gelten.

In einer weiteren Ausführungsform weisen die erfindungsgemäßen organischen Moleküle eine Struktur der Formel Va-1 oder Formel Va-2 auf:

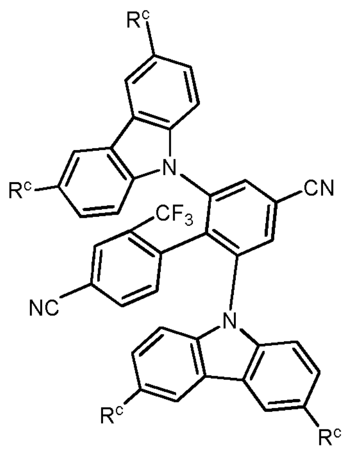

Formel Va-1

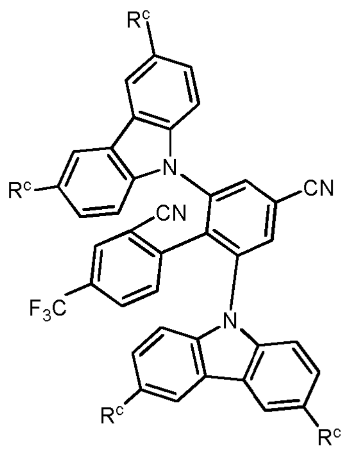

Formel Va-2

wobei die oben genannten Definitionen gelten.

In einer weiteren Ausführungsform weisen die erfindungsgemäßen organischen Moleküle eine Struktur der Formel Va-1 auf, wobei die oben genannten Definitionen gelten.

In einer weiteren Ausführungsform weisen die erfindungsgemäßen organischen Moleküle eine Struktur der Formel Vb-1 oder Formel Vb-2 auf:

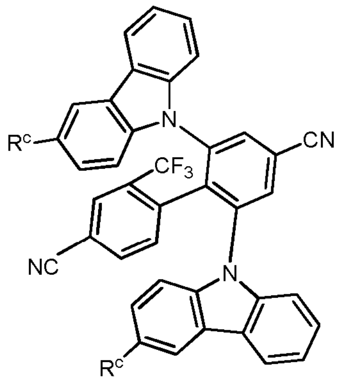

Formel Vb-1

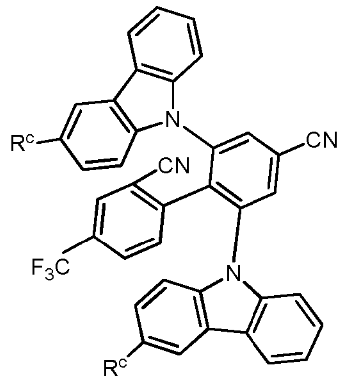

Formel Vb-2

wobei die oben genannten Definitionen gelten.

In einer weiteren Ausführungsform weisen die erfindungsgemäßen organischen Moleküle eine Struktur der Formel Vb-1 auf, wobei die oben genannten Definitionen gelten.

In einer weiteren Ausführungsform weisen die erfindungsgemäßen organischen Moleküle eine Struktur der Formel Vc-1 oder Formel Vc-2 auf:

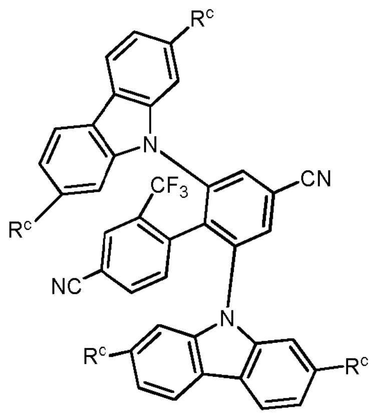

Formel Vc-1

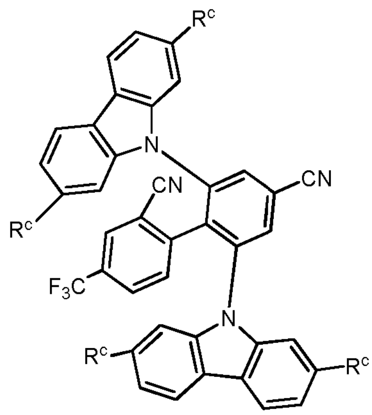

Formel Vc-2

wobei die oben genannten Definitionen gelten.

In einer weiteren Ausführungsform weisen die erfindungsgemäßen organischen Moleküle eine Struktur der Formel Vc-1 auf, wobei die oben genannten Definitionen gelten.

In einer weiteren Ausführungsform weisen die erfindungsgemäßen organischen Moleküle eine Struktur der Formel Vd-1 oder Formel Vd-2 auf:

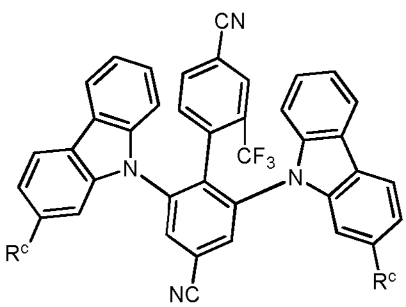

Formel Vd-1

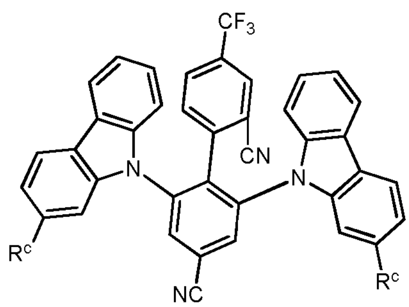

Formel Vd-2

wobei die oben genannten Definitionen gelten.

In einer weiteren Ausführungsform weisen die erfindungsgemäßen organischen Moleküle eine Struktur der

Formel Vd-1 auf, wobei die oben genannten Definitionen gelten.

In einer weiteren Ausführungsform weisen die erfindungsgemäßen organischen Moleküle eine Struktur der Formel Ve-1 oder Formel Ve-2 auf:

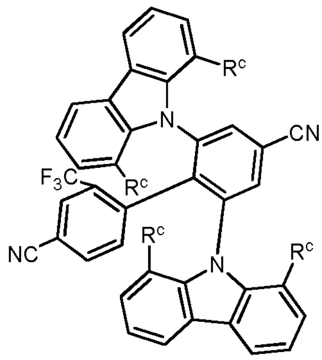

Formel Ve-1

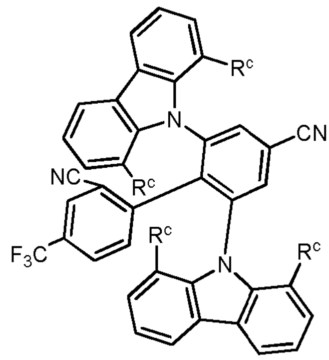

Formel Ve-2

wobei die oben genannten Definitionen gelten.

In einer weiteren Ausführungsform weisen die erfindungsgemäßen organischen Moleküle eine Struktur der Formel Ve-1 auf, wobei die oben genannten Definitionen gelten.

In einer weiteren Ausführungsform weisen die erfindungsgemäßen organischen Moleküle eine Struktur der Formel Vf-1 oder Formel Vf-2 auf:

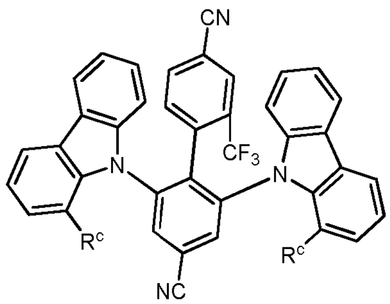

Formel Vf-1

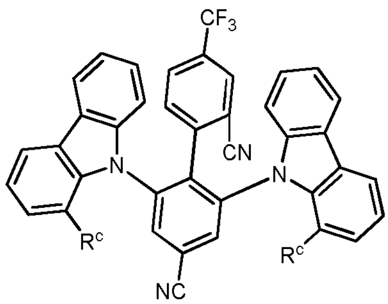

Formel Vf-2

wobei die oben genannten Definitionen gelten.

In einer weiteren Ausführungsform weisen die erfindungsgemäßen organischen Moleküle eine Struktur der Formel Vf-1 auf, wobei die oben genannten Definitionen gelten.

In einer weiteren Ausführungsform weisen die erfindungsgemäßen organischen Moleküle eine Struktur der Formel Vg-1 oder Formel Vg-2 auf:

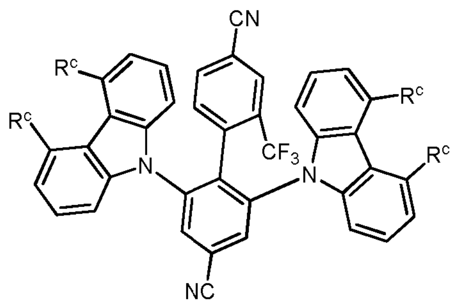

Formel Vg-1

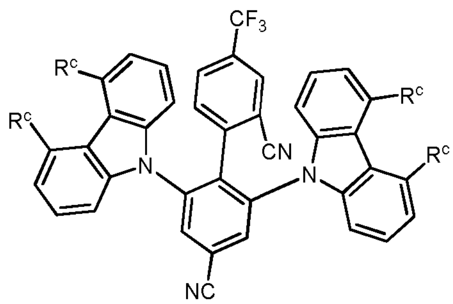

Formel Vg-2

wobei die oben genannten Definitionen gelten.

In einer weiteren Ausführungsform weisen die erfindungsgemäßen organischen Moleküle eine Struktur der

Formel Vg-1 auf, wobei die oben genannten Definitionen gelten.

In einer weiteren Ausführungsform weisen die erfindungsgemäßen organischen Moleküle eine Struktur der Formel Vh-1 oder Formel Vh-2 auf:

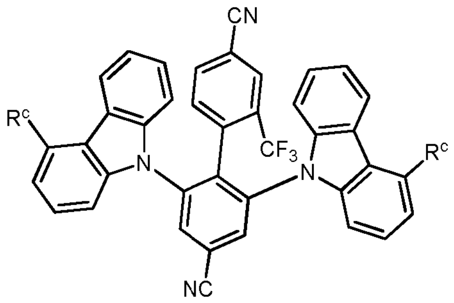

Formel Vh-1

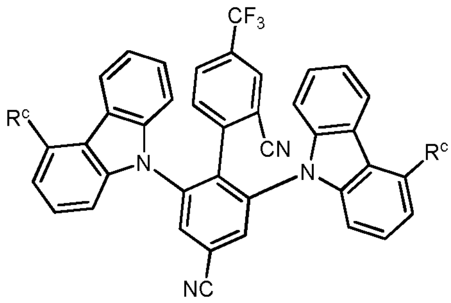

Formel Vh-2

wobei die oben genannten Definitionen gelten.

In einer weiteren Ausführungsform weisen die erfindungsgemäßen organischen Moleküle eine Struktur der Formel Vh-1 auf, wobei die oben genannten Definitionen gelten.

In einer Ausführungsform weisen die erfindungsgemäßen organischen Moleküle eine Struktur der Formel VI-1 oder Formel VI-2 auf:

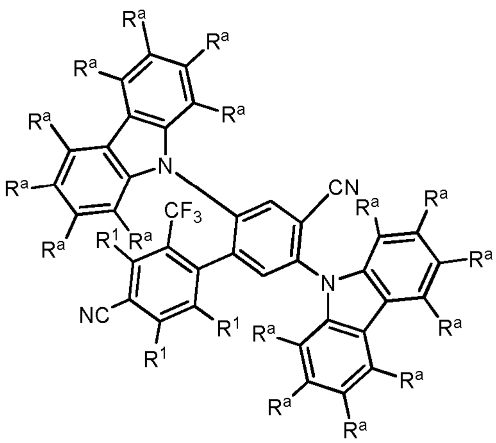

Formel VI-1

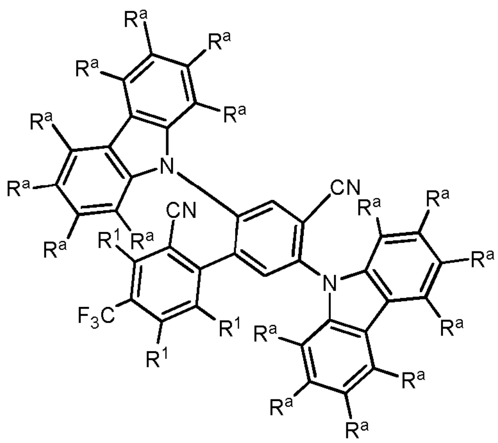

Formel VI-2

wobei die oben genannten Definitionen gelten.

In einer Ausführungsform weisen die erfindungsgemäßen organischen Moleküle eine Struktur der Formel VI-1 auf, wobei die oben genannten Definitionen gelten.

In einer weiteren Ausführungsform weisen die erfindungsgemäßen organischen Moleküle eine Struktur der Formel VIa-1 oder Formel VIa-2 auf:

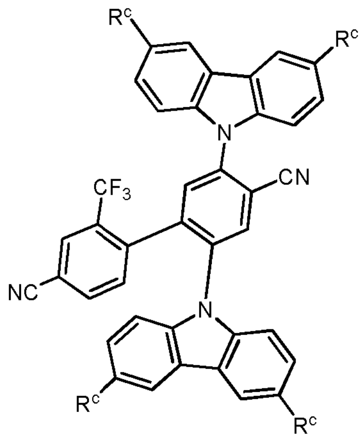

Formel VIa-1

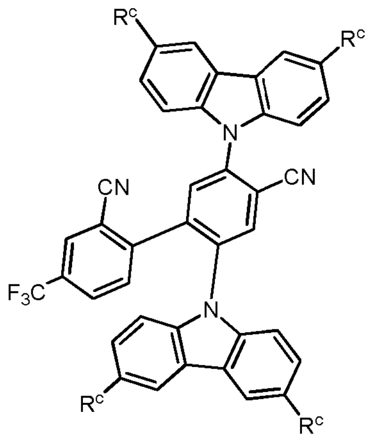

Formel VIa-2

wobei die oben genannten Definitionen gelten.

In einer weiteren Ausführungsform weisen die erfindungsgemäßen organischen Moleküle eine Struktur der Formel VIa-1 auf, wobei die oben genannten Definitionen gelten.

In einer weiteren Ausführungsform weisen die erfindungsgemäßen organischen Moleküle eine Struktur der Formel VIb-1 oder Formel VIb-2 auf:

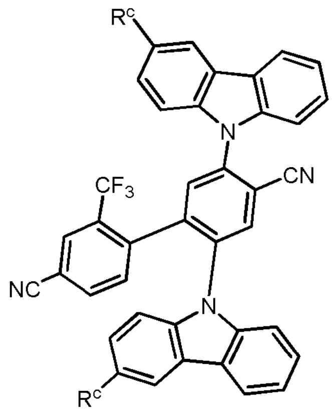

Formel VIb-1

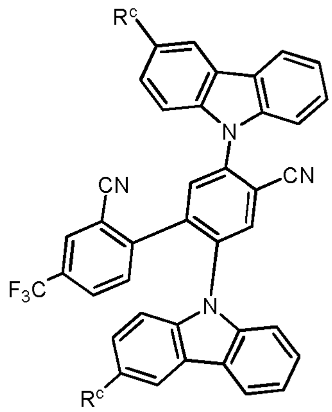

Formel VIb-2

wobei die oben genannten Definitionen gelten.

In einer weiteren Ausführungsform weisen die erfindungsgemäßen organischen Moleküle eine Struktur der Formel VIb-1 auf, wobei die oben genannten Definitionen gelten.

In einer weiteren Ausführungsform weisen die erfindungsgemäßen organischen Moleküle eine Struktur der Formel VIc-1 oder Formel VIc-2 auf:

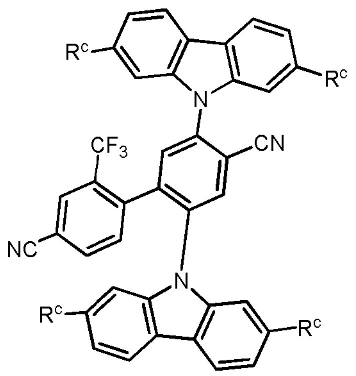

Formel VIc-1

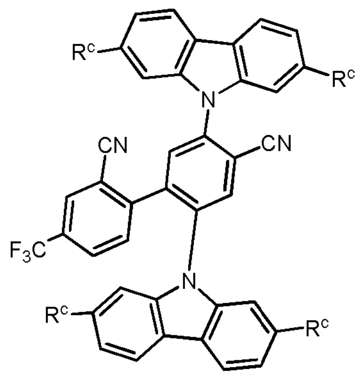

Formel VIc-2

wobei die oben genannten Definitionen gelten.

In einer weiteren Ausführungsform weisen die erfindungsgemäßen organischen Moleküle eine Struktur der Formel VIc-1 auf, wobei die oben genannten Definitionen gelten.

In einer weiteren Ausführungsform weisen die erfindungsgemäßen organischen Moleküle eine Struktur der Formel VId-1 oder Formel VId-2 auf:

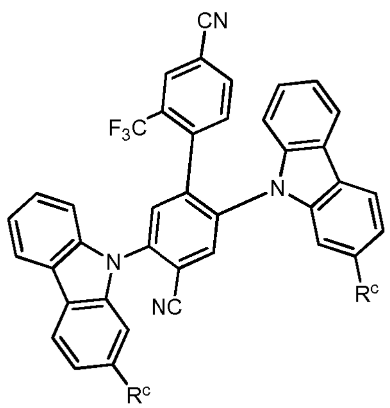

Formel VId-1

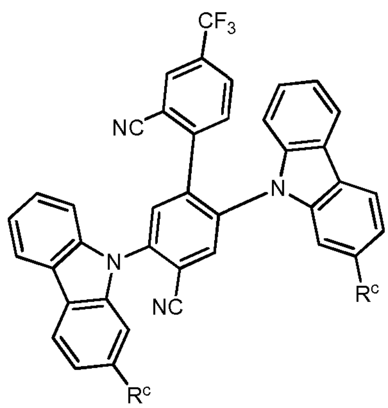

Formel VId-2

wobei die oben genannten Definitionen gelten.

In einer weiteren Ausführungsform weisen die erfindungsgemäßen organischen Moleküle eine Struktur der Formel VId-1 auf, wobei die oben genannten Definitionen gelten.

In einer weiteren Ausführungsform weisen die erfindungsgemäßen organischen Moleküle eine Struktur der Formel VIe-1 oder Formel VIe-2 auf:

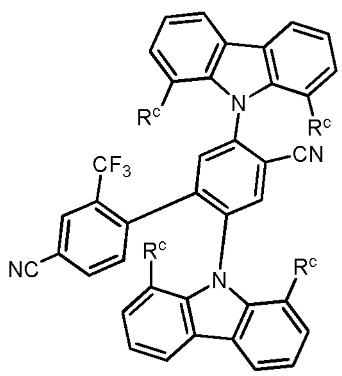

Formel VIe-1

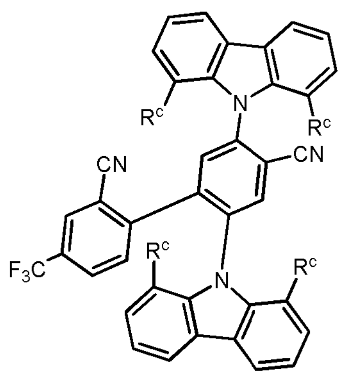

Formel VIe-2

wobei die oben genannten Definitionen gelten.

In einer weiteren Ausführungsform weisen die erfindungsgemäßen organischen Moleküle eine Struktur der Formel VIe-1 auf, wobei die oben genannten Definitionen gelten.

In einer weiteren Ausführungsform weisen die erfindungsgemäßen organischen Moleküle eine Struktur der Formel VIf-1 oder Formel VIf-2 auf:

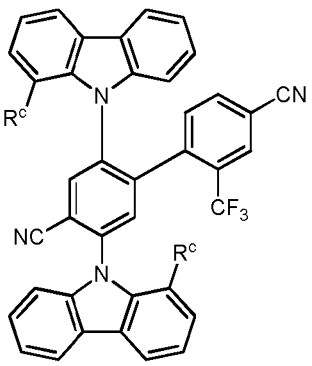

Formel VIf-1

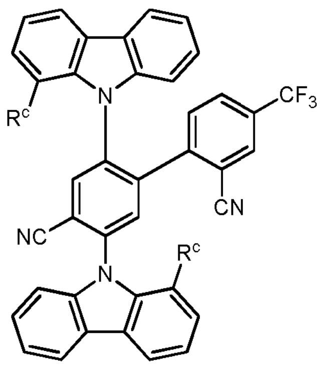

Formel VIf-2

wobei die oben genannten Definitionen gelten.

In einer weiteren Ausführungsform weisen die erfindungsgemäßen organischen Moleküle eine Struktur der Formel VIf-1 auf, wobei die oben genannten Definitionen gelten.

In einer weiteren Ausführungsform weisen die erfindungsgemäßen organischen Moleküle eine Struktur der Formel VIg-1 oder Formel VIg-2 auf:

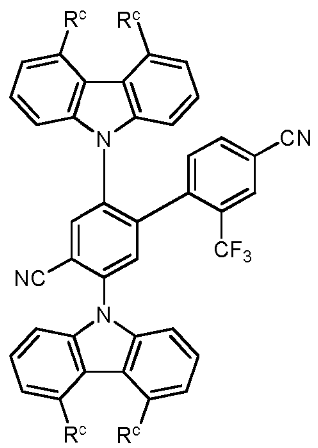

Formel VIg-1

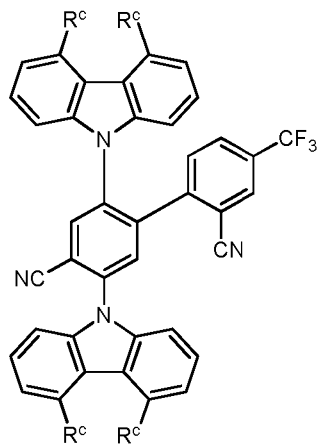

Formel VIg-2

wobei die oben genannten Definitionen gelten.

In einer weiteren Ausführungsform weisen die erfindungsgemäßen organischen Moleküle eine Struktur der Formel VIg-1 auf, wobei die oben genannten Definitionen gelten.

In einer weiteren Ausführungsform weisen die erfindungsgemäßen organischen Moleküle eine Struktur der Formel VIh-1 oder Formel VIh-2 auf:

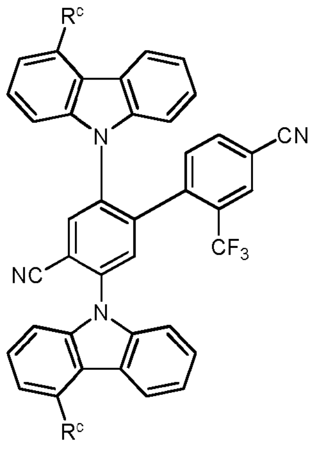

Formel VIh-1

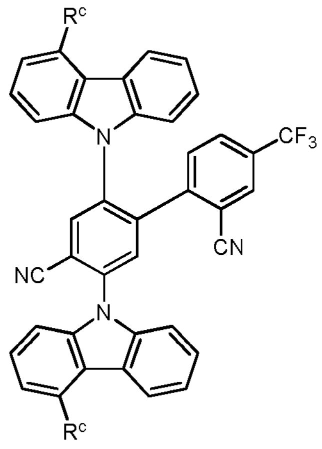

Formel VIh-2

wobei die oben genannten Definitionen gelten.

In einer weiteren Ausführungsform weisen die erfindungsgemäßen organischen Moleküle eine Struktur der Formel VIh-1 auf, wobei die oben genannten Definitionen gelten.

In einer Ausführungsform weisen die erfindungsgemäßen organischen Moleküle eine Struktur der Formel VII-1 oder Formel VII-2 auf:

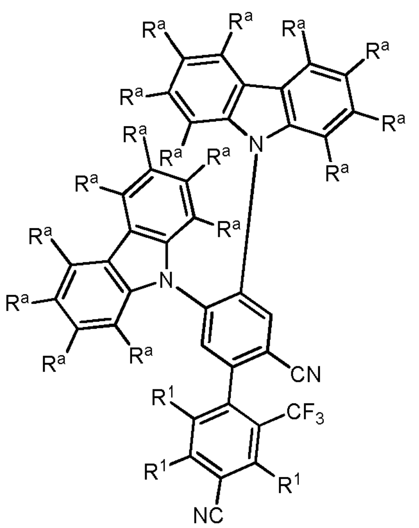

Formel VII-1

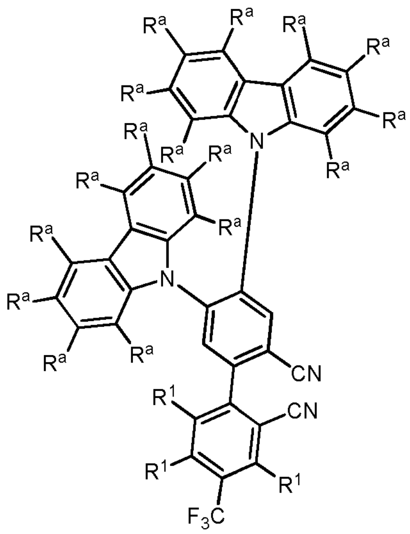

Formel VII-2

wobei die oben genannten Definitionen gelten.

In einer Ausführungsform weisen die erfindungsgemäßen organischen Moleküle eine Struktur der Formel VII-1 auf, wobei die oben genannten Definitionen gelten.

In einer weiteren Ausführungsform weisen die erfindungsgemäßen organischen Moleküle eine Struktur der Formel VIIa-1 oder Formel VIIa-2 auf:

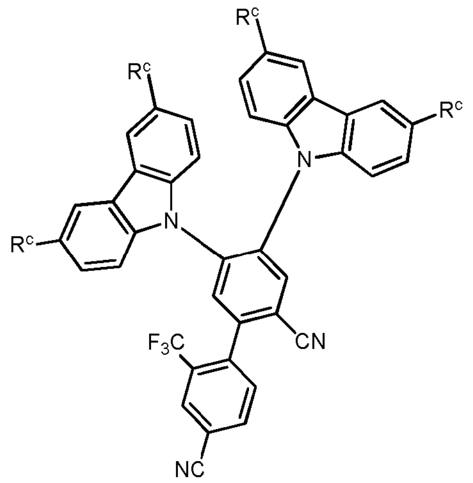

Formel VIIa-1

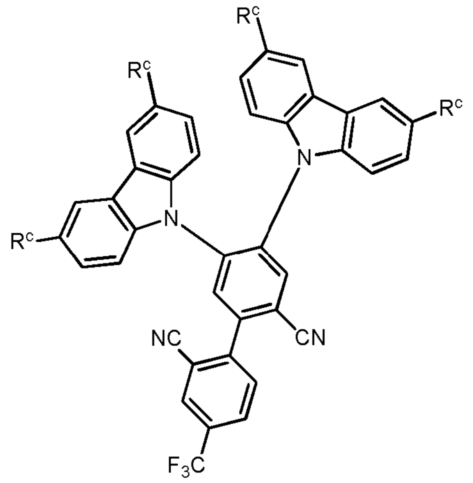

Formel VIIa-2

wobei die oben genannten Definitionen gelten.

In einer weiteren Ausführungsform weisen die erfindungsgemäßen organischen Moleküle eine Struktur der Formel VIIa-1 auf, wobei die oben genannten Definitionen gelten.

In einer weiteren Ausführungsform weisen die erfindungsgemäßen organischen Moleküle eine Struktur der Formel VIIb-1 oder Formel VIIb-2 auf:

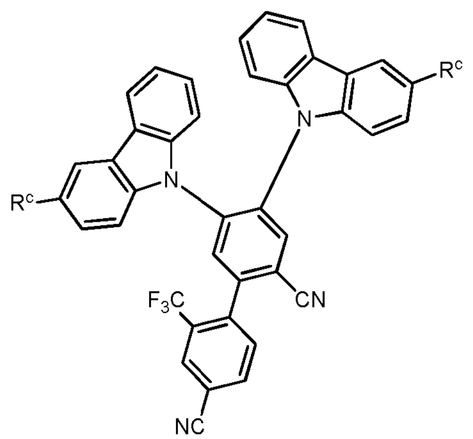

Formel VIIb-1

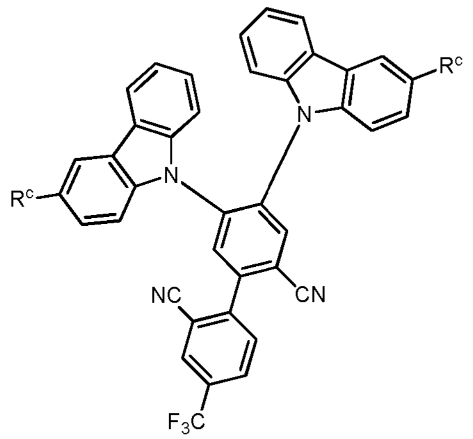

Formel VIIb-2

wobei die oben genannten Definitionen gelten.

In einer weiteren Ausführungsform weisen die erfindungsgemäßen organischen Moleküle eine Struktur der Formel VIIb-1 auf, wobei die oben genannten Definitionen gelten.

In einer weiteren Ausführungsform weisen die erfindungsgemäßen organischen Moleküle eine Struktur der Formel VIIc-1 oder Formel VIIc-2 auf:

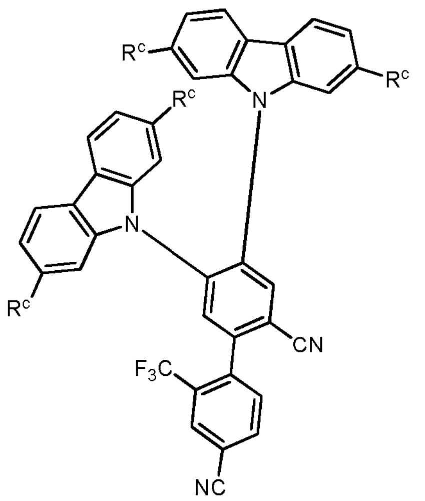

Formel VIIc-1

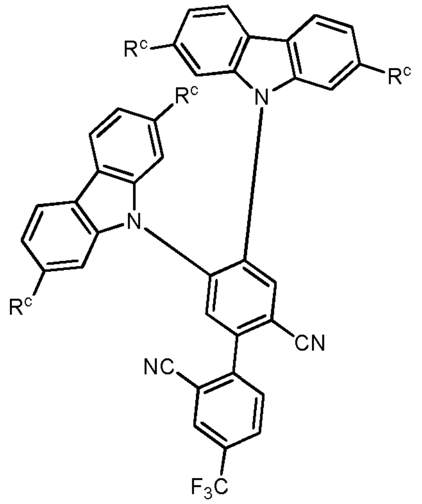

Formel VIIc-2

wobei die oben genannten Definitionen gelten.

In einer weiteren Ausführungsform weisen die erfindungsgemäßen organischen Moleküle eine Struktur der Formel VIIc-1 auf, wobei die oben genannten Definitionen gelten.

In einer weiteren Ausführungsform weisen die erfindungsgemäßen organischen Moleküle eine Struktur der Formel VIId-1 oder Formel VIId-2 auf:

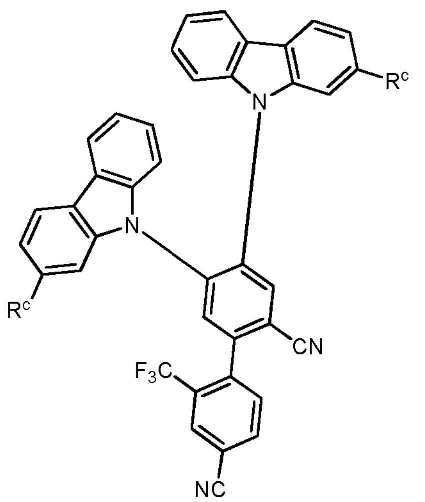

Formel VIId-1

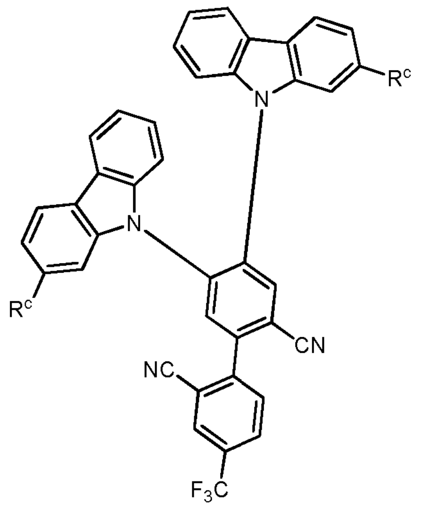

Formel VIId-2

wobei die oben genannten Definitionen gelten.

In einer weiteren Ausführungsform weisen die erfindungsgemäßen organischen Moleküle eine Struktur der Formel VIId-1 auf, wobei die oben genannten Definitionen gelten.

In einer weiteren Ausführungsform weisen die erfindungsgemäßen organischen Moleküle eine Struktur der Formel VIIe-1 oder Formel VIIe-2 auf:

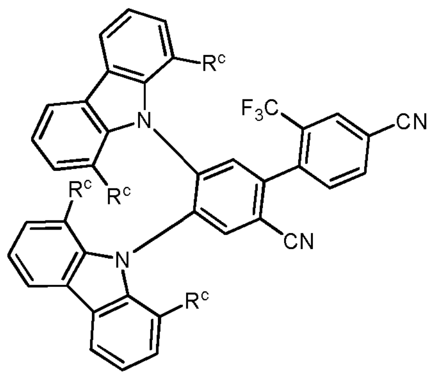

Formel VIIe-1

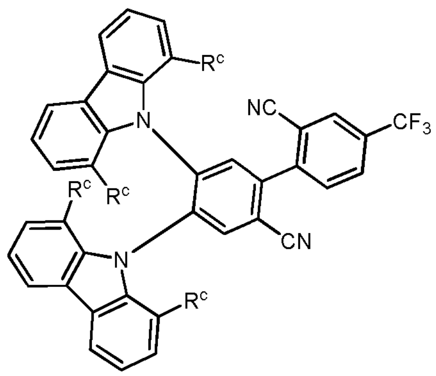

Formel VIIe-2

wobei die oben genannten Definitionen gelten.

In einer weiteren Ausführungsform weisen die erfindungsgemäßen organischen Moleküle eine Struktur der Formel VIIe-1 auf, wobei die oben genannten Definitionen gelten.

In einer weiteren Ausführungsform weisen die erfindungsgemäßen organischen Moleküle eine Struktur der Formel VIIf-1 oder Formel VIIf-2 auf:

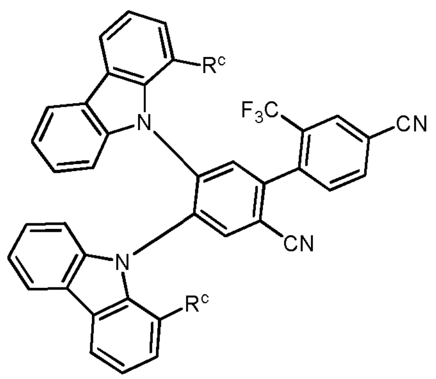

Formel VIIf-1

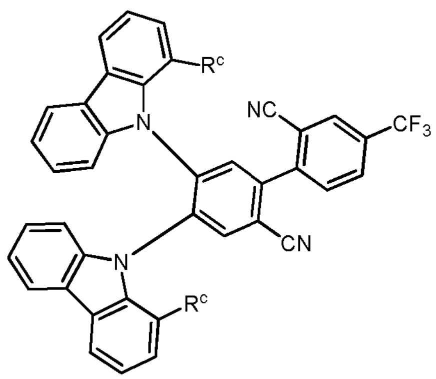

Formel VIIf-2

wobei die oben genannten Definitionen gelten.

In einer weiteren Ausführungsform weisen die erfindungsgemäßen organischen Moleküle eine Struktur der Formel VIIf-1 auf, wobei die oben genannten Definitionen gelten.

In einer weiteren Ausführungsform weisen die erfindungsgemäßen organischen Moleküle eine Struktur der Formel VIIg-1 oder Formel VIIg-2 auf:

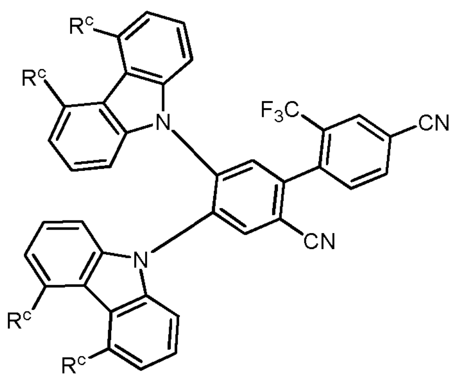

Formel VIIg-1

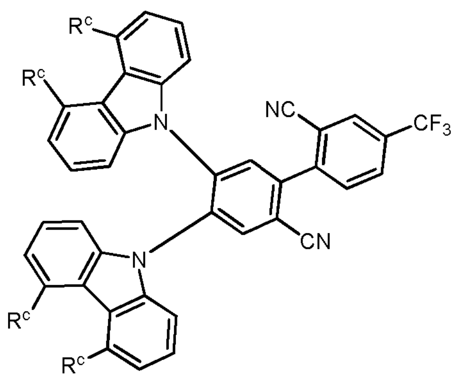

Formel VIIg-2

wobei die oben genannten Definitionen gelten.

In einer weiteren Ausführungsform weisen die erfindungsgemäßen organischen Moleküle eine Struktur der Formel VIIg-1 auf, wobei die oben genannten Definitionen gelten.

In einer weiteren Ausführungsform weisen die erfindungsgemäßen organischen Moleküle eine Struktur der Formel VIIh-1 oder Formel VIIh-2 auf:

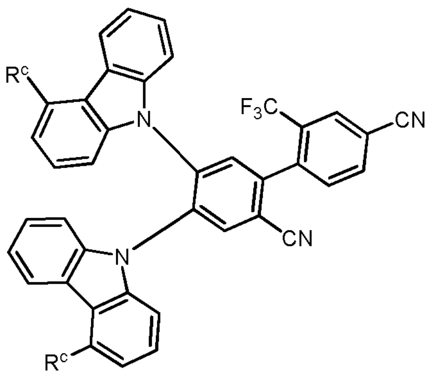

Formel VIIh-1

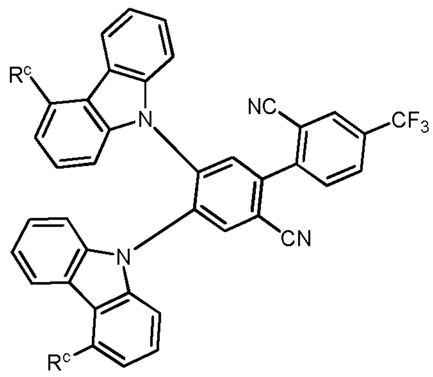

Formel VIIh-2

wobei die oben genannten Definitionen gelten.

In einer weiteren Ausführungsform weisen die erfindungsgemäßen organischen Moleküle eine Struktur der Formel VIIh-1 auf, wobei die oben genannten Definitionen gelten.

In einer weiteren Ausführungsform weisen die erfindungsgemäßen organischen Moleküle eine Struktur der Formel VIII-1 oder Formel VIII-2 auf:

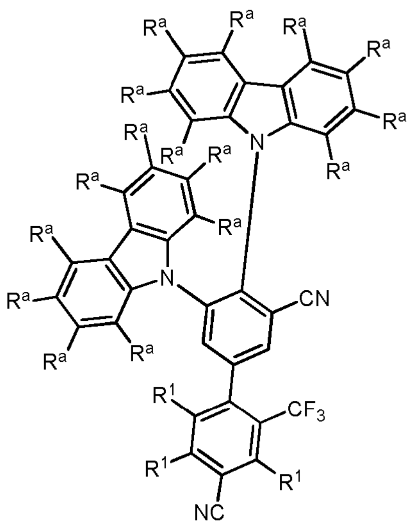

Formel VIII-1

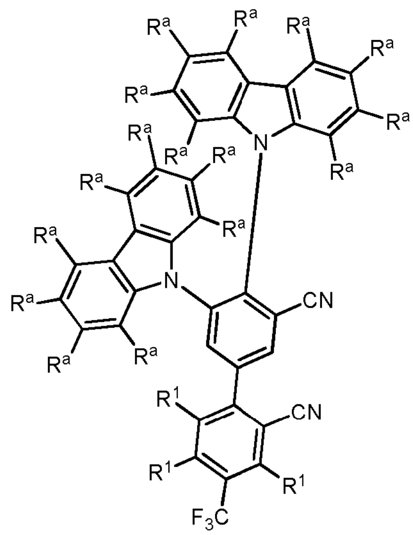

Formel VIII-2

wobei die oben genannten Definitionen gelten.

In einer weiteren Ausführungsform weisen die erfindungsgemäßen organischen Moleküle eine Struktur der Formel VIII-1 auf, wobei die oben genannten Definitionen gelten.

In einer Ausführungsform ist $R^c$ bei jedem Auftreten unabhängig voneinander ausgewählt aus der Gruppe bestehend aus Me, $^i$Pr, $^t$Bu, Ph, das jeweils mit einem oder mehreren Resten ausgewählt aus Me, $^i$Pr, $^t$Bu, CN, $CF_3$ oder Ph substituiert sein kann, und Carbazolyl, das jeweils mit einem oder mehreren Resten ausgewählt aus Me, $^i$Pr, $^t$Bu, oder Ph substituiert sein kann, Triazinyl, das jeweils mit einem oder mehreren Resten ausgewählt aus Me, $^i$Pr, $^t$Bu, CN, $CF_3$ oder Ph substituiert sein kann, und $N(Ph)_2$ ist.

Im Sinne dieser Erfindung enthält eine Arylgruppe 6 bis 60 aromatische Ringatome; eine Heteroarylgruppe enthält 5 bis 60 aromatische Ringatome, von denen mindestens eines ein Heteroatom darstellt. Die Heteroatome sind insbesondere N, O und/oder S. Werden in der Beschreibung bestimmter Ausführungsformen der Erfindung andere, von der genannten Definition abweichende Definitionen angegeben, beispielsweise bezüglich der Zahl der aromatischen

Ringatome oder der enthaltenen Heteroatome, so gelten diese.

Unter einer Arylgruppe bzw. Heteroarylgruppe wird ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,3,5-Triazin, 1,2,3,4-Tetrazin, Purin, Pteridin, Indolizin und Benzothiadiazol oder Kombinationen der genannten Gruppen.

Unter einer cyclischen Alkyl-, Alkoxy- oder Thioalkoxygruppe wird hier eine monocyclische, eine bicyclische oder eine polycyclische Gruppe verstanden.

Im Rahmen der vorliegenden Erfindung werden unter einer $C_1$- bis $C_{40}$-Alkylgruppe, in der auch einzelne H-Atome oder $CH_2$-Gruppen durch die oben genannten Gruppen oder mit den oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluor-methyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-0ctyl)-cyclohex-1-yl- und 1-(n-Decyl)-cyclohex-1-yl-verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer $C_1$- bis $C_{40}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Eine Ausführungsform der Erfindung betrifft organische Moleküle, welche einen $\Delta E(S_1-T_1)$-Wert zwischen dem untersten angeregten Singulett ($S_1$)- und dem darunter liegenden Triplett ($T_1$)-Zustand von nicht höher als 5000 $cm^{-1}$, insbesondere nicht höher als 3000 $cm^{-1}$, oder nicht höher als 1500 $cm^{-1}$ oder 1000 $cm^{-1}$ aufweisen und/oder eine Emissionslebensdauer von höchstens 150 $\mu$s, insbesondere von höchstens 100 $\mu$s, von höchsten 50 $\mu$s, oder von höchstens 10 $\mu$s aufweisen und/oder eine Hauptemissionsbande mit einer Halbwertsbreite kleiner als 0,55 eV, insbesondere kleiner als 0,50 eV, kleiner als 0,48 eV, oder kleiner als 0,45 eV aufweisen.

Die organischen Moleküle zeigen insbesondere ein Emissionsmaximum zwischen 420 und 500 nm, zwischen 430 und 480 nm, insbesondere zwischen 450 und 470 nm.

Die Moleküle weisen insbesondere einen "blue material index" (BMI), den Quotienten aus der PLQY (in %) und ihrer $CIE_y$-Farbkoordinate des von dem erfindungsgemäßen Moleküls emittierten Lichts, von größer 150, insbesondere von größer 200, von größer 250 oder von größer 300 auf.

In einem weiteren Aspekt betrifft die Erfindung ein Verfahren zur Herstellung eines erfindungsgemäßen organischen Moleküls der hier beschriebenen Art (mit einer eventuellen Folgeumsetzung), wobei ein in 2,5,6-Position $R^1$-substituiertes 4-Brom-3-trifluormethylbenzonitril oder ein in 3,4,6-Position $R^1$-substituiertes 2-Brom-5-trifluormethylbenzonitril als Edukt eingesetzt wird

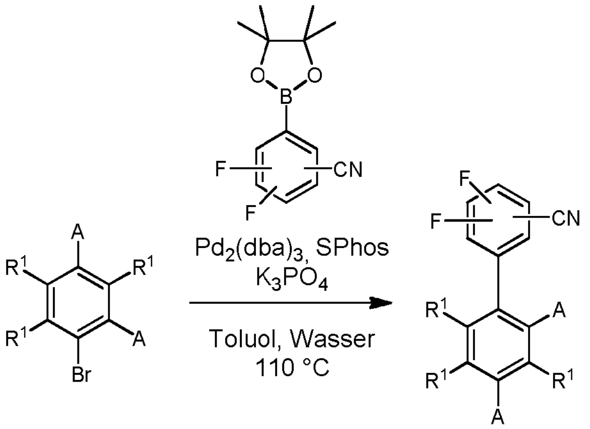


E1

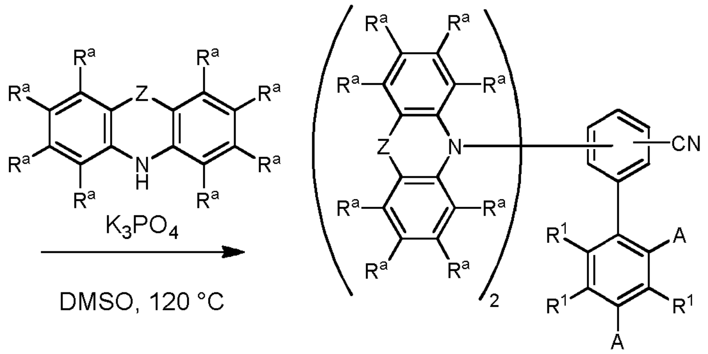


Im obigen Schema ist eines der beiden A gleich $CF_3$ und das andere A ist gleich CN. In einer Ausführungsform des obigen Schemas ist die chemische Gruppe CN des Cyanodifluorphenylboronsäureesters durch $CF_3$ ersetzt.

Erfindungsgemäß kann statt einem Boronsäureester auch eine Boronsäure verwendet werden.

In einer Ausführungsform wird eine in 2,5,6-Position $R^1$-substituierte 4-Brom-3-trifluormethylbenzonitril als Edukt mit einem Cyano-difluorphenylboronsäureester oder einer entsprechenden Cyano-difluorphenylboronsäure in einer Palladium-katalysierten Kreuzkupplungsreaktion umgesetzt. Hierbei können erfindungsgemäß beispielhaft 4-Cyano-2,6-difluorphenylboronsäureester, 4-Cyano-2,5-difluorphenylboronsäureester, 4-Cyano-3,5-difluorphenylboronsäureester, 3-Cyano-2,4-difluorphenylboronsäureester, 3-Cyano-4,5-difluorphenylboronsäureester und 2-Cyano-4,5-difluorphenylboronsäureester oder 4-Cyano-2,6-difluorphenylboronsäure, 4-Cyano-2,5-difluorphenylboronsäure, 4-Cyano-3,5-difluorphenylboronsäure, 3-Cyano-2,4-difluorphenylboronsäure, 3-Cyano-4,5-difluorphenylboronsäure und 2-Cyano-4,5-difluorphenylboronsäure eingesetzt werden. Das Produkt wird durch Deprotonierung des entsprechenden Amins und anschließender nukleophiler Substitution der zwei Fluorgruppen erhalten. Hierbei wird ein Stickstoffheterozyklus im Sinne einer nukleophilen aromatischen Substitution mit einem Edukt E1 umgesetzt. Typische Bedingungen beinhalten die Verwendung einer Base wie beispielweise tribasisches Kaliumphosphat oder Natriumhydrid in einem aprotischen polaren Lösungsmittel wie beispielweise Dimethylsulfoxid (DMSO) oder N,N-Dimethylformamid (DMF).

Erfindungsgemäß kann statt einem in 2,5,6-Position $R^1$-substituierte 4-Brom-3-trifluormethylbenzonitril als Edukt ein in 3,4,6-Position $R^1$-substituierte 2-Brom-5-trifluormethylbenzonitril als Edukt verwendet werden.

In einem weiteren Aspekt betrifft die Erfindung die Verwendung der organischen Moleküle als lumineszierender Emitter oder als Hostmaterial in einer organischen optoelektronischen Vorrichtung, insbesondere wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

In einem weiteren Aspekt betrifft die Erfindung eine Zusammensetzung aufweisend oder bestehend aus:

(a) mindestens einem erfindungsgemäßen organischen Molekül, insbesondere als Emitter und/oder Host, und
(b) mindestens ein, d. h. ein oder mehrere Emitter- und/oder Hostmaterialien, die von dem erfindungsgemäßen organischen Molekül verschiedenen ist bzw. sind und
(c) optional eine oder mehreren Farbstoffen und/ oder einem oder mehreren organischen Lösungsmitteln.

In einer Ausführungsform besteht die erfindungsgemäße Zusammensetzung aus einem erfindungsgemäßen organischen Molekül und einem oder mehreren Hostmaterialien. Das oder die Hostmaterialen weisen insbesondere erste angeregte Triplett ($T_1$)-Energieniveaus auf, die energetisch höher liegen als die ersten angeregten Triplett ($T_1$)-Energieniveaus des erfindungsgemäßen organischen Moleküls und weisen erste angeregte Singulett ($S_1$)-Energieniveaus auf, die energetisch höher liegen als die Singulett ($S_1$)-Energieniveaus des erfindungsgemäßen organischen Moleküls.

Die Orbitalenergien und die Energien der angeregten Zustände können entweder mittels experimenteller Methoden oder durch die Anwendung quantenchemischer Methoden, insbesondere Rechnungen der Dichte-Funktional-Theorie, bestimmt werden. Die Energie des höchst besetzten Orbitals $E^{HOMO}$ wird, wie dem Fachmann bekannt, über Cyclovoltammetrie mit einer Genauigkeit von 0.1 eV bestimmt. Die Energie des niedrigsten unbesetzten Orbitals $E^{LUMO}$ wird über die Summe von $E^{HOMO}$ und $E^{gap}$ berechnet, wobei $E^{gap}$ wie folgt bestimmt wird: Für ein Hostmolekül wird für $E^{gap}$, falls nicht anders erwähnt, die Energie verwendet, bei der die Emission eines Films mit 10% Host (Massenprozent) in Polymethylmethacrylat (PMMA) einsetzt. Für ein Emittermolekül wird $E^{gap}$ als die Energie bestimmt, an der sich das Anregungs- und das Emissionsspektrum eines Films mit 10% Emitter (Massenprozent) in PMMA schneiden.

Die Energie des ersten angeregten Triplett-Zustands $T_1$ wird über die Energie bestimmt, bei der die Emission bei niedriger Temperatur, typischerweise 77 K, einsetzt. Für ein Hostmolekül für das die Energiedifferenz zwischen dem ersten angeregten Singulett-Zustand und dem ersten angeregten Triplett-Zustand sich um mehr als 0.4 eV unterscheidet, ist die Phosphoreszenz typischerweise im Steady-State-Spektrum in 2-Me-THF sichtbar. Die Triplettenergie kann daher als die Energie bestimmt werden, bei der das Phosphoreszenzspektrum einsetzt. Für TADF-Emittermoleküle wird die Energie des ersten angeregten Triplett-Zustands $T_1$ über die Energie bestimmt, bei der das verzögerte Emissionsspektrum bei 77 K einsetzt, welches falls nicht anders erwähnt, in einem Film mit 10 % Emittermolekül (Massenprozent) in PMMA gemessen wird. Für Host- und Emittermoleküle wird die Energie des ersten angeregten Singulett-Zustands S, mit der Energie bestimmt, bei der das Emissionsspektrums einsetzt, welches falls nicht anders erwähnt, in einem Film mit 10 % Hostmolekül oder Emittermolekül (Massenprozent) in PMMA gemessen wird.

In einer Ausführungsform weist die Zusammensetzung neben dem erfindungsgemäßen organischen Molekül ein elektronendominantes und ein lochdominantes Hostmaterial auf. Das höchste besetzte Orbital (HOMO) des lochdominanten Hostmaterials liegt energetisch insbesondere höher als das HOMO des elektronendominanten Hostmaterials und das niedrigste unbesetzte Orbital (LUMO) des lochdominanten Hostmaterials liegen energetisch insbesondere höher als das LUMO des elektronendominanten Hostmaterials. In einerweiteren Ausführungsform liegt das HOMO des lochdominanten Hostmaterials energetisch über dem HOMO des erfindungsgemäßen organischen Moleküls, während das LUMO des elektronendominanten Hostmaterials energetisch unter dem LUMO des erfindungsgemäßen organischen Moleküls liegt. Um Exciplex-Formation zwischen Emitter und Hostmaterial oder Hostmaterialien zu vermeiden, sollten die Materialien so gewählt sein, dass die Energieabstände zwischen dem LUMO des elektronendominanten Hostmaterials und dem LUMO des erfindungsgemäßen organischen Moleküls insbesondere weniger als 0,5 eV, bevorzugt weniger als 0,3 eV, noch bevorzugter weniger als 0,2 eV beträgt. Der Energieabstand zwischen dem HOMO des lochdominanten Hostmaterials und dem HOMO des erfindungsgemäßen organischen Moleküls beträgt insbesondere weniger als 0,5 eV, bevorzugt weniger als 0,3 eV, noch bevorzugter weniger als 0,2 eV.

In einer weiteren Ausführungsform weist die Zusammensetzung neben dem erfindungsgemäßen organischen Molekül ein elektronendominantes Hostmaterial auf. Die Energiedifferenz zwischen dem höchsten besetzten Orbital (HOMO) des erfindungsgemäßen organischen Moleküls und dem HOMO des elektronendominanten Hostmaterials ist zwischen -0,5 eV und 0,5 eV, bevorzugt -0,3 eV und 0,3 eV, noch bevorzugter -0,2 eV und 0,2 eV, oder sogar zwischen -0,1 eV und 0,1 eV.

In einer weiteren Ausführungsform weist die Zusammensetzung neben dem erfindungsgemäßen organischen Molekül ein elektronendominantes Hostmaterial auf. Die Energiedifferenz zwischen dem niedrigsten unbesetzten Orbital (LUMO) des erfindungsgemäßen organischen Moleküls und dem LUMO des elektronendominanten Hostmaterials ist zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV.

In einer weiteren Ausführungsform weist die Zusammensetzung neben dem erfindungsgemäßen organischen Molekül ein lochdominantes Hostmaterial auf. Die Energiedifferenz zwischen dem höchsten besetzten Orbital (HOMO) des erfindungsgemäßen organischen Moleküls und dem HOMO des lochdominanten Hostmaterials ist zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV.

In einer weiteren Ausführungsform weist die Zusammensetzung neben dem erfindungsgemäßen organischen Molekül ein lochdominantes Hostmaterial auf. Die Energiedifferenz zwischen dem niedrigsten unbesetzten Orbital (LUMO) des erfindungsgemäßen organischen Moleküls und dem LUMO des lochdominanten Hostmaterials ist zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV.

In einer Ausführungsform weist die Zusammensetzung neben dem erfindungsgemäßen organischen Molekül ein elektronendominantes und ein lochdominantes Hostmaterial auf. Die Energiedifferenz zwischen dem höchsten besetzen Orbital (HOMO) des erfindungsgemäßen organischen Moleküls und dem HOMO des elektronendominanten Hostmaterials ist zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV und die Energiedifferenz zwischen dem niedrigsten unbesetzten Orbital (LUMO) des erfindungsgemäßen organischen Moleküls und dem LUMO des elektronendominanten Hostmaterials ist zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV. Entsprechend ist die Energiedifferenz zwischen dem höchsten besetzen Orbital (HOMO) des erfindungsgemäßen organischen Moleküls und dem HOMO des lochdominanten Hostmaterials zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV und die Energiedifferenz zwischen dem niedrigsten unbesetzten Orbital (LUMO) des erfindungsgemäßen organischen Moleküls und dem LUMO des lochdominanten Hostmaterials zwischen -0,5 eV und 0,5 eV, bevorzugt zwischen -0,3 eV und 0,3 eV, noch bevorzugter zwischen -0,2 eV und 0,2 eV, oder sogar zwischen -0,1 eV und 0,1 eV.

In einem weiteren Aspekt betrifft die Erfindung eine organische optoelektronische Vorrichtung, die ein erfindungsgemäßes organisches Molekül oder eine erfindungsgemäße Zusammensetzung aufweist. Die organische optoelektronische Vorrichtung ist insbesondere ausgeformt als eine Vorrichtung ausgewählt aus der Gruppe bestehend aus organischer lichtemittierender Diode (OLED); lichtemittierender elektrochemischer Zelle; OLED-Sensor, insbesondere nicht hermetisch nach außen abgeschirmten Gas- und Dampf-Sensoren; organischer Diode; organischer Solarzelle; organischem Transistor; organischem Feldeffekttransistor; organischem Laser und Down-Konversion-Element.

Eine organische optoelektronische Vorrichtung aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein erfindungsgemäßes organisches Molekül aufweist, stellt einen weitere Ausführungsform der Erfindung dar.

In einer Ausführungsform handelt es sich bei der optoelektronischen Vorrichtung um eine OLED. Eine typische OLED weist beispielsweise folgenden Schichtaufbau auf:

1. Substrat (Trägermaterial)
2. Anode
3. Lochinjektionsschicht (hole injection layer, HIL)
4. Lochtransportschicht (hole transport layer, HTL)
5. Elektronenblockierschicht (electron blocking layer, EBL)
6. Emitterschicht (emitting layer, EML)
7. Lochblockierschicht (hole blocking layer, HBL)
8. Elektronenleitschicht (electron transport layer, ETL)
9. Elektroneninjektionsschicht (electron injection layer, EIL)
10. Kathode.

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

Gemäß einer Ausführungsform ist mindestens eine Elektrode des organischen Bauelements transluzent ausgebildet. Hier wird mit "transluzent" eine Schicht bezeichnet, die durchlässig für sichtbares Licht ist. Dabei kann die transluzente Schicht klar durchscheinend, also transparent, oder zumindest teilweise Licht absorbierend und/oder teilweise Licht streuend sein, so dass die transluzente Schicht beispielsweise auch diffus oder milchig durchscheinend sein kann. Insbesondere ist eine hier als transluzent bezeichnete Schicht möglichst transparent ausgebildet, so dass insbesondere die Absorption von Licht so gering wie möglich ist.

Gemäß einer weiteren Ausführungsform weist das organische Bauelement, insbesondere eine OLED, einen invertierten Aufbau auf. Der invertierte Aufbau zeichnet sich dadurch aus, dass sich die Kathode auf dem Substrat befindet und die anderen Schichten entsprechend invertiert aufgebracht werden:

1. Substrat (Trägermaterial)
2. Kathode
3. Elektroneninjektionsschicht (electron injection layer, EIL)
4. Elektronenleitschicht (electron transport layer, ETL)
5. Lochblockierschicht (hole blocking layer, HBL)
6. Emissionsschicht bzw. Emitterschicht (emitting layer, EML)
7. Elektronenblockierschicht (electron blocking layer, EBL)
8. Lochtransportschicht (hole transport layer, HTL)
9. Lochinjektionsschicht (hole injection layer, HIL)
10. Anode

Dabei sind einzelne Schichten lediglich in optionaler Weise vorhanden. Weiterhin können mehrere dieser Schichten zusammenfallen. Und es können einzelne Schichten mehrfach im Bauteil vorhanden sein.

In einer Ausführungsform wird bei der invertierten OLED die Anodenschicht des typischen Aufbaus, z.B. eine ITO-Schicht (Indium-Zinn-Oxid), als Kathode geschaltet.

Gemäß einer weiteren Ausführungsform weist das organische Bauelement, insbesondere eine OLED, einen gestapelten Aufbau auf. Hierbei werden die einzelnen OLEDs übereinander und nicht wie üblich nebeneinander angeordnet. Durch einen gestapelten Aufbau kann die Erzeugung von Mischlicht ermöglicht werden. Beispielsweise kann dieser Aufbau bei der Erzeugung von weißem Licht eingesetzt werden, für dessen Erzeugung das gesamte sichtbare Spektrum typischerweise durch die Kombination des emittierten Lichts von blauen, grünen und roten Emittern abgebildet wird. Weiterhin können bei praktisch gleicher Effizienz und identischer Leuchtdichte signifikant längere Lebensdauern im Vergleich zu üblichen OLEDs erzielt werden. Für den gestapelten Aufbau wird optional eine sogenannte Ladungserzeugungsschicht (charge generation layer, CGL) zwischen zwei OLEDs eingesetzt. Diese besteht aus einer n-dotierten und einem p-dotierten Schicht, wobei die n-dotierte Schicht typischerweise näher an der Anode aufgebracht wird.

In einer Ausführungsform - einer sogenannten Tandem-OLED - treten zwei oder mehr Emissionsschichten zwischen Anode und Kathode auf. In einer Ausführungsform sind drei Emissionsschichten übereinander angeordnet, wobei eine Emissionsschicht rotes Licht emittiert, eine Emissionsschicht grünes Licht emittiert und eine Emissionsschicht blaues Licht emittiert und optional weitere Ladungserzeugungs-, Blockier- oder Transportschichten zwischen den einzelnen Emissionsschichten aufgebracht sind. In einer weiteren Ausführungsform werden die jeweiligen Emissionsschichten direkt angrenzend aufgebracht. In einer weiteren Ausführungsform befindet sich jeweils eine Ladungserzeugungsschicht zwischen den Emissionsschichten. Weiterhin können in einer OLED direkt angrenzende und durch Ladungserzeugungsschichten getrennte Emissionsschichten kombiniert werden.

Über den Elektroden und den organischen Schichten kann weiterhin noch eine Verkapselung angeordnet sein. Die Verkapselung kann beispielsweise in Form eines Glasdeckels oder in Form einer Dünnschichtverkapselung ausgeführt sein.

Als Trägermaterial der optoelektronischen Vorrichtung kann beispielsweise Glas, Quarz, Kunststoff, Metall, ein Siliziumwafer oder jedes andere geeignete feste oder flexible, optional durchsichtige Material dienen. Das Trägermaterial kann beispielsweise ein oder mehrere Materialien in Form einer Schicht, einer Folie, einer Platte oder einem Laminat aufweisen.

Als Anode der optoelektronischen Vorrichtung können beispielsweise transparente leitende Metalloxide wie beispielsweise ITO (Indium-Zinn-Oxid), Zinkoxid, Zinnoxid, Cadmiumoxid, Titanoxid, Indiumoxid oder Aluminiumzinkoxid (AZO), $Zn_2SnO_4$, CdSnOs, ZnSnOs, $MgIn_2O_4$, GaInOs, $Zn_2In_2O_5$ oder $In_4Sn_3O_{12}$ oder Mischungen unterschiedlicher transparenter leitender Oxide dienen.

Als Materialien einer HIL können beispielsweise PEDOT:PSS (Poly-3,4-ethylendioxythiophen:Polystyrolsulfonsäure), PEDOT (Poly-3,4-ethylendioxythiophen), m-MTDATA (4,4',4"-Tris[phenyl(m-tolyl)amino]triphenylamin), Spiro-TAD (2,2',7,7'-Tetrakis(N,N-diphenylamino)-9,9-spirobifluoren), DNTPD (4,4'-Bis[N-[4-{N,N-bis(3-methylphenyl)amino}phenyl]-N-phenylamino]biphenyl), NPB (N,N'-Bis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzol), MeO-TPD (N,N,N',N'-Tetrakis(4-methoxyphenyl)benzol), HAT-CN (1,4,5,8,9,11-Hexaazatriphenylen-hexacarbonitril) oder Spiro-NPD (N,N'-diphenyl-N,N'-Bis-(1-naphthyl)-9,9'-spirobifluorene-2,7-diamin) dienen. Beispielhaft ist die Schichtdicke 10-80 nm. Desweiteren können kleine Moleküle können verwendet werden (z. B. Kupfer-Phthalocyanin (CuPc z. B. 10 nm dick)) oder Metalloxide wie beispielhaft MoOs, $V_2O_5$.

Als Materialien einer HTL können tertiäre Amine, Carbazolderivate, mit Polystyrolsulfonsäure dotiertes Polyethylendioxythiophen, mit Camphersulfonsäure dotiertes Polyanilin poly-TPD (Poly(4-butylphenyl-diphenyl-amin)), [alpha]-NPD (Poly(4-butylphenyl-diphenyl-amin)), TAPC (4,4'-Cyclohexyliden-bis[N,N-bis(4-methylphenyl)benzenamin]), TCTA (Tris(4-carbazoyl-9-ylphenyl)amin), 2-TNATA (4,4',4"-Tris[2-naphthyl(phenyl)amino]triphenylamin), Spiro-TAD, DNTPD, NPB, NPNPB, MeO-TPD, HAT-CN oder TrisPcz (9,9'-Diphenyl-6-(9-phenyl-9H-carbazol-3-yl)-9H,9'H-3,3'-bicarbazol) dienen. Beispielhaft ist die Schichtdicke 10 nm bis 100 nm.

Die HTL kann eine p-dotierte Schicht aufweisen, die einen anorganischen oder organischen Dotierstoff in einer organischen löcherleitenden Matrix aufweist. Als anorganischer Dotierstoff können beispielsweise Übergangsmetalloxide wie etwa Vanadiumoxid, Molybdänoxid oder Wolframoxid genutzt werden. Als organische Dotierstoffe können beispielsweise Tetrafluorotetracyanoquinodimethan (F4-TCNQ), Kupfer-Pentafluorobenzoat (Cu(I)pFBz) oder Übergangsmetallkomplexe verwendet werden. Beispielhaft ist die Schichtdicke 10 nm bis 100 nm.

Als Materialien einer Elektronenblockierschicht können beispielsweise mCP (1,3-Bis(carbazol-9-yl)benzol), TCTA, 2-TNATA, mCBP (3,3-Di(9H-carbazol-9-yl)biphenyl), tris-Pcz (9,9'-Diphenyl-6-(9-phenyl-9H-carbazol-3-yl)-9H,9'H-3,3'-bicarbazol), CzSi (9-(4-tert-Butylphenyl)-3,6-bis(triphenylsilyl)-9H-carbazol) oder DCB (N,N'-Dicarbazolyl-1,4-dimethylbenzol) dienen. Beispielhaft ist die Schichtdicke 10 nm bis 50 nm.

Die Emitter-Schicht EML oder Emissionsschicht besteht aus oder enthält Emittermaterial oder eine Mischung aufweisend mindestens zwei Emittermaterialien und optional ein oder mehreren Hostmaterialien. Geeignete Hostmaterialien sind beispielsweise mCP, TCTA, 2-TNATA, mCBP, CBP (4,4'-Bis-(N-carbazolyl)-biphenyl), Sif87 (Dibenzo[b,d]thiophen-2-yltriphenylsilan), Sif88 (Dibenzo[b,d]thiophen-2-yl)diphenylsilan) oder DPEPO (Bis[2-((oxo)diphenylphosphino)phenyl]ether). Für im Grünen oder im Roten emittierendes Emittermaterial oder einer Mischung aufweisend mindestens zwei Emittermaterialien eignen sich die gängigen Matrixmaterialien wie CBP. Für im Blauen emittierendes Emittermaterial oder einer Mischung aufweisend mindestens zwei Emittermaterialien können UHG-Matrixmaterialien (Ultra-High energy Gap Materialien) (siehe z. B. M.E. Thompson et al., Chem. Mater. 2004, 16, 4743) oder andere sogenannten Wide-Gap-Matrixmaterialien eingesetzt werden. Beispielhaft ist die Schichtdicke 10 nm bis 250 nm.

Die Lochblockierschicht HBL kann beispielsweise BCP (2,9-Dimethyl-4,7-diphenyl-1,10-phenanthrolin = Bathocuproin), Bis-(2-methyl-8-hydroxychinolinato)-(4-phenylphenolato)-aluminium(III) (BAlq), Nbphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid) oder TCB/TCP (1,3,5-Tris(N-carbazolyl)benzol/ 1,3,5-tris(carbazol)-9-yl) benzol) aufweisen. Beispielhaft ist die Schichtdicke 10 nm bis 50 nm.

Die Elektronentransportschicht ETL kann beispielsweise Materialien auf Basis von $AlQ_3$, TSPO1, BPyTP2 (2,7-Di(2,2'-bipyridin-5-yl)triphenyl), Sif87, Sif88, BmPyPhB (1,3-Bis[3,5-di(pyridin-3-yl)phenyl]benzol) oder BTB (4,4'-Bis-[2-(4,6-diphenyl-1,3,5-triazinyl)]-1,1'-biphenyl) aufweisen. Beispielhaft ist die Schichtdicke 10 nm bis 200 nm.

Als Materialien einer dünnen Elektroneninjektionsschicht EIL können beispielsweise CsF, LiF, 8-Hydroxyquinolinolatolithium (Liq), $Li_2O$, $BaF_2$, MgO oder NaF verwendet werden.

Als Materialien der Kathodenschicht können Metalle oder Legierungen dienen, beispielsweise Al, Al > AlF, Ag, Pt, Au, Mg, Ag:Mg. Typische Schichtdicken betragen 100 nm bis 200 nm. Insbesondere werden ein oder mehrere Metalle verwendet, die stabil an der Luft sind und/oder die selbstpassivierend, beispielsweise durch Ausbildung einer dünnen schützenden Oxidschicht, sind.

Als Materialien zu Verkapselung sind beispielsweise Aluminiumoxid, Vanadiumoxid, Zinkoxid, Zirkoniumoxid, Titanoxid, Hafniumoxid, Lanthanoxid, Tantaloxid geeignet.

In einer Ausführungsform der erfindungsgemäßen organischen optoelektronischen Vorrichtung ist das erfindungsgemäße organische Molekül als Emissionsmaterial in einer lichtemittierenden Schicht EML eingesetzt, wobei es entweder als Reinschicht oder in Kombination mit einem oder mehreren Hostmaterialien eingesetzt ist.

Eine Ausführungsform der Erfindung betrifft organische optoelektronische Vorrichtungen, welche eine externe Quanteneffizienz (EQE) bei 1000 cd/m$^2$ von größer 5 %, insbesondere von größer 8 %, insbesondere von größer 10 %, oder von größer 13 %, oder von größer 16 % und insbesondere von größer 20 % und/oder ein Emissionsmaximum bei einer Wellenlänge zwischen 420 nm und 500 nm, insbesondere zwischen 430 nm und 490 nm, oder zwischen 440 nm und 480 nm und insbesondere zwischen 450 nm und 470 nm und/oder einen LT80 Wert bei 500 cd/m$^2$ von größer 30 h, insbesondere von größer 70 h, oder von größer 100 h, oder von größer 150 h und insbesondere von größer 200 h aufweisen.

Der Massenanteil des erfindungsgemäßen organischen Moleküls an der Emitter-Schicht EML beträgt in einer weiteren Ausführungsform in einer lichtemittierenden Schicht in optischen Licht emittierenden Vorrichtungen, insbesondere in OLEDs, zwischen 1 % und 80 %. In einer Ausführungsform der erfindungsgemäßen organischen optoelektronischen Vorrichtung ist die lichtemittierende Schicht auf ein Substrat aufgebracht, wobei bevorzugt eine Anode und eine Kathode auf das Substrat aufgebracht sind und die lichtemittierende Schicht zwischen Anode und Kathode aufgebracht ist.

Die lichtemittierende Schicht kann in einer Ausführungsform ausschließlich ein erfindungsgemäßes organisches Molekül in 100 % Konzentration aufweisen, wobei die Anode und die Kathode auf das Substrat aufgebracht sind, und die lichtemittierende Schicht zwischen Anode und Kathode aufgebracht ist.

In einer Ausführungsform der erfindungsgemäßen organischen optoelektronischen Vorrichtung sind eine löcher- und elektroneninjizierende Schicht zwischen Anode und Kathode, und eine löcher- und elektronentransportierende Schicht zwischen löcher- und elektroneninjizierender Schicht, und die lichtemittierende Schicht zwischen löcher- und elektronentransportierender Schicht aufgebracht.

Die organische optoelektronische Vorrichtung weist in einer weiteren Ausführungsform der Erfindung auf: ein

Substrat, eine Anode, eine Kathode und mindestens je eine löcher- und elektroneninjizierende Schicht, und mindestens je eine löcher- und elektronentransportierende Schicht, und mindestens eine lichtemittierende Schicht, die erfindungsgemäßes organisches Molekül und ein oder mehrere Hostmaterialen aufweist, deren Triplett ($T_1$)-Energieniveaus energetisch höher liegen als die Triplett ($T_1$)-Energieniveaus des organischen Moleküls und deren Singulett ($S_1$)-Energieniveaus energetisch höher liegen als die Singulett ($S_1$)-Energieniveaus des organischen Moleküls, wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierender Schicht aufgebracht ist, und die lichtemittierende Schicht zwischen löcher- und elektronentransportierender Schicht aufgebracht ist.

In einem weiteren Aspekt betrifft die Erfindung ein Verfahren zur Herstellung eines optoelektronischen Bauelements. Dabei wird ein erfindungsgemäßes organisches Molekül verwendet.

In einer Ausführungsform umfasst das Herstellungsverfahren die Verarbeitung des erfindungsgemäßen organischen Moleküls mittels eines Vakuumverdampfungsverfahrens oder aus einer Lösung.

Erfindungsgemäß ist auch ein Verfahren zur Herstellung einer erfindungsgemäßen optoelektronischen Vorrichtung, bei dem mindestens eine Schicht der optoelektronischen Vorrichtung

- mit einem Sublimationsverfahren beschichtet wird,
- mit einem OVPD (Organic Vapor Phase Deposition) Verfahren beschichtet wird,
- mit einer Trägergassublimation beschichtet wird, und/oder
- aus Lösung oder mit einem Druckverfahren hergestellt wird.

Bei der Herstellung der erfindungsgemäßen optoelektronischen Vorrichtung werden bekannte Verfahren eingesetzt. Generell werden die Schichten einzeln auf ein geeignetes Substrat in aufeinanderfolgenden Abscheideverfahrensschritten aufgebracht. Bei der Gasphasenabscheidung können die gebräuchlichen Methoden, wie thermische Verdampfung, chemische Gasphasenabscheidung (CVD), physikalische Gasphasenabscheidung (PVD) angewendet werden. Für active matrix OLED (AMOLED) Displays erfolgt die Abscheidung auf eine AMOLED Backplane als Substrat.

Alternativ können Schichten aus Lösungen oder Dispersionen in geeigneten Lösungsmitteln aufgebracht werden. Beispielhafte geeignete Beschichtungsverfahren sind Rotationsbeschichtung (spin-coating), Tauchbeschichtung (dip-coating) und Strahldruckmethoden. Die einzelnen Schichten können erfindungsgemäß sowohl über dasselbe als auch über jeweils verschiedene Beschichtungsmethoden hergestellt werden.

**Beispiele**

Allgemeines Syntheseschema I

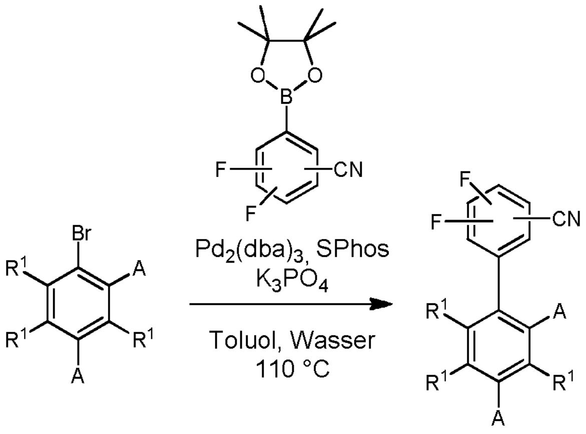


**E1**

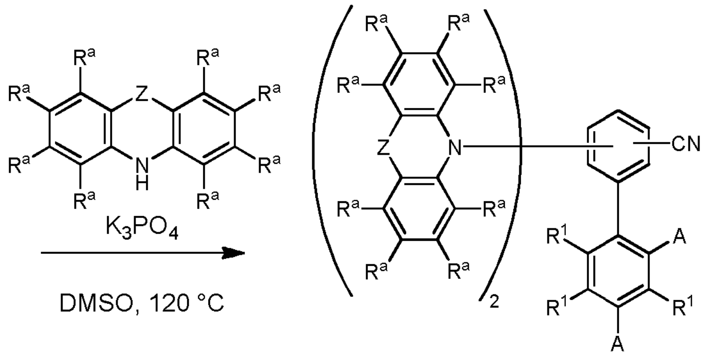


*Allgemeine Synthesevorschrift **AAV1-1:***

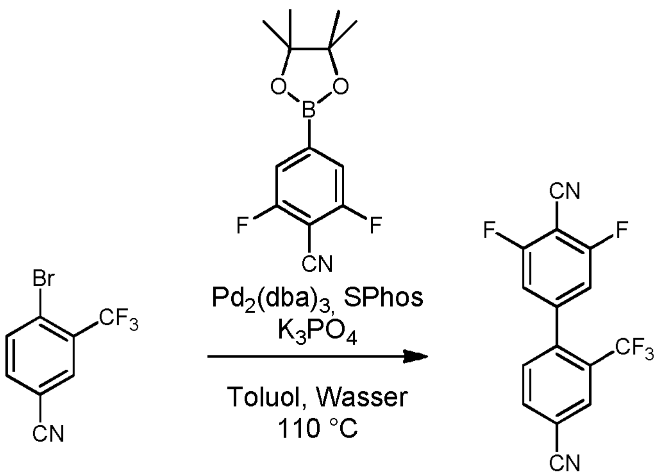


**Z1**

4-Brom-3-trifluormethylbenzonitril (1,00 Äquivalente), 4-Cyano-3,5-difluorphenylboronsäureester (1,20 Äquivalente), Pd$_2$(dba)$_3$ (0,01 Äquivalente), 2-Dicyclohexylphosphino-2',6'-dimethoxybiphenyl (SPhos) (0,04 Äquivalente) und tribasisches Kaliumphosphat (2,50 Äquivalente) werden unter Stickstoff in einem Toluol/Wasser-Gemisch (Verhältnis 10:1) bei 110 °C für 17 h gerührt. Durch typische Aufreinungsmethoden wie Extraktion oder Filtration und anschließendem Entfernen des jeweiligen Lösungsmittels wird das Produkt als Feststoff erhalten. Durch Umkristallisation und/oder Säulenchromatographie kann das Produkt bei Bedarf weiter aufgereinigt werden.

Erfindungsgemäß kann statt einem Boronsäureester auch eine entsprechende Boronsäure verwendet werden.

Statt Toluol können weitere geeignete Lösungsmittel wie z. B. Dioxan eingesetzt werden.

*Allgemeine Synthesevorschrift **AAV2-1:***

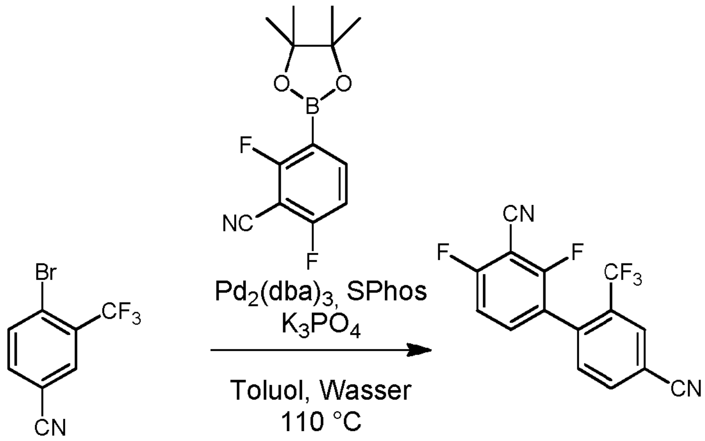


**Z2**

Die Synthese von **Z2** erfolgt analog ***AAV1-1,*** wobei 4-Brom-3-trifluormethylbenzonitril mit 3-Cyano-2,4-difluorphenylboronsäureester umgesetzt wird.

*Allgemeine Synthesevorschrift **AAV3-1**:*

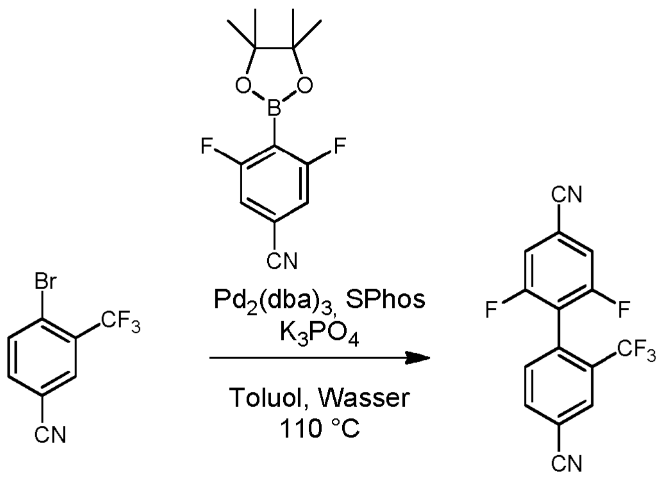


**Z3**

Die Synthese von **Z3** erfolgt analog ***AAV1-1,*** wobei 4-Brom-3-trifluormethylbenzonitril mit 4-Cyano-2,6-difluorphenylboronsäureester umgesetzt wird.

*Allgemeine Synthesevorschrift **AAV4-1:***

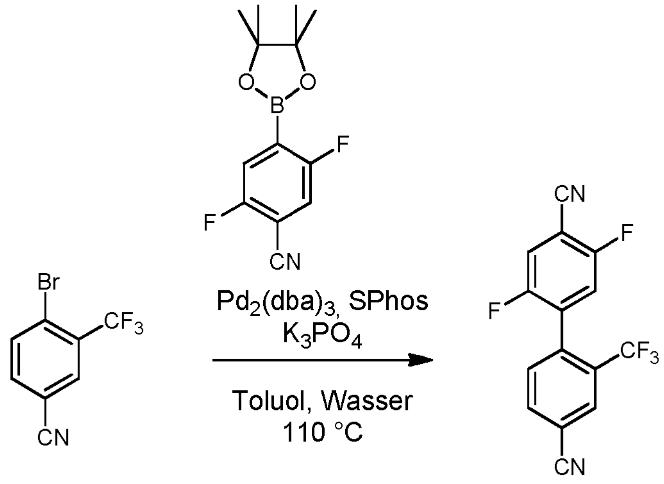


**Z4**

Die Synthese von **Z4** erfolgt analog ***AAV1-1,*** wobei 4-Brom-3-trifluormethylbenzonitril mit 4-Cyano-2,5-difluorphenylboronsäureester umgesetzt wird.

*Allgemeine Synthesevorschrift* ***AAV5-1*:**

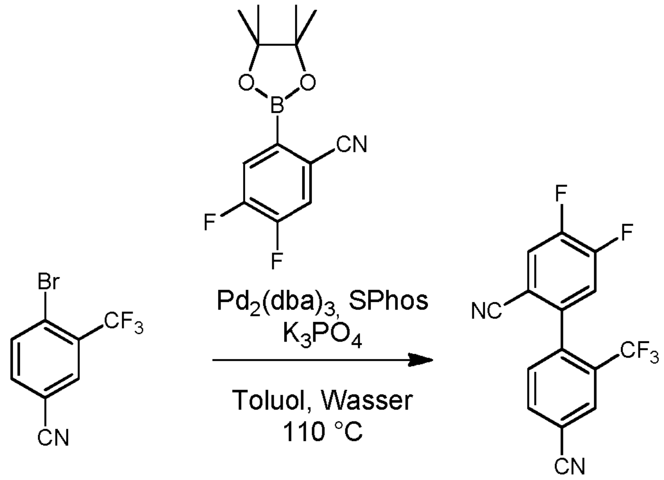


**Z5**

Die Synthese von **Z5** erfolgt analog ***AAV1-1,*** wobei 4-Brom-3-trifluormethylbenzonitril mit 2-Cyano-4,5-difluorphenylboronsäureester umgesetzt wird.

*Allgemeine Synthesevorschrift* ***AAV6-1*:**

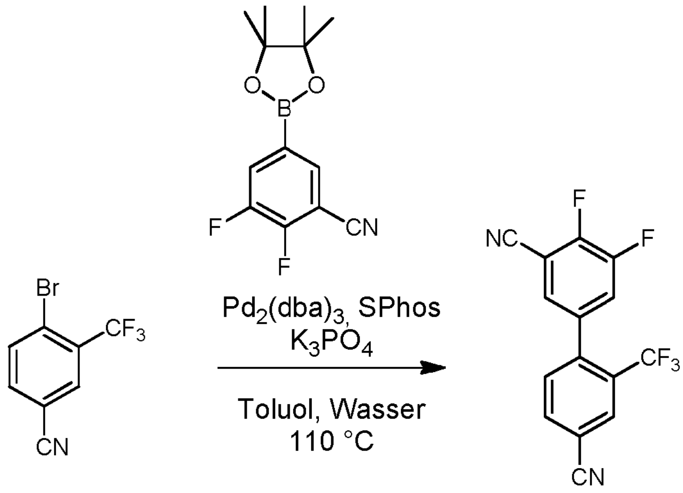


**Z6**

Die Synthese von **Z6** erfolgt analog ***AAV1-1,*** wobei 4-Brom-3-trifluormethylbenzonitril mit 3-Cyano-4,5-difluorphenylboronsäureester umgesetzt wird.

*Allgemeine Synthesevorschrift **AA V1-2:***

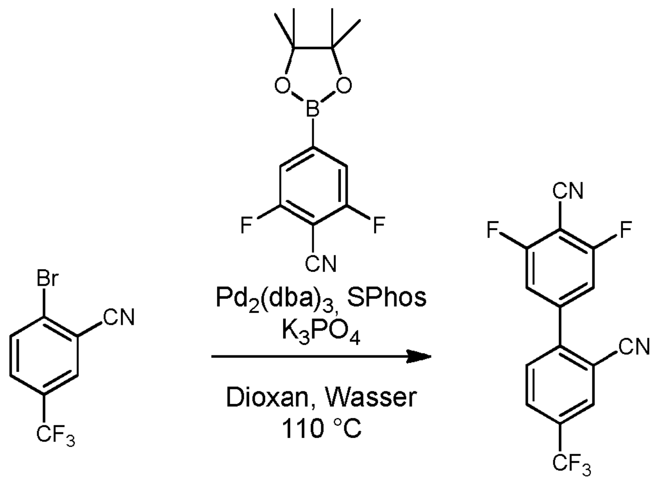


**Z7**

2-Brom-5-trifluormethylbenzonitril (1,00 Äquivalente), 4-Cyano-3,5-difluorphenylboronsäureester (1,00 Äquivalente), $Pd_2(dba)_3$ (0,01 Äquivalente), 2-Dicyclohexylphosphino-2',6'-dimethoxybiphenyl (SPhos) (0,08 Äquivalente), Kaliumacetat (2,00 Äquivalente) und tribasisches Kaliumphosphat (5,00 Äquivalente) werden unter Stickstoff in Dioxan bei 110 °C für 17 h gerührt. Durch typische Aufreinungsmethoden wie Extraktion, Filtration und/oder Säulenchromatographie und anschließendem Entfernen des jeweiligen Lösungsmittels wird das Produkt als Feststoff erhalten. Durch Umkristallisation und/oder Sublimation kann das Produkt bei Bedarf weiter aufgereinigt werden

Erfindungsgemäß kann statt einem Boronsäureester auch eine entsprechende Boronsäure verwendet werden. Statt Dioxan können weitere geeignete Lösungsmittel wie z.B. Toluol eingesetzt werden.

*Allgemeine Synthesevorschrift **AAV2-2**:*

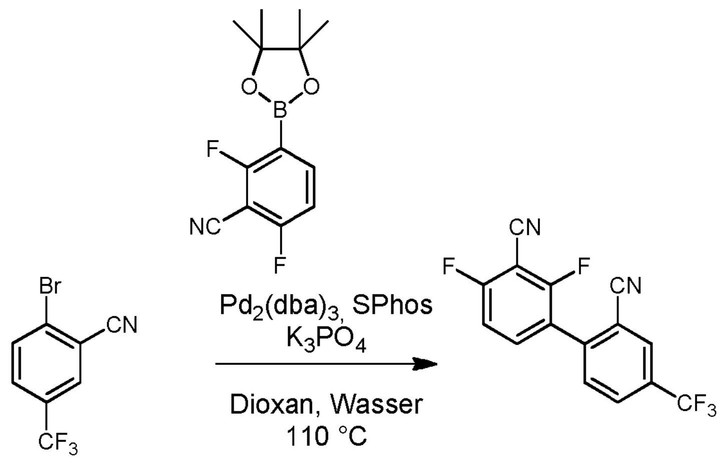


**Z8**

Die Synthese von **Z8** erfolgt analog ***AAV1-2*** wobei 2-Brom-5-trifluormethylbenzonitril mit 3-Cyano-2,4-difluorphenylboronsäureester umgesetzt wird.

*Allgemeine Synthesevorschrift **AAV3-2***:

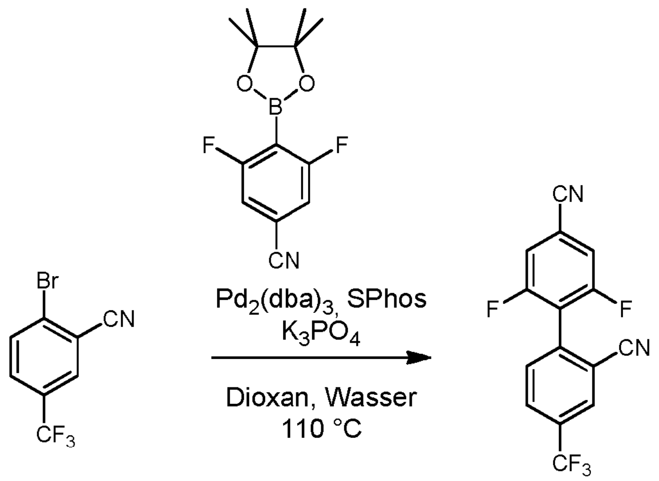


**Z9**

Die Synthese von **Z9** erfolgt analog ***AAV1-2***, wobei 2-Brom-5-trifluormethylbenzonitril mit 4-Cyano-2,6-difluorphenylboronsäureester umgesetzt wird.

*Allgemeine Synthesevorschrift **AAV4-2:***

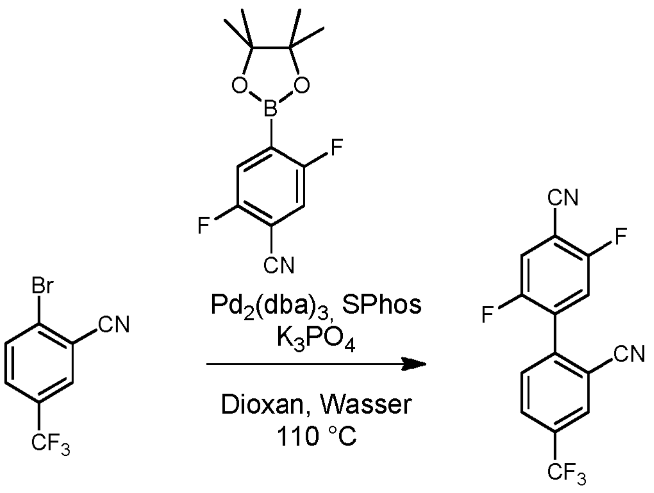


**Z10**

Die Synthese von **Z10** erfolgt analog ***AAV1-2,*** wobei 2-Brom-5-trifluormethylbenzonitril mit 4-Cyano-2,5-difluorphenylboronsäureester umgesetzt wird.

*Allgemeine Synthesevorschrift* ***AAV5-2*****:**

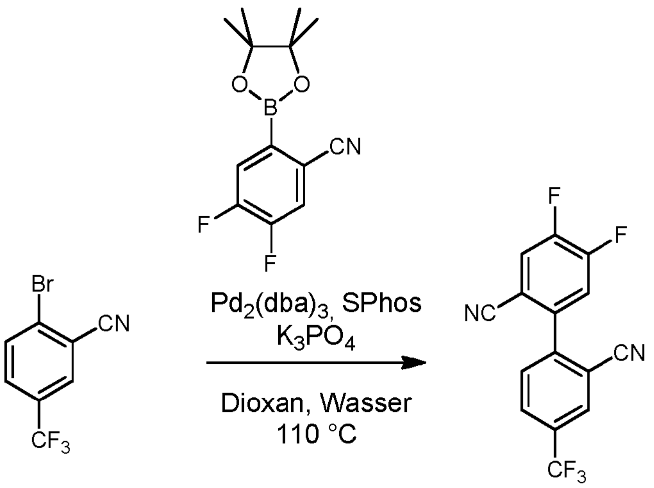


**Z11**

Die Synthese von **Z11** erfolgt analog ***AAV1-2,*** wobei 2-Brom-5-trifluormethylbenzonitril mit 2-Cyano-4,5-difluorphenylboronsäureester umgesetzt wird.

*Allgemeine Synthesevorschrift* ***AAV6-2*****:**

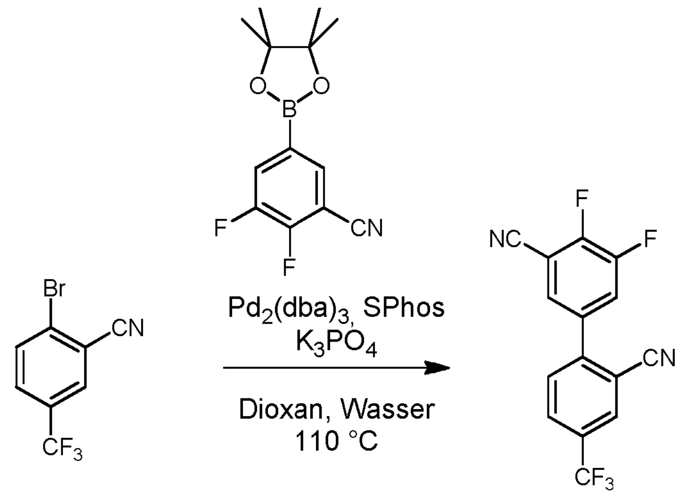


**Z12**

Die Synthese von **Z12** erfolgt analog ***AAV1-2,*** wobei 2-Brom-5-trifluormethylbenzonitril mit 2-Cyano-4,5-difluorphenylboronsäureester umgesetzt wird.

*Allgemeine Synthesevorschrift* ***AAV7*:**

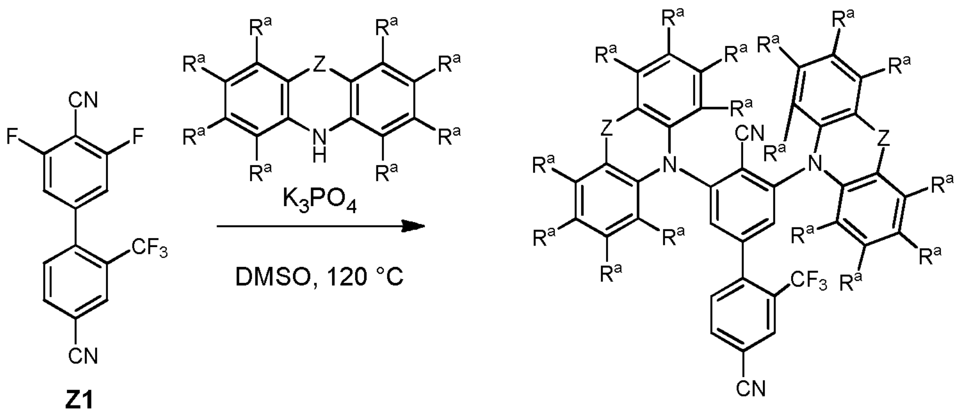


**Z1**

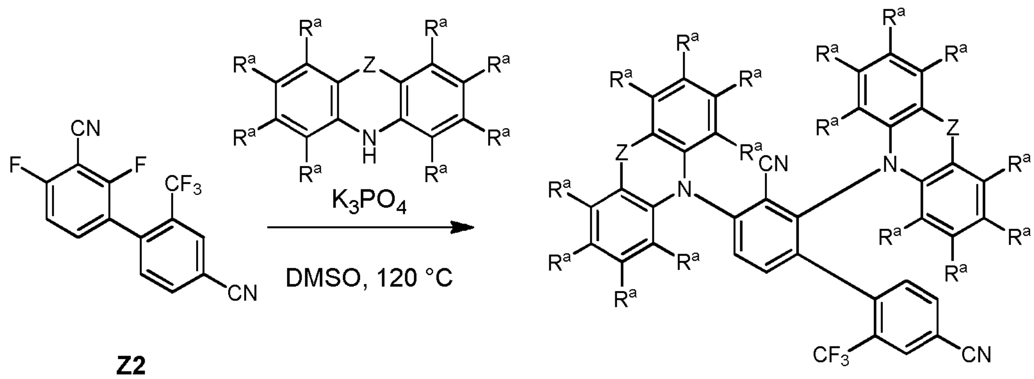


**Z2**

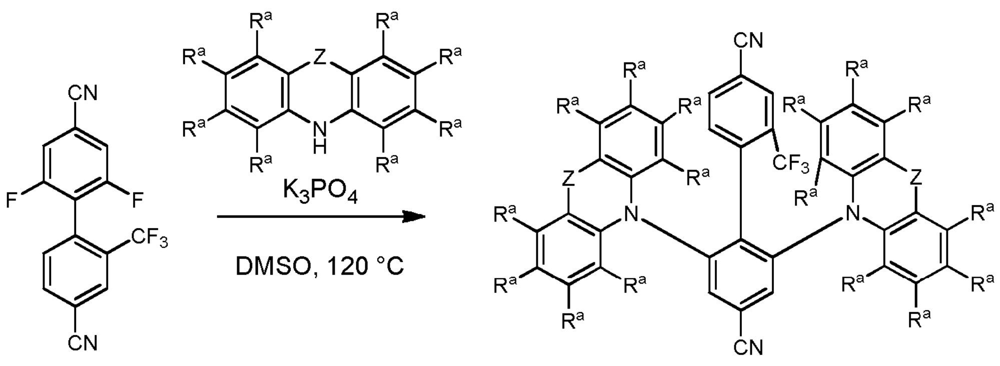


Z3

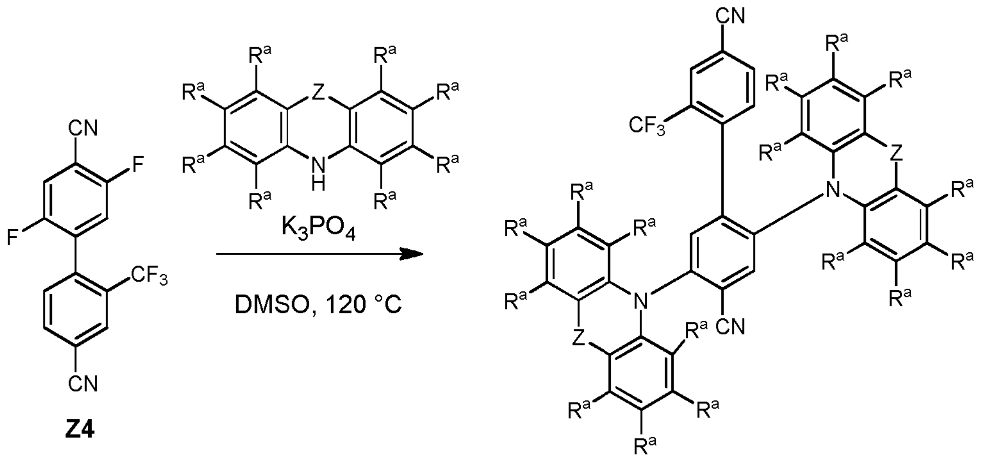


Z4

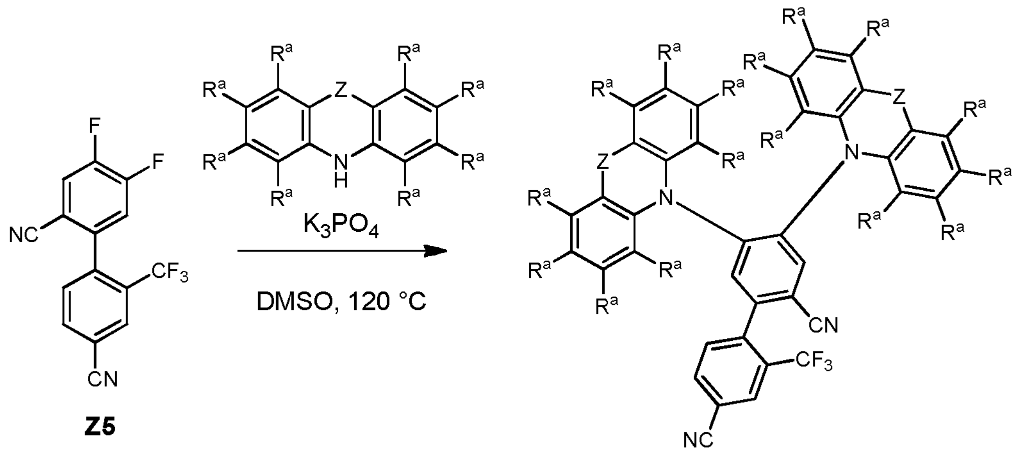


Z5

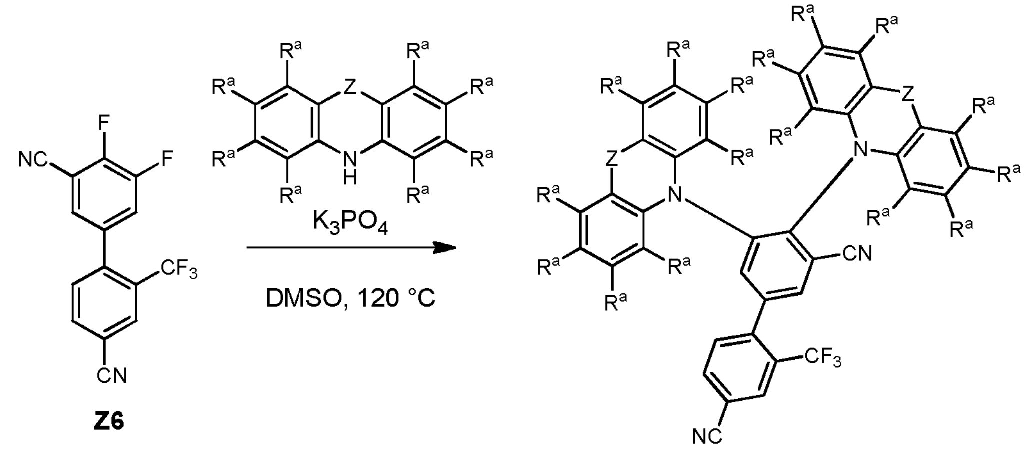
$K_3PO_4$
DMSO, 120 °C

**Z6**

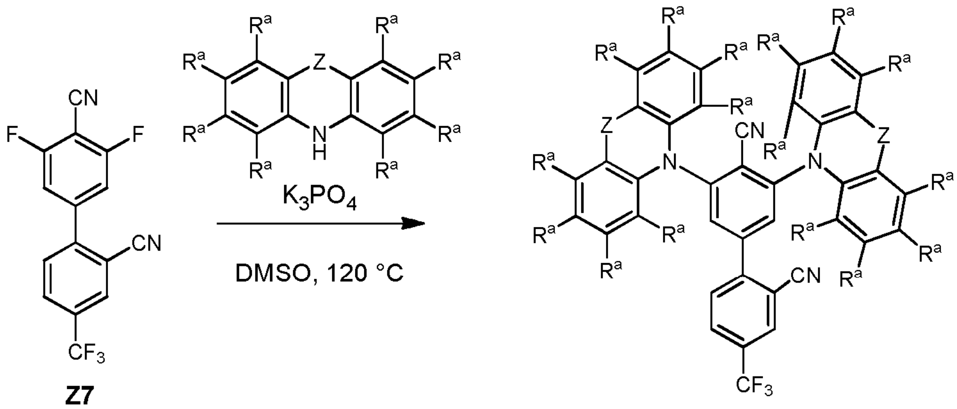
$K_3PO_4$
DMSO, 120 °C

**Z7**

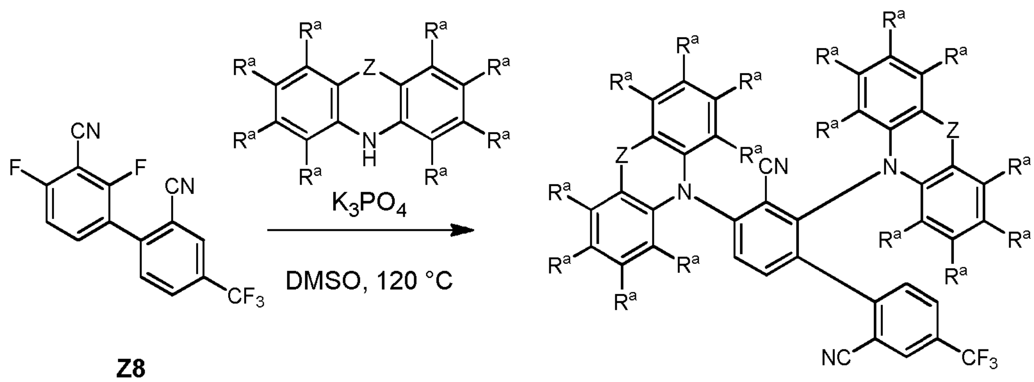
$K_3PO_4$
DMSO, 120 °C

**Z8**

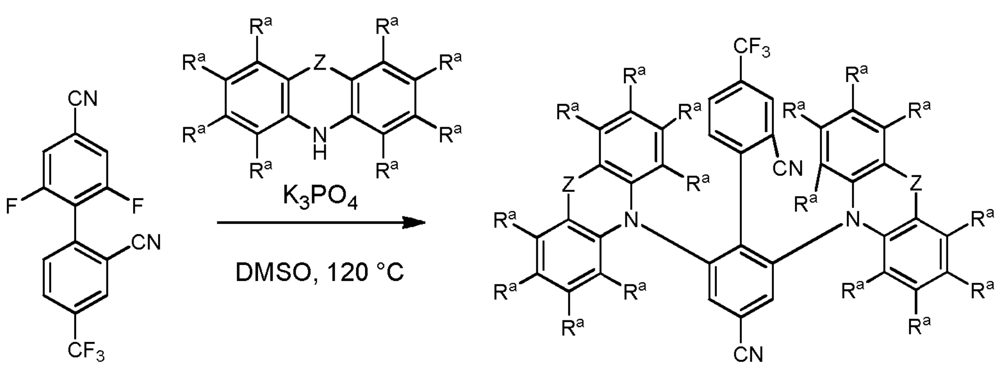
$K_3PO_4$
DMSO, 120 °C

**Z9**

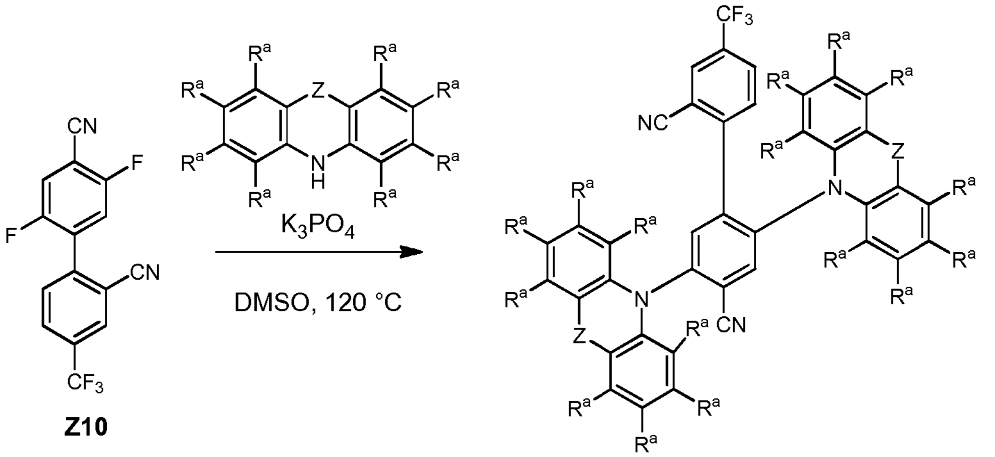


**Z10**

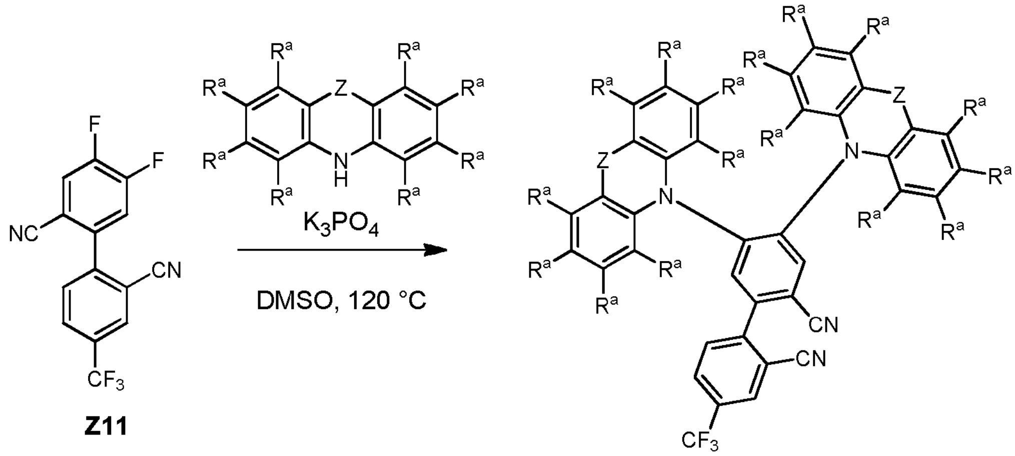


**Z11**

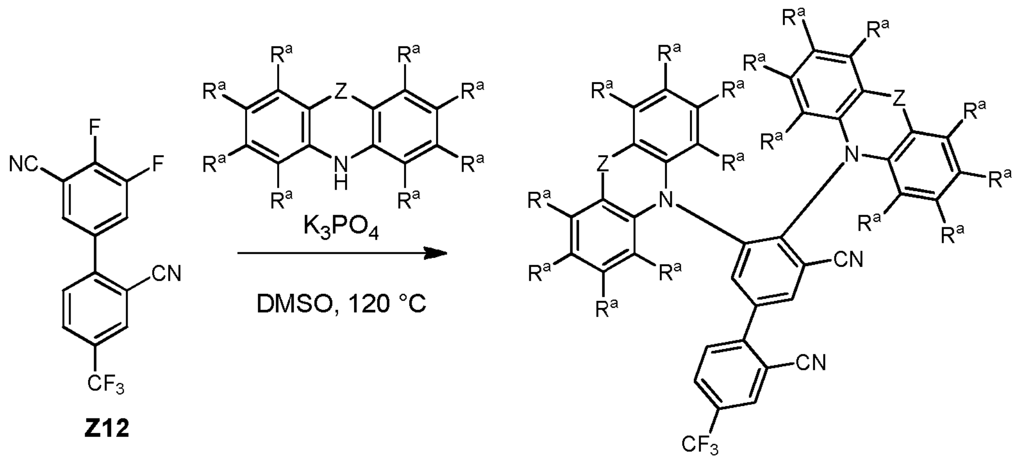


**Z12**

**Z1, Z2, Z3, Z4, Z5, Z6, Z7, Z8, Z9, Z10, Z11** oder **Z12** (jeweils 1,00 Äquivalente), das entsprechende Donor-Molekül D-H (2,00 Äquivalente) und tribasisches Kaliumphosphat (4,00 Äquivalente) werden unter Stickstoff in DMSO suspendiert und bei 120 °C gerührt (16 h). Anschließend wird die Reaktionsmischung in Wasser gegeben. Der ausgefallene Feststoff wird filtriert und mit Wasser gewaschen. Der ausgefallene Feststoff wird in Dichlormethan gelöst, über Natriumsulfat getrocknet, filtriert und das Lösungsmittel anschließend entfernt. Das Rohprodukt wird schließlich durch Umkristallisation aus Ethanol oder über Flashchromatographie gereinigt. Das Produkt wird als Feststoff erhalten.

Im speziellen entspricht D-H einem 3,6-substituierten Carbazol (z. B. 3,6-Dimethylcarbazol, 3,6-Diphenylcarbazol, 3,6-Di-tert-butylcarbazol), einem 2,7-substituierten Carbazol (z. B. 2,7-Dimethylcarbazol, 2,7-Diphenylcarbazol, 2,7-Di-tert-butylcarbazol), einem 1,8-substituierten Carbazol (z. B. 1,8-Dimethylcarbazol, 1,8-Diphenylcarbazol, 1,8-Di-tert-butylcarbazol), einem 1-substituierten Carbazol (z. B. 1-Methylcarbazol, 1-Phenylcarbazol, 1-tert-Butylcarbazol),

einem 2-substituierten Carbazol (z. B. 2-Methylcarbazol, 2-Phenylcarbazol, 2-tert-Butylcarbazol) oder einem 3-substituierten Carbazol (z. B. 3-Methylcarbazol, 3-Phenylcarbazol, 3-tert-Butylcarbazol).

**Allgemeine experimentelle Verfahren**

*Cyclovoltammetrie*

Cyclovoltammogramme von $10^{-3}$ molaren Lösungen der organischen Moleküle in Dichlormethan und Tetrabutylammoniumhexafluorophosphat (0.1 mol/l) als Leitelektrolyt wurden aufgenommen mit Hilfe eines Model 600D Series Electrochemical Analyzer mit Workstation (CH Instruments) bei einer Scanrate von 100 mV $s^{-1}$. Die Messungen wurden durchgeführt bei Raumtemperatur, unter Schutzgas-Atmosphäre mit einer Dreielektroden-Anordnung (Arbeits- und Gegenelektrode: Pt-Wire. Referenzelektrode: Pt-Wire) und kalibriert gegen $FeCp_2/FeCp_2^+$ als internem Standard. HOMO- und LUMO-Daten wurden korrigiert mittels Ferrocen als internem Standard gegen SCE (Saturated Calomel Electrode) und nach folgender Formel berechnet:

$$E_{CV-HOMO} = -(1.4 * 0.978 * (Ox/V - Ferrocen/V) + 4.6)\ eV.$$

*Rechnungen nach der Dichtefunktionaltheorie*

Für die Optimierung der Molekülstrukturen wurde das BP86-Funktional verwendet, wobei die resolution-of-identity-Näherung (RI) zum Einsatz kam. Anregungsenergien wurden für die mit BP86 optimierten Strukturen mit der Time-Dependent DFT-Methode (TD-DFT) unter Verwendung des B3LYP-Funktionals berechnet. In allen Rechnungen wurden def2-SV(P)-Basissätze und ein m4-Grid wurden zur numerischen Integration verwendet. Alle DFT-Rechnungen wurden mit dem Turbomole-Programmpaket (Version 6.5) (TURBOMOLE V6.4 2012, a development of University of Karlsruhe and Forschungszentrum Karlsruhe GmbH, 1989-2007, TURBOMOLE GmbH, since 2007; available from http://www.turbomole.com) durchgeführt.

**Photophysikalische Messungen**

*Probenvorbereitung, Film: Spin-Coating*

Gerät: Spin150, SPS euro.

Die Probenkonzentration entsprach 10 mg/ml, angesetzt in einem geeigneten Lösungsmittel. Programm: 1) 3 s bei 400 U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenzspektroskopie und TCSPC

Steady-state Emissionsspektroskopie wurde mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" *(Time-correlated single-photon counting,* TCSPC)-Option. Emissions- und Anregungsspektren wurden korrigiert durch Standardkorrekturkurven.

Die Emissionsabklingzeiten wurden ebenfalls auf diesem System gemessen unter Verwendung der TCSPC-Methode mit dem FM-2013 Zubehör und einem TCSPC-Hub von Horiba Yvon Jobin. Anregungsquellen:

NanoLED 370 (Wellenlänge: 371 nm, Pulsdauer: 1,1 ns)
NanoLED 290 (Wellenlänge: 294 nm, Pulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben.

Quanteneffizienzbestimmung

Die Messung der Photolumineszenzquantenausbeute (PLQY) erfolgte mittels eines *Absolute PL Quantum*

*Yield Measurement* C9920-03G-Systems der *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.

Das Emissionsmaximum wird in nm, die Quantenausbeute Φ in % und die CIE-Farbkoordinaten als x,y-Werte angegeben.

Die Photolumineszenzquantenausbeute wurde nach folgendem Protokoll bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorptionsmaximum des organischen Moleküls bestimmt und mit diesem angeregt.
3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbiert} = \frac{\int \frac{\lambda}{hc} \left[Int_{emittiert}^{Probe}(\lambda) - Int_{absorbiert}^{Probe}(\lambda)\right] d\lambda}{\int \frac{\lambda}{hc} \left[Int_{emittiert}^{Referenz}(\lambda) - Int_{absorbiert}^{Referenz}(\lambda)\right] d\lambda}$$

mit der Photonenzahl $n_{photon}$ und der Intensität Int.

Alle photophysikalischen Messungen wurden in dem jeweils in den Beispielen angegebenen Medium und bei Raumtemperatur bestimmt.

*Herstellung und Charakterisierung von organischen Elektrolumineszenzvorrichtungen aus der Gasphase*

Mit den erfindungsgemäßen organischen Molekülen können OLED-Devices mittels Vakuum-Sublimationstechnik erstellt werden. Enthält eine Schicht mehrere Komponenten, so ist das Verhältnis dieser in Massenprozent angegeben.

Diese noch nicht optimierten OLEDs können standardmäßig charakterisiert werden; hierfür werden die Elektrolumineszenzspektren, die externe Quanteneffizienz (gemessen in %) in Abhängigkeit von der Helligkeit, berechnet aus dem von der Fotodiode detektiertem Licht, und dem Strom aufgenommen. Aus dem zeitlichen Verlauf der Elektrolumineszenzspektren kann die Lebensdauer der OLEDs bestimmt werden. Der angegebene LT50-Wert entspricht hierbei der Zeit, bei der die Leuchtdichte auf 50 % des Startwertes abgefallen ist. Analog entspricht der LT70-Wert der Zeit, bei der die Leuchtdichte auf 70 % des Startwertes abgefallen ist.

Die angegebenen Werte ergeben sich aus dem Durchschnitt der verschiedenen Pixel einer OLED. Die abgebildeten Spektren zeigen jeweils eine Messreihe eines Pixels.

**HPLC-MS:**

HPLC-MS Spektroskopie wurde mit einer HPLC-Anlage der Firma Agilent (1100er Serie) mit einem angeschlossenen MS-Detektor (Thermo LTQ XL) gemessen. Für die HPLC wurde eine RP Säule 4,6mm x 150mm, Partikelgröße 5,0μm von Waters verwendet. Es wurde ohne Vorsäule und bei Raumtemperatur mit den Lösemitteln Acetonitril, Wasser und Tetrahydrofuran in diesen Konzentrationen gearbeitet:

| | | |
|---|---|---|
| Lösemittel A: | $H_2O$ (90%) | MeCN (10%) |
| Lösemittel B: | $H_2O$ (10%) | MeCN (90%) |
| Lösemittel C: | THF (50%) | MeCN (50%) |

Es wurde mit einem Einspritzvolumen von 15 μL und einer Konzentration von 0.5mg/ml gearbeitet.

| **Fluss [ml/min]** | **Zeit [min]** | **A[%]** | **B[%]** | **C[%]** |
|---|---|---|---|---|
| 3 | 0.00 | 40 | 50 | 10 |

(fortgesetzt)

| Fluss [ml/min] | Zeit [min] | A[%] | B[%] | C[%] |
|---|---|---|---|---|
| 3 | 10.00 | 15 | 25 | 60 |
| 3 | 14.00 | 15 | 25 | 60 |
| 3 | 14.01 | 40 | 50 | 10 |
| 3 | 18.00 | 40 | 50 | 10 |
| 3 | 19.00 | 40 | 50 | 10 |

Die Ionisation der Probe erfolgt durch APCI (atmospheric pressure chemical ionization).

**Beispiel 1**

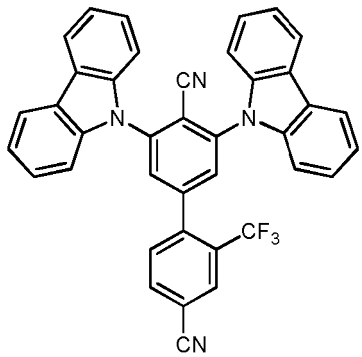

Beispiel **1** wurde nach AAV1-1 (Ausbeute 54%) und AAV7 (Ausbeute 79%) hergestellt.
MS (HPLC-MS), m/z (Retentionszeit): 602.18 (6.11 min)
Figur 1 zeigt das Emissionsspektrum von Beispiel 1 (10 % in PMMA). Das Emissionsmaximum liegt bei 456 nm. Die Photolumineszenzquantenausbeute (PLQY) beträgt 88% und die Halbwertsbreite beträgt 0,47 eV.

**Beispiel 2**

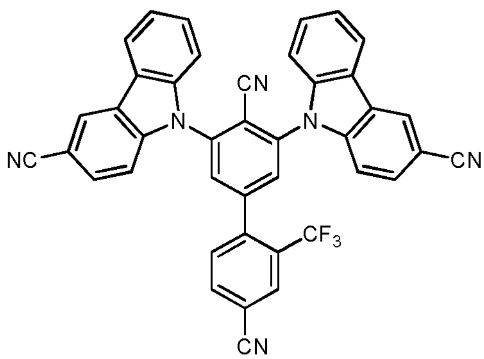

Beispiel **2** wurde nach AAV1-1 (Ausbeute 88%) und AAV7 (Ausbeute 90%) hergestellt.
MS (HPLC-MS), m/z (Retentionszeit): 652.2 (4.69 min)
Figur 2 zeigt das Emissionsspektrum von Beispiel **2** (10 % in PMMA). Das Emissionsmaximum liegt bei 437 nm. Die Photolumineszenzquantenausbeute (PLQY) beträgt 61% und die Halbwertsbreite beträgt 0,49 eV.

**Beispiel 3**

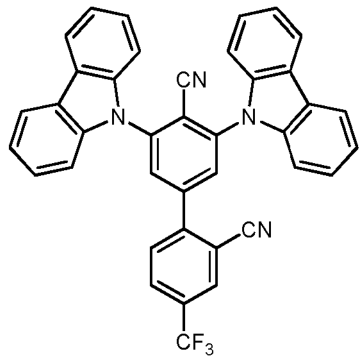

Beispiel **3** wurde nach AAV1-2 (Ausbeute 54%) und AAV7 (Ausbeute 58%) hergestellt.
MS (HPLC-MS), m/z (Retentionszeit): 602.21 (6.23 min)
Figur 3 zeigt das Emissionsspektrum von Beispiel **3** (10 % in PMMA). Das Emissionsmaximum liegt bei 474 nm. Die Photolumineszenzquantenausbeute (PLQY) beträgt 80% und die Halbwertsbreite beträgt 0,45 eV. Die Emissionsabklingzeit beträgt 39 μs.

**Beispiel 4**

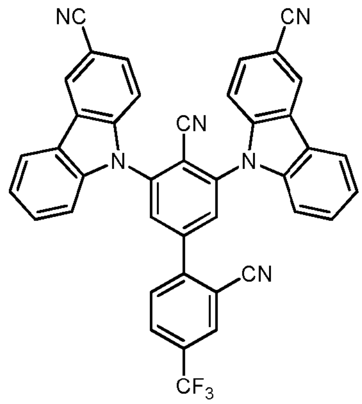

Beispiel **3** wurde nach AAV1-2 (Ausbeute 54%) und AAV7 (Ausbeute 49%) hergestellt.
Figur 4 zeigt das Emissionsspektrum von Beispiel **4** (10 % in PMMA). Das Emissionsmaximum liegt bei 452 nm. Die Photolumineszenzquantenausbeute (PLQY) beträgt 69% und die Halbwertsbreite beträgt 0,46 eV.

**Beispiel 5**

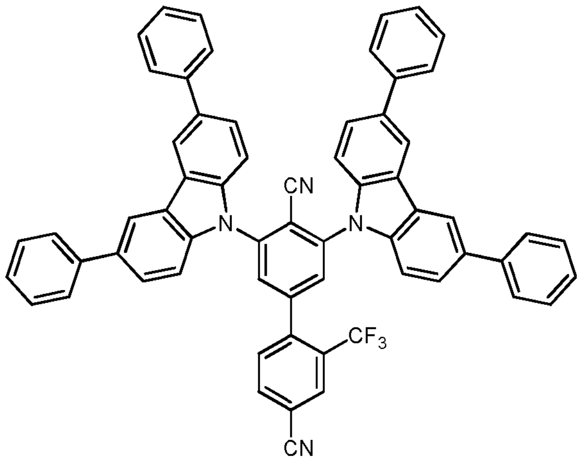

Beispiel **5** wurde nach AAV1-1 (Ausbeute 88%) und AAV7 (Ausbeute 16%) hergestellt.

Figur 5 zeigt das Emissionsspektrum von Beispiel **5** (10 % in PMMA). Das Emissionsmaximum liegt bei 489 nm. Die Photolumineszenzquantenausbeute (PLQY) beträgt 80% und die Halbwertsbreite beträgt 0,46 eV. Die Emissionsabklingzeit beträgt 11 μs.

**Beispiel 6**

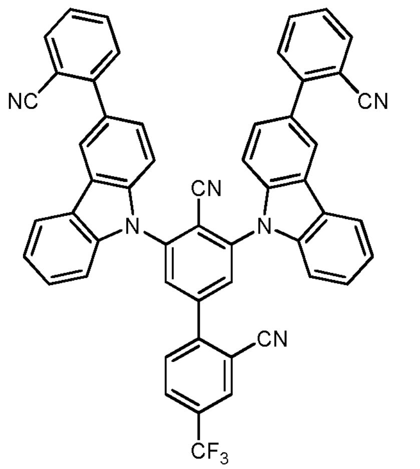

Beispiel **6** wurde nach AAV1-2 (Ausbeute 54%) und AAV7 (Ausbeute 45%) hergestellt.

Figur 6 zeigt das Emissionsspektrum von Beispiel **6** (10 % in PMMA). Das Emissionsmaximum liegt bei 474 nm. Die Photolumineszenzquantenausbeute (PLQY) beträgt 66% und die Halbwertsbreite beträgt 0,45 eV. Die Emissionsabklingzeit beträgt 87 μs.

**Beispiel 7**

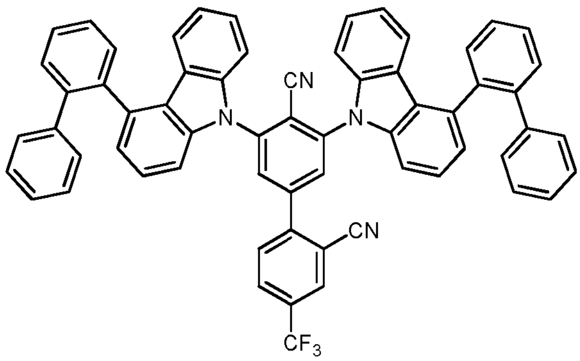

Beispiel **7** wurde nach AAV1-2 (Ausbeute 54%) und AAV7 (Ausbeute 57%) hergestellt.

Figur 7 zeigt das Emissionsspektrum von Beispiel **7** (10 % in PMMA). Das Emissionsmaximum liegt bei 477 nm. Die Photolumineszenzquantenausbeute (PLQY) beträgt 84% und die Halbwertsbreite beträgt 0,44 eV. Die Emissionsabklingzeit beträgt 55 µs.

**Beispiel 8**

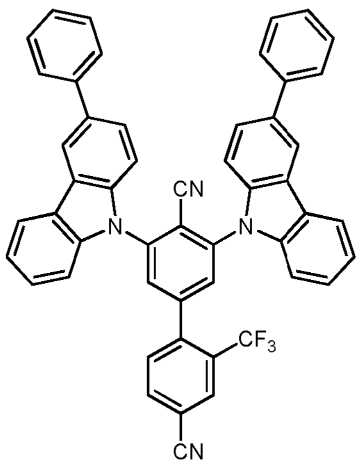

Beispiel **8** wurde nach AAV1-1 (Ausbeute 88%) und AAV7 (Ausbeute 51%) hergestellt.

Figur 8 zeigt das Emissionsspektrum von Beispiel **8** (10 % in PMMA). Das Emissionsmaximum liegt bei 481 nm. Die Photolumineszenzquantenausbeute (PLQY) beträgt 75% und die Halbwertsbreite beträgt 0,48 eV. Die Emissionsabklingzeit beträgt 13 µs.

**Beispiel 9**

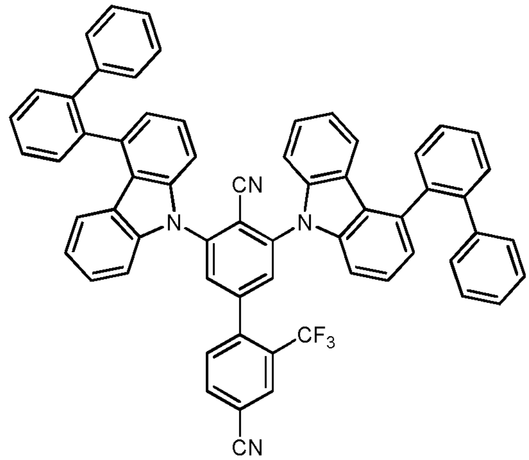

Beispiel **9** wurde nach AAV1-1 (Ausbeute 88%) und AAV7 (Ausbeute 58%) hergestellt.

Figur 9 zeigt das Emissionsspektrum von Beispiel **9** (10 % in PMMA). Das Emissionsmaximum liegt bei 466 nm. Die Photolumineszenzquantenausbeute (PLQY) beträgt 66% und die Halbwertsbreite beträgt 0,47 eV.

**Beispiel 10**

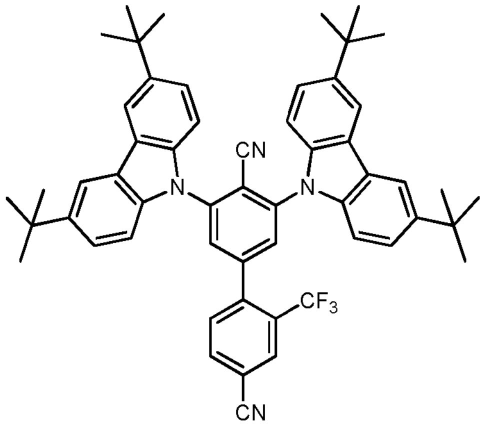

Beispiel **10** wurde nach AAV1-1 (Ausbeute 88%) und AAV7 (Ausbeute 71%) hergestellt.

Figur 10 zeigt das Emissionsspektrum von Beispiel **10** (10 % in PMMA). Das Emissionsmaximum liegt bei 480 nm. Die Photolumineszenzquantenausbeute (PLQY) beträgt 81% und die Halbwertsbreite beträgt 0,47 eV. Die Emissionsabklingzeit beträgt 7 µs.

**Beispiel 11**

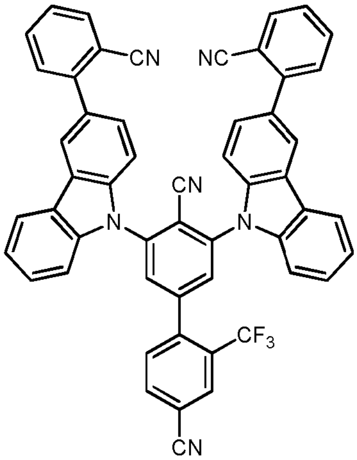

Beispiel **11** wurde nach AAV1-1 (Ausbeute 88%) und AAV7 (Ausbeute 25%) hergestellt.

Figur 11 zeigt das Emissionsspektrum von Beispiel **11** (10 % in PMMA). Das Emissionsmaximum liegt bei 462 nm. Die Photolumineszenzquantenausbeute (PLQY) beträgt 79% und die Halbwertsbreite beträgt 0,47 eV.

**Beispiel 12**

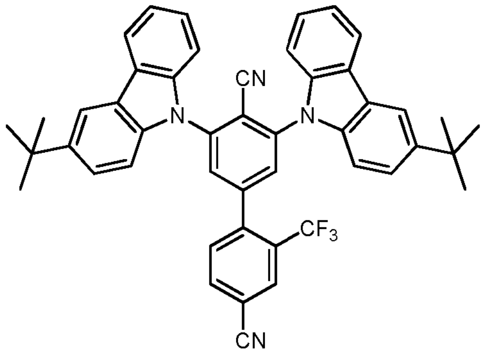

Beispiel **12** wurde nach AAV1-1 (Ausbeute 88%) und AAV7 (Ausbeute 59%) hergestellt.

Figur 12 zeigt das Emissionsspektrum von Beispiel **12** (10 % in PMMA). Das Emissionsmaximum liegt bei 473 nm. Die Photolumineszenzquantenausbeute (PLQY) beträgt 89% und die Halbwertsbreite beträgt 0,47 eV. Die Emissionsabklingzeit beträgt 14 µs.

**Beispiel D1**

Beispiel **1** wurde in dem OLED-Bauteil **D1** mit folgendem Aufbau getestet (Anteil des erfindungsgemäßen Moleküls und des Hostmoleküls an der Emissionsschicht ist jeweils in Massenprozent angegeben):

| **Schicht** | **Dicke** | **Material** |
|---|---|---|
| **10** | 100 nm | Al |
| **9** | 2 nm | Liq |
| **8** | 30 nm | TPBi |
| **7** | 10 nm | DPEPO |
| **6** | 20 nm | **Beispiel 1** (10%):DPEPO (90%) |
| **5** | 10 nm | CzSi |

(fortgesetzt)

| Schicht | Dicke | Material |
|---|---|---|
| **4** | 20 nm | TCTA |
| **3** | 70 nm | NPB |
| **2** | 20 nm | m-MTDATA |
| **1** | 130 nm | ITO |
| **Substrat** | | Glas |

Das Emissionsmaximum liegt bei 468 nm, CIEx wurde mit 0,17 und die CIEy mit 0,24 bei 6 V bestimmt. Die EQE bei 1000 cd/$m^2$ beträgt 7,9 ± 0,2 %.

**Beispiel D2**

Das OLED-Bauteil **D2** wurde analog zu OLED-Bauteil **D1** gefertigt, nur dass Beispiel **1** durch Beispiel **3** in der Emissionsschicht ersetzt wurde.
Das Emissionsmaximum liegt bei 475 nm, CIEx wurde mit 0,19 und die CIEy mit 0,31 bei 6 V bestimmt. Die EQE bei 1000 cd/$m^2$ beträgt 8,0 ± 0,3 %.

**Beispiel D3**

Beispiel **3** wurde in dem OLED-Bauteil **D3** mit folgendem Aufbau getestet (Anteil des erfindungsgemäßen Moleküls und der Hostmoleküle an der Emissionsschicht ist jeweils in Massenprozent angegeben):

| Schicht | Dicke | Material |
|---|---|---|
| **8** | 100 nm | Al |
| **7** | 2 nm | Liq |
| **6** | 30 nm | NBPhen |
| **5** | 10 nm | T2T |
| **4** | 20 nm | **Beispiel 3** (20%): mCBP (65%): T2T (15%) |
| **3** | 10 nm | TCTA |
| **2** | 80 nm | NPB |
| **1** | 130 nm | ITO |
| **Substrat** | | Glas |

Das Emissionsmaximum liegt bei 475 nm, CIEx wurde mit 0,19 und die CIEy mit 0,34 bei 6 V bestimmt. Die EQE bei 1000 cd/$m^2$ beträgt 11,2 ± 0,1 %.

**Beispiel D4**

Beispiel **7** wurde in dem OLED-Bauteil **D4** mit folgendem Aufbau getestet:

| Schicht | Dicke | Material |
|---|---|---|
| **9** | 100 nm | Al |
| **8** | 2 nm | Liq |
| **7** | 30 nm | NBPhen |
| **6** | 10 nm | T2T |
| **5** | 30 nm | **Beispiel 7** (20%): mCBP (60%): T2T (20%) |

(fortgesetzt)

| Schicht | Dicke | Material |
|---|---|---|
| **4** | 8 nm | mCBP |
| **3** | 10 nm | TCTA |
| **2** | 62 nm | NPB |
| **1** | 130 nm | ITO |
| **Substrat** | | Glas |

Das Emissionsmaximum liegt bei 480 nm, CIEx wurde mit 0,18 und die CIEy mit 0,35 bei 6 V bestimmt. Die EQE bei 1000 cd/$m^2$ beträgt 16,9 $\pm$ 0,1 %.

**Beispiel D5**

Das OLED-Bauteil **D5** wurde analog zu OLED-Bauteil **D4** gefertigt mit dem Unterschied, dass Schicht 5 wie folgt aufgebaut war: **Beispiel 8** (20%): mCBP (70%): T2T (10%).

Das Emissionsmaximum liegt bei 475 nm, CIEx wurde mit 0,20 und die CIEy mit 0,30 bei 6 V bestimmt. Die EQE bei 1000 cd/$m^2$ beträgt 11,4 $\pm$ 0,1 %.

**Beispiel D6**

Das OLED-Bauteil **D6** wurde analog zu OLED-Bauteil **D4** gefertigt mit dem Unterschied, dass Beispiel **7** durch Beispiel **12** ersetzt wurde.

Das Emissionsmaximum liegt bei 475 nm, CIEx wurde mit 0,19 und die CIEy mit 0,29 bei 6 V bestimmt. Die EQE bei 1000 cd/$m^2$ beträgt 8,6 $\pm$ 0,2 %.

**Weitere Beispiele erfindungsgemäßer organischer Moleküle:**

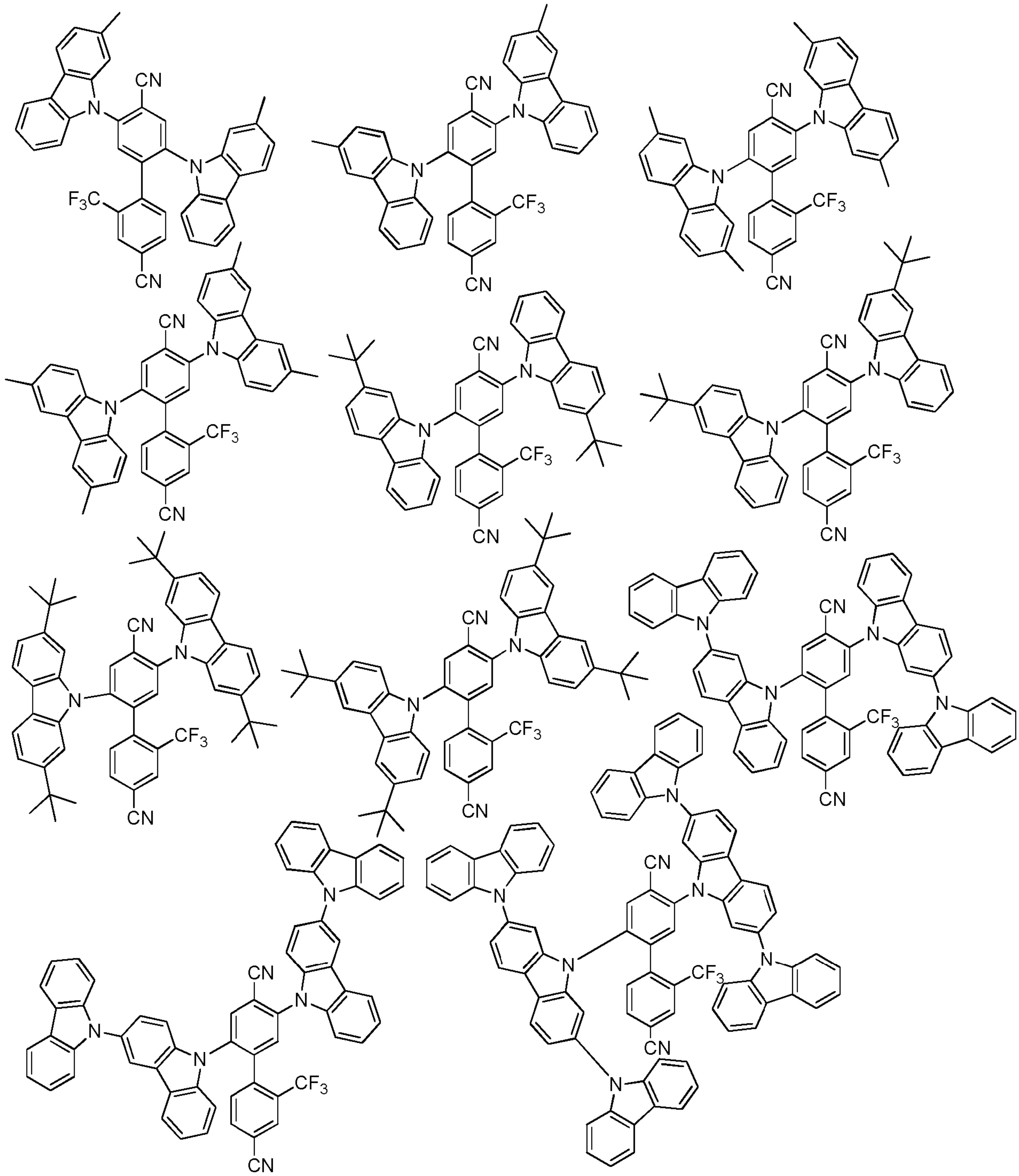

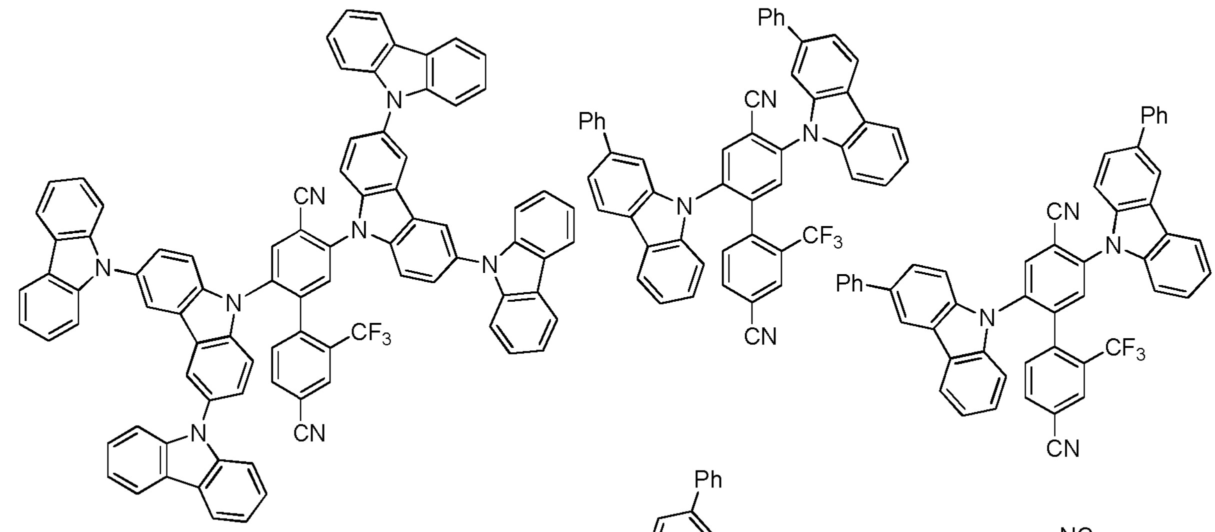

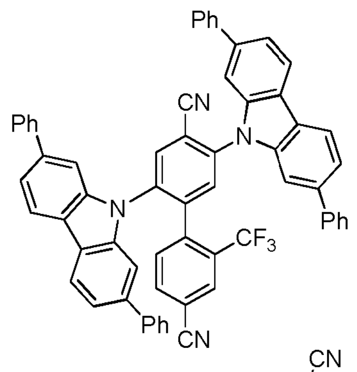

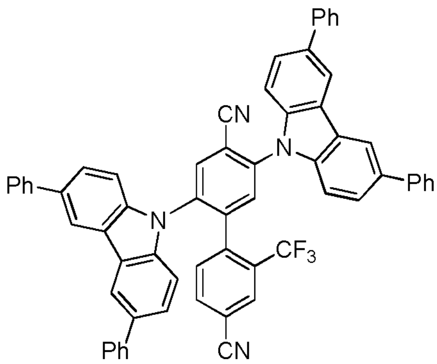

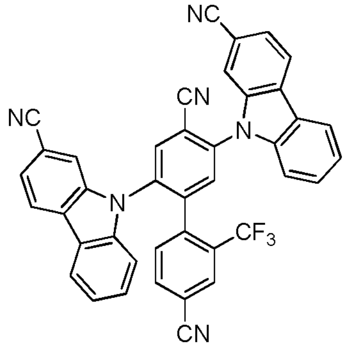

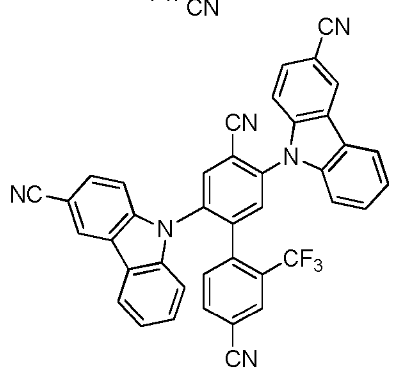

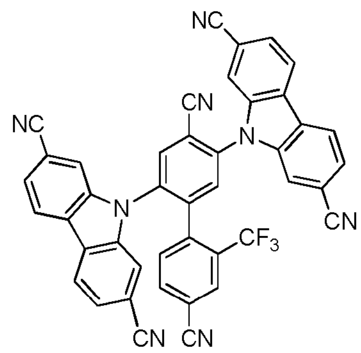

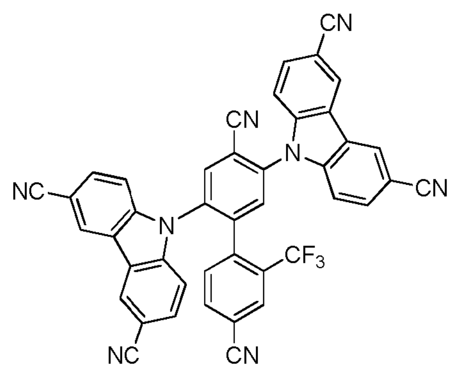

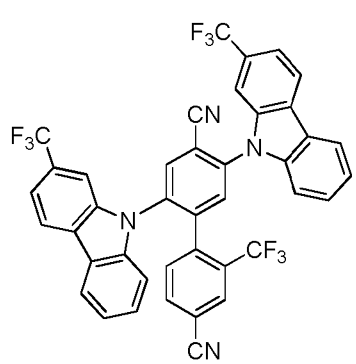

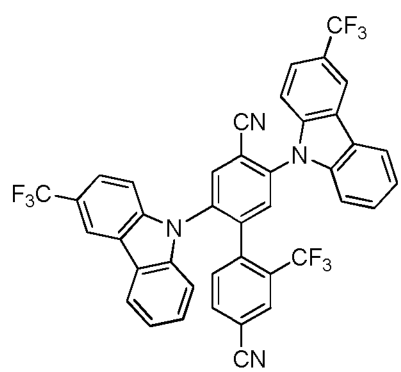

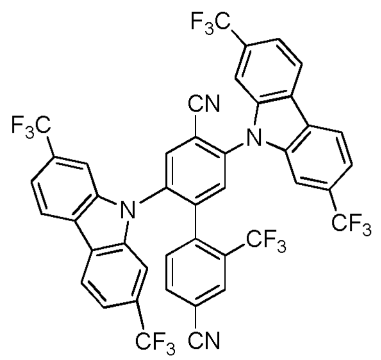

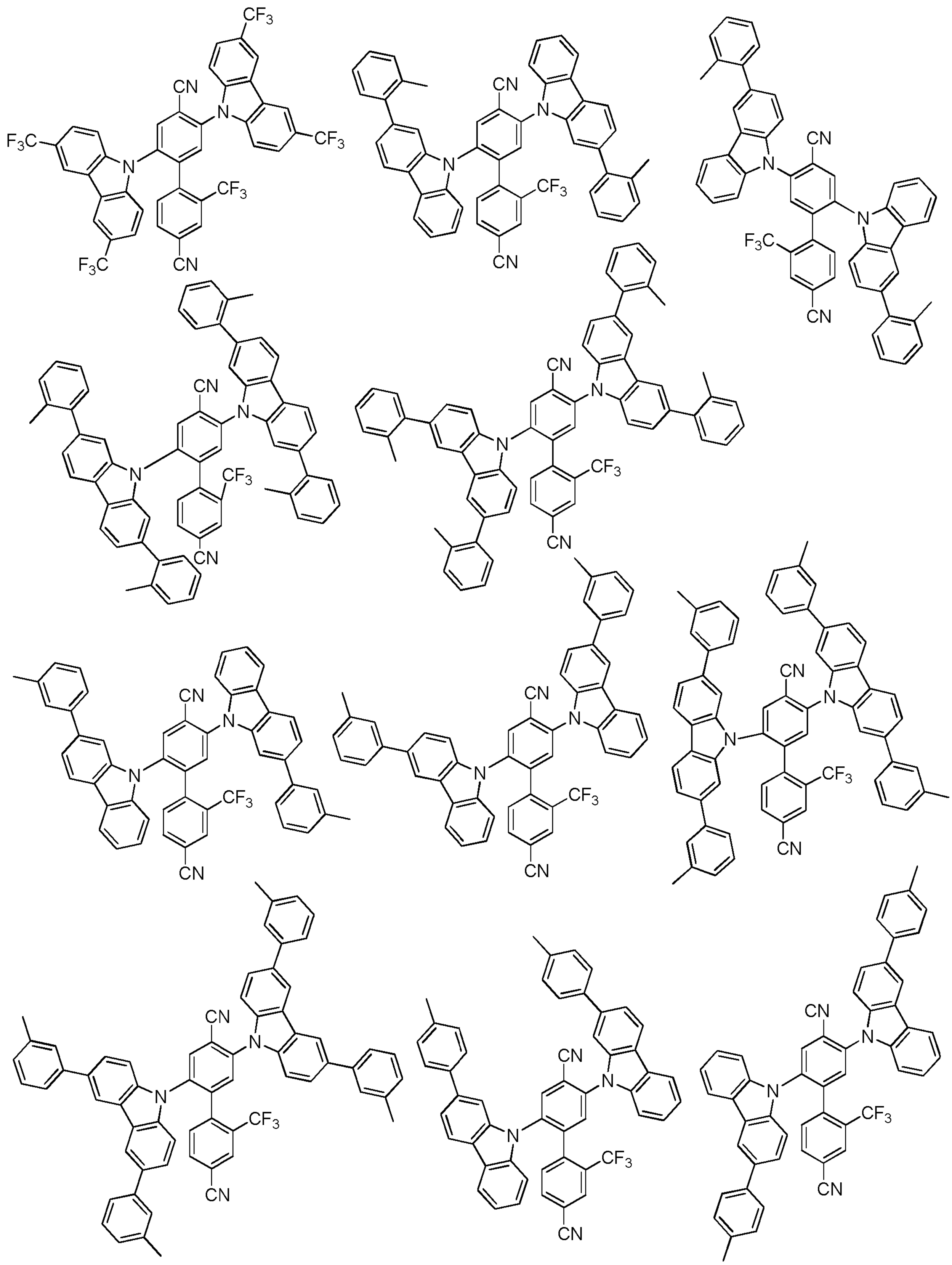

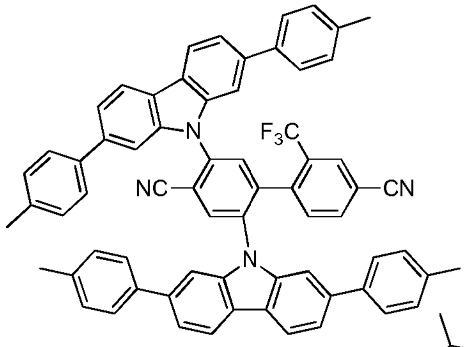

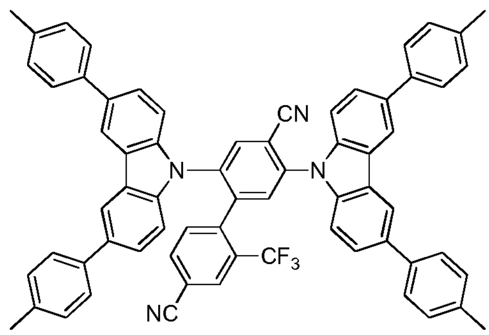

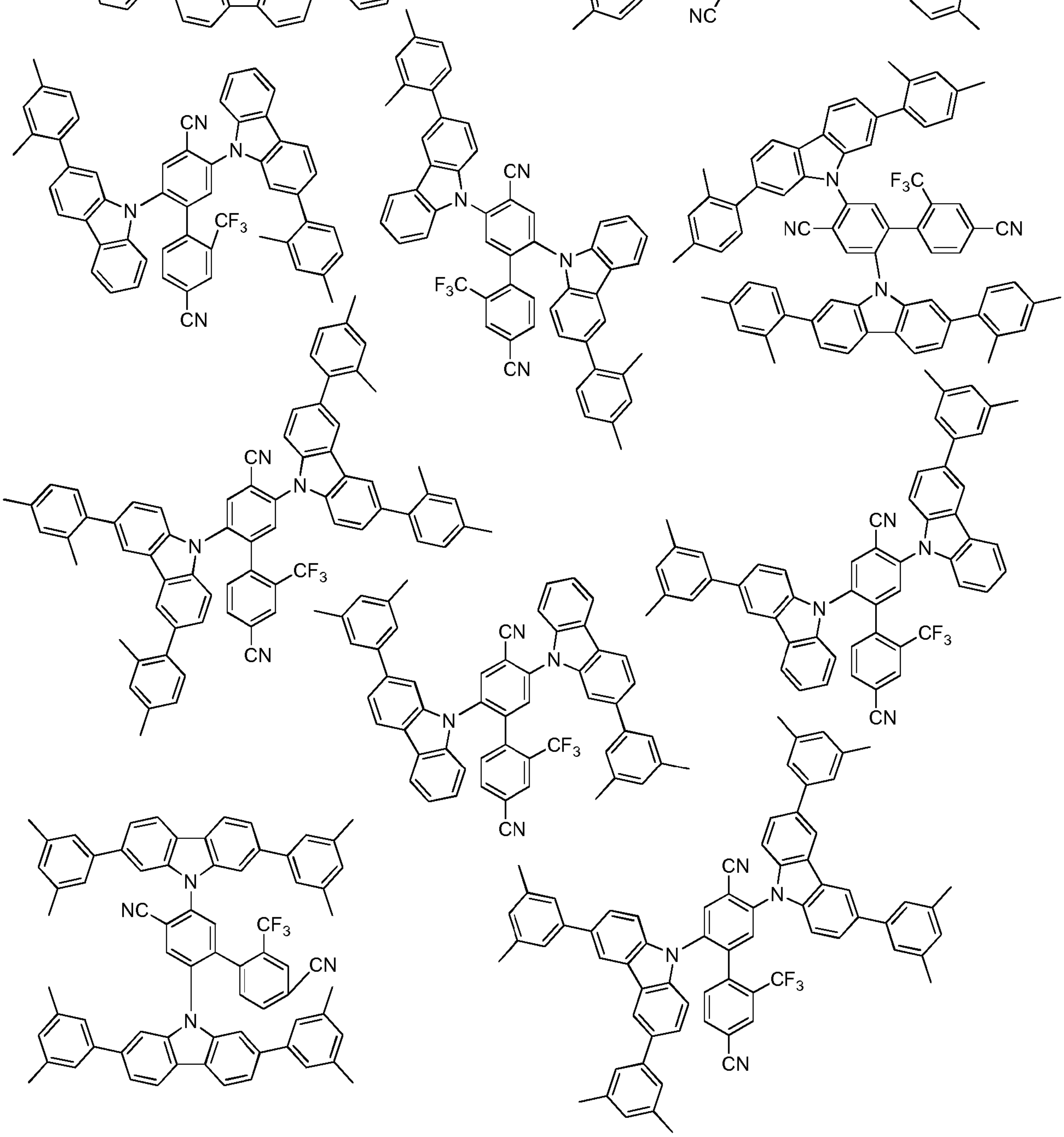

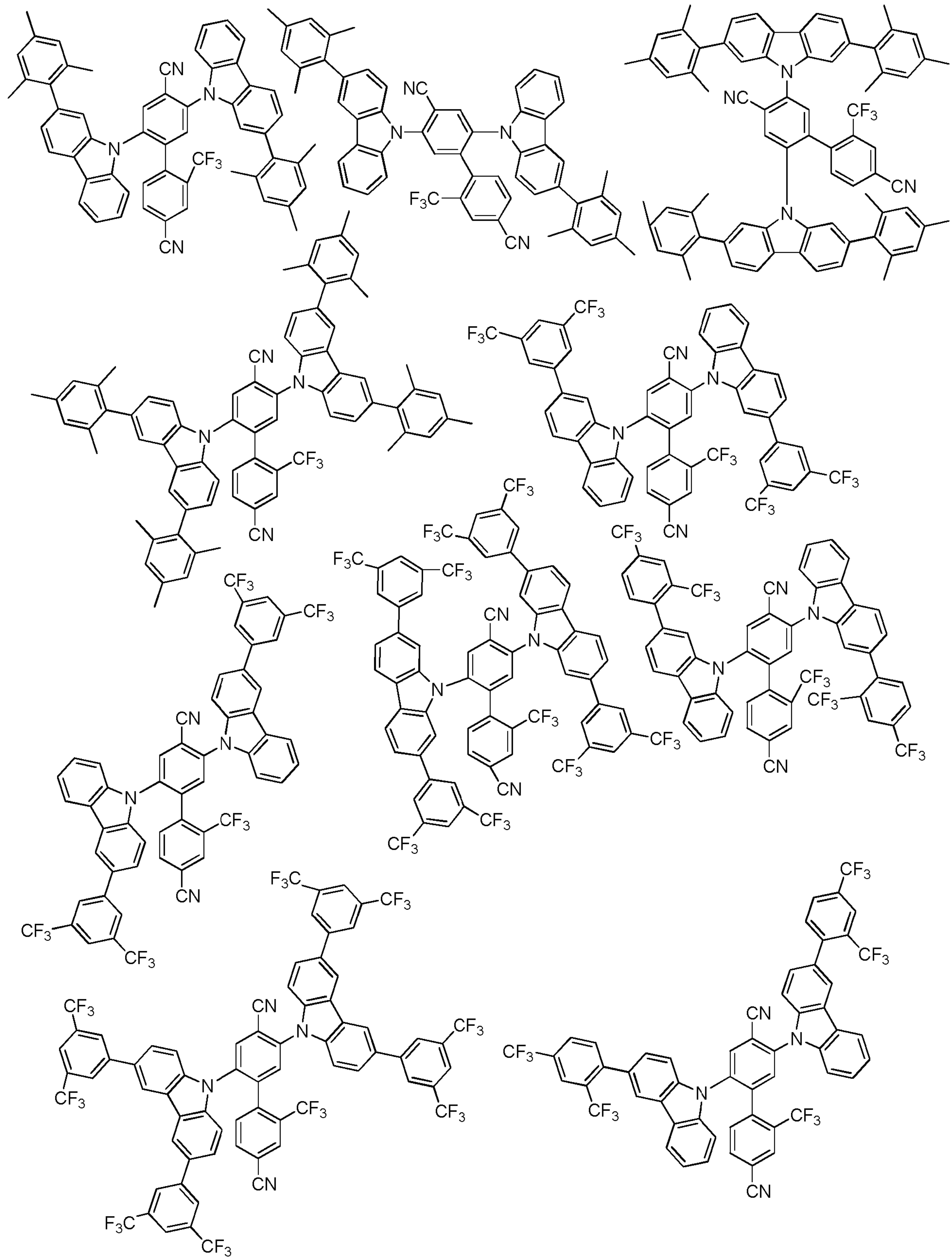

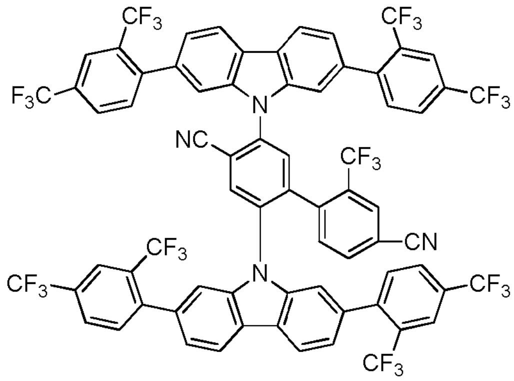

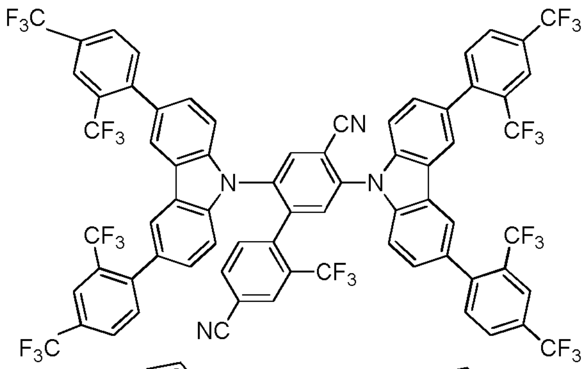

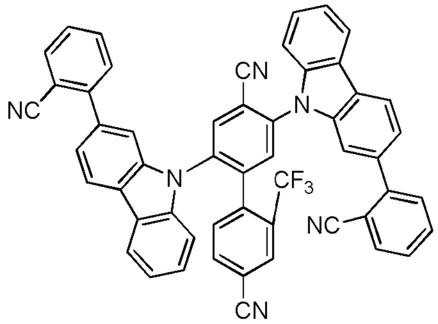

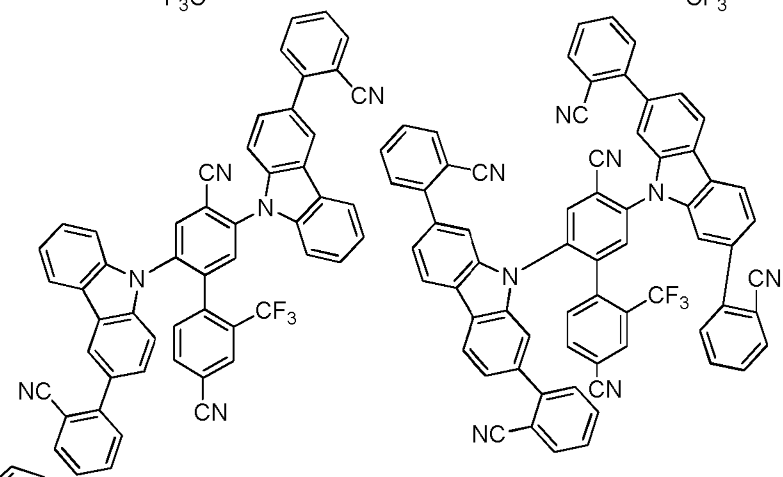

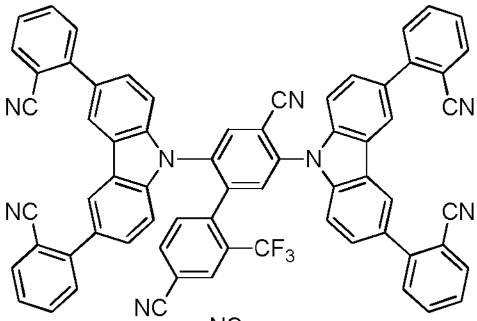

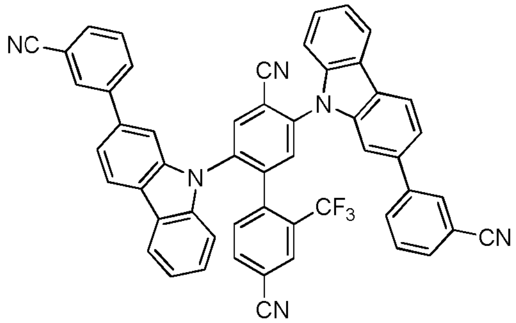

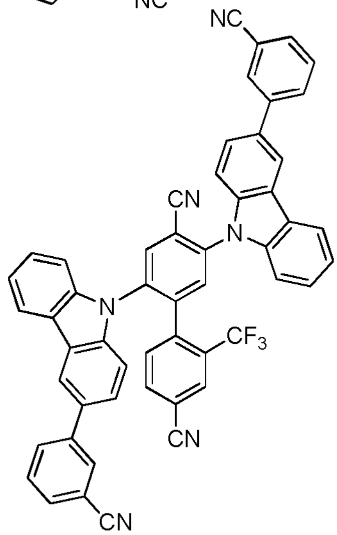

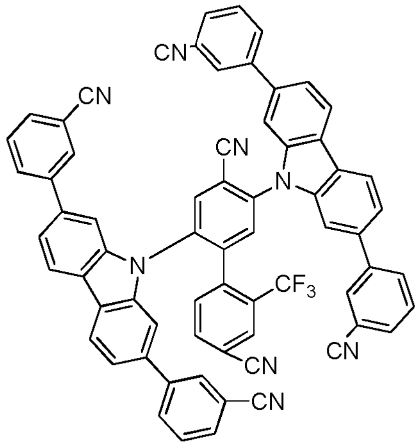

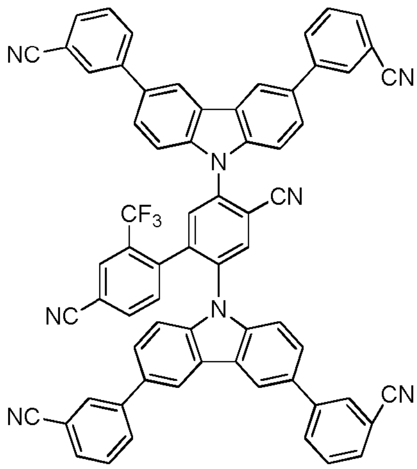

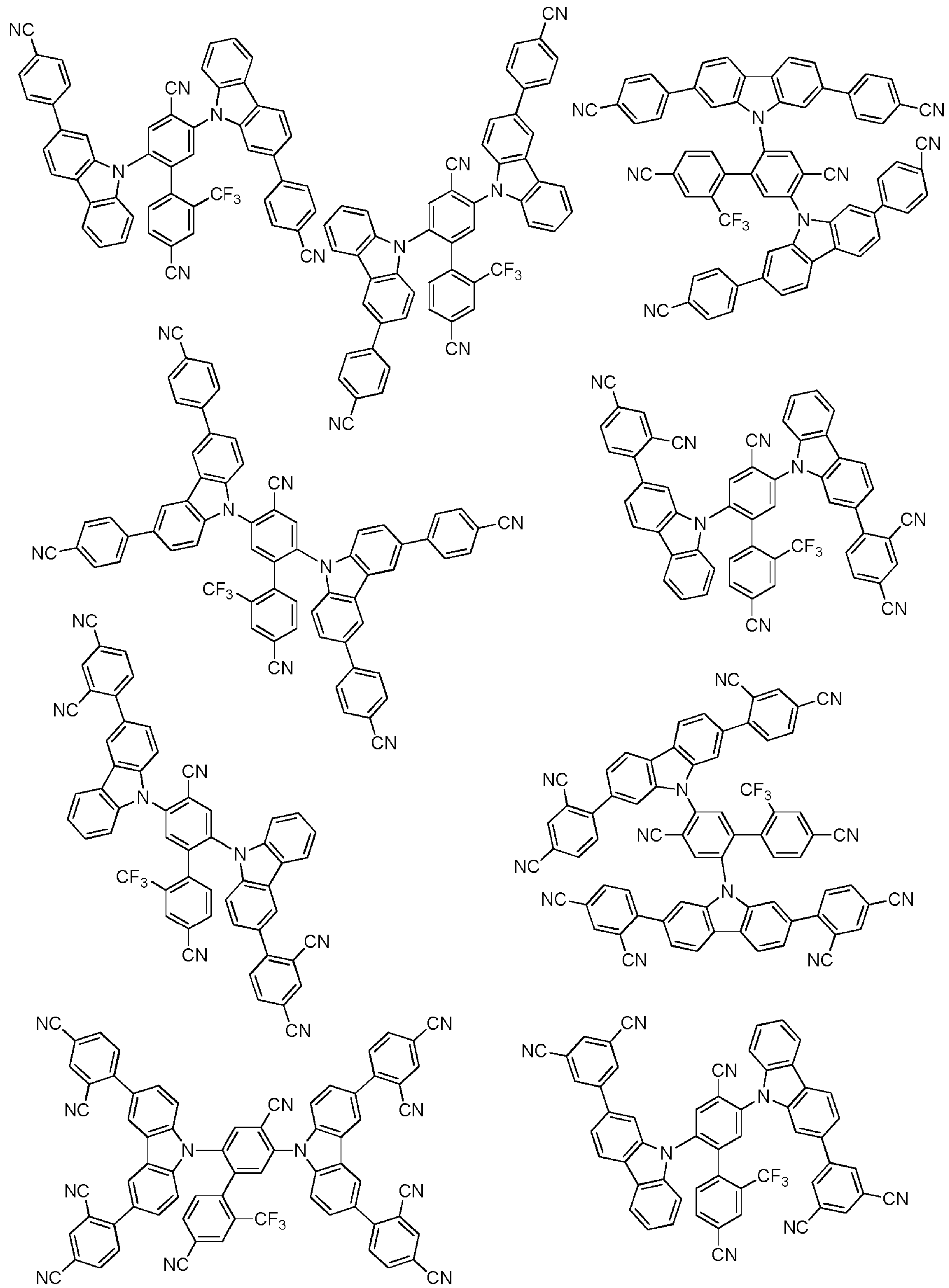

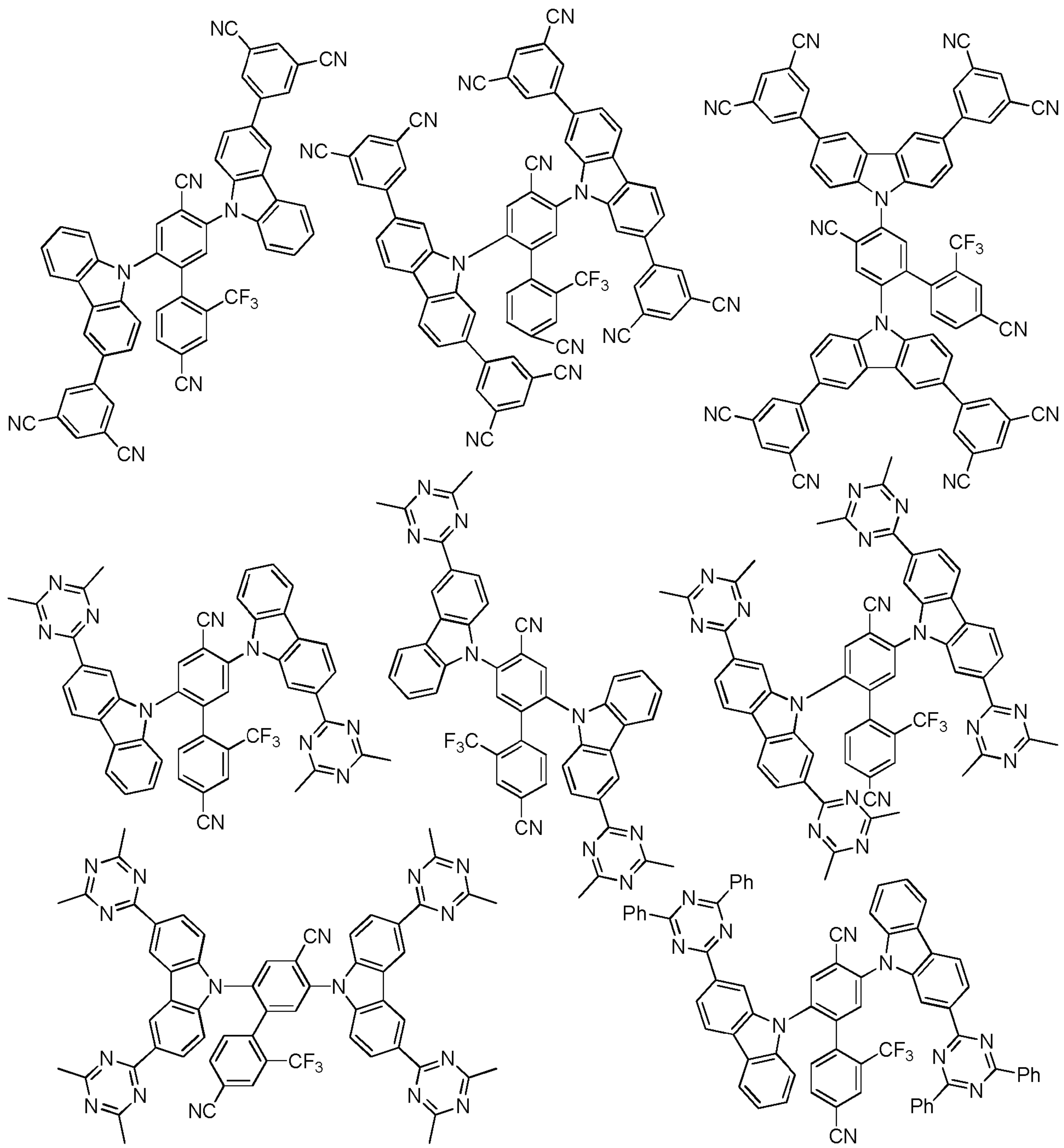

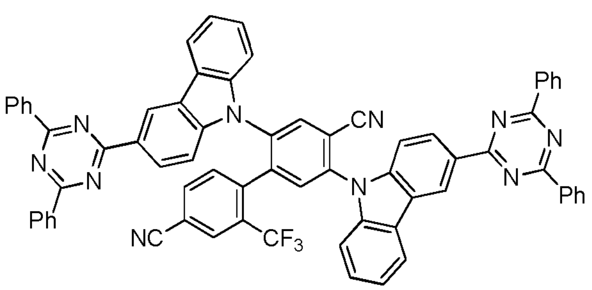

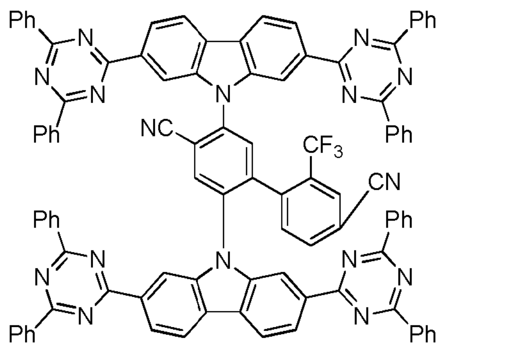

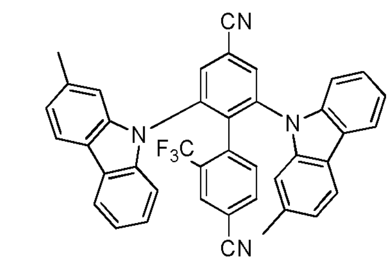

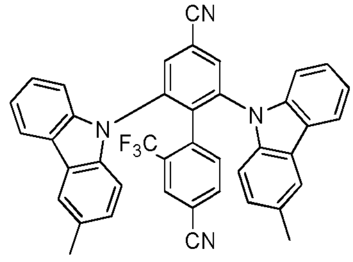

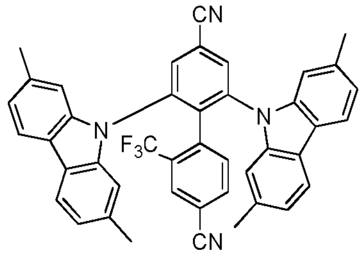

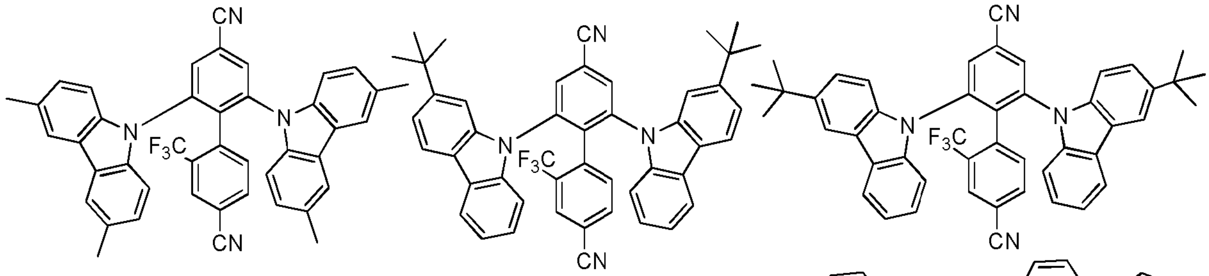

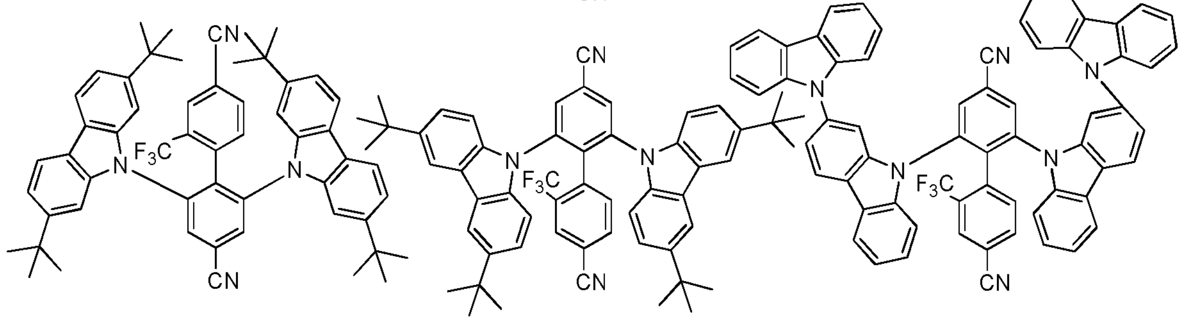

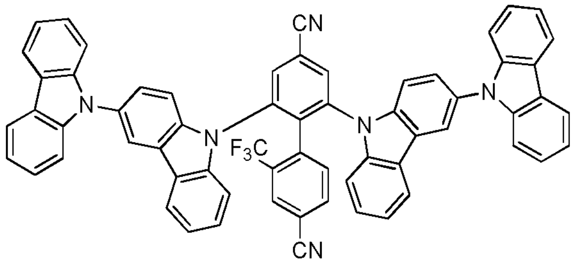

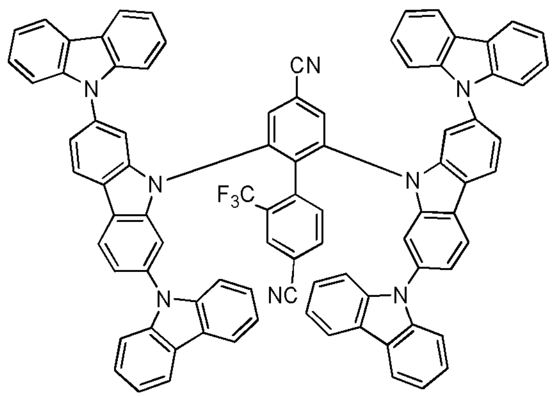

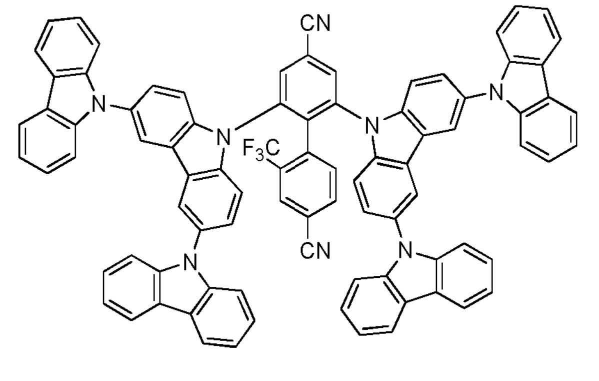

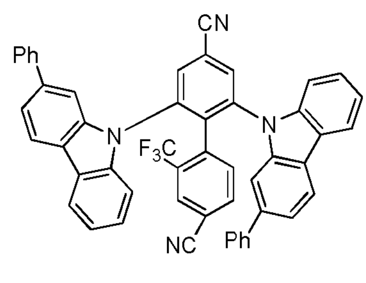

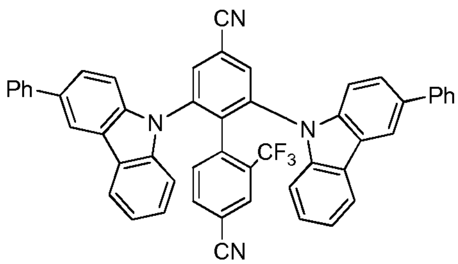

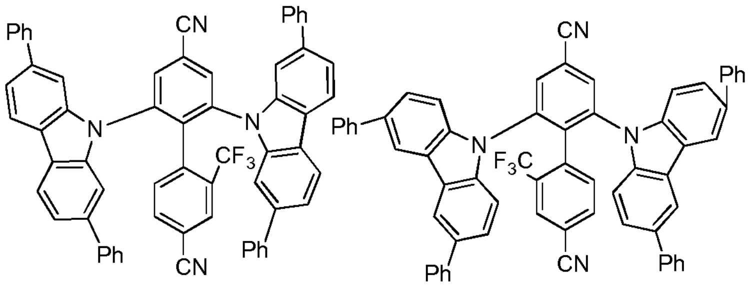

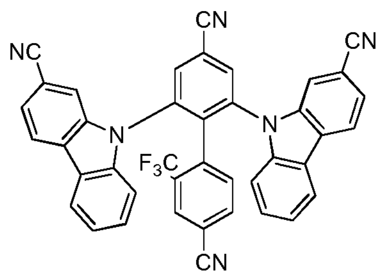

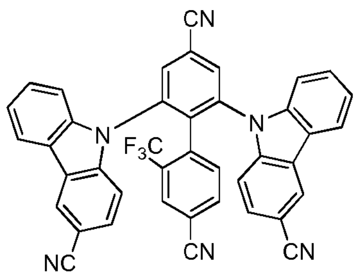

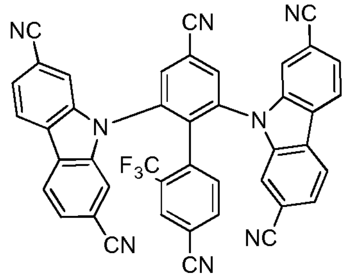

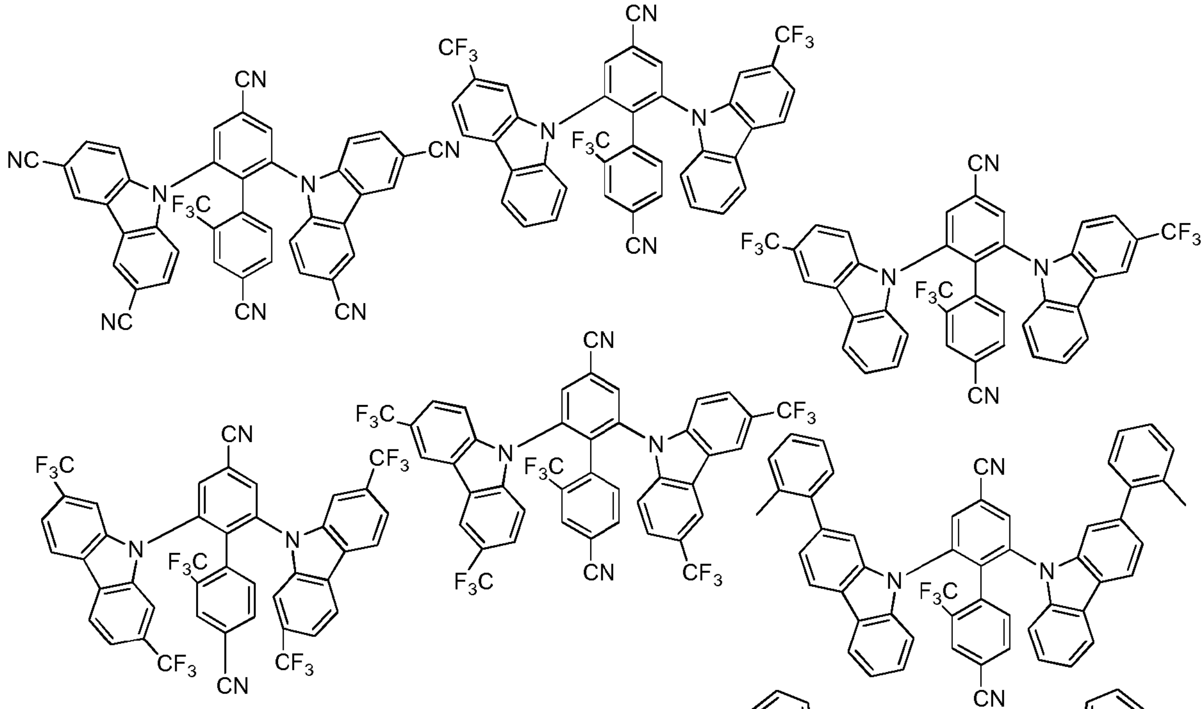

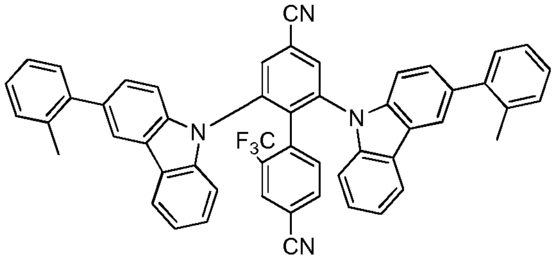

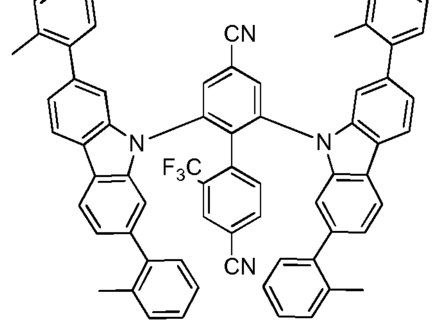

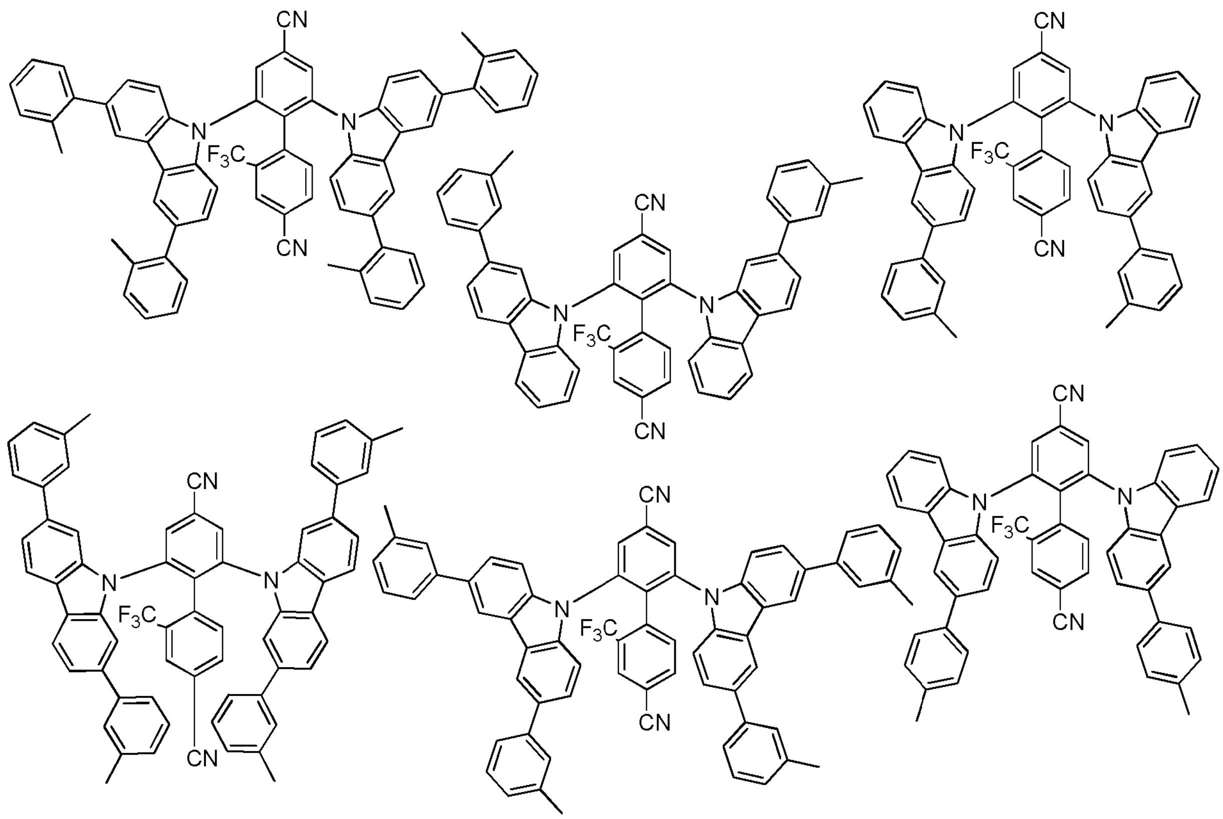

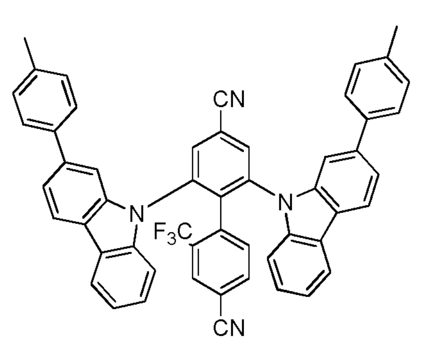

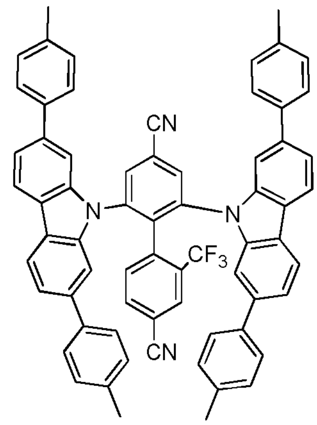

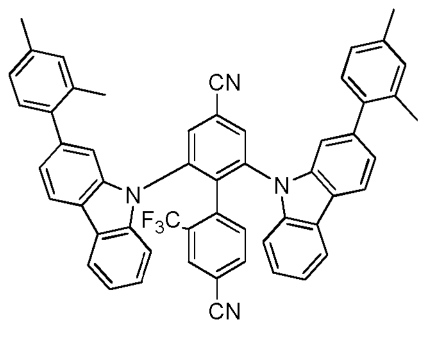

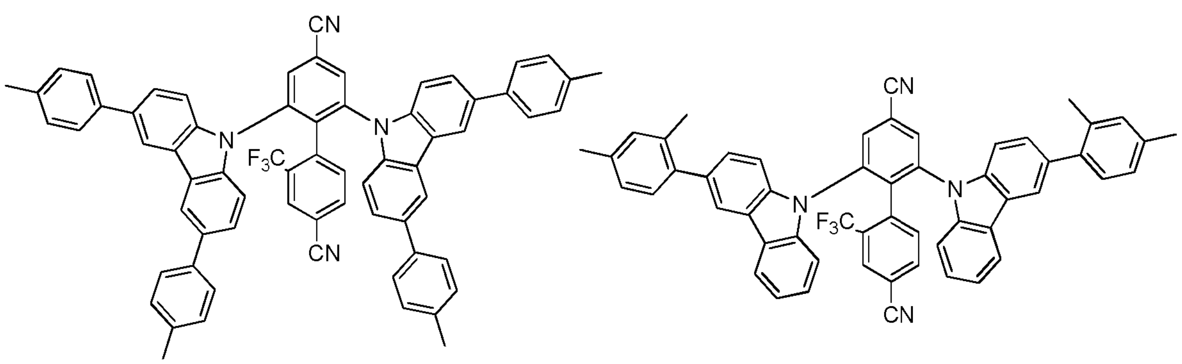

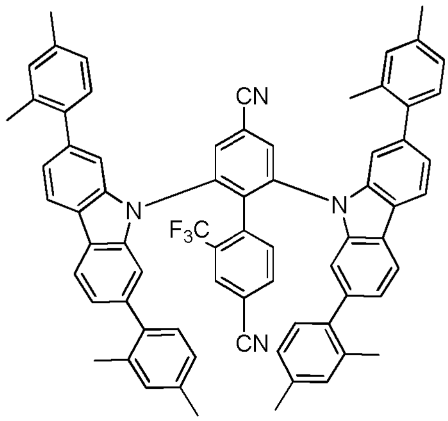

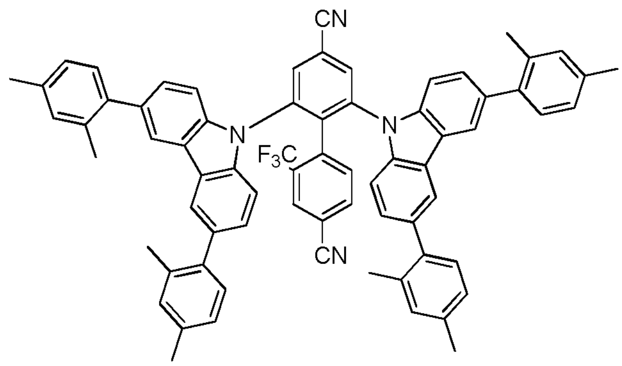

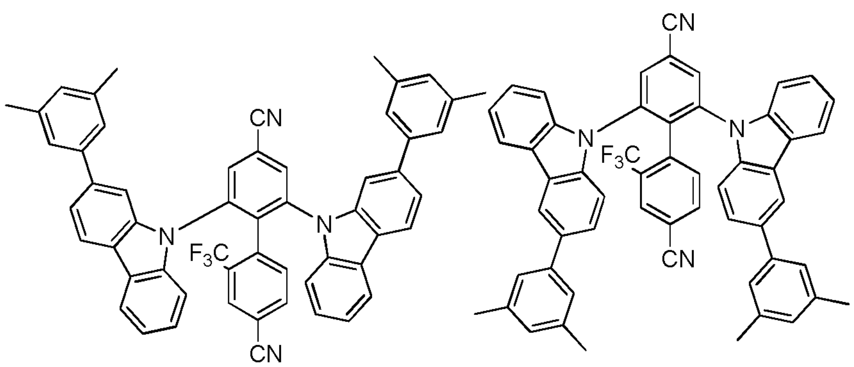

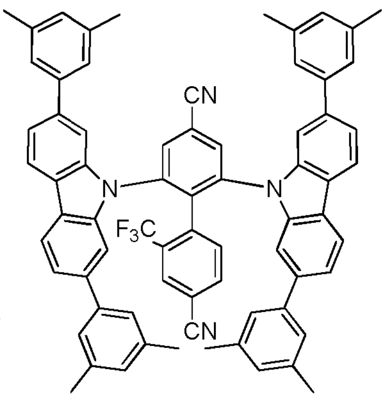

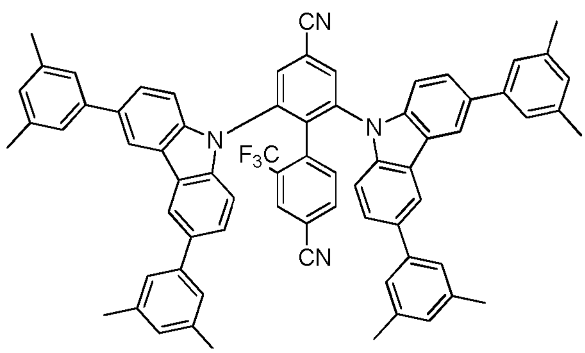

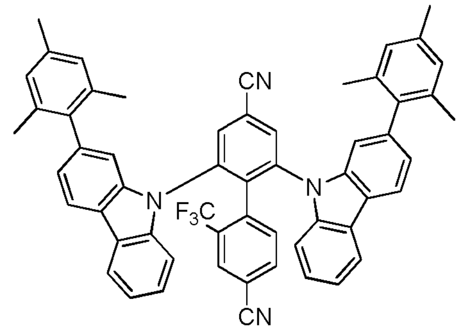

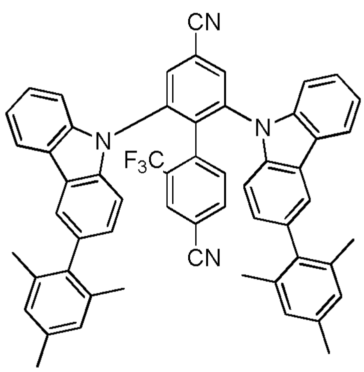

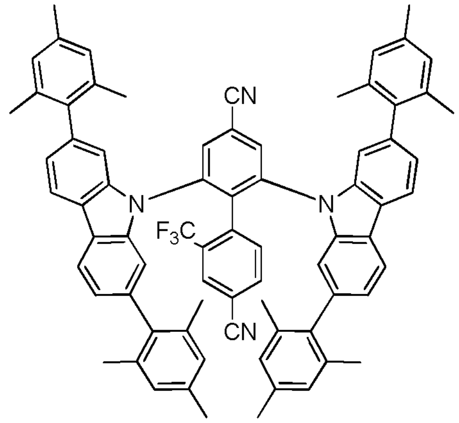

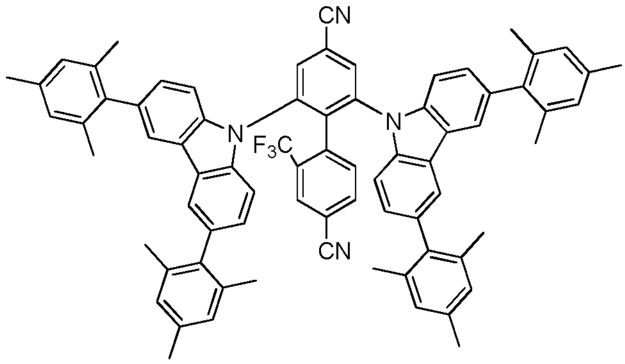

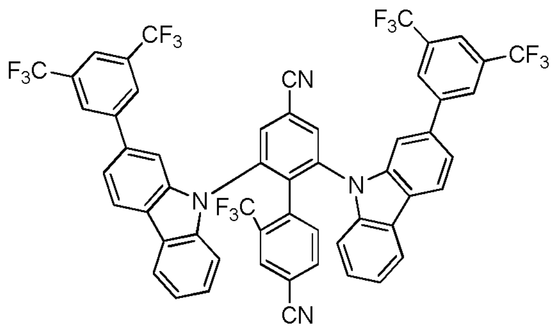

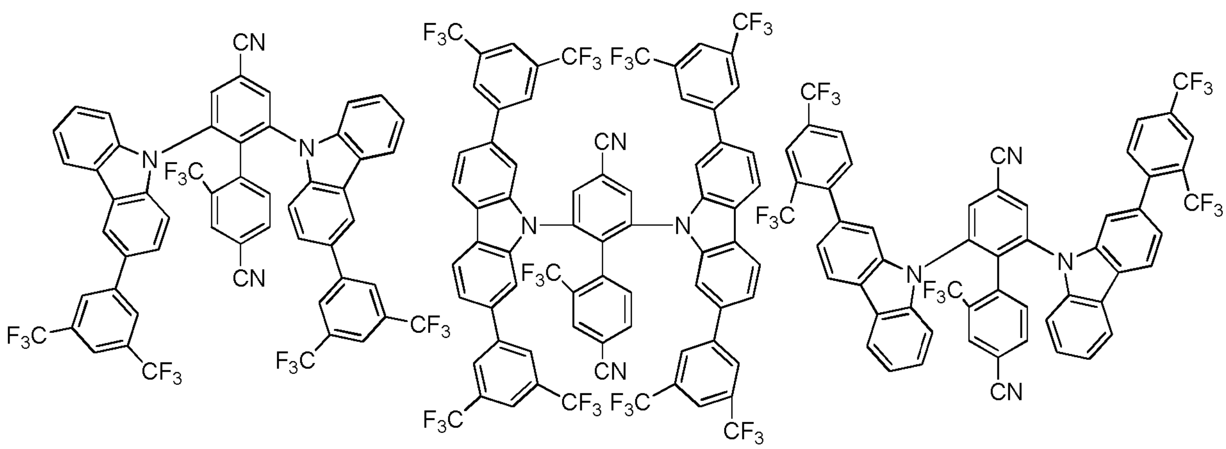

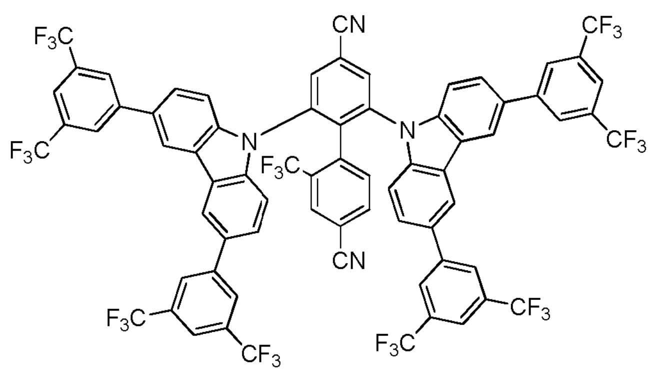

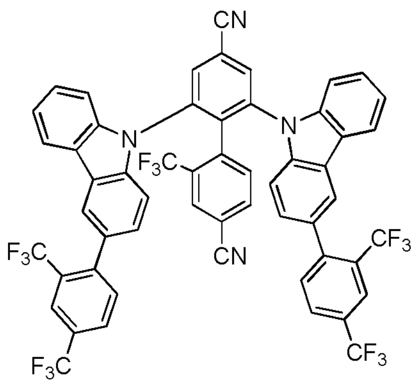

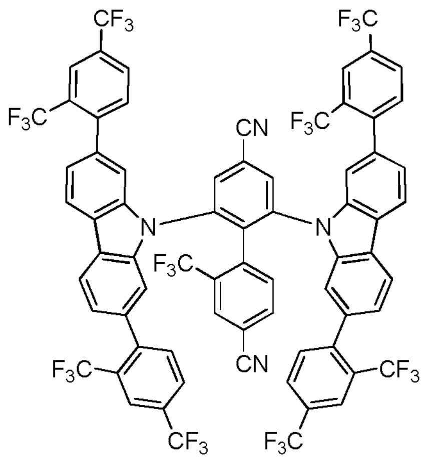

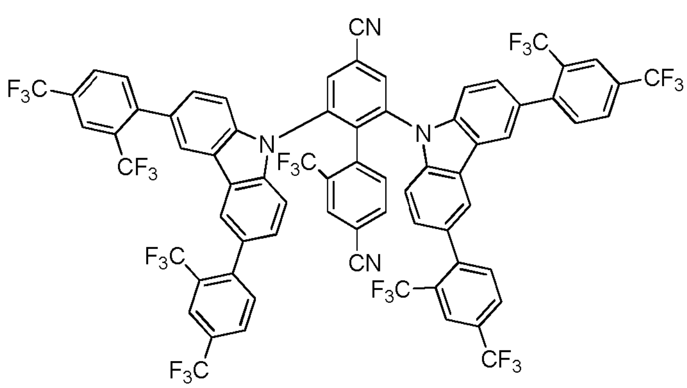

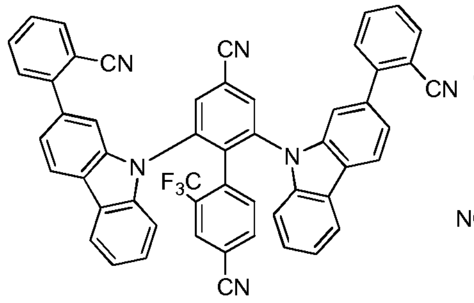

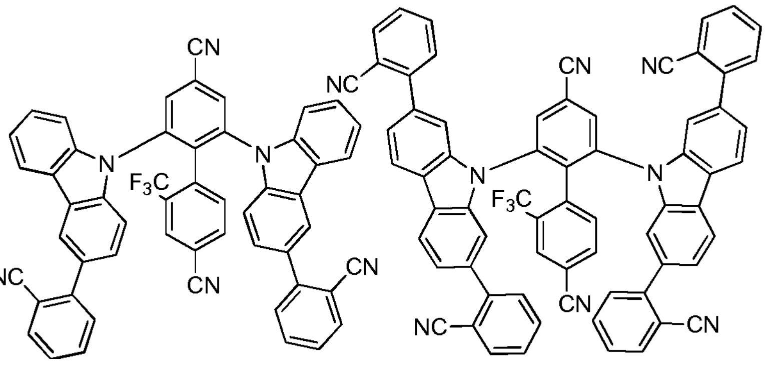

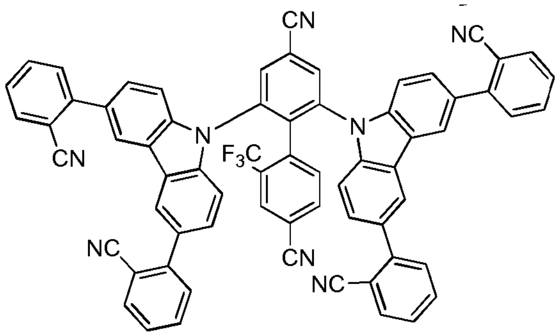

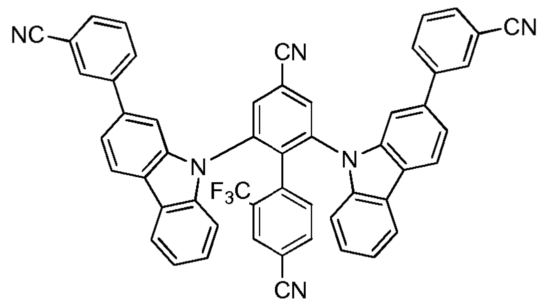

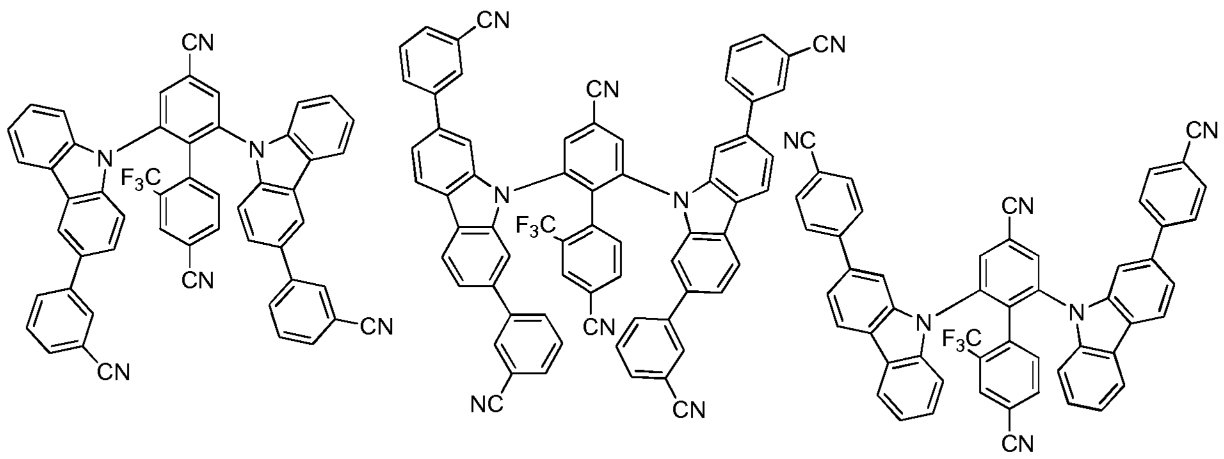

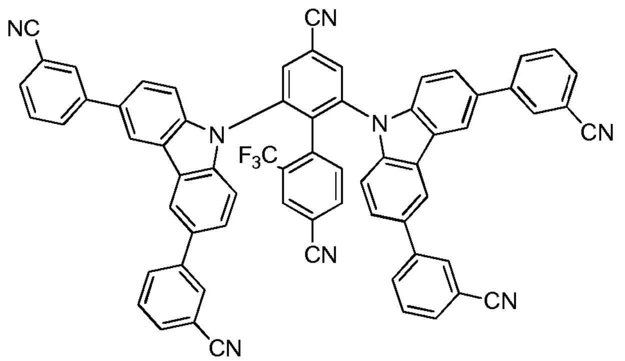

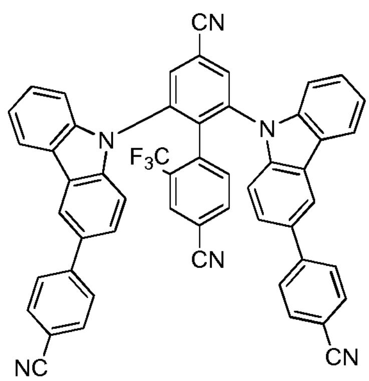

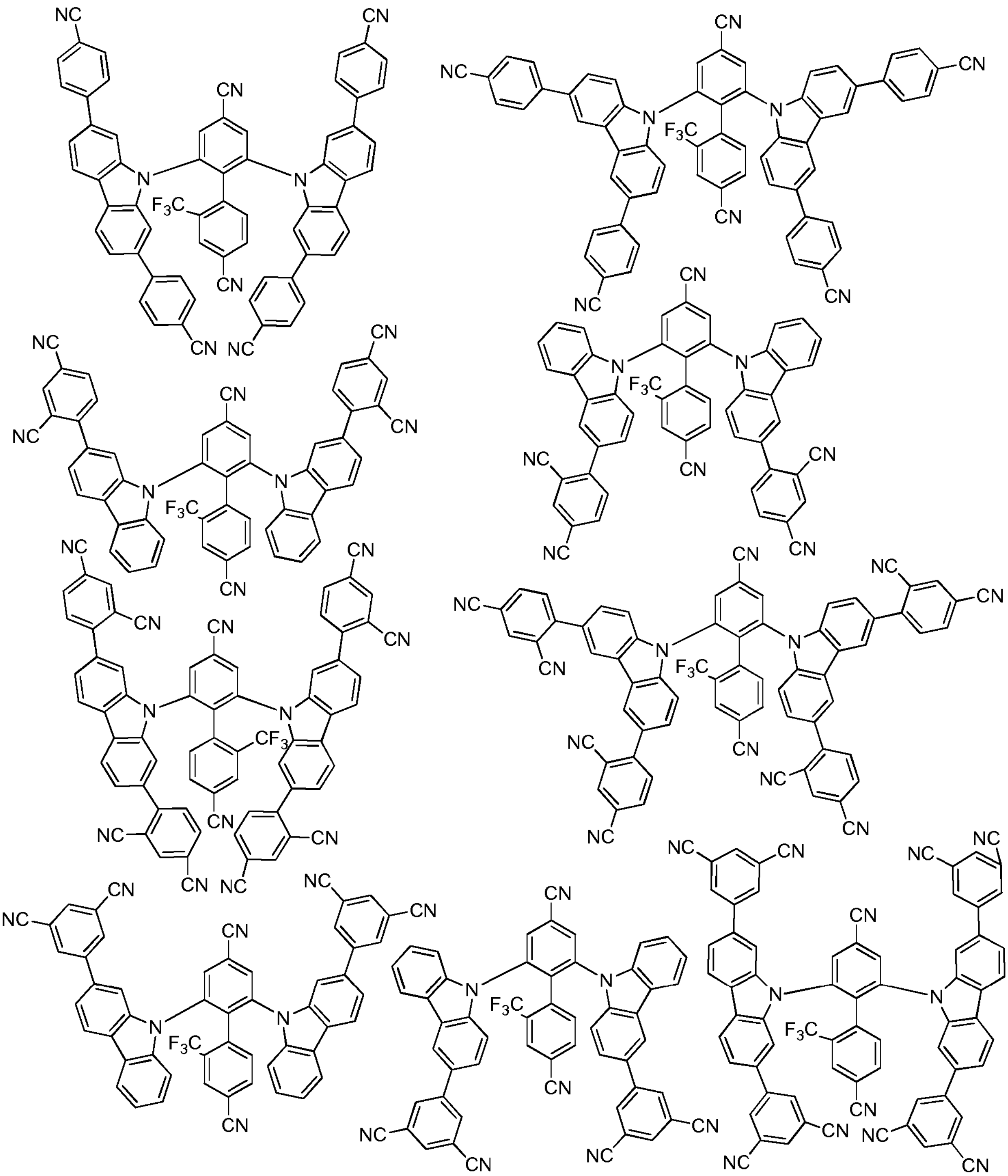

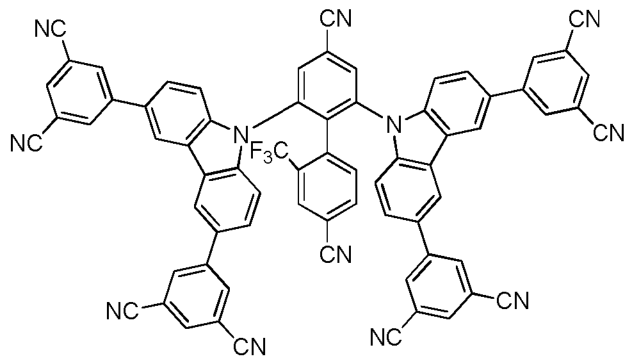

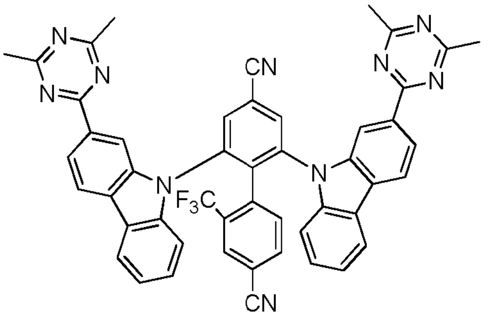

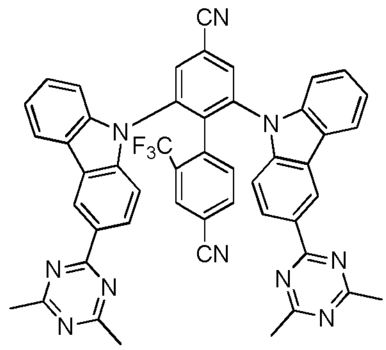

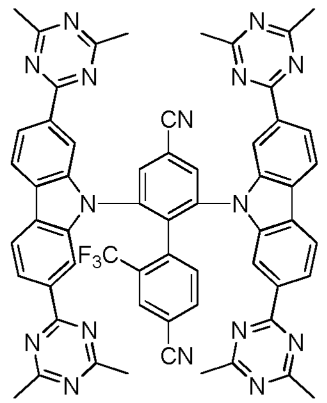

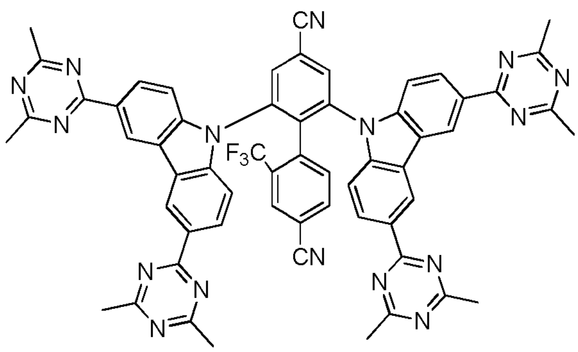

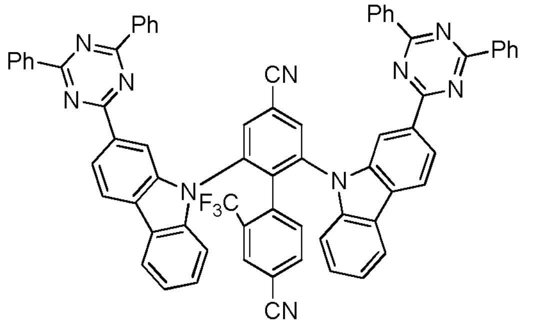

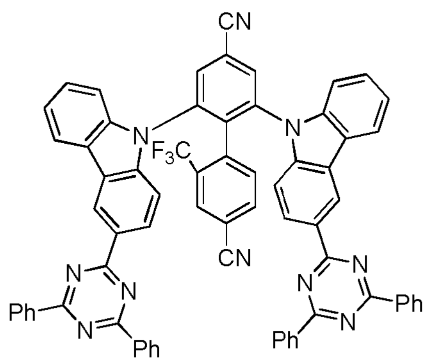

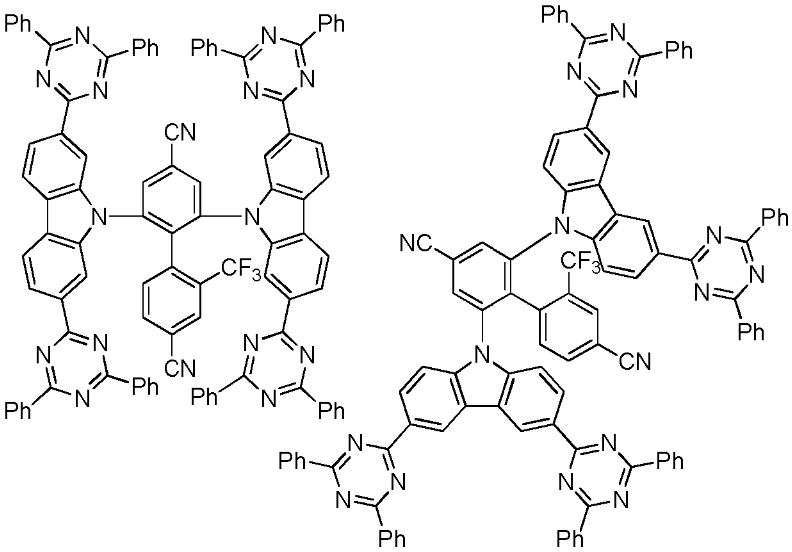

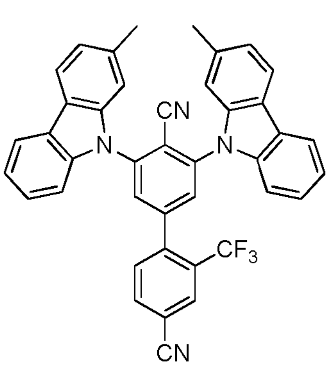

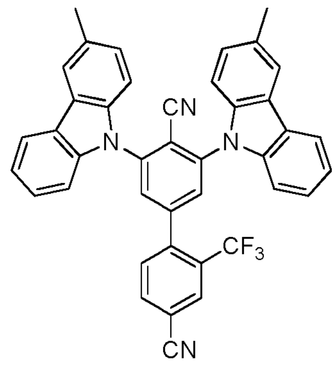

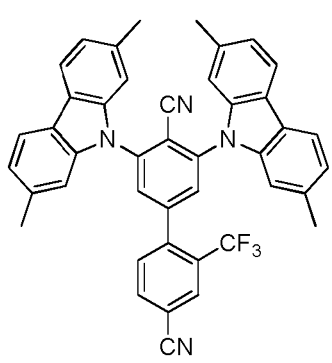

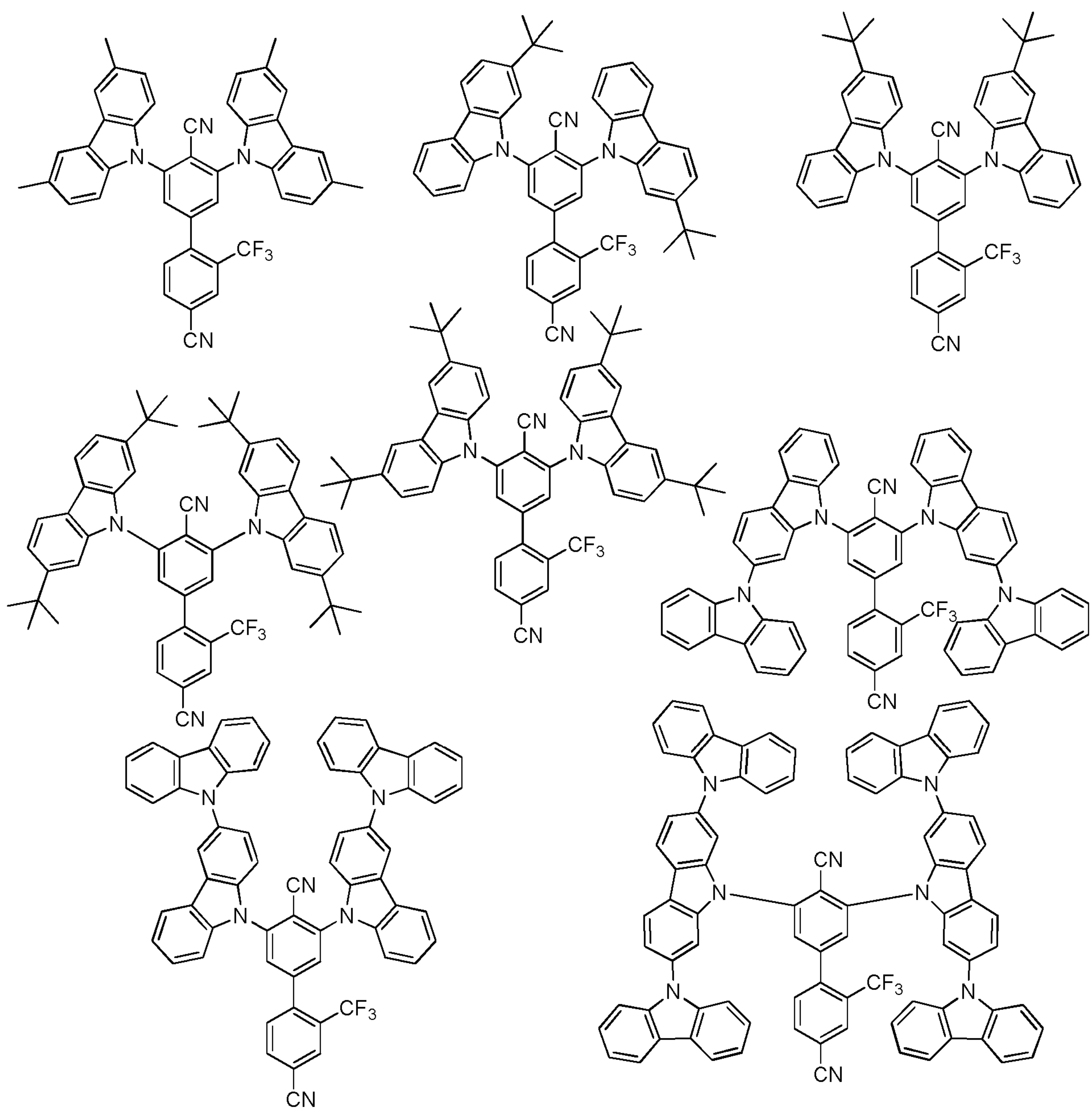

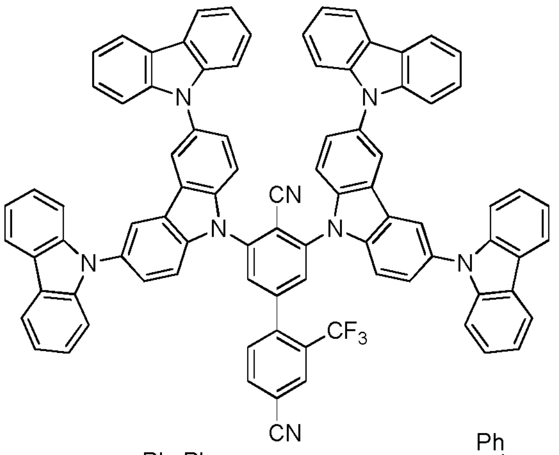
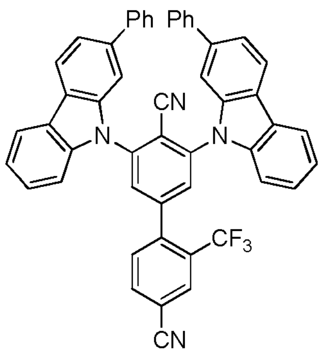
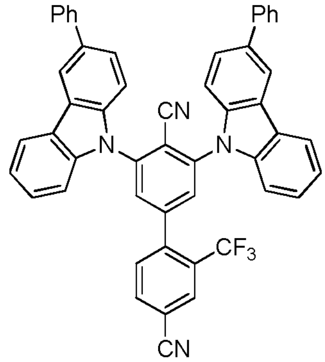
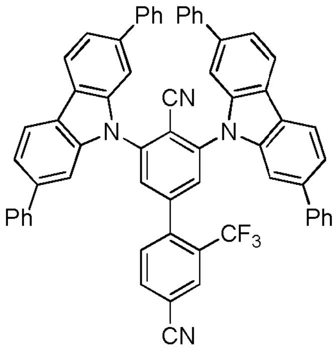
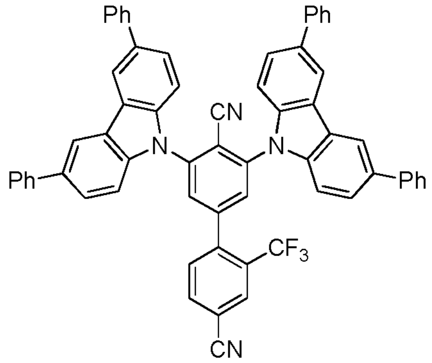
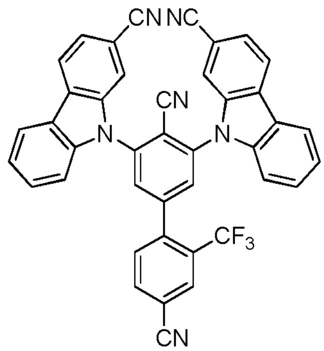
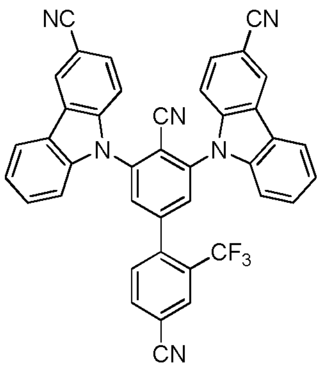
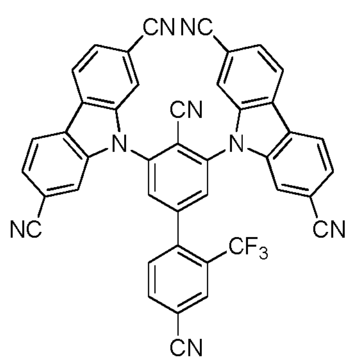
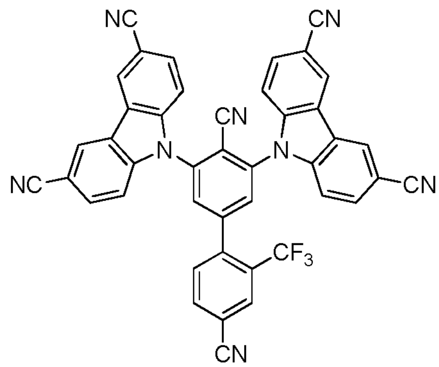
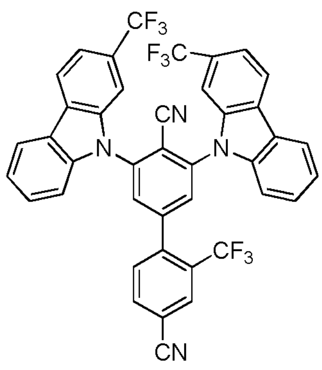
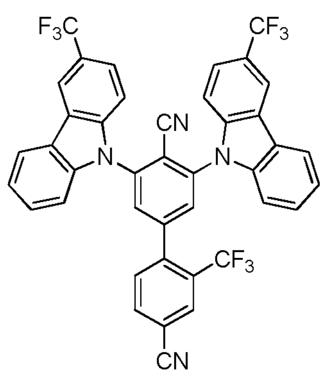
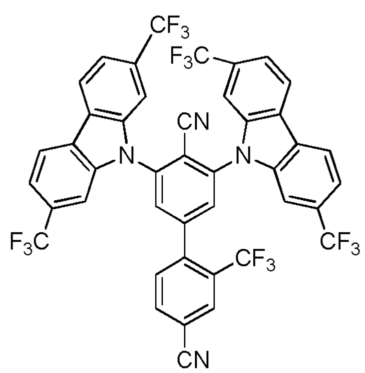

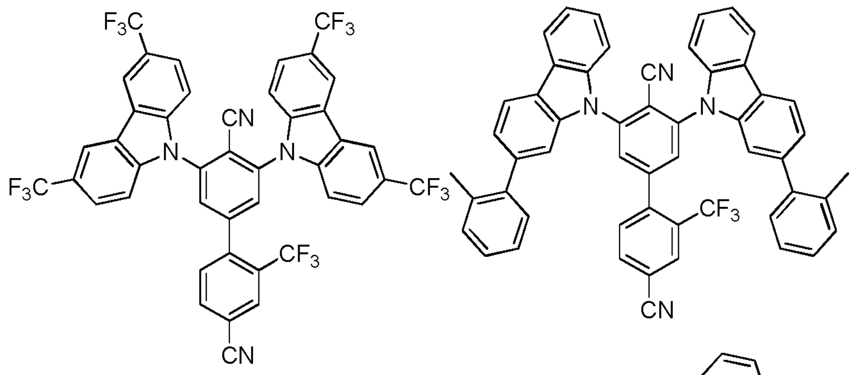

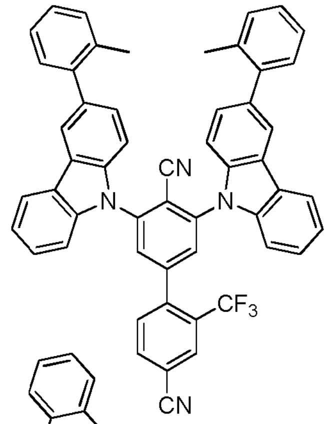

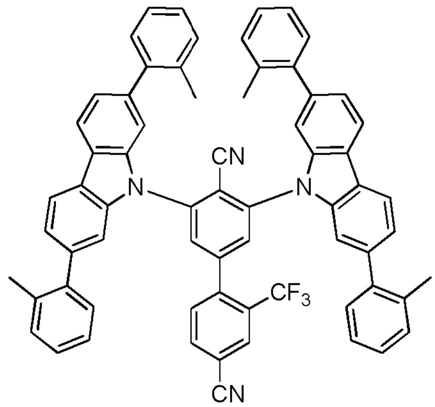

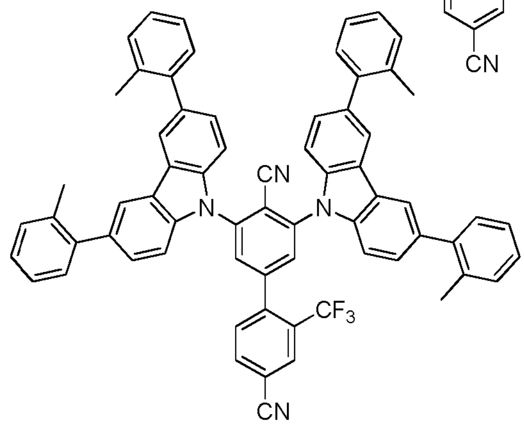

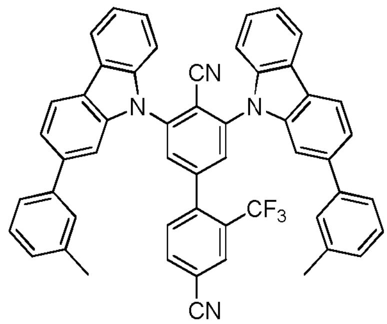

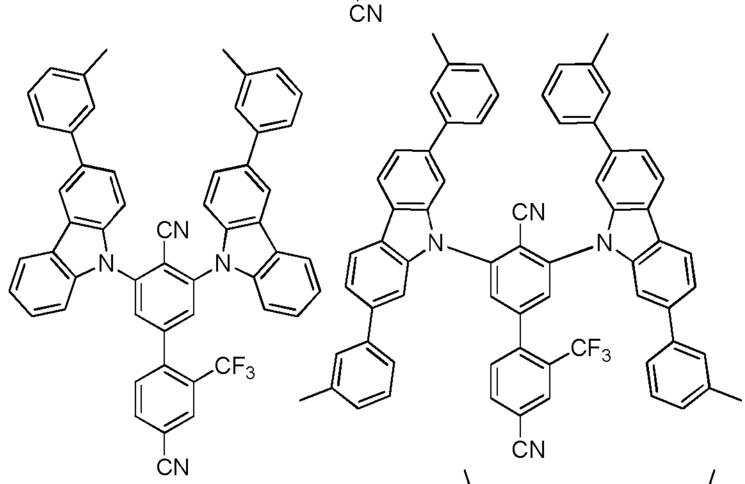

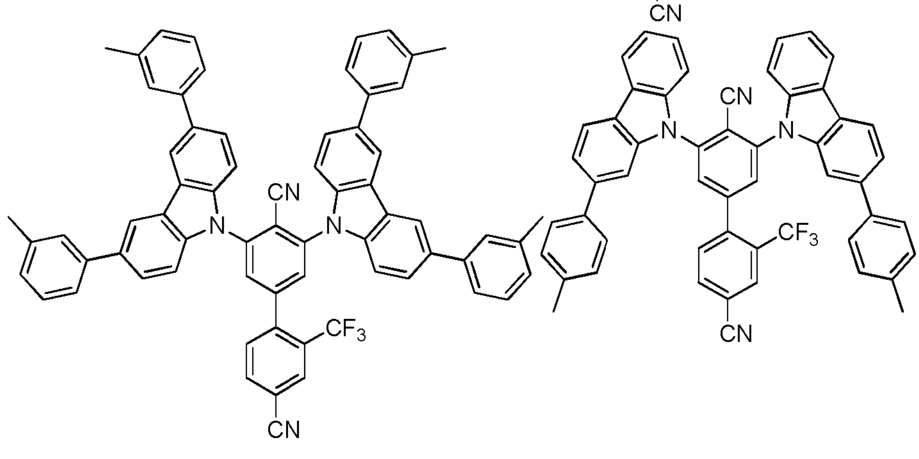

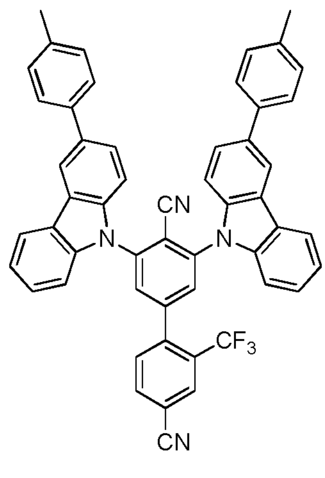

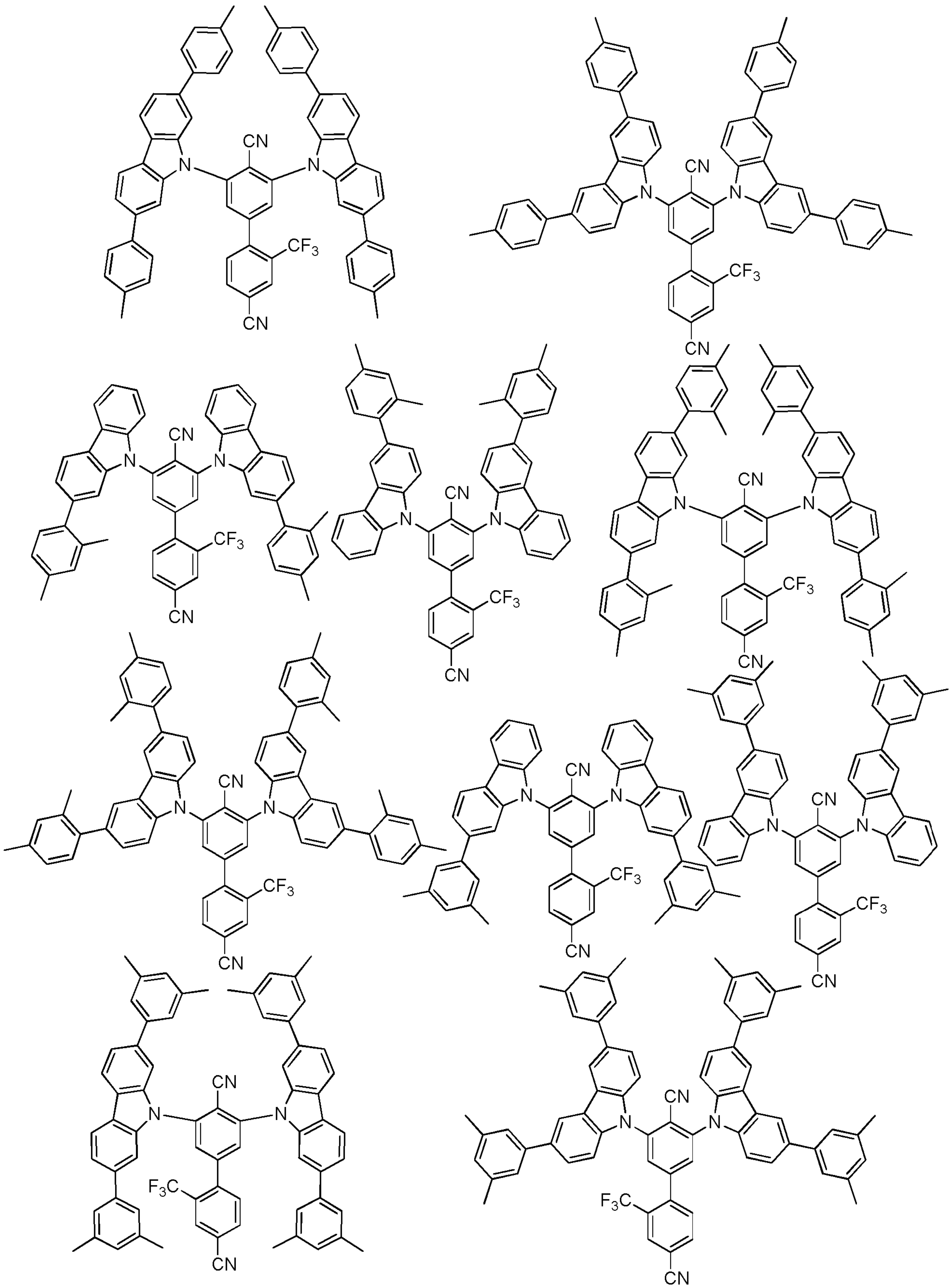

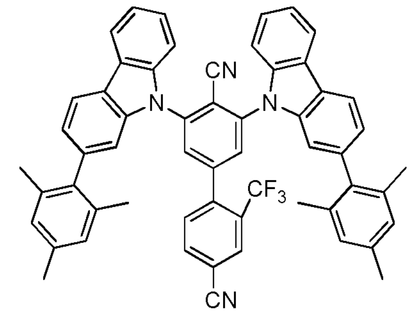

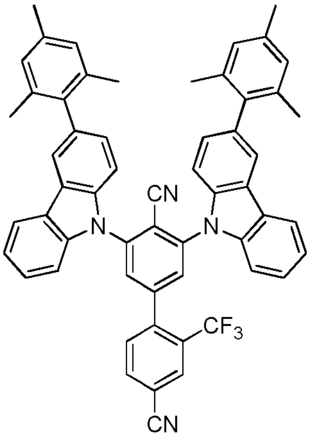

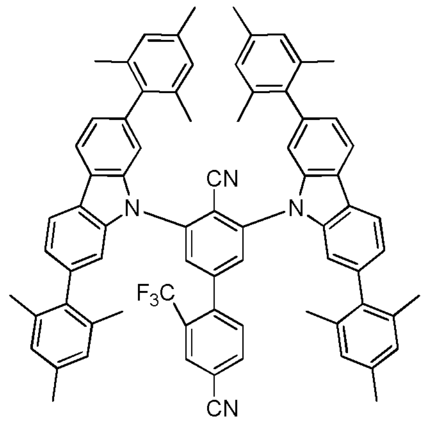

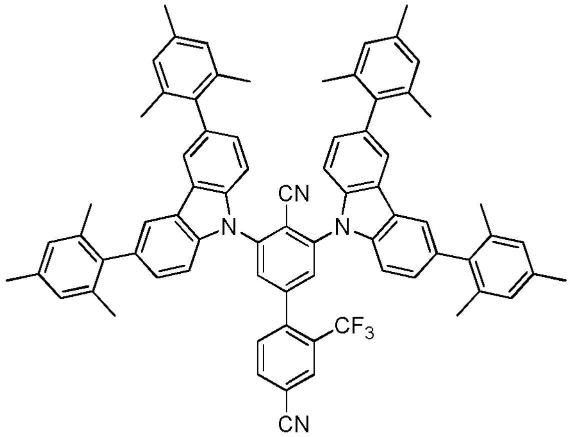

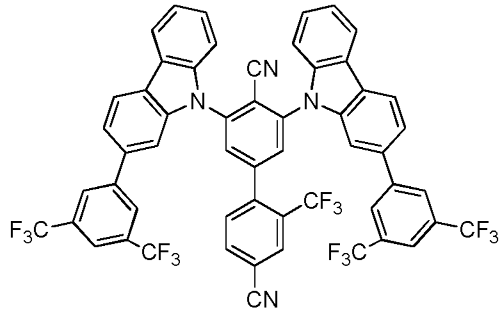

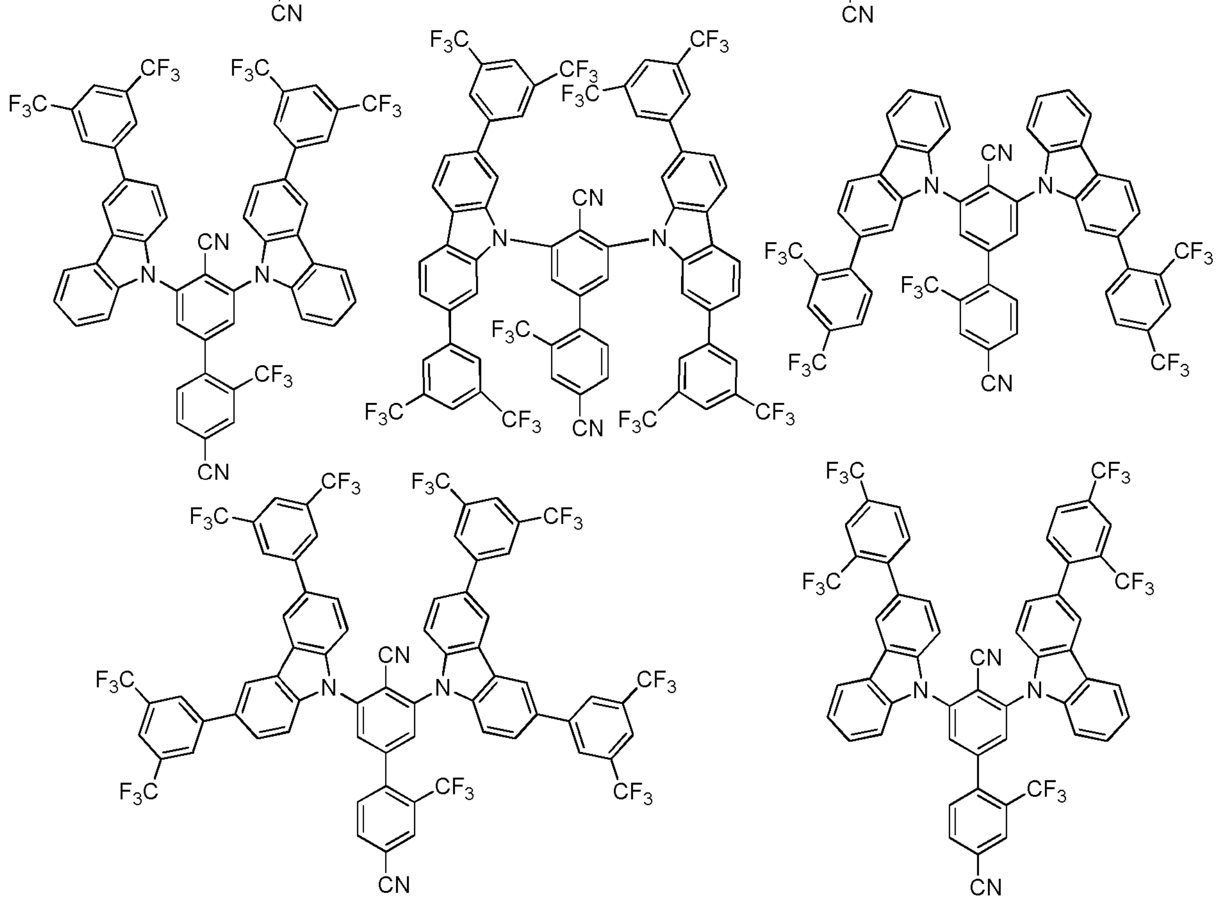

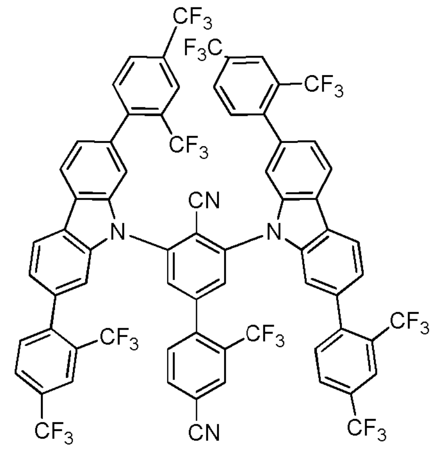

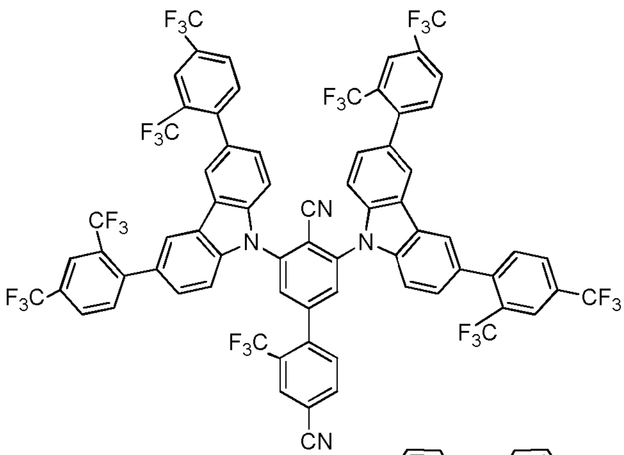

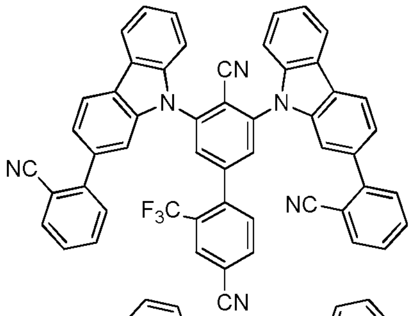

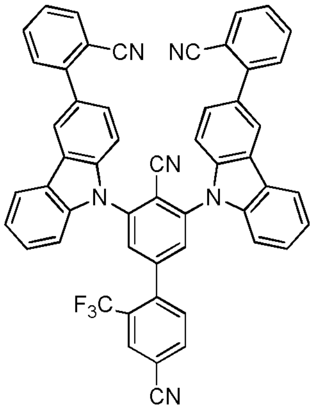

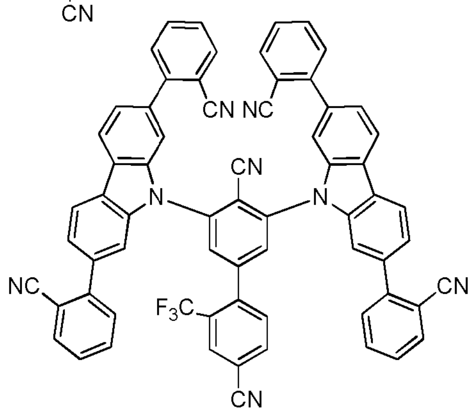

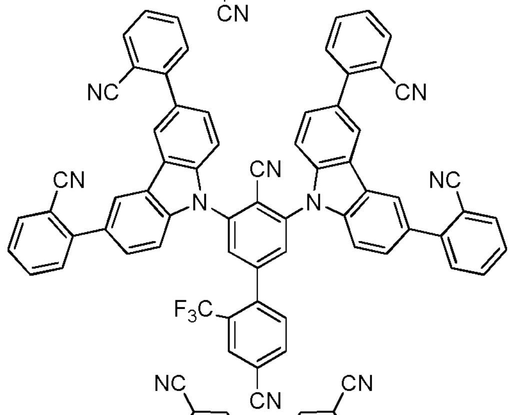

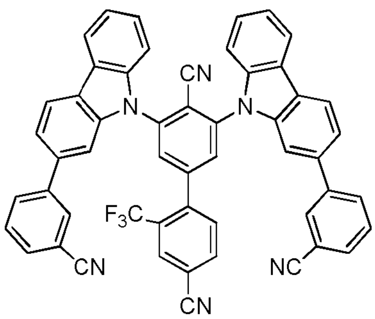

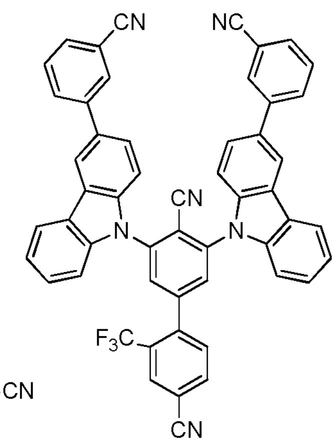

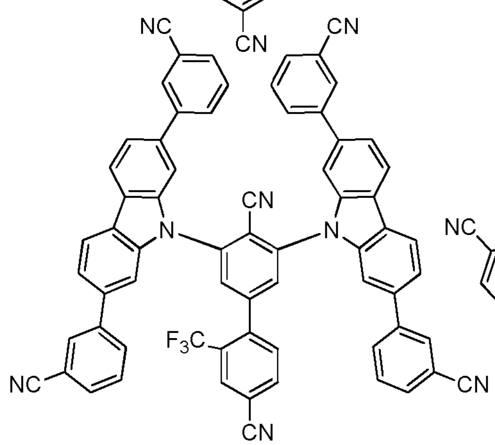

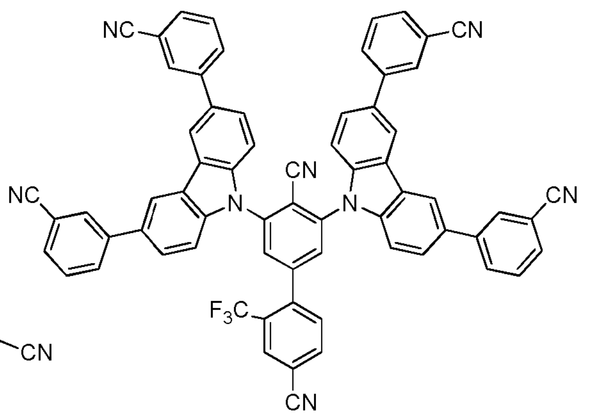

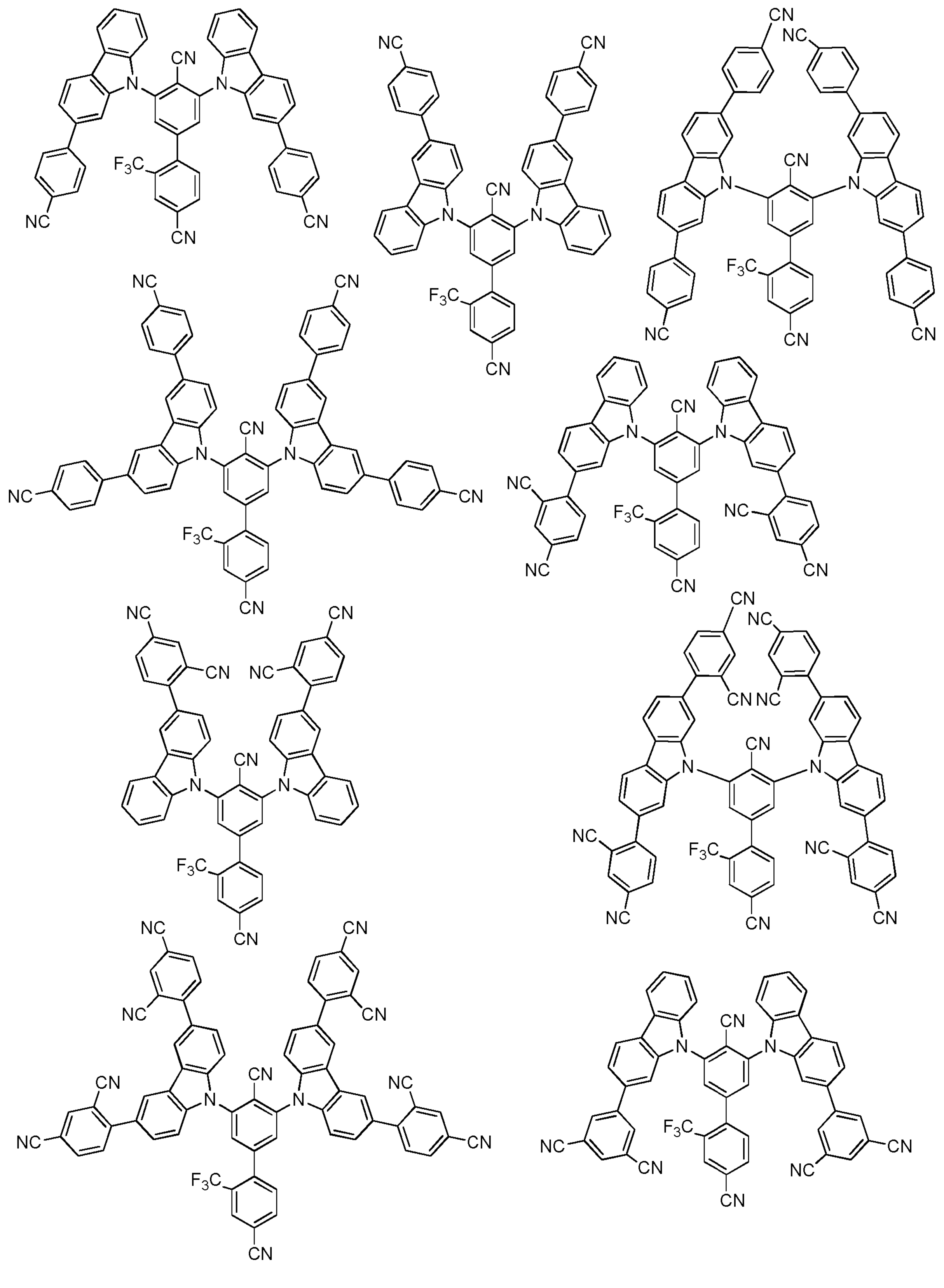

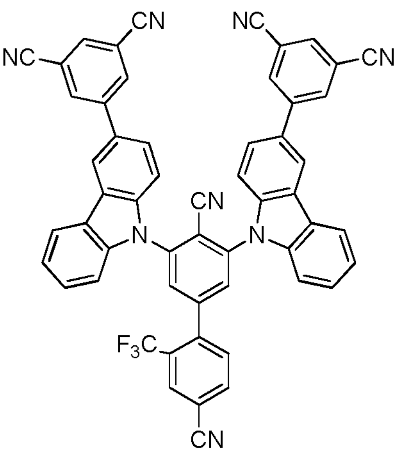

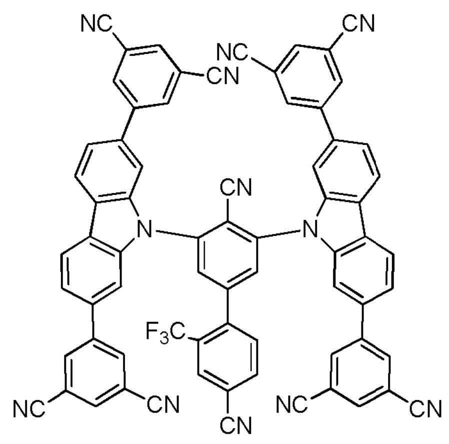

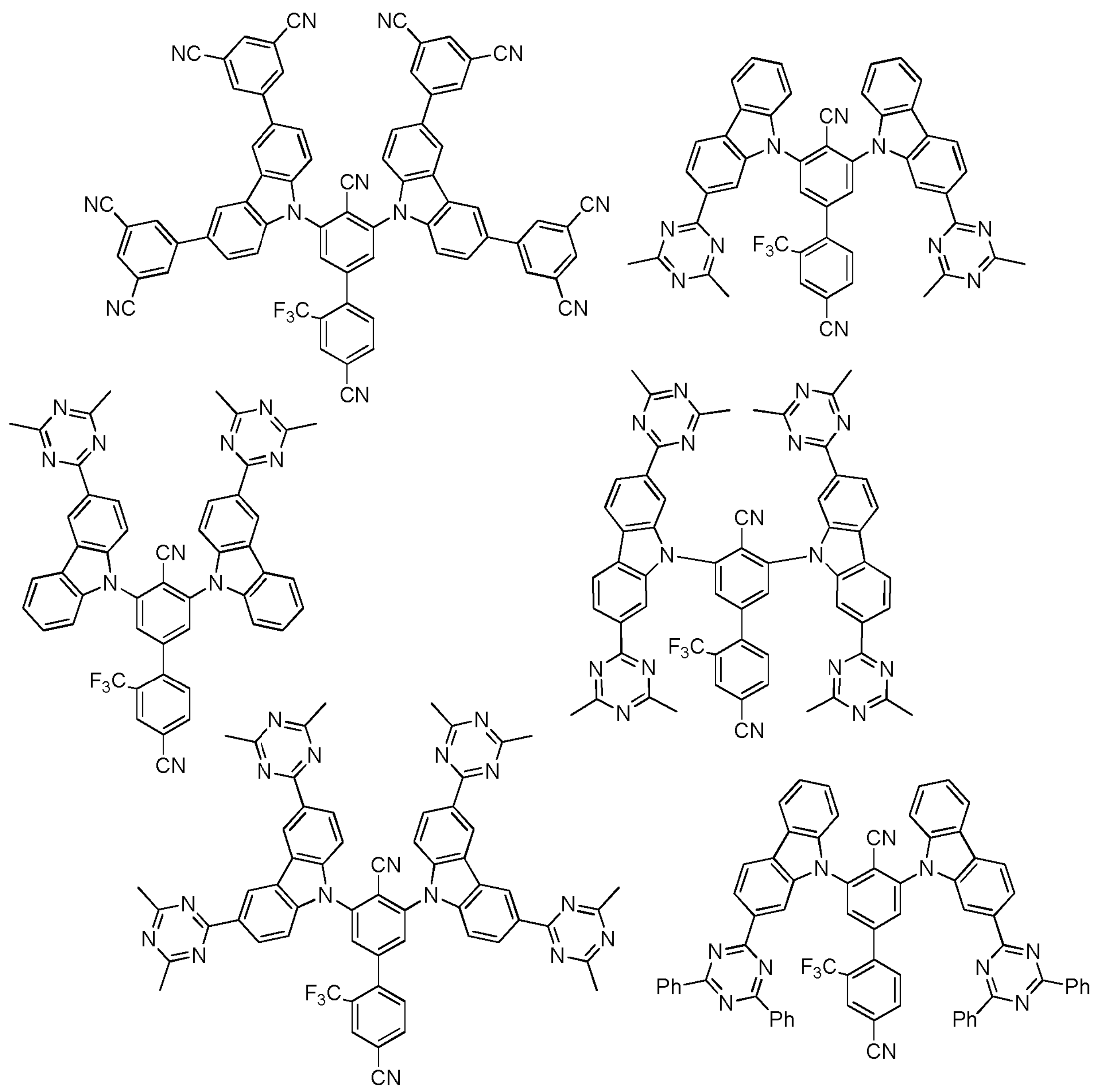

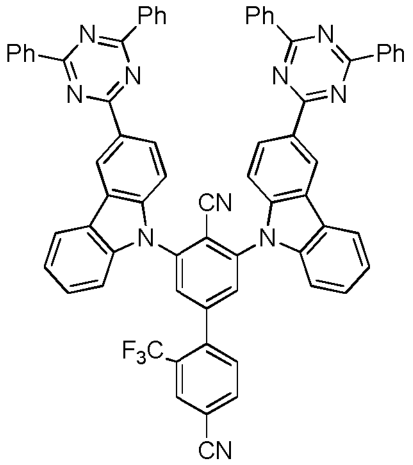

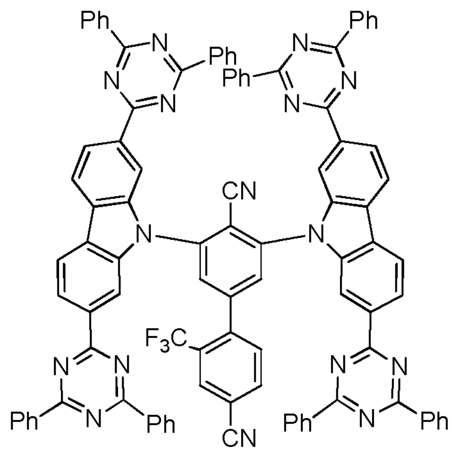

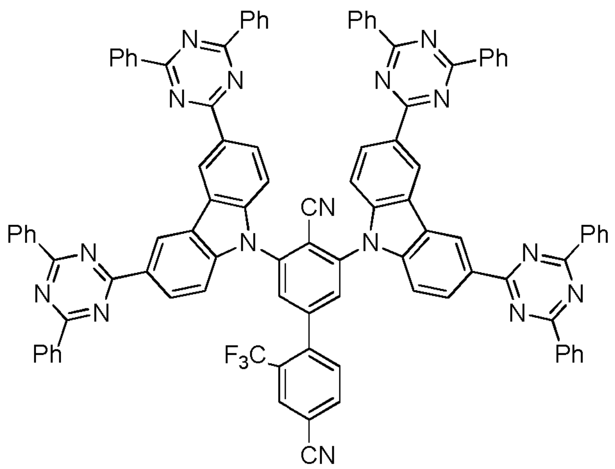

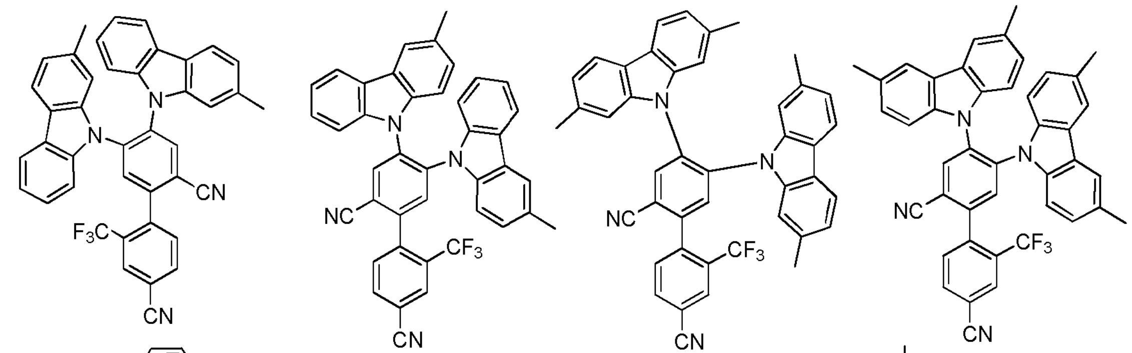

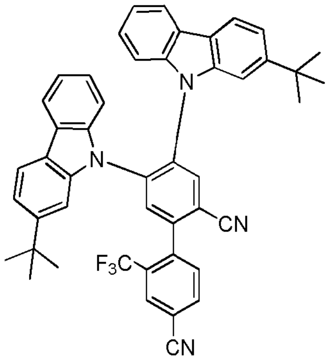

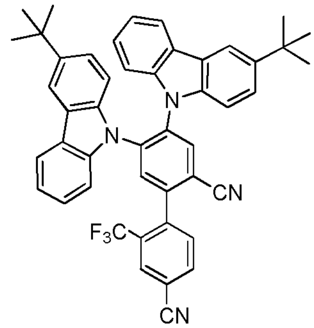

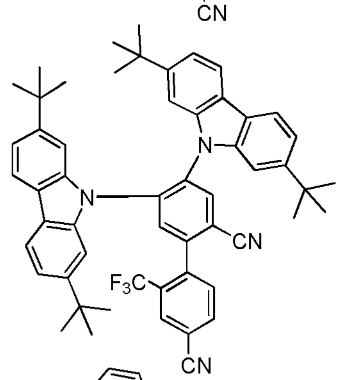

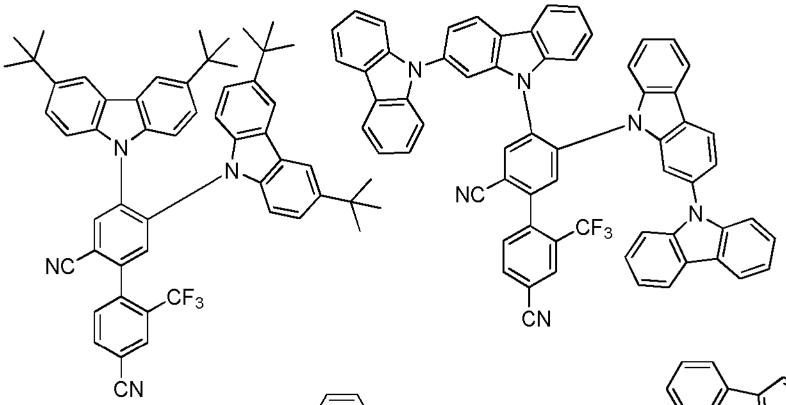

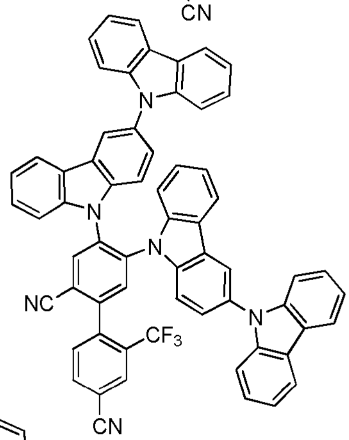

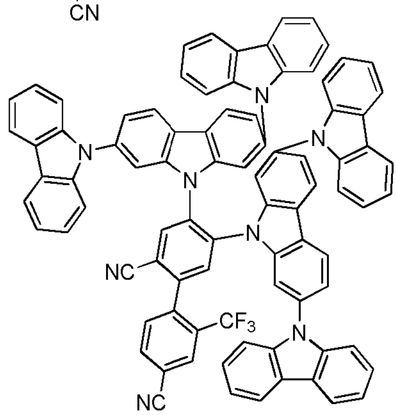

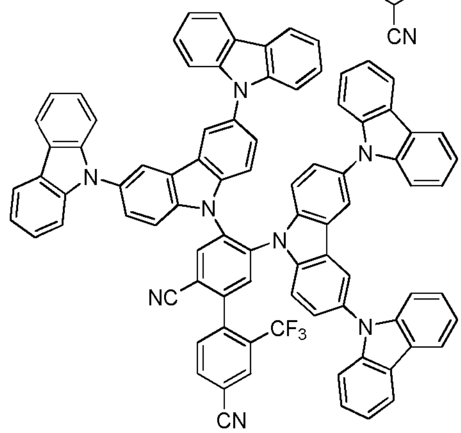

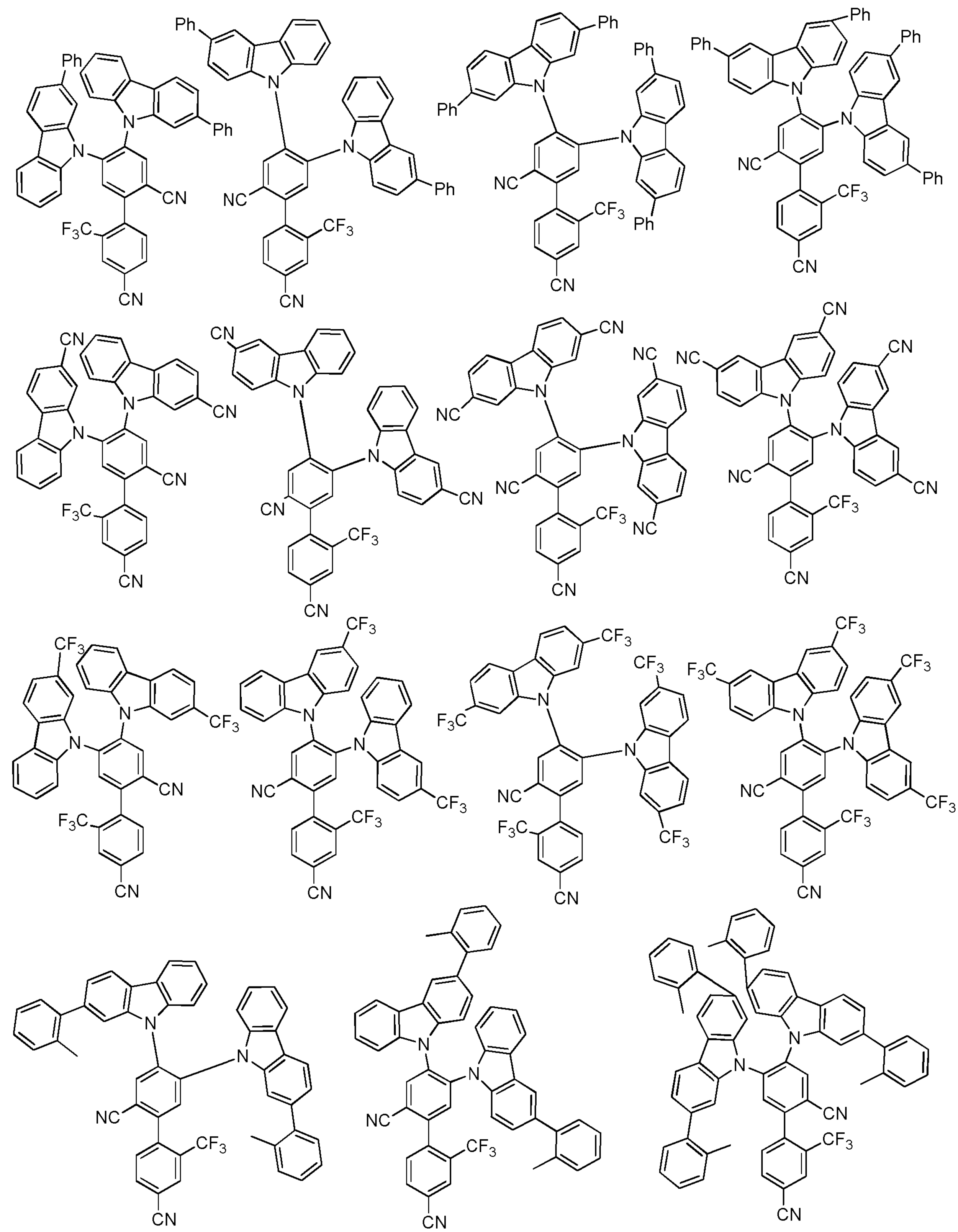

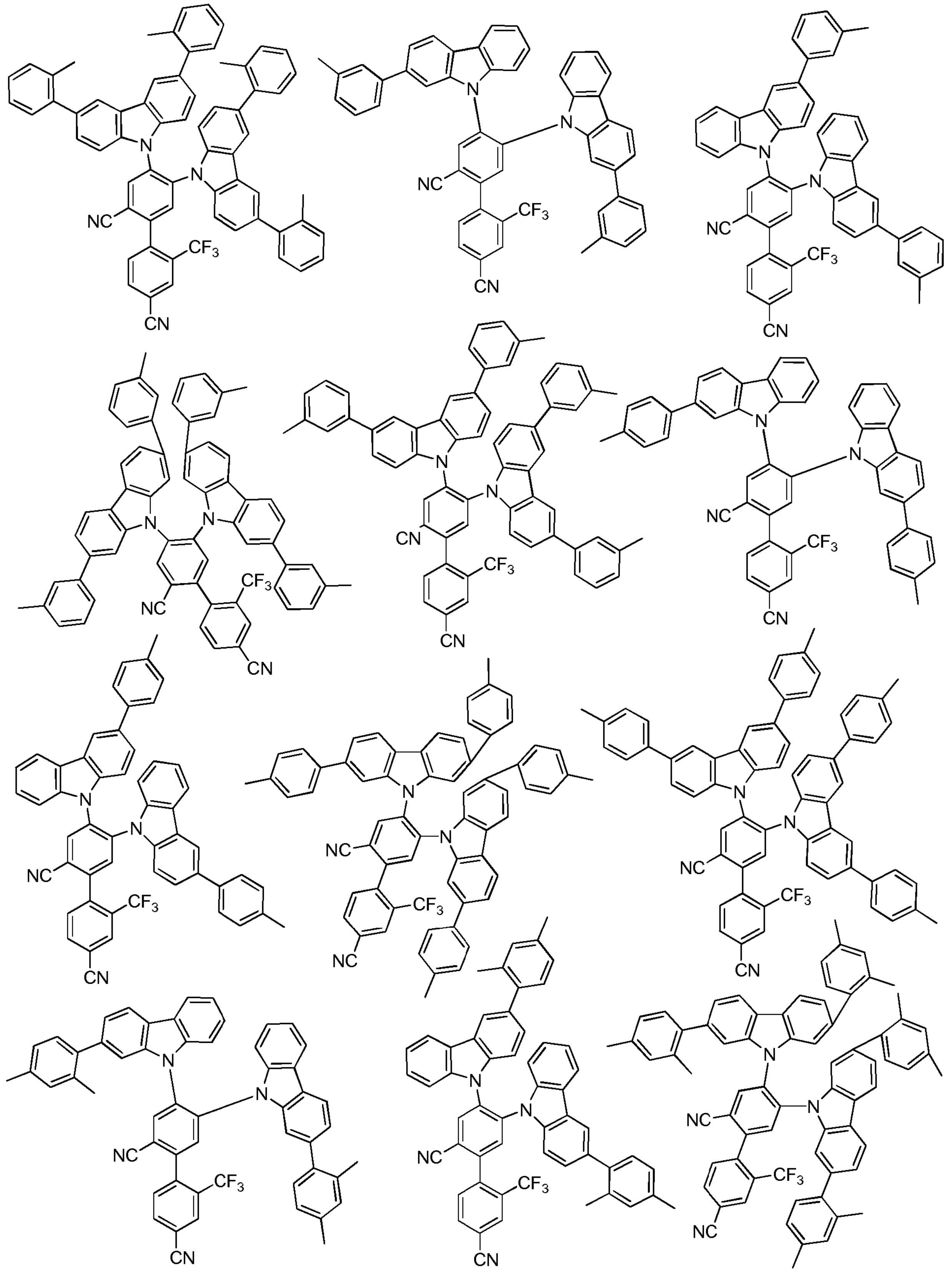

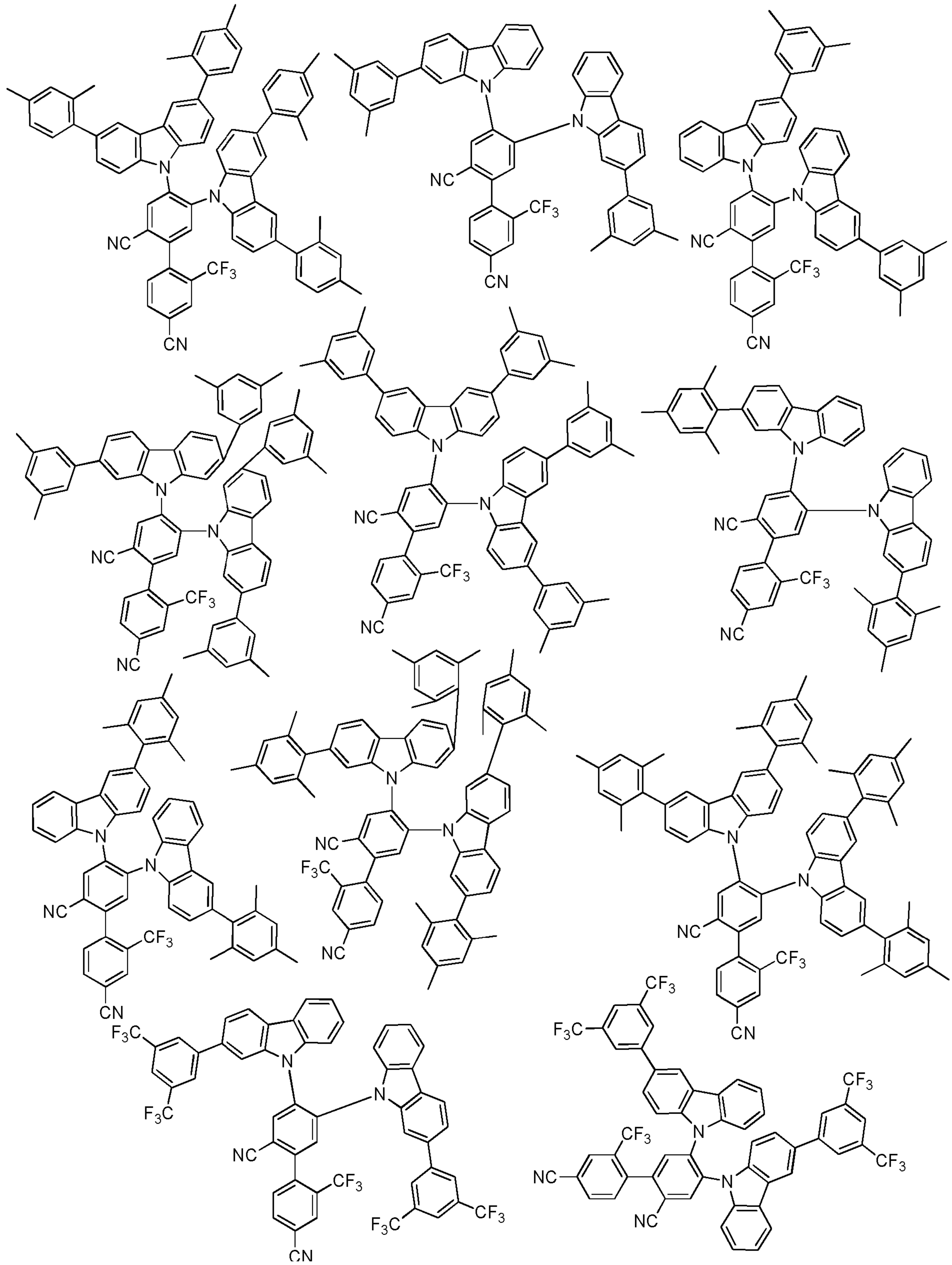

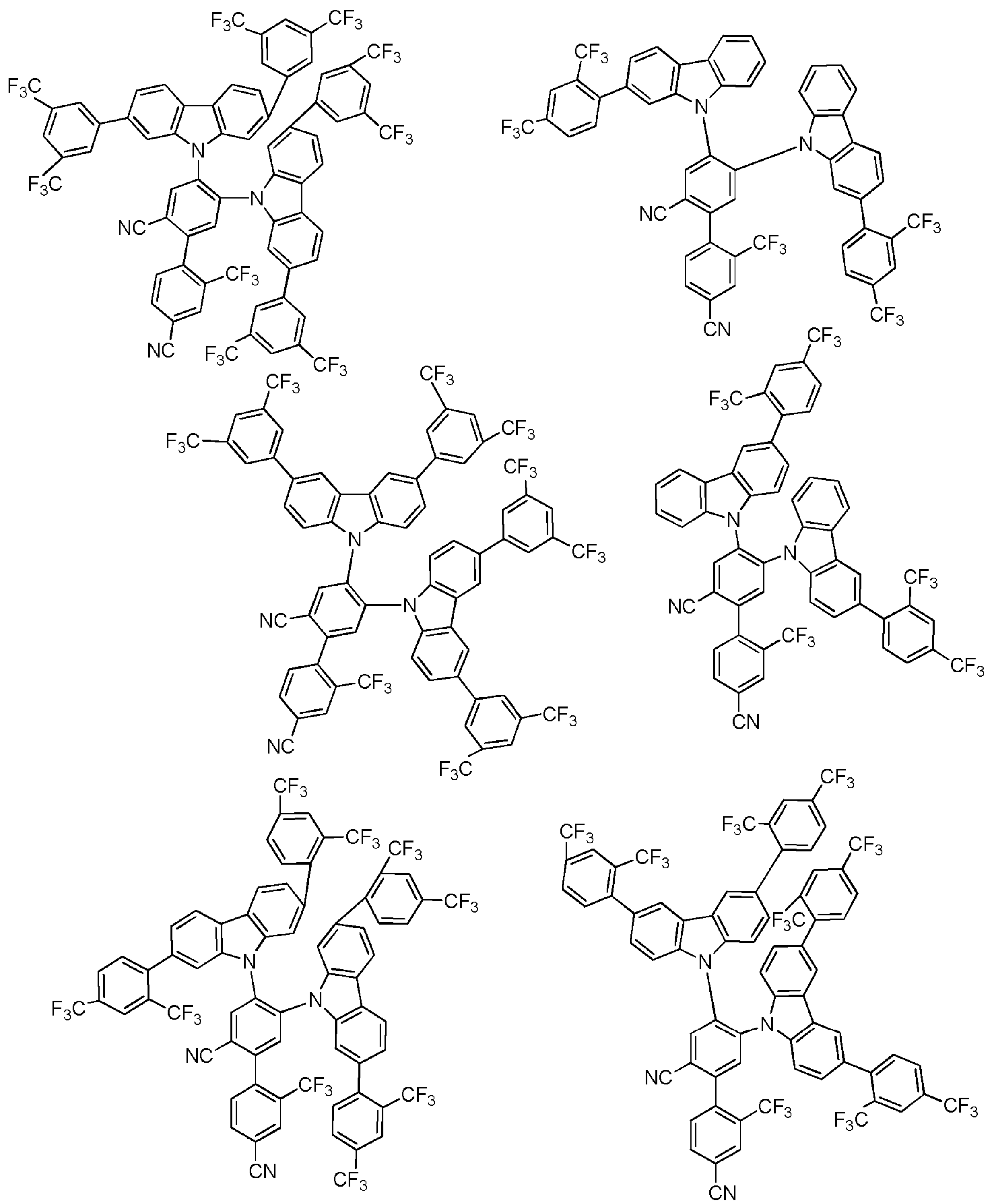

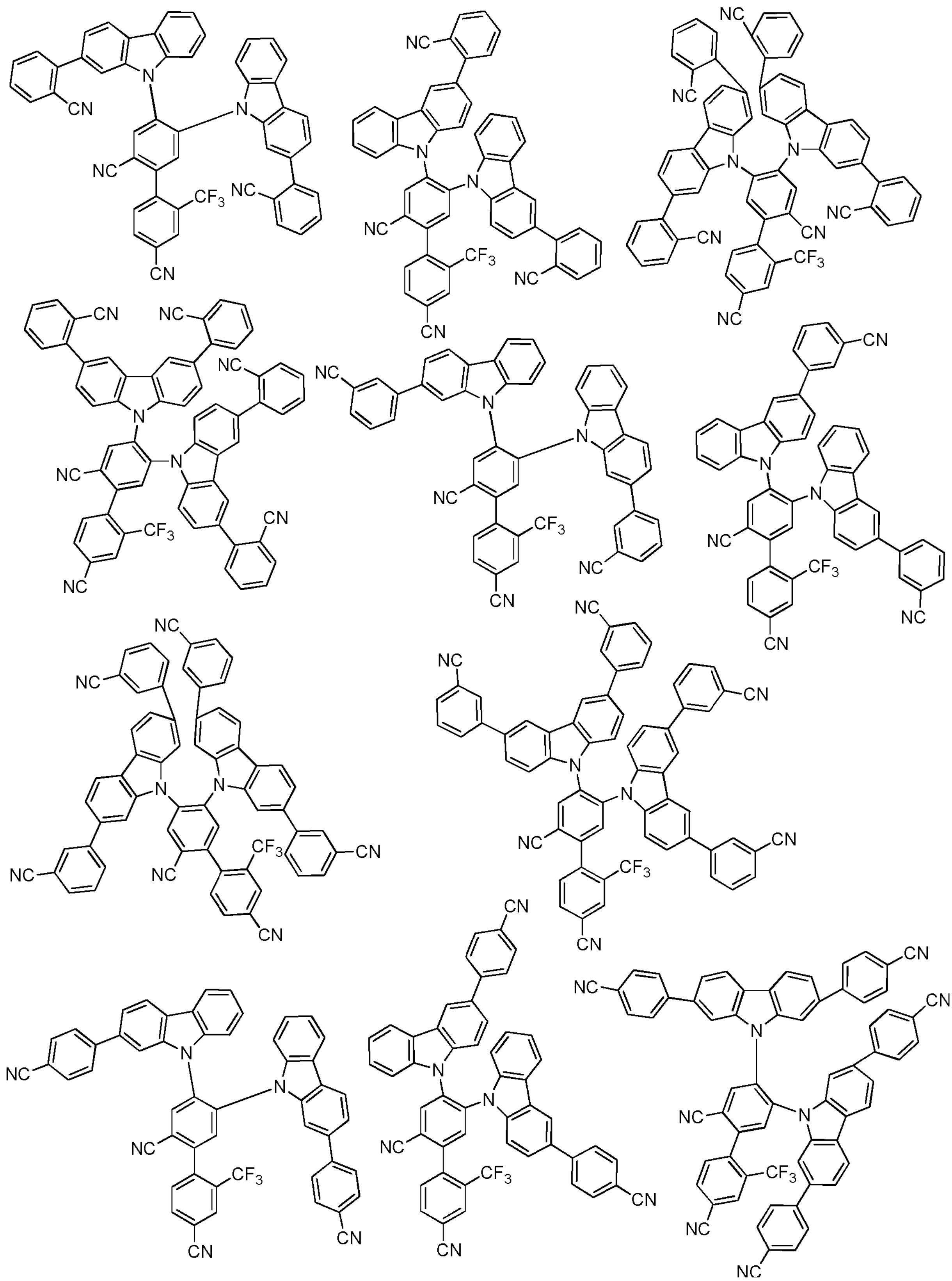

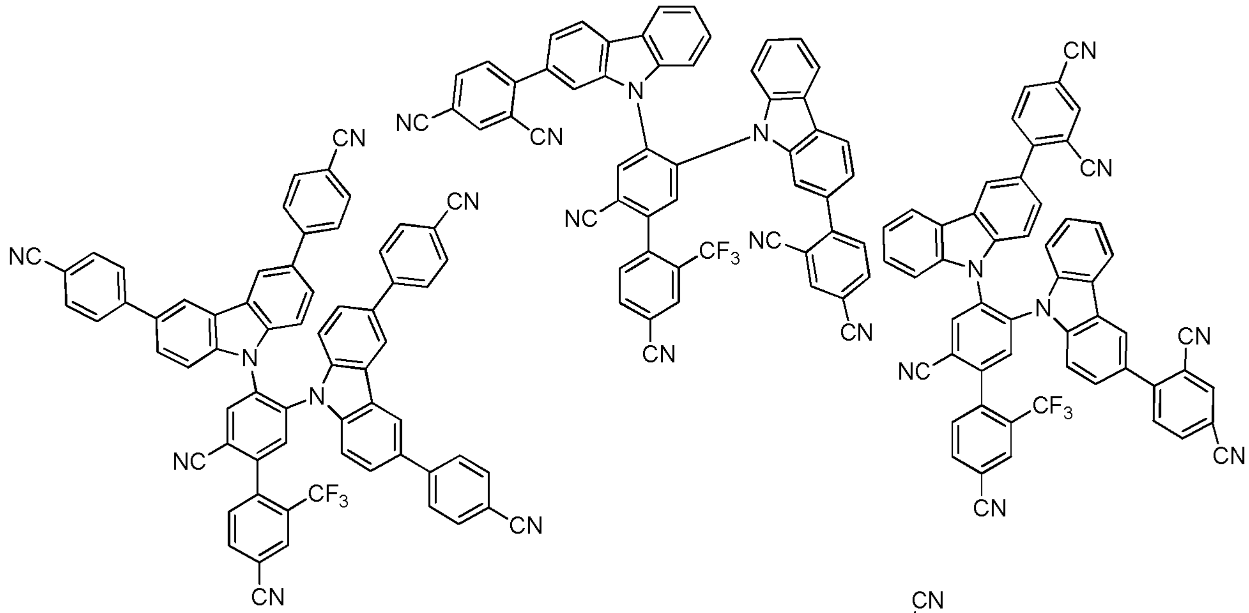

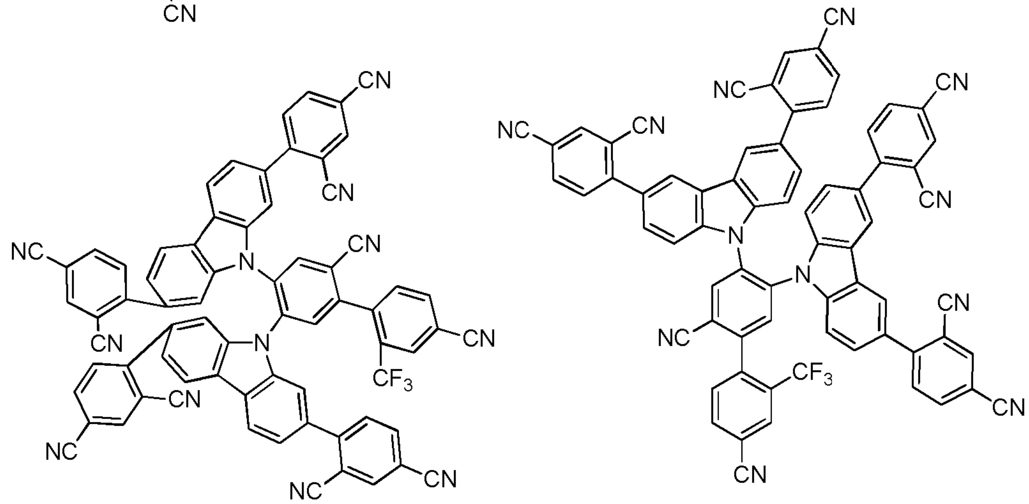

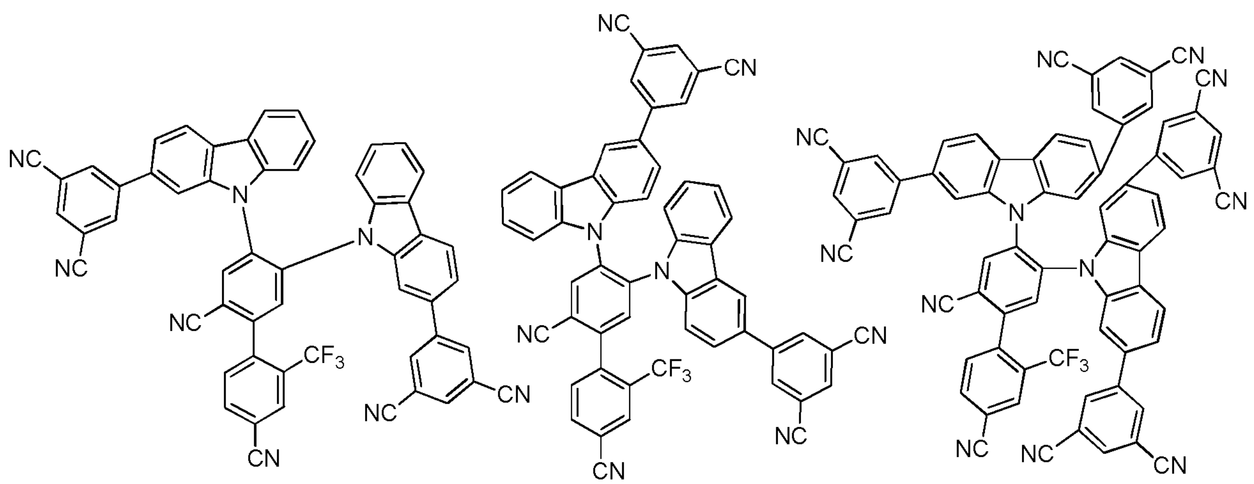

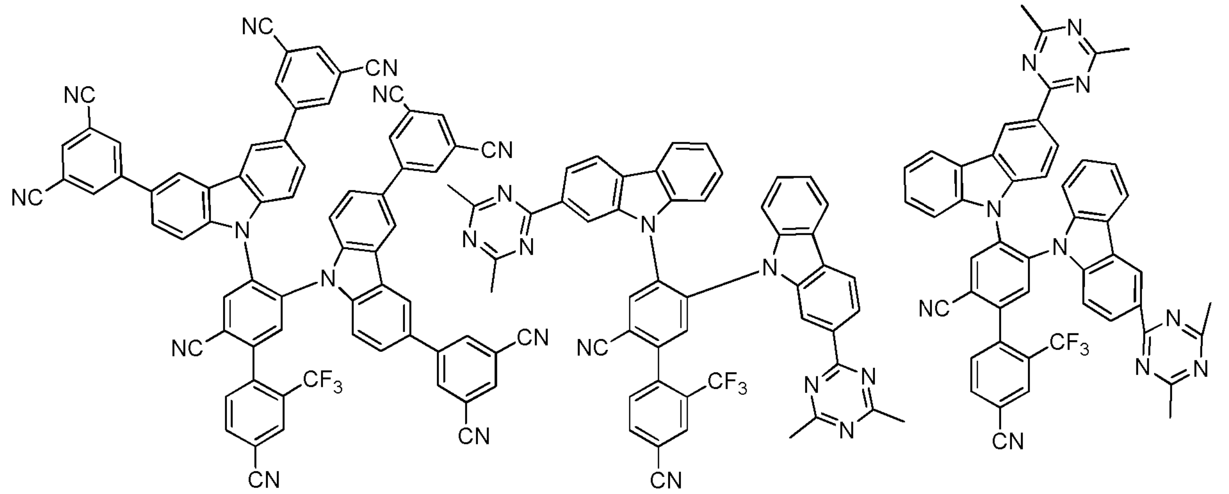

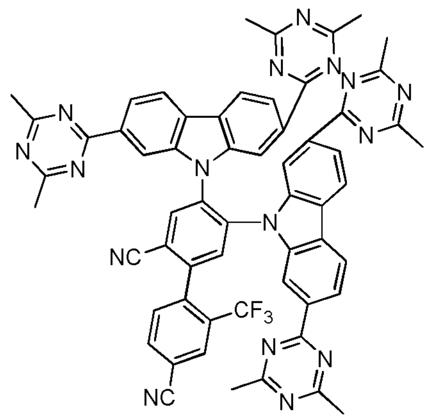

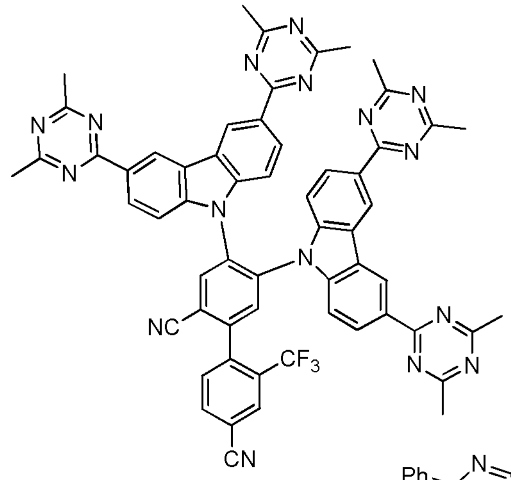

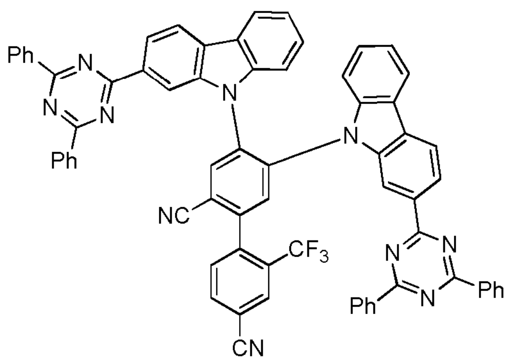

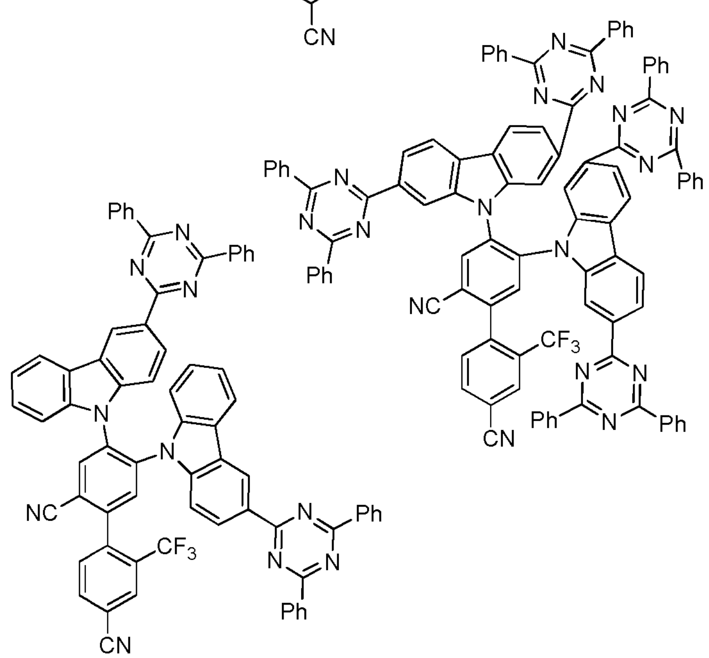

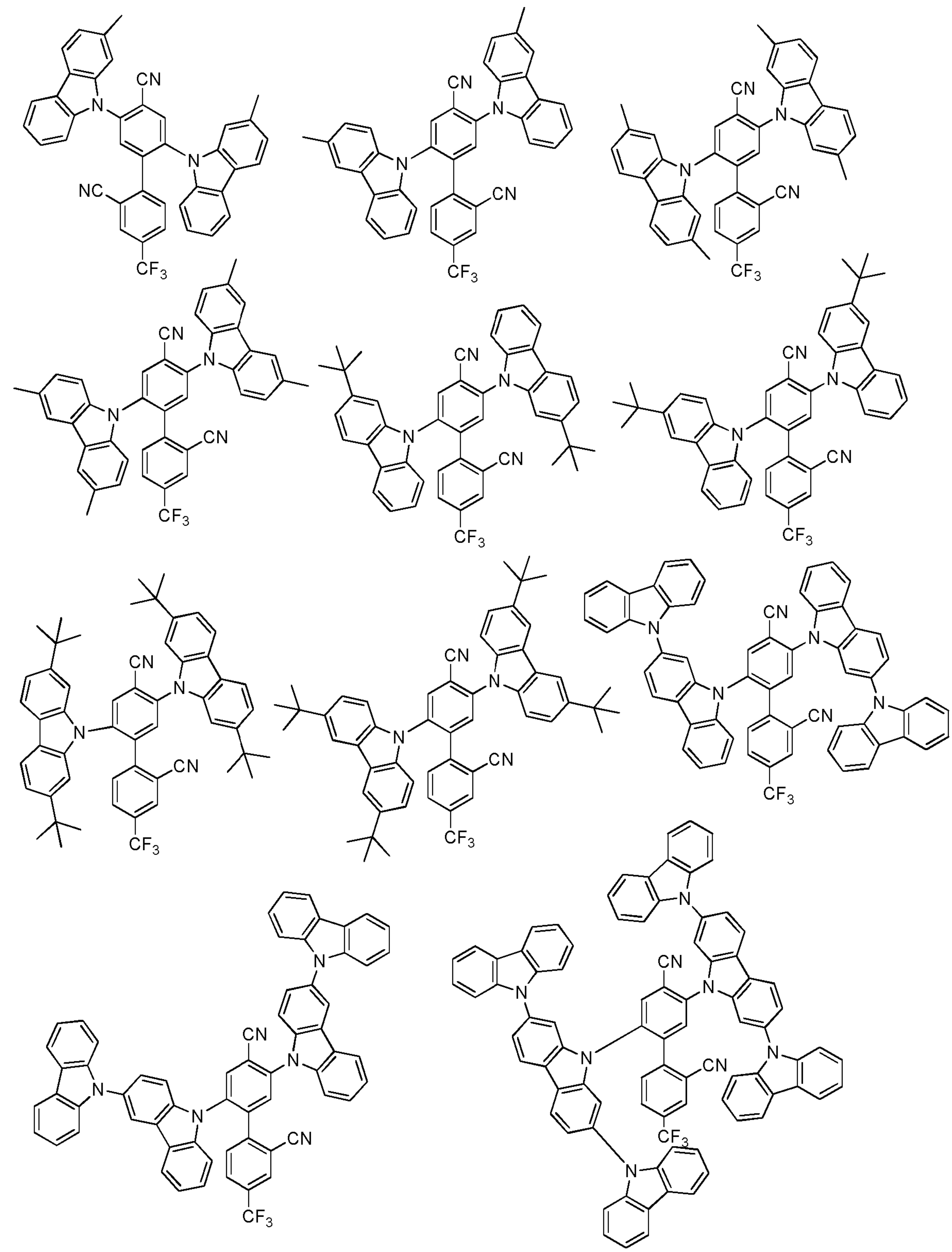

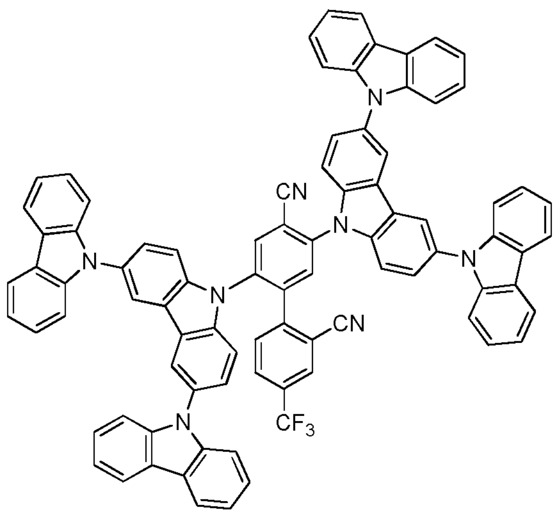

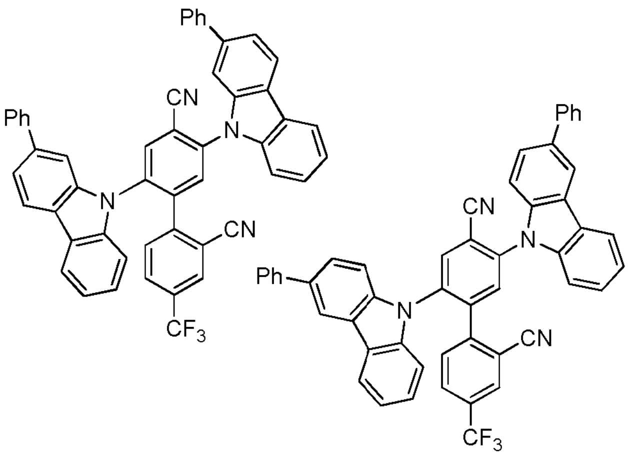

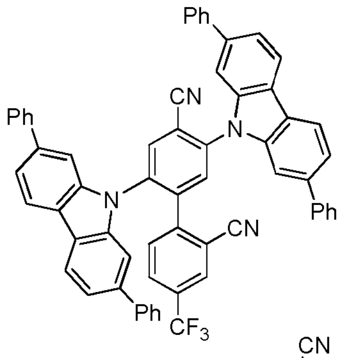

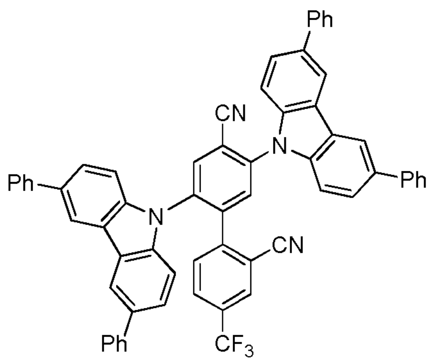

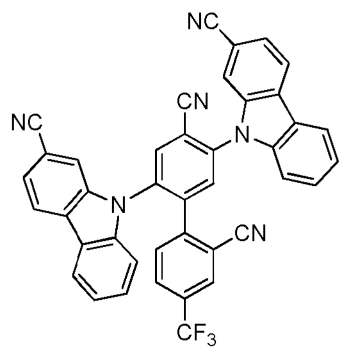

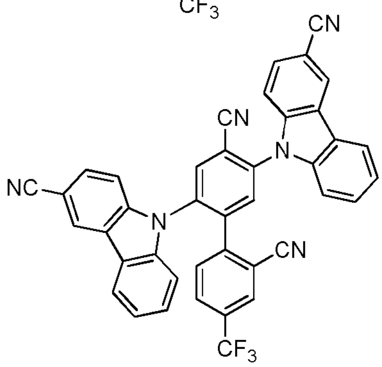

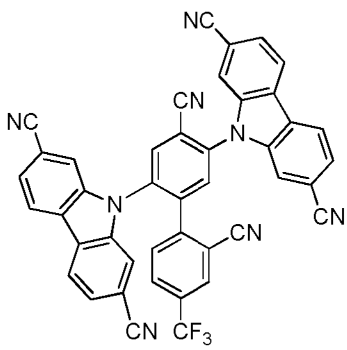

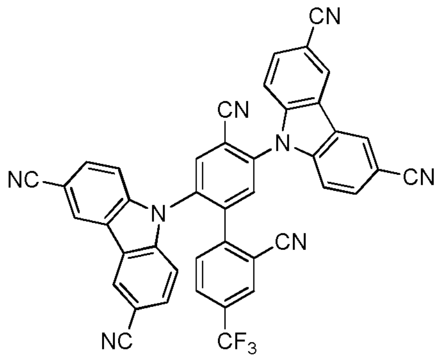

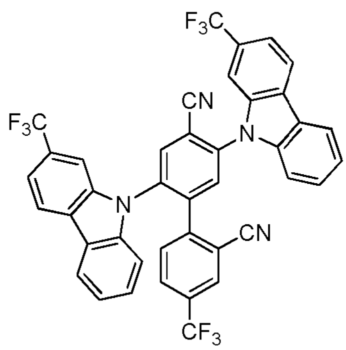

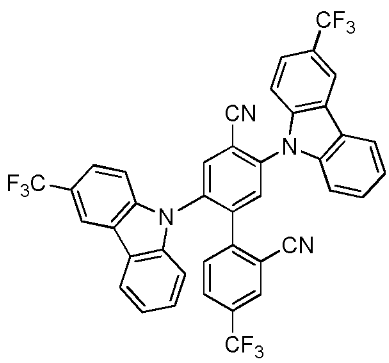

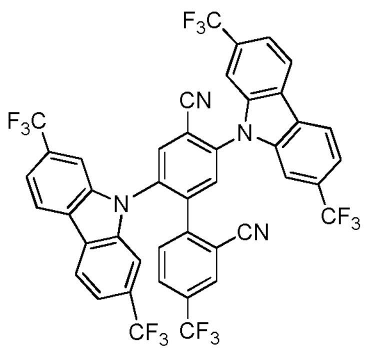

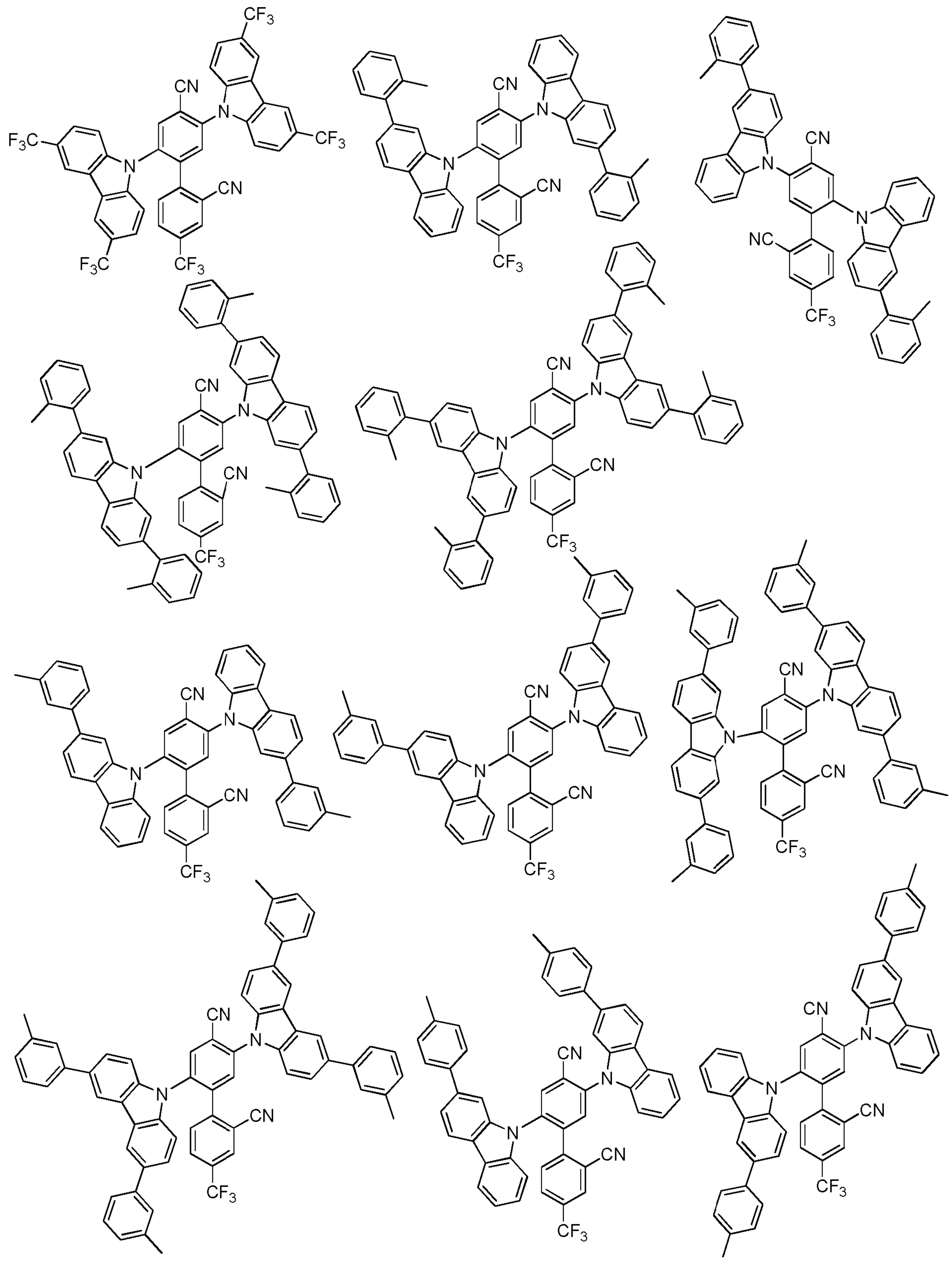

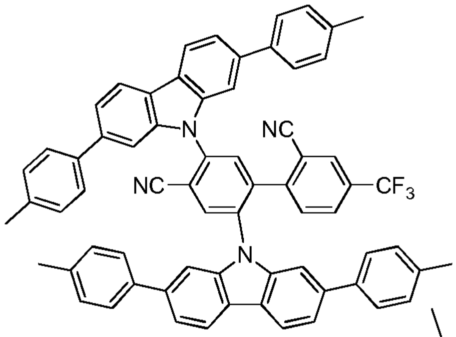

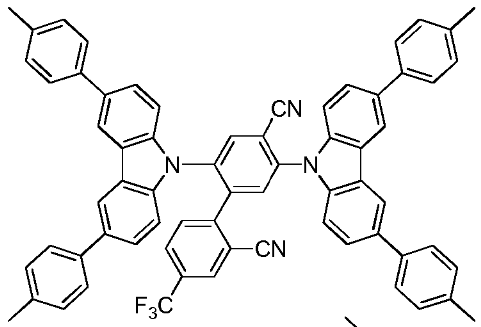

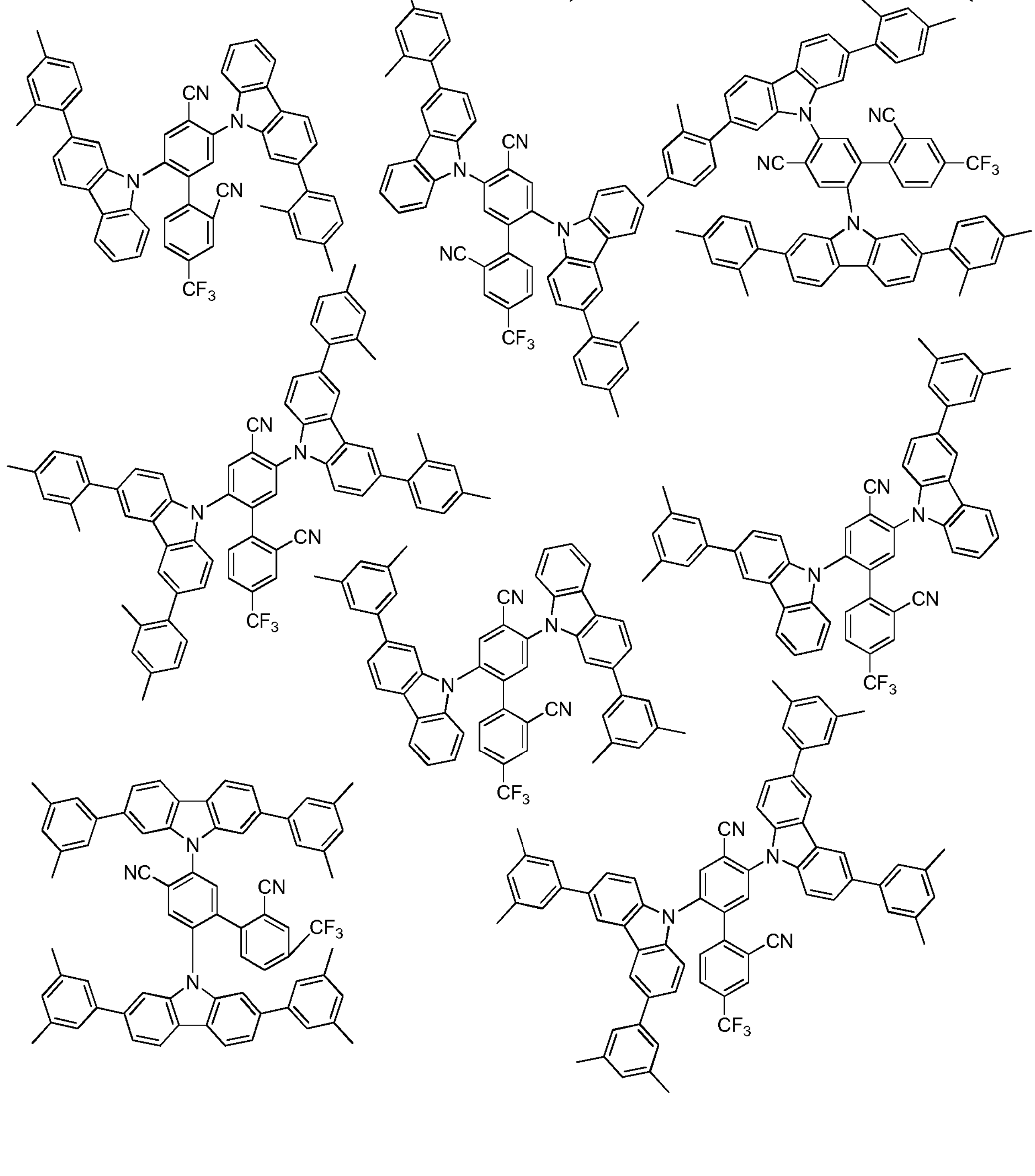

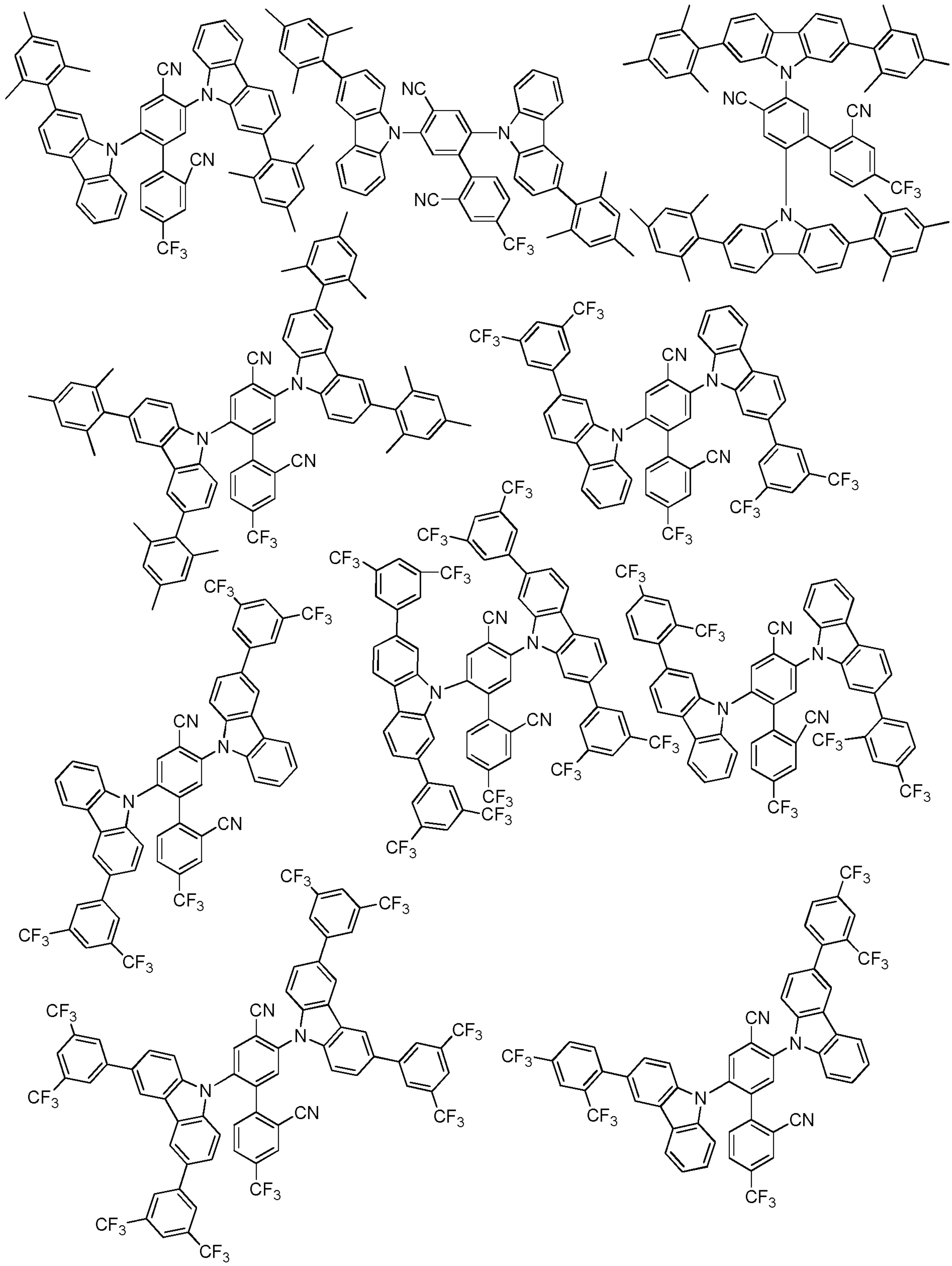

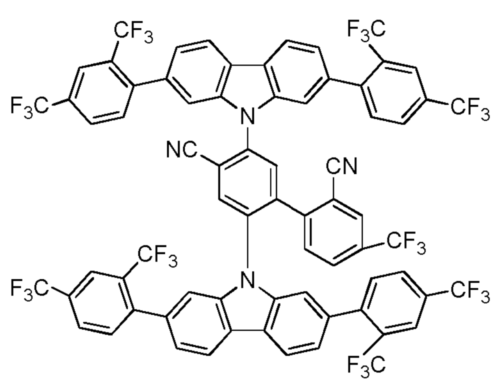

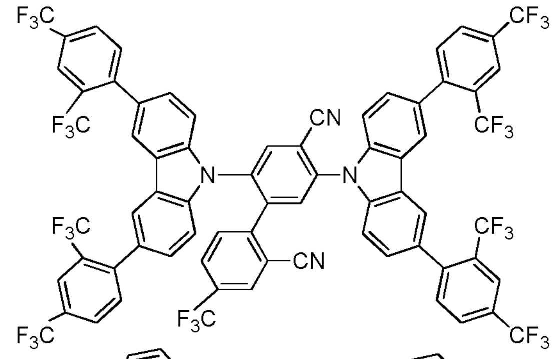

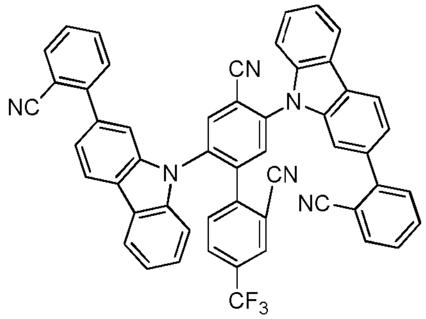

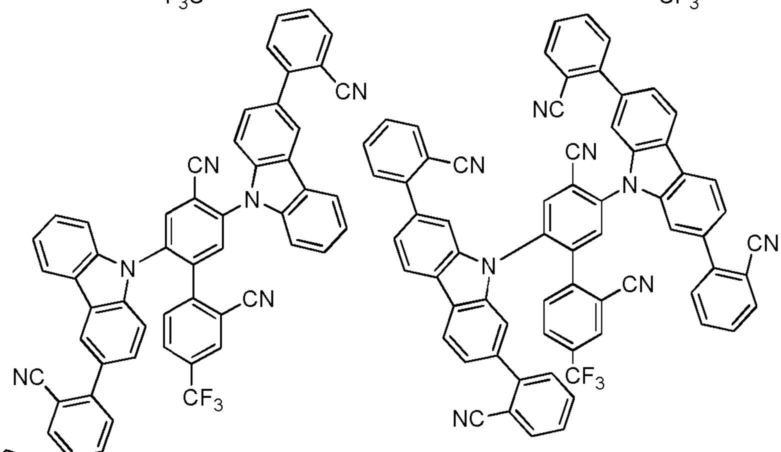

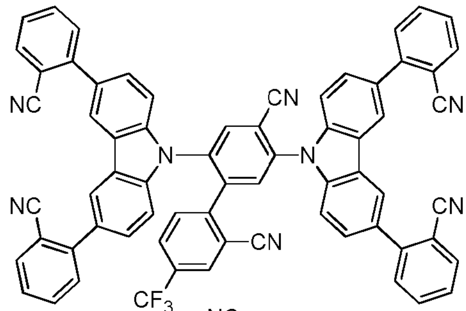

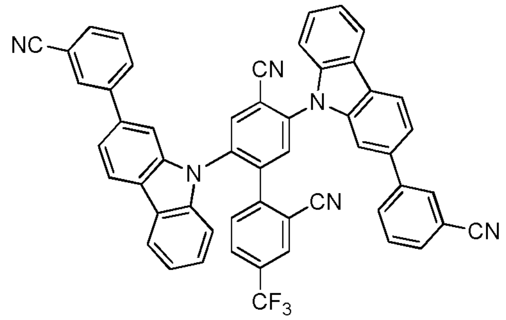

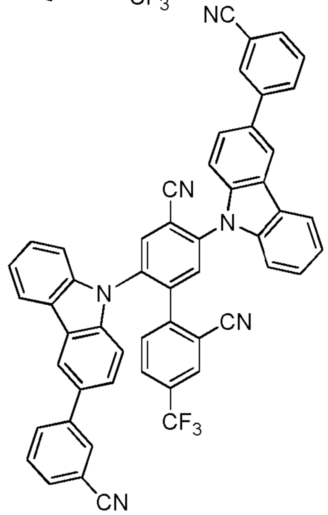

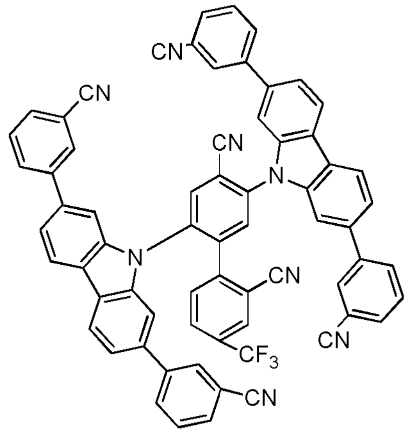

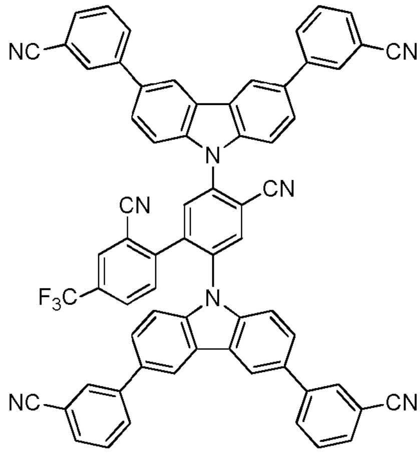

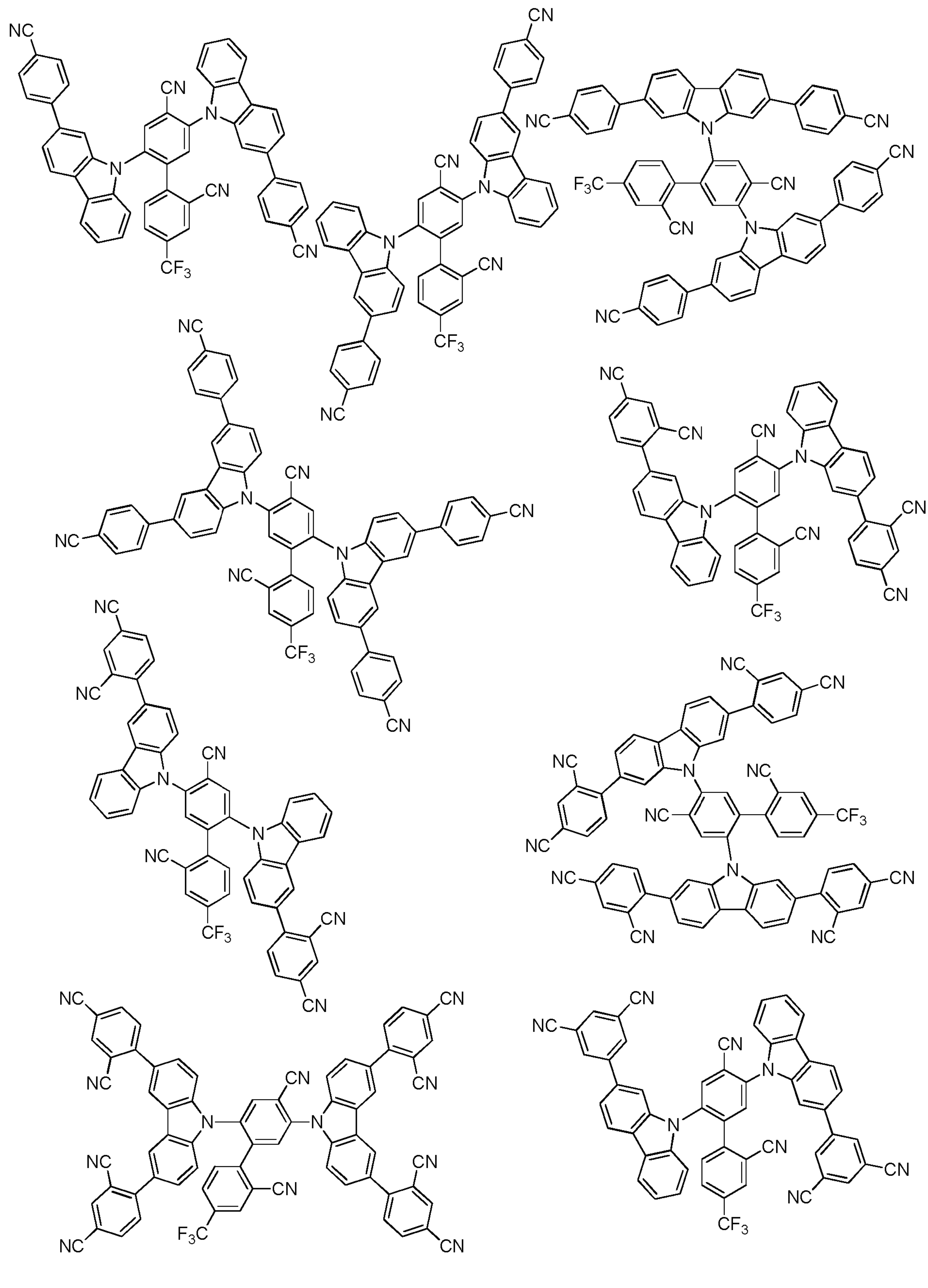

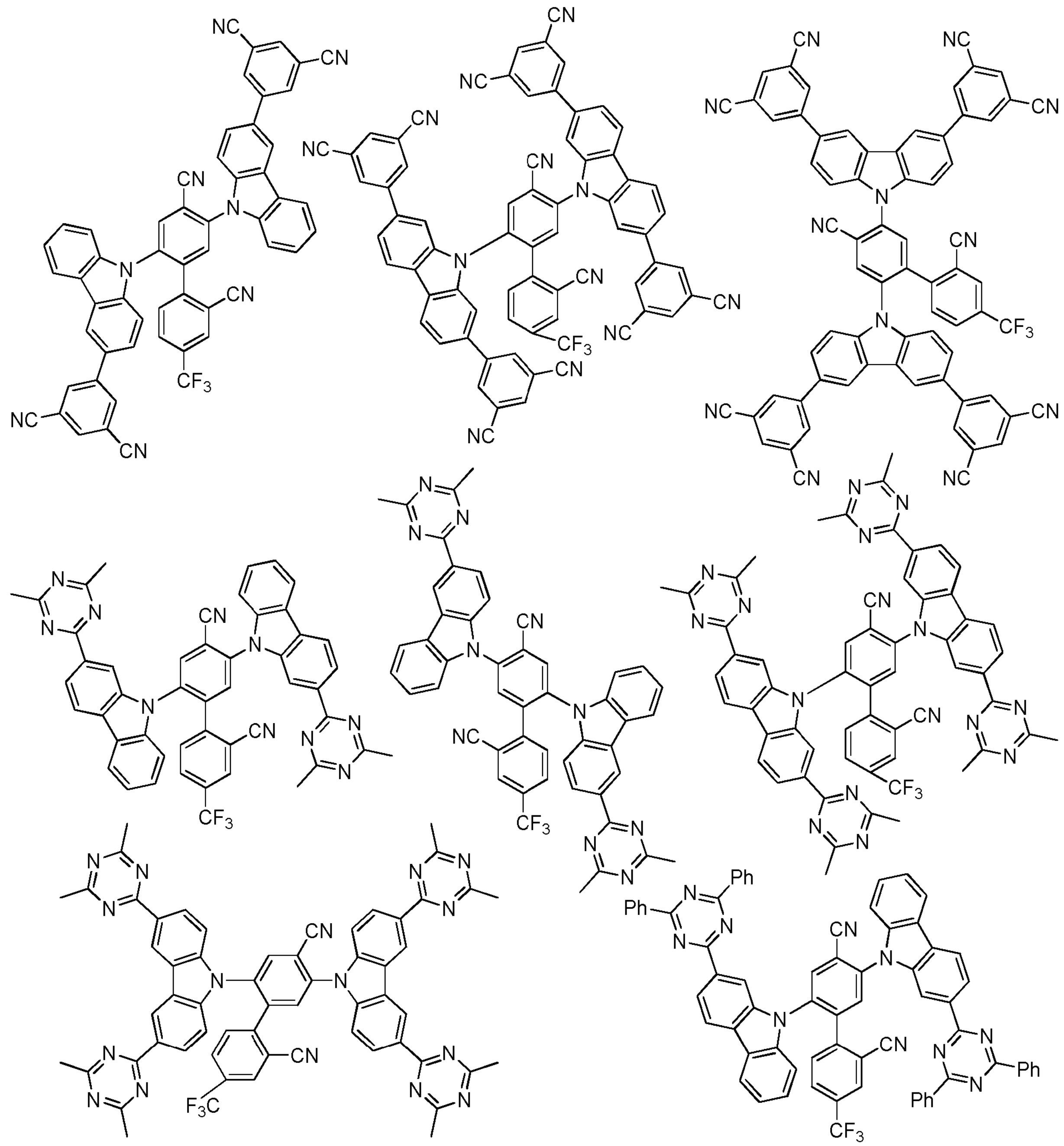

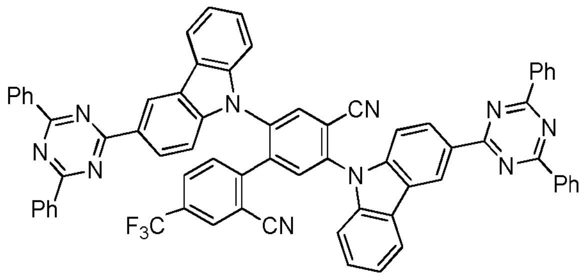

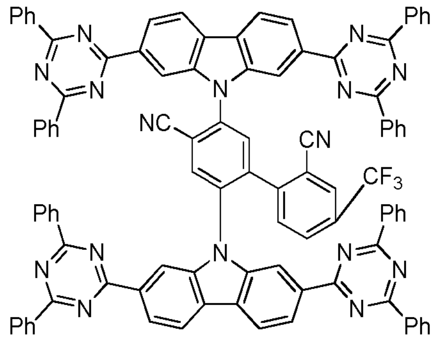

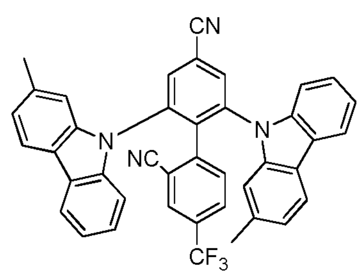

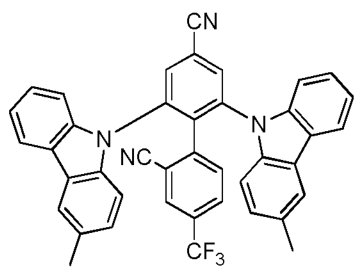

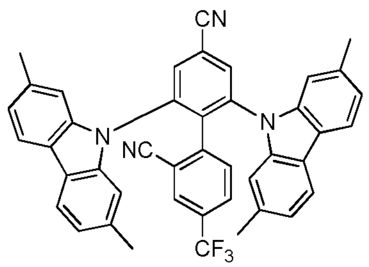

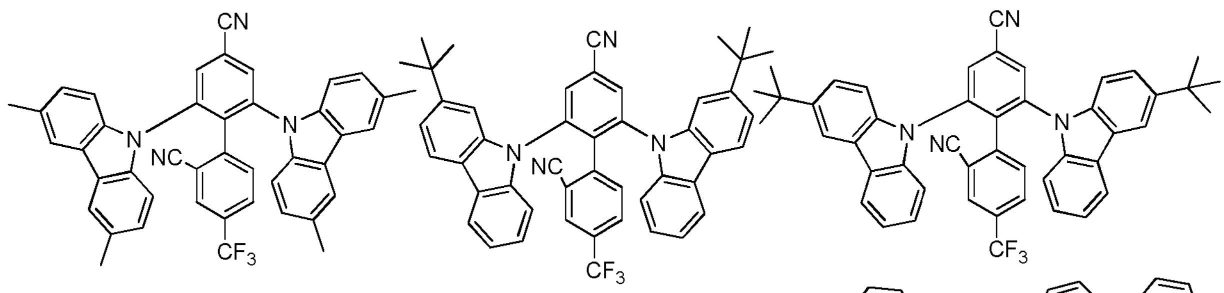

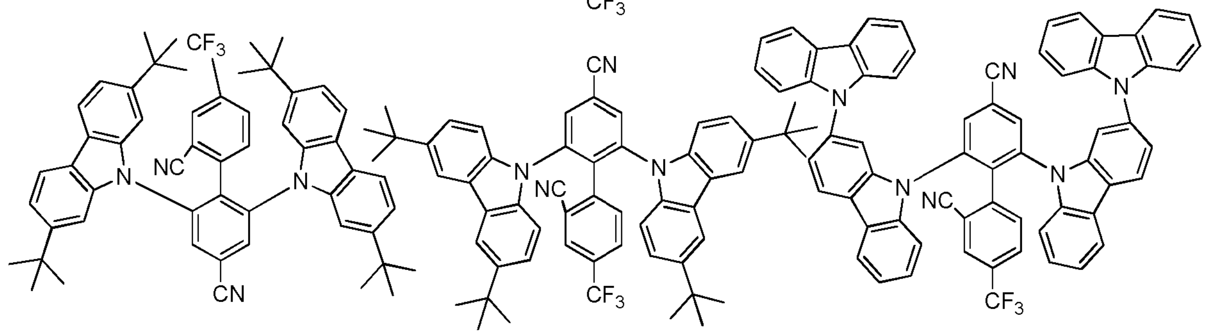

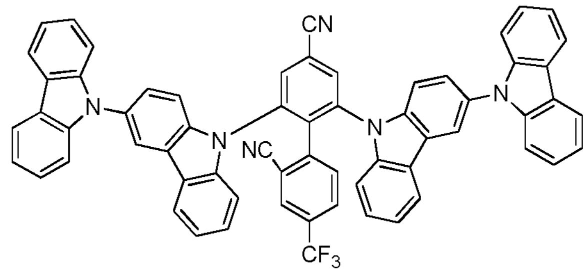

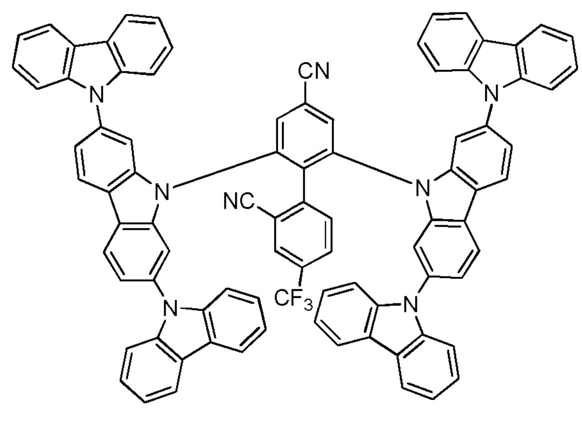

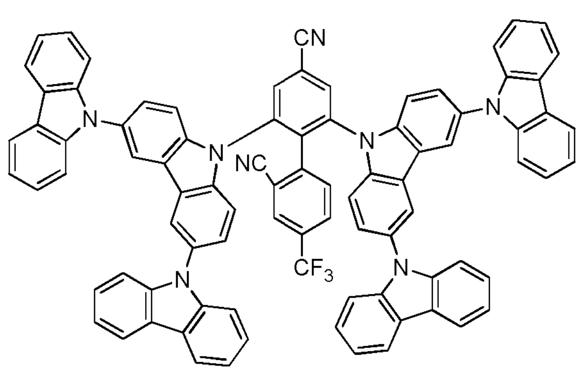

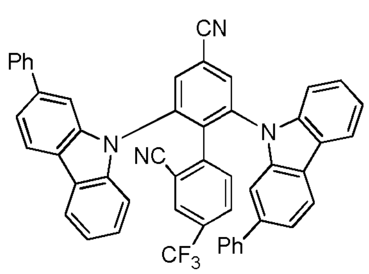

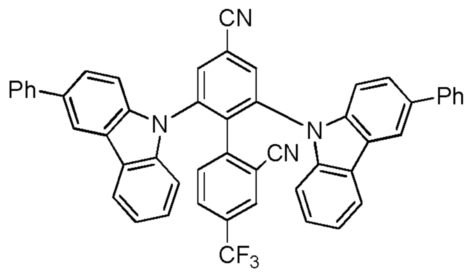

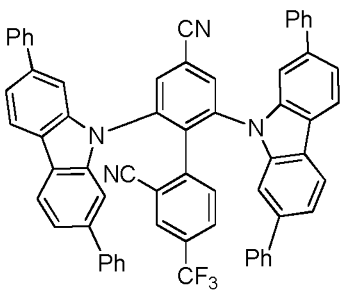

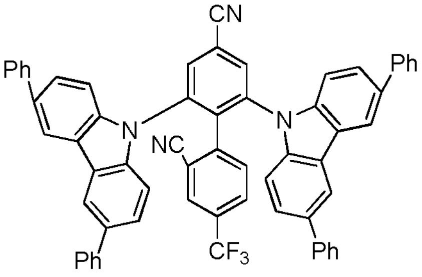

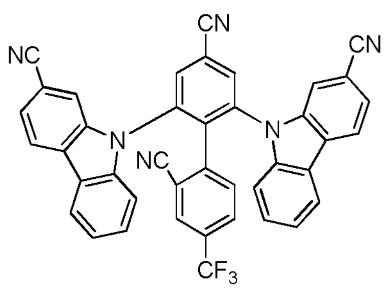

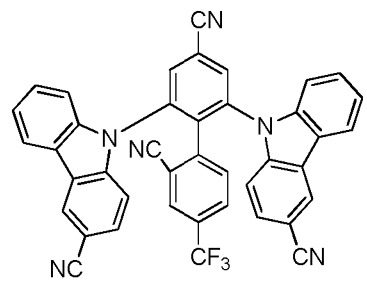

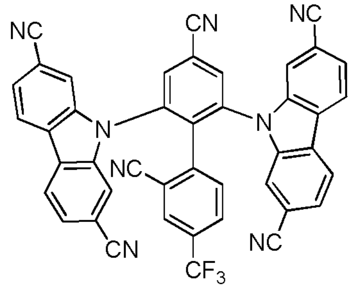

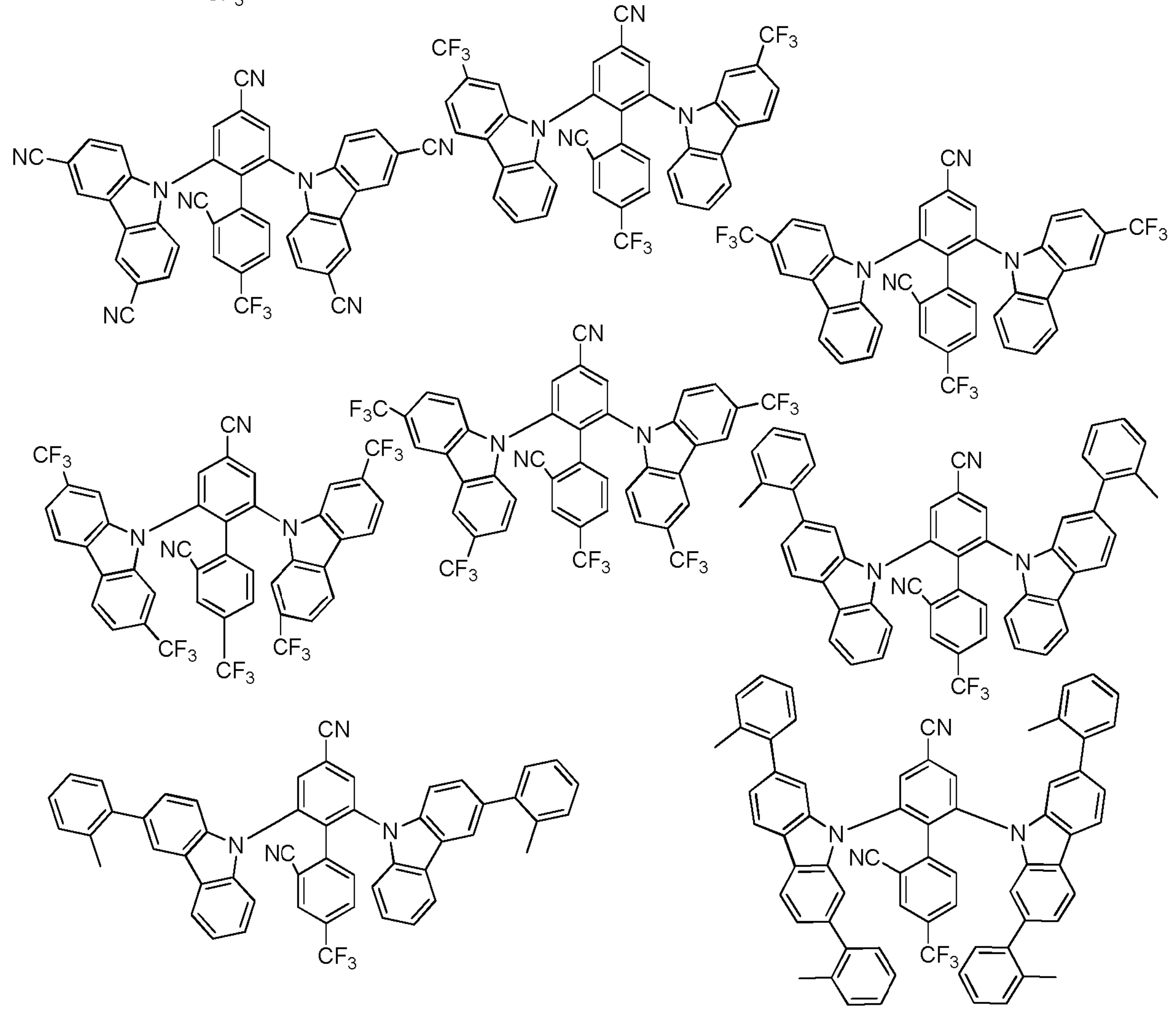

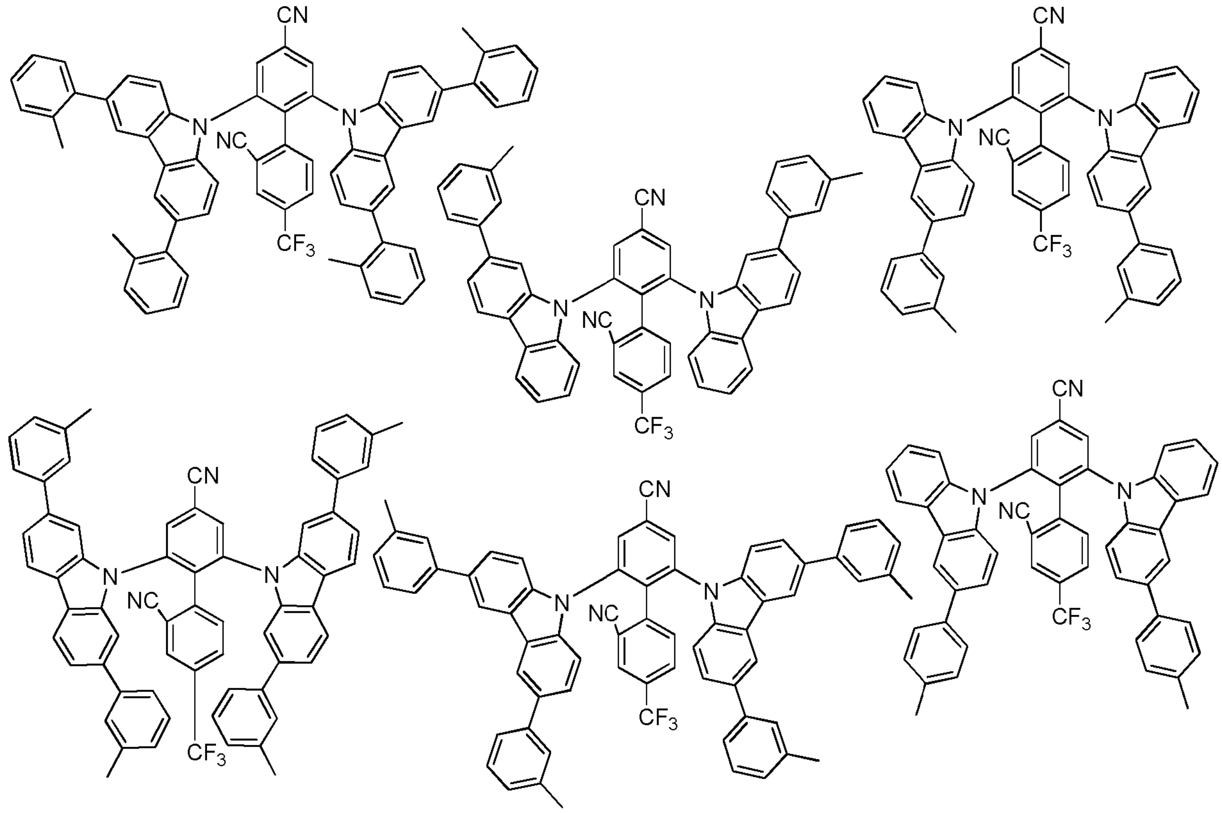

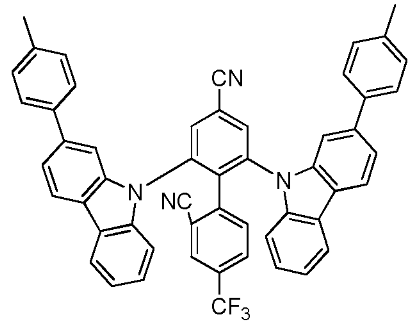

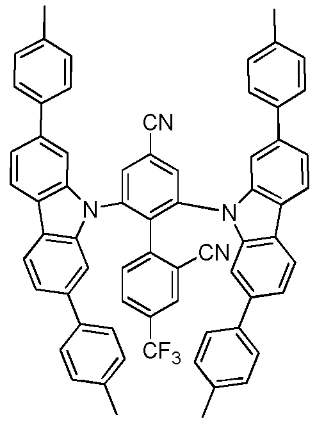

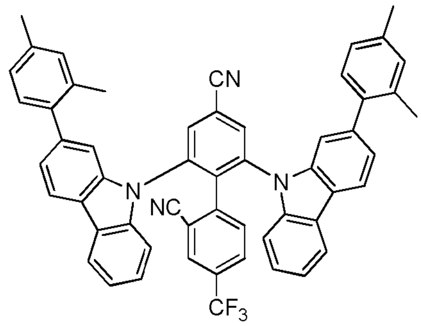

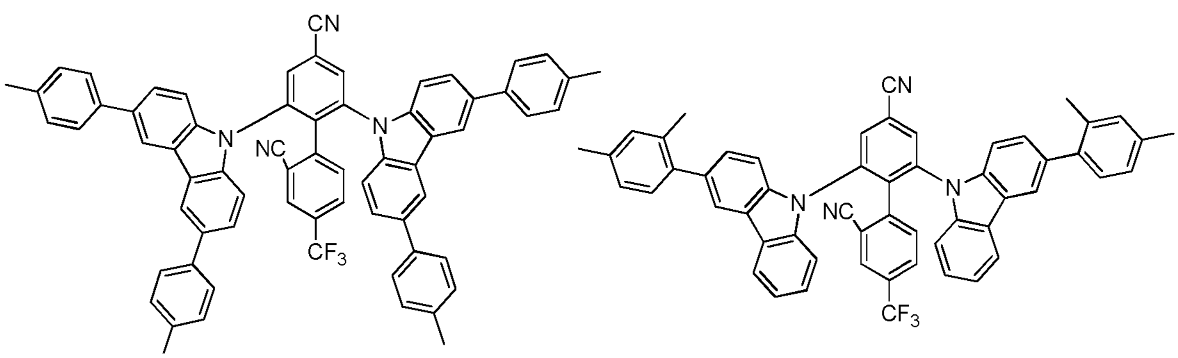

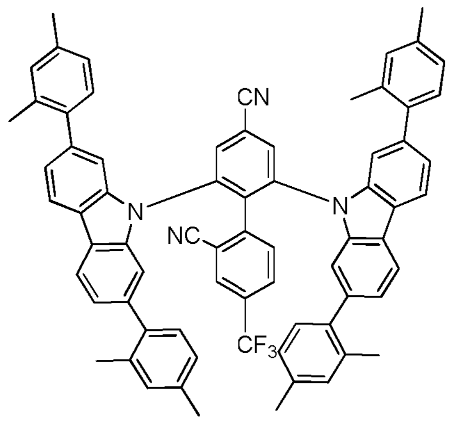

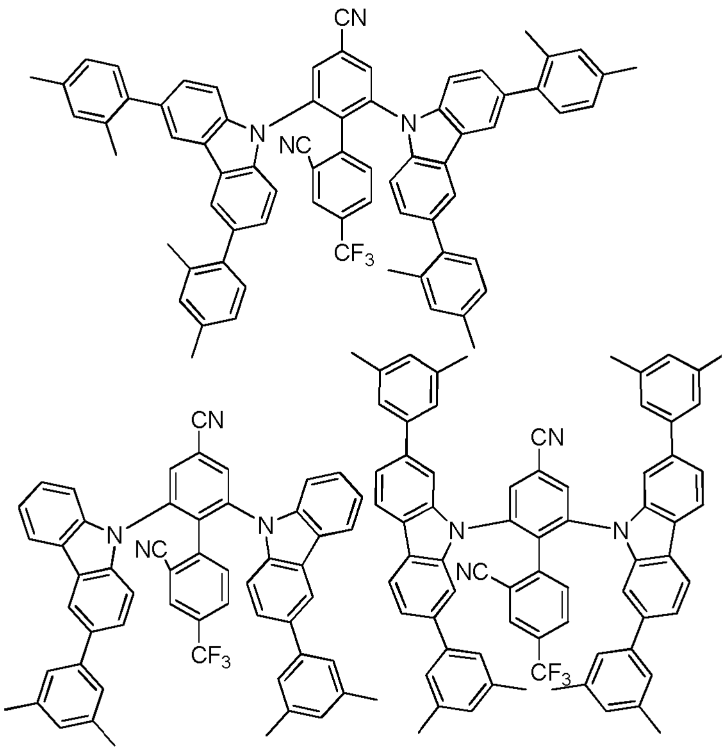

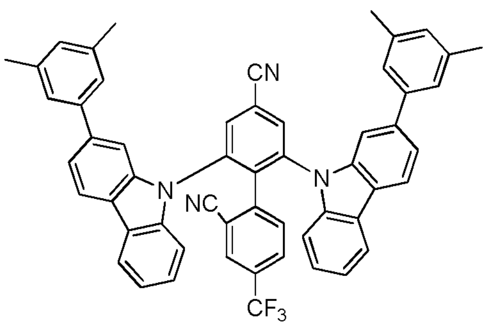

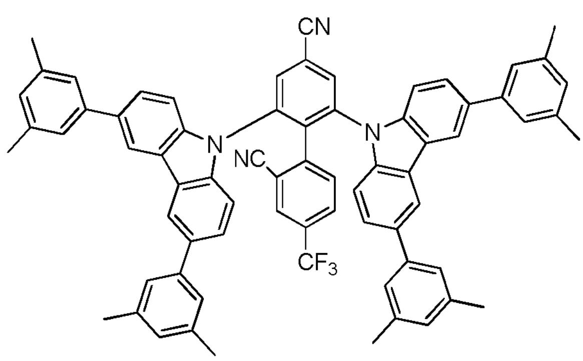

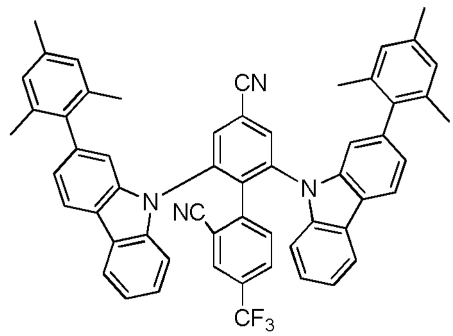

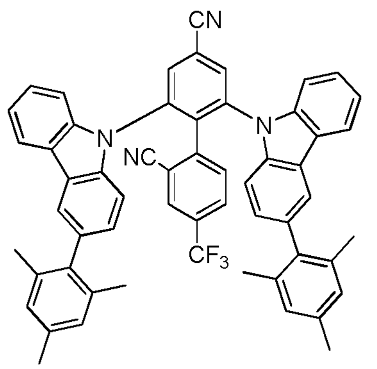

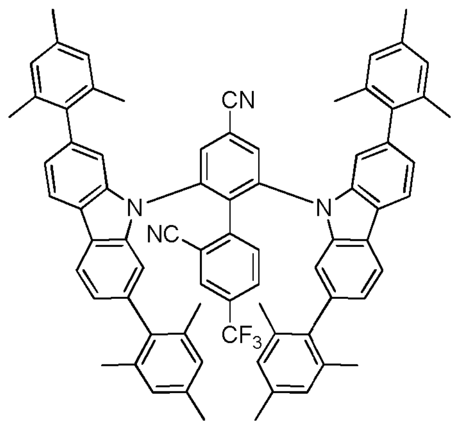

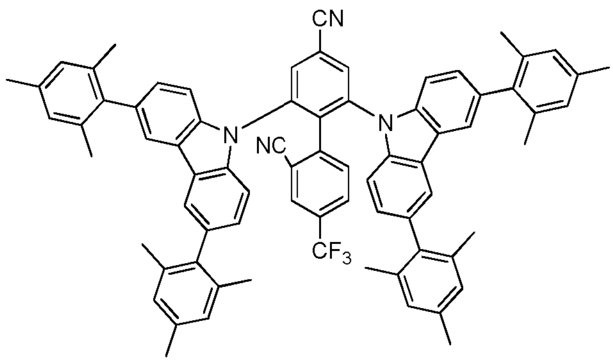

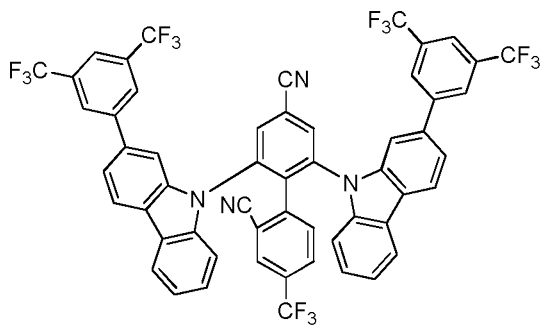

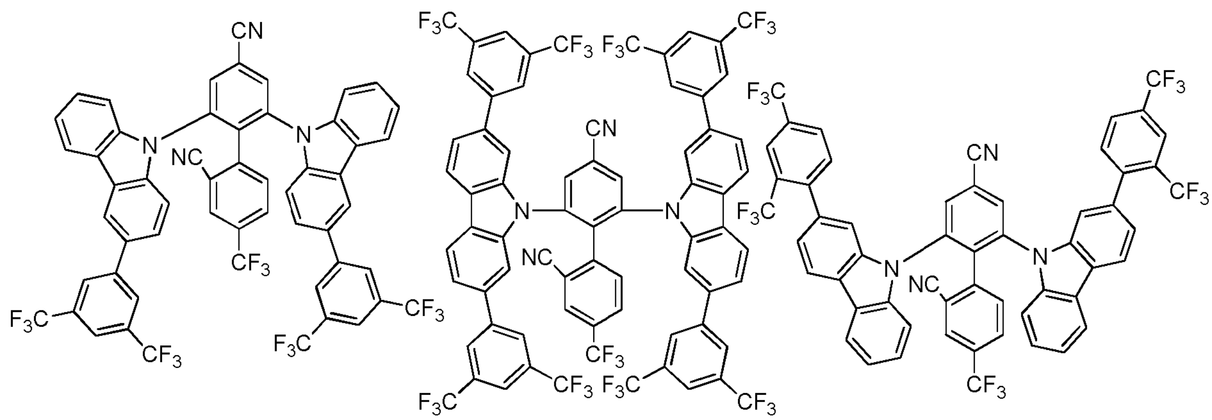

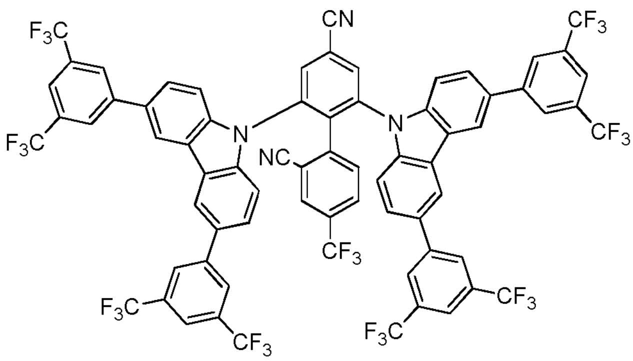

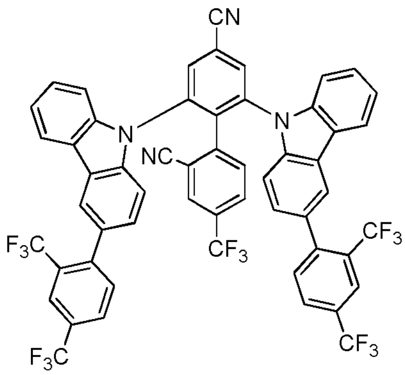

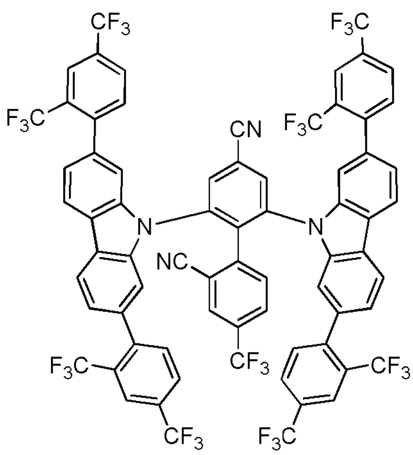

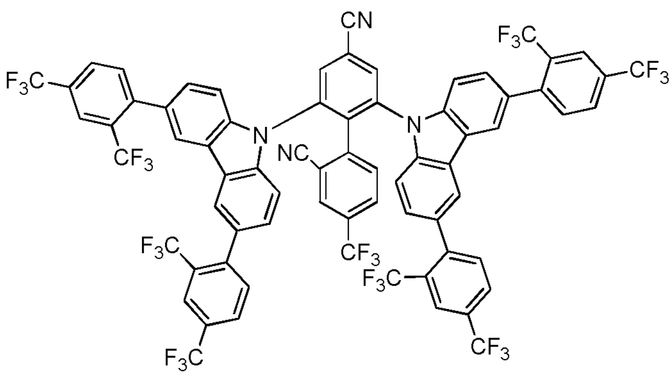

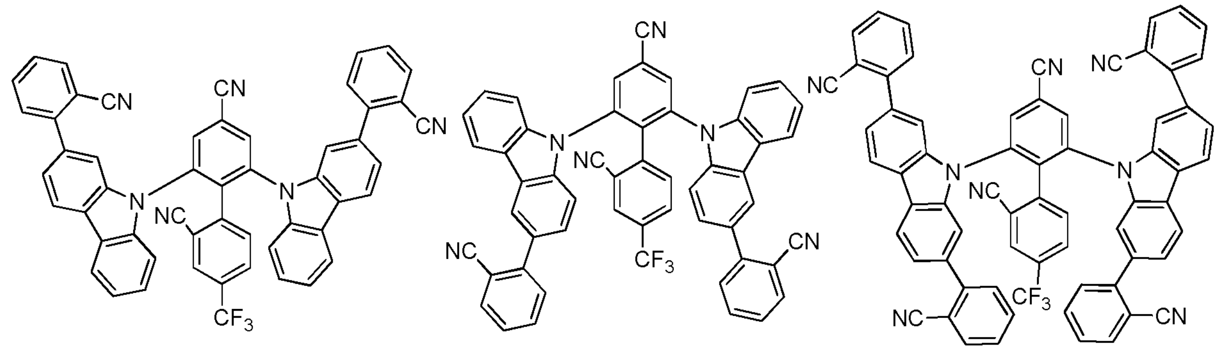

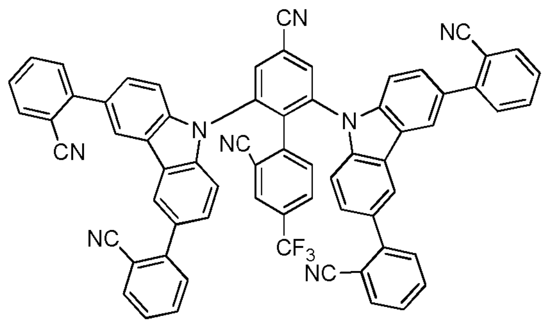

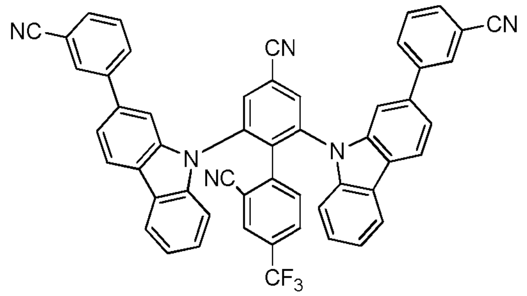

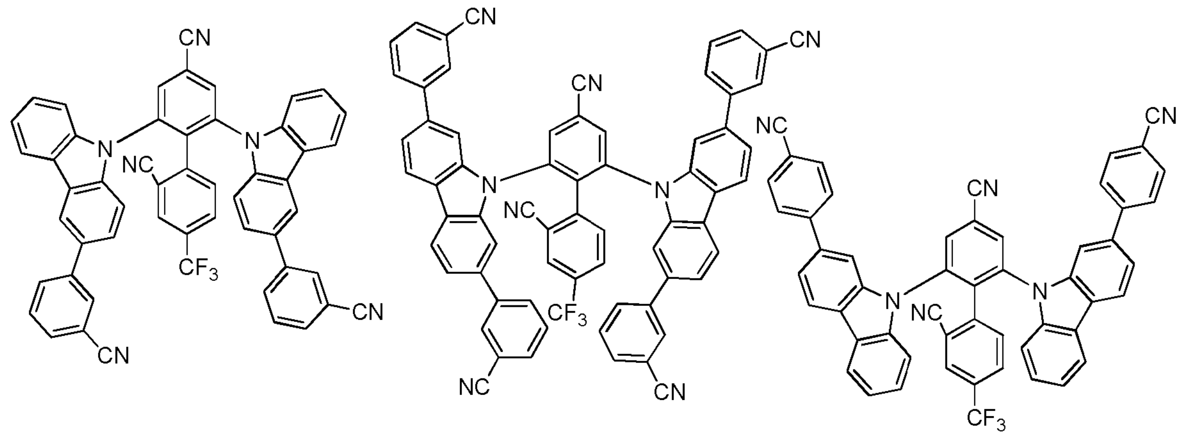

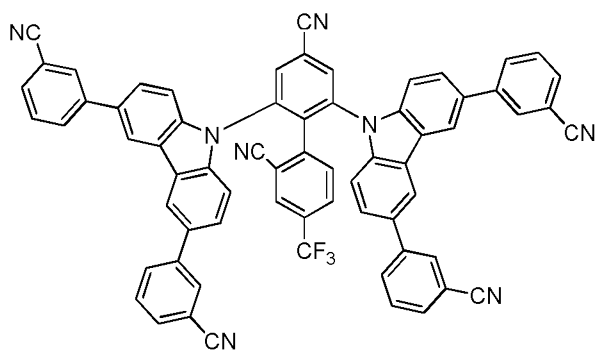

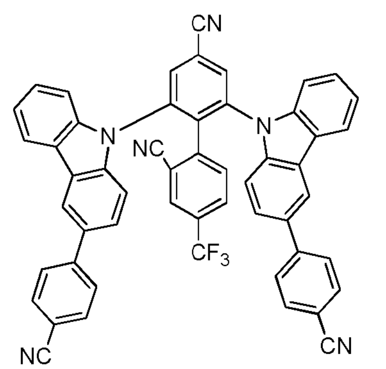

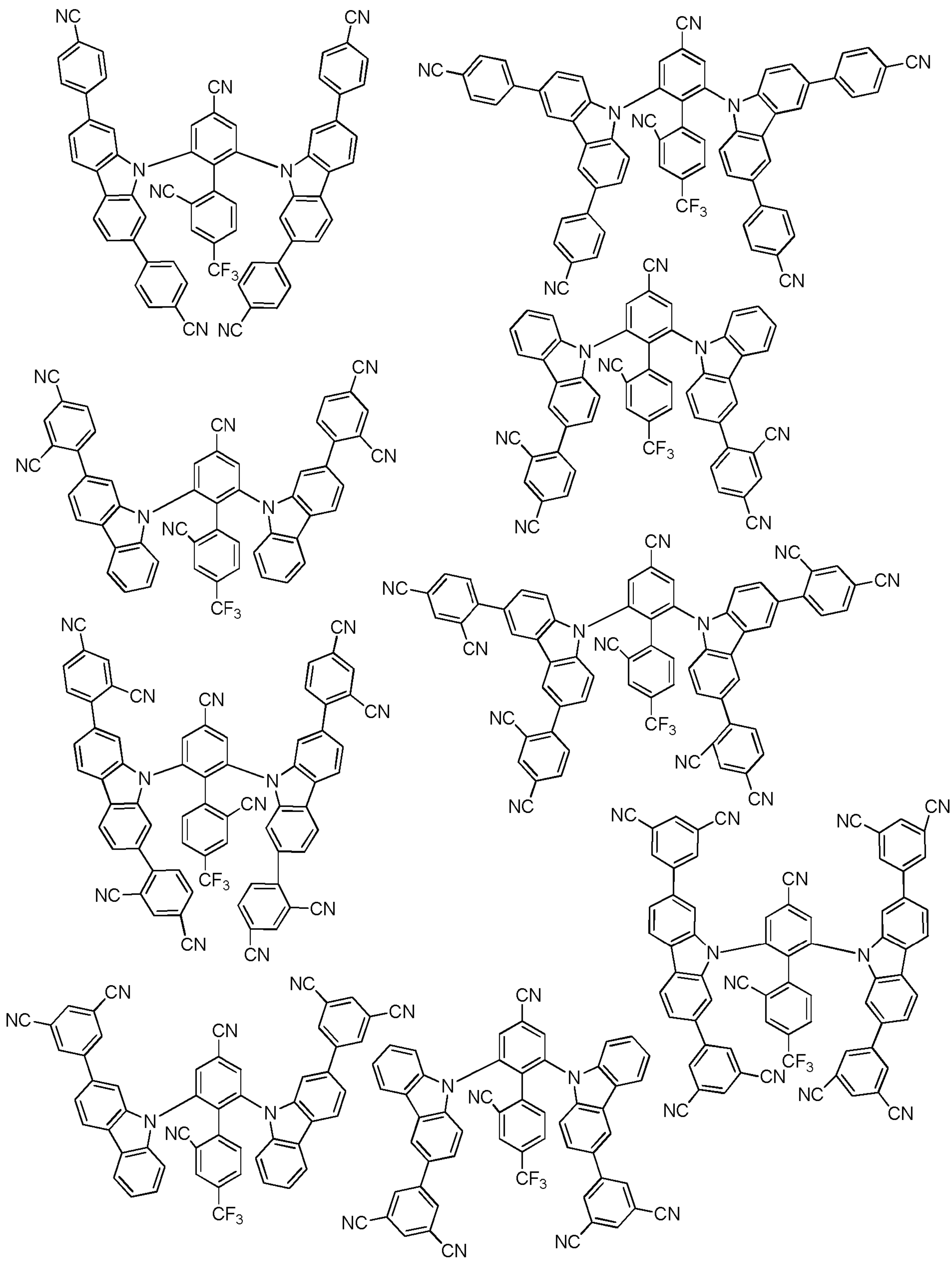

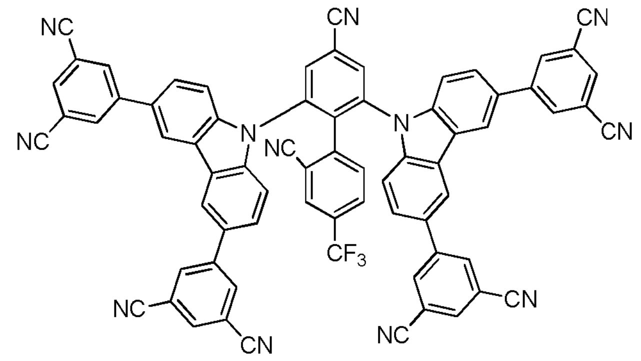

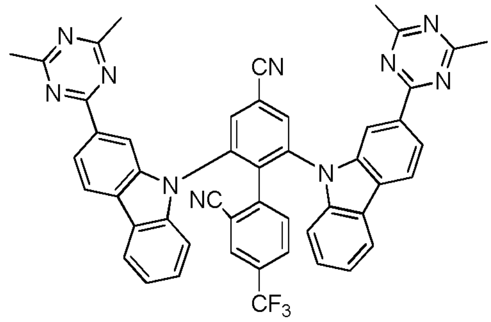

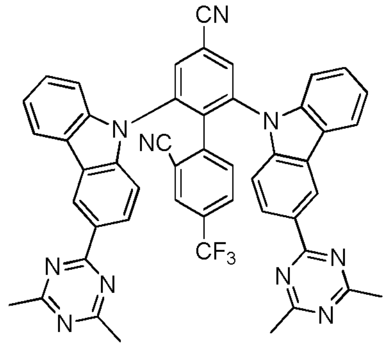

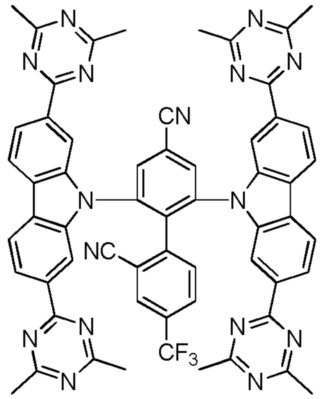

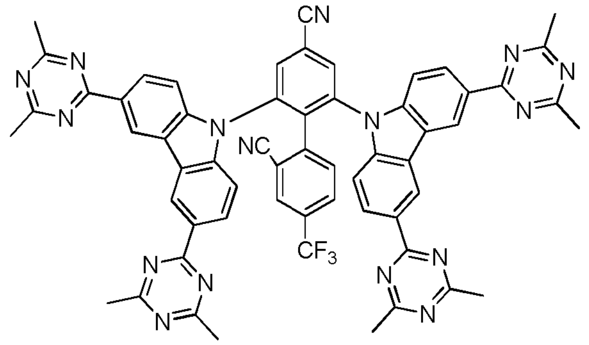

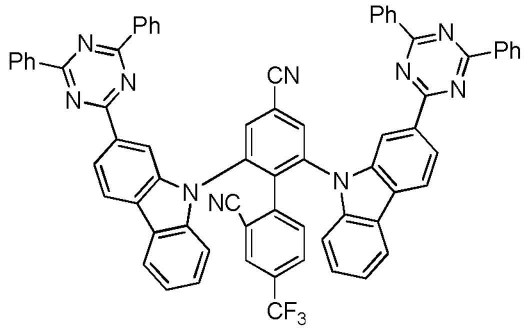

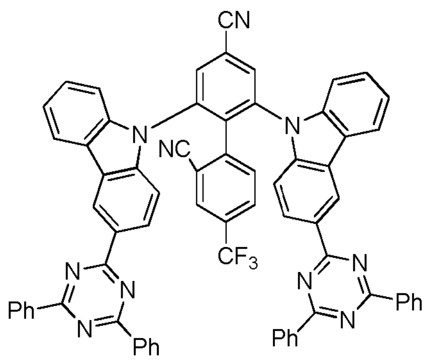

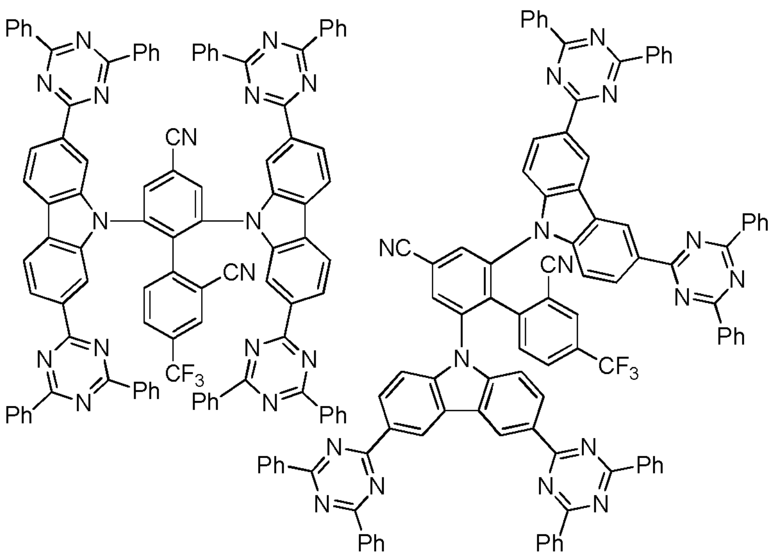

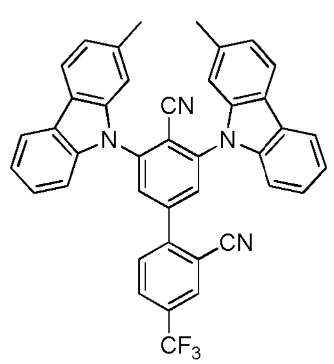

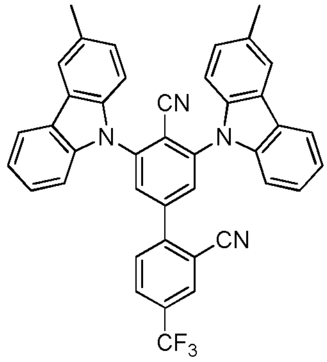

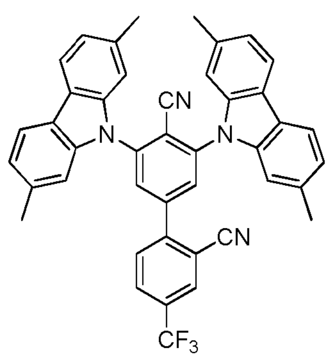

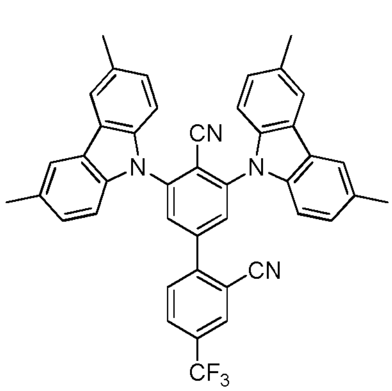

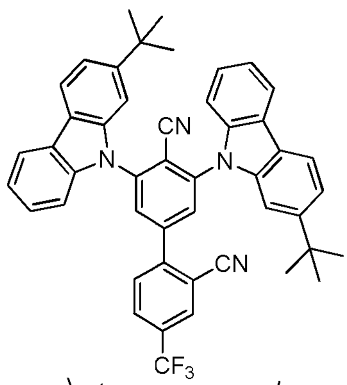

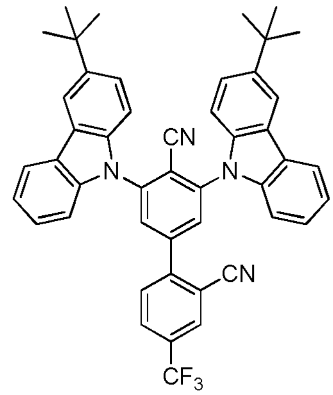

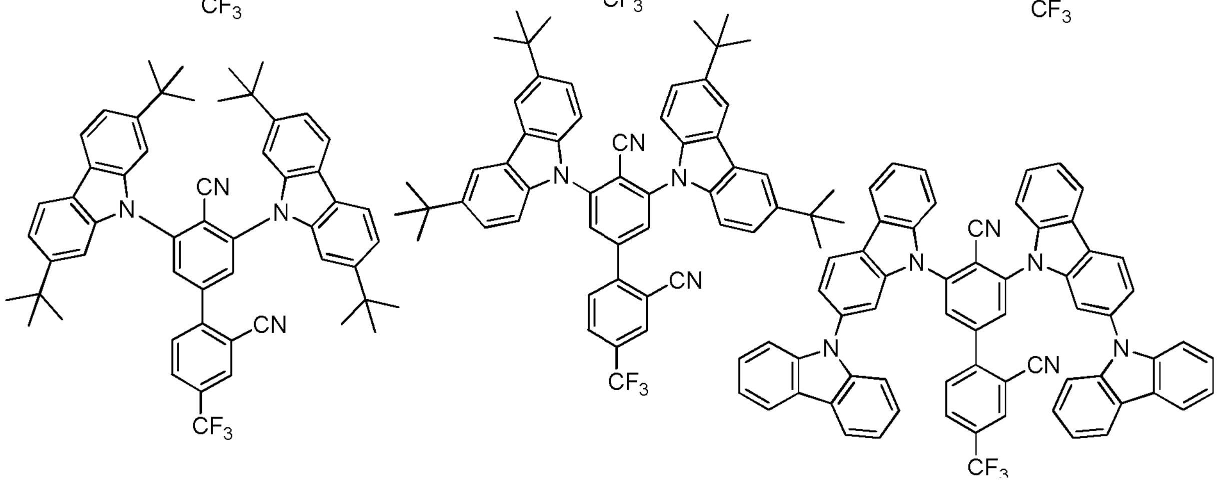

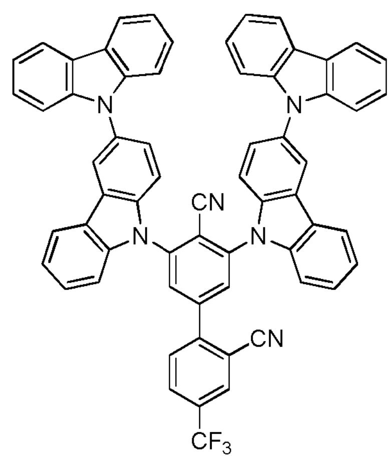

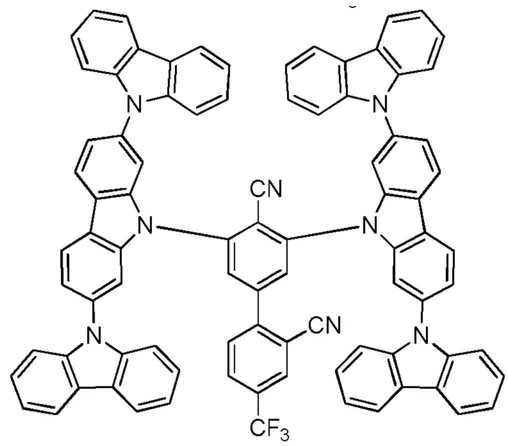

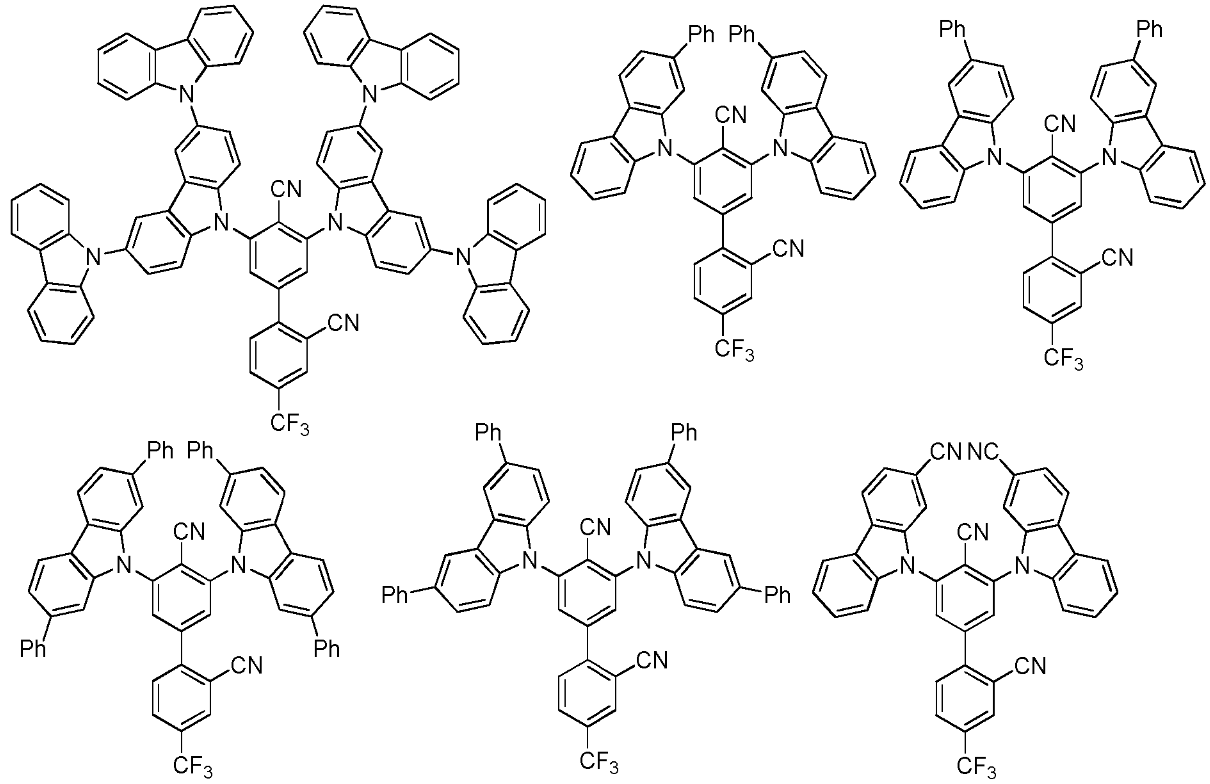

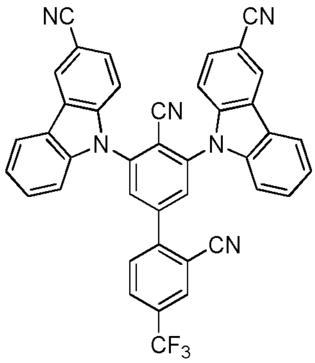

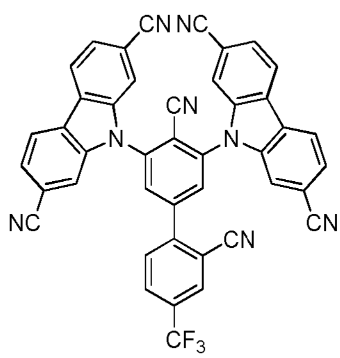

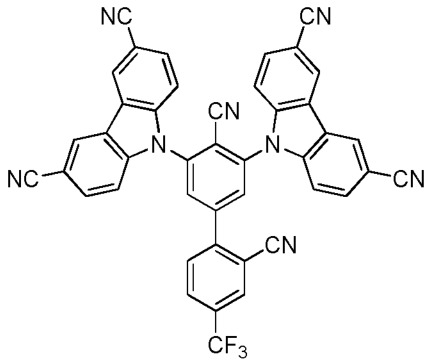

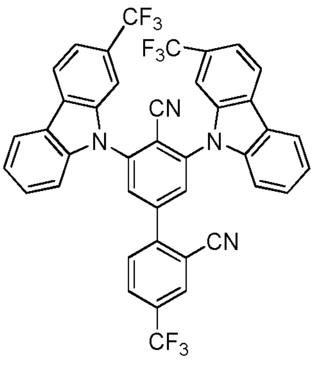

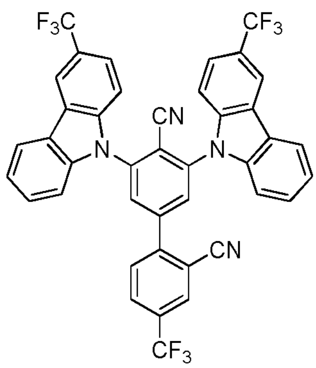

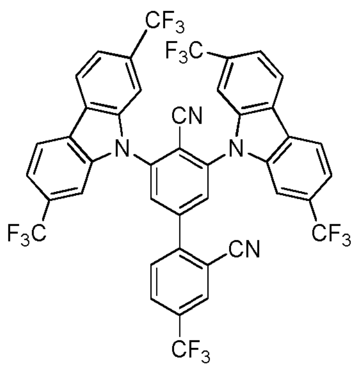

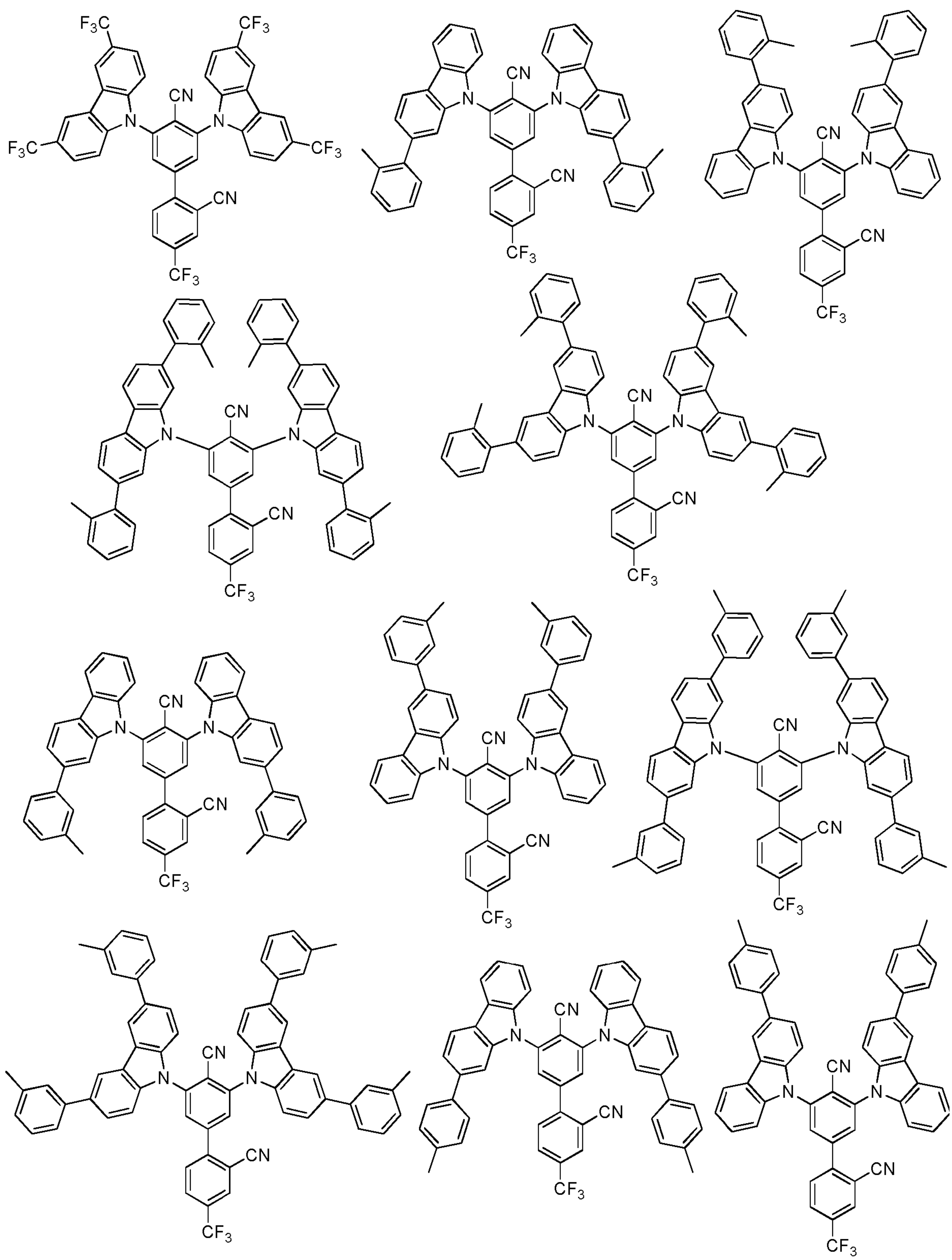

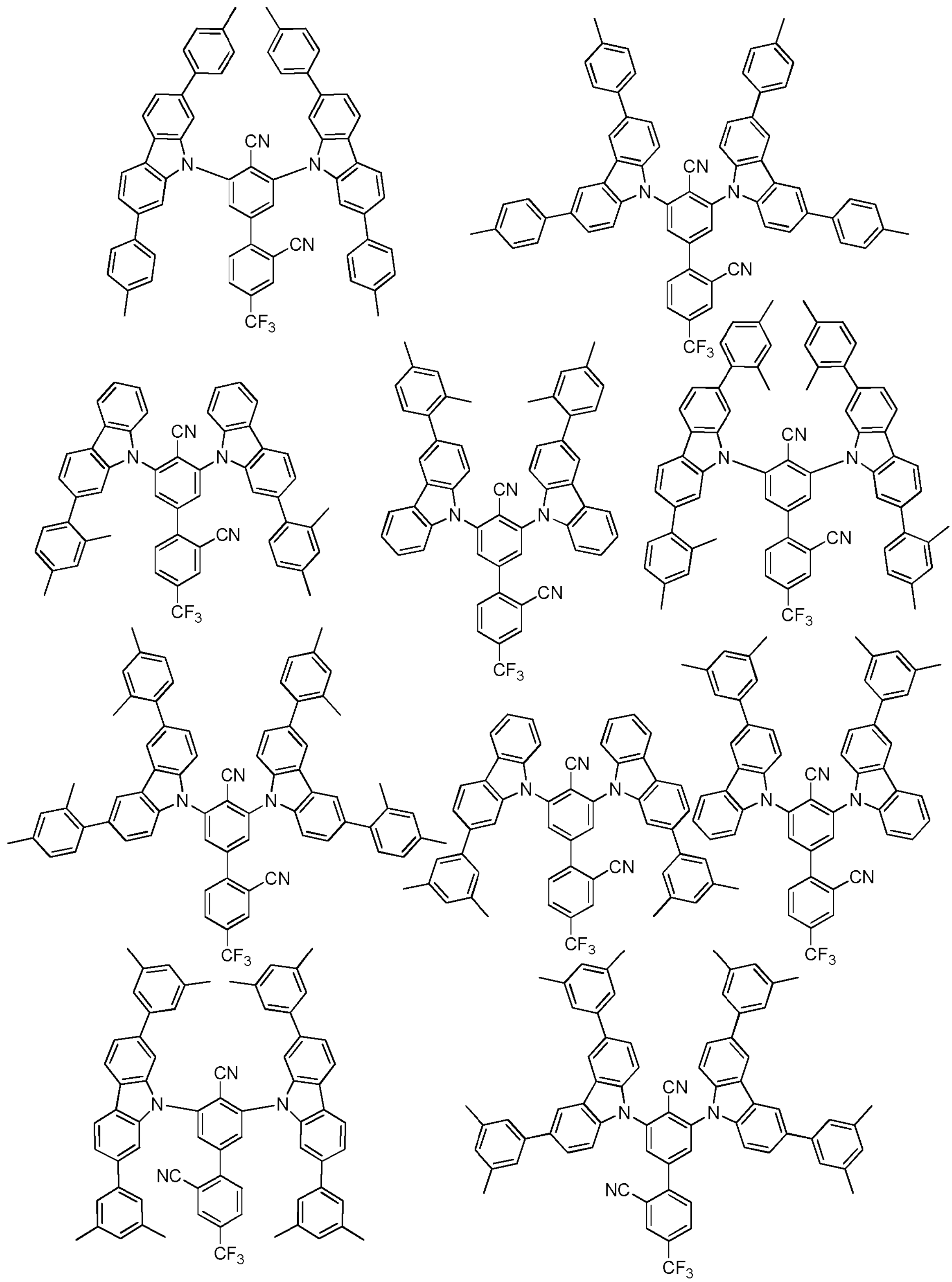

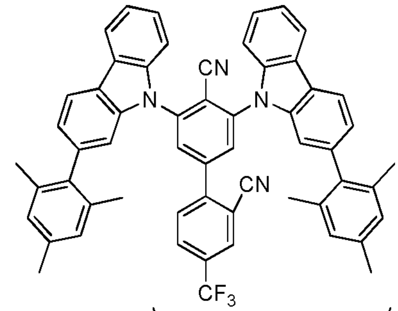
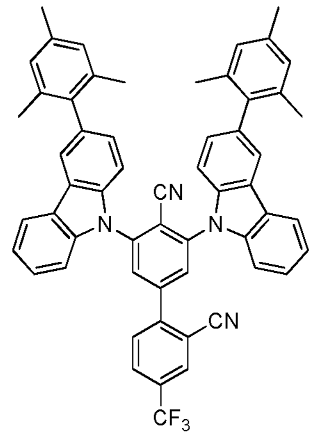
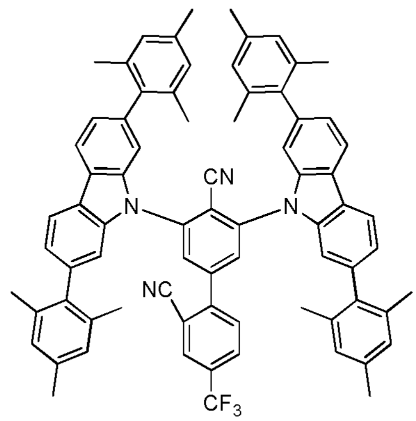
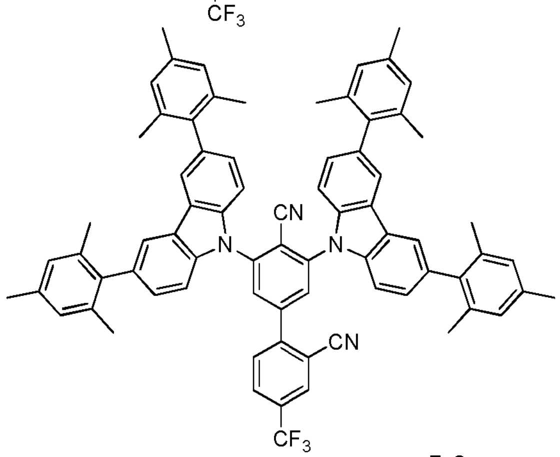
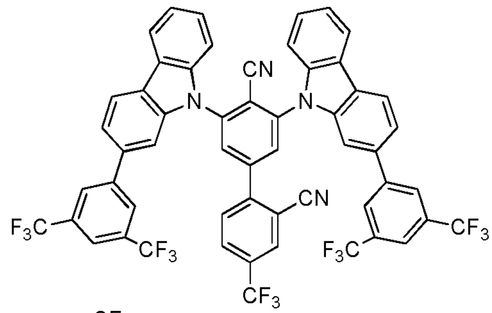
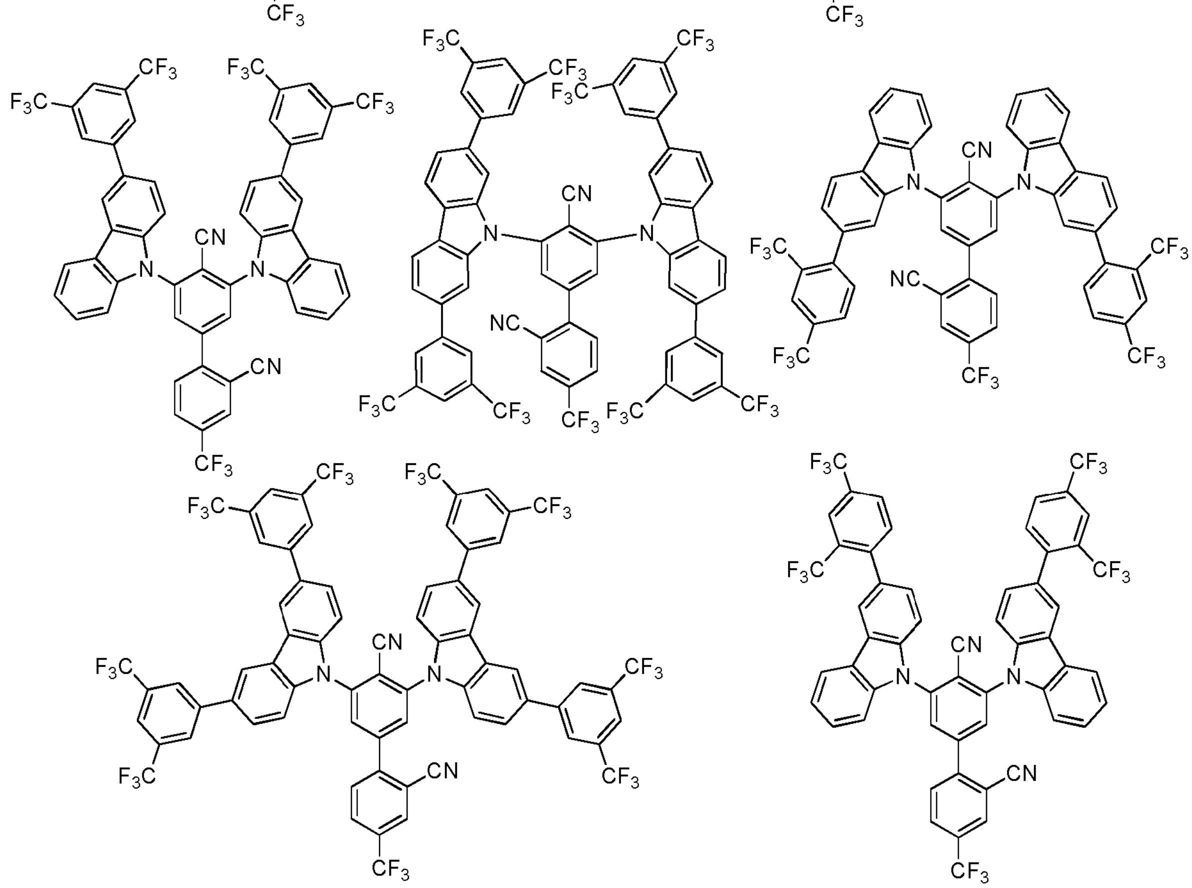

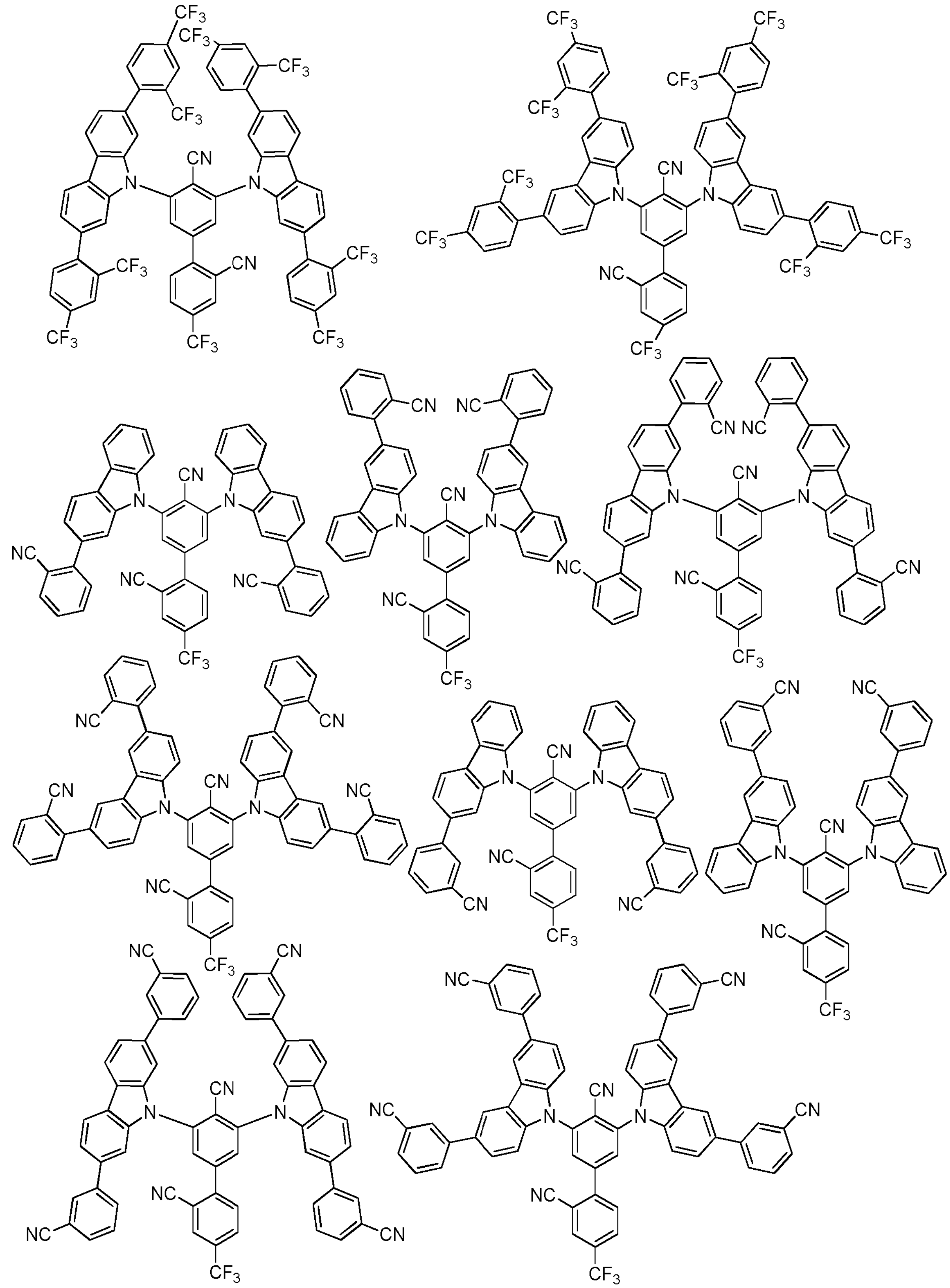

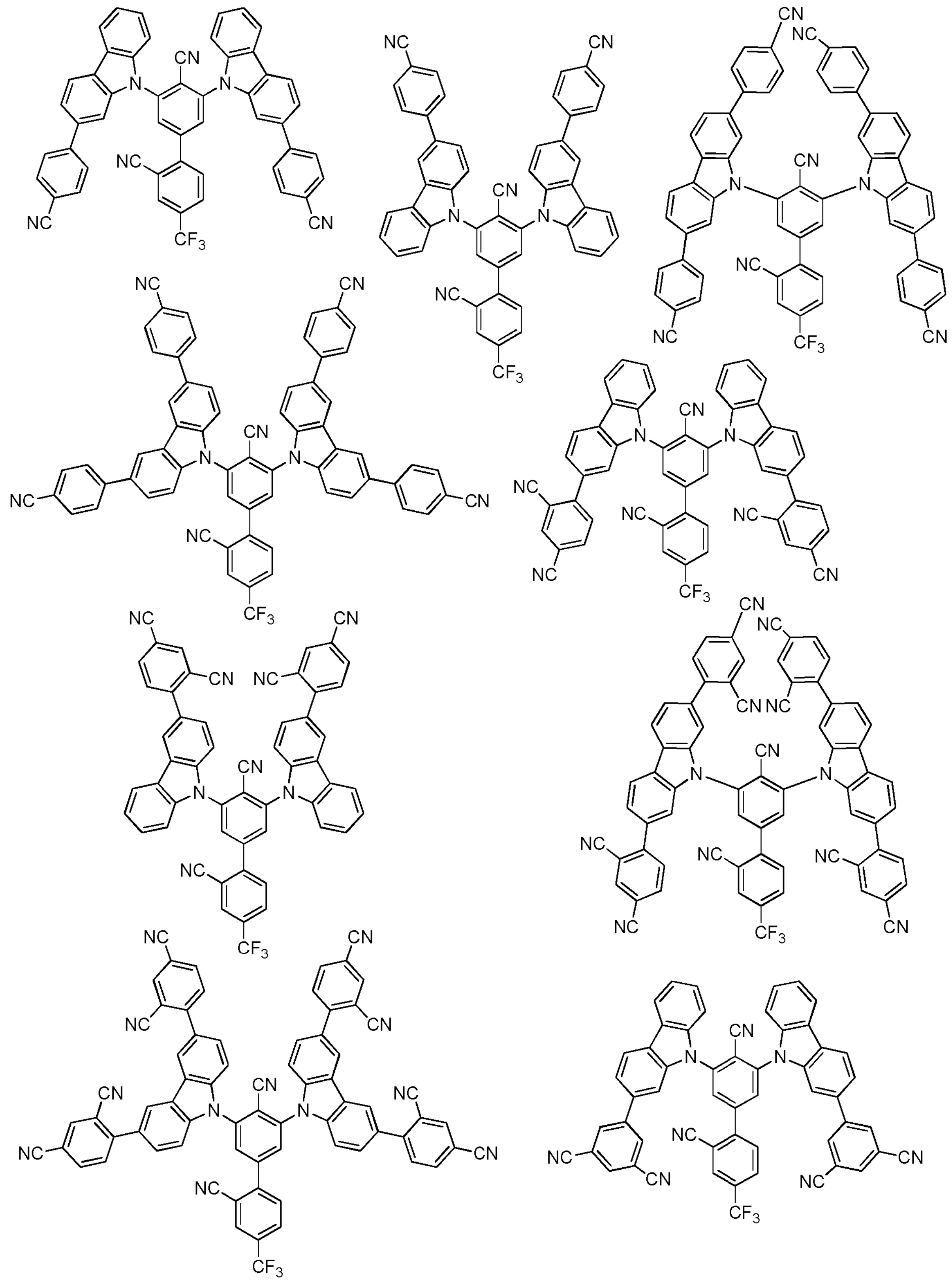

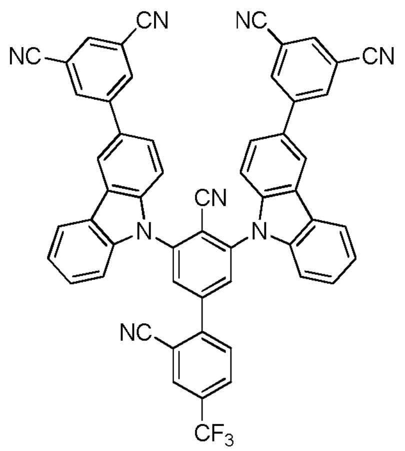

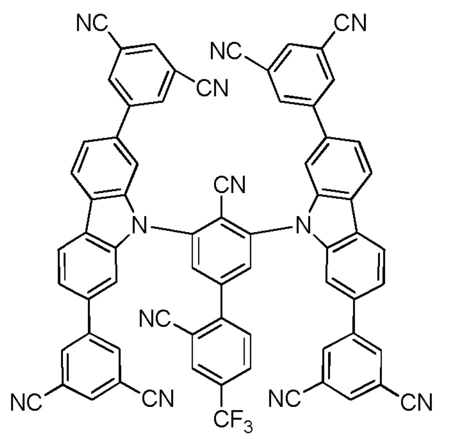

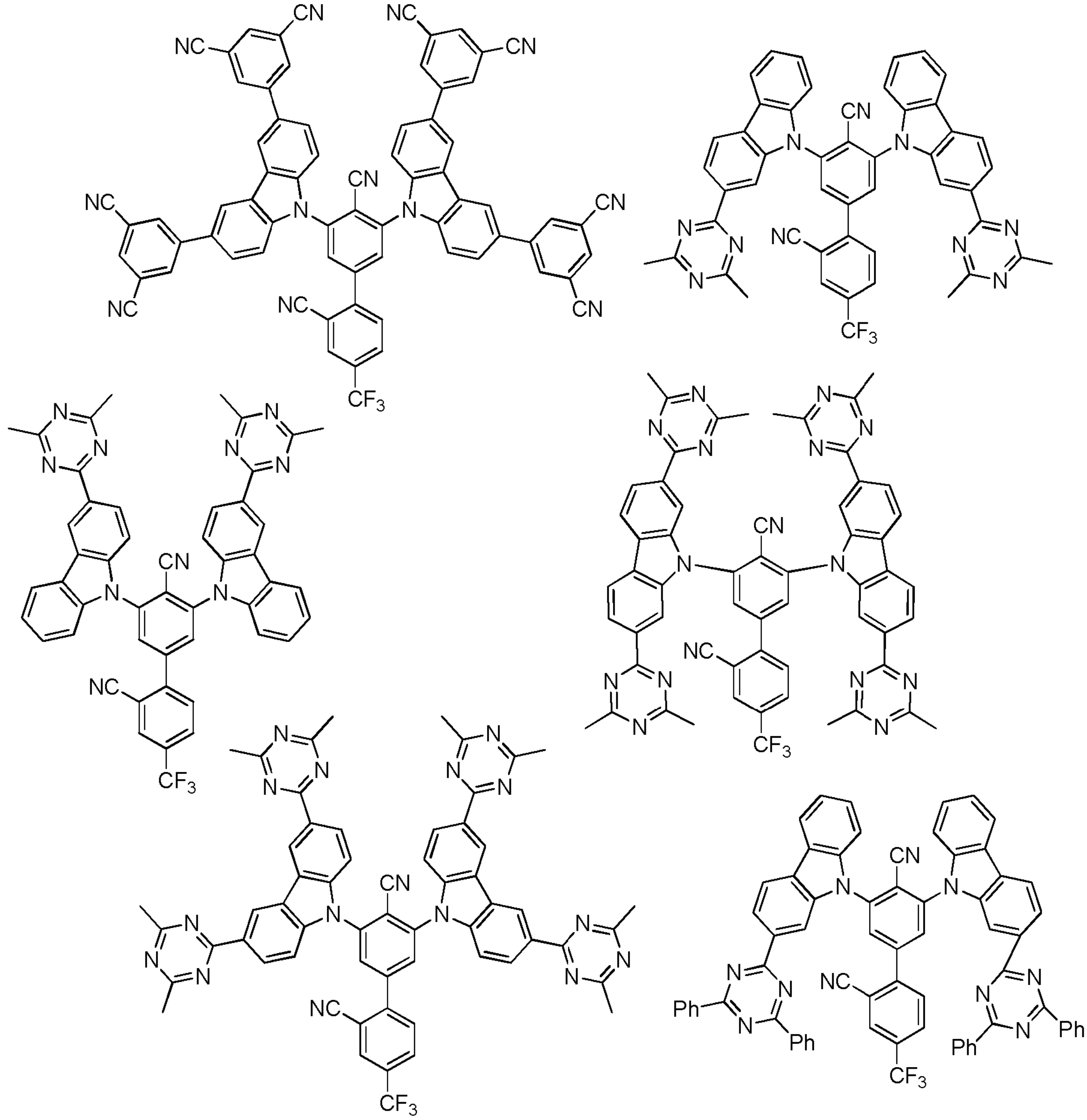

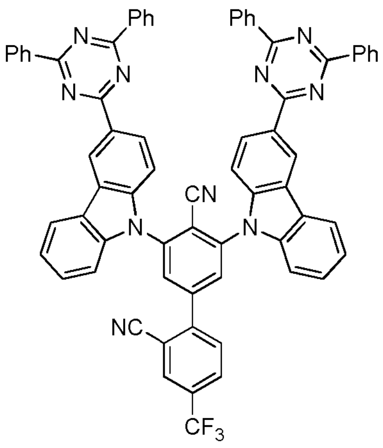

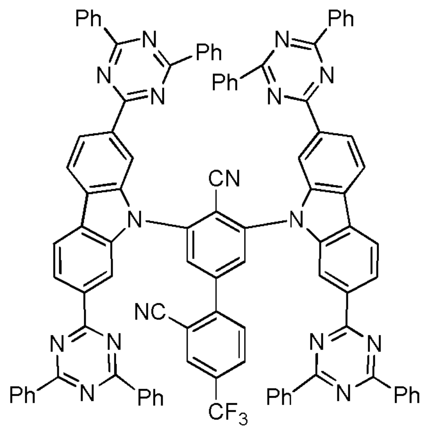

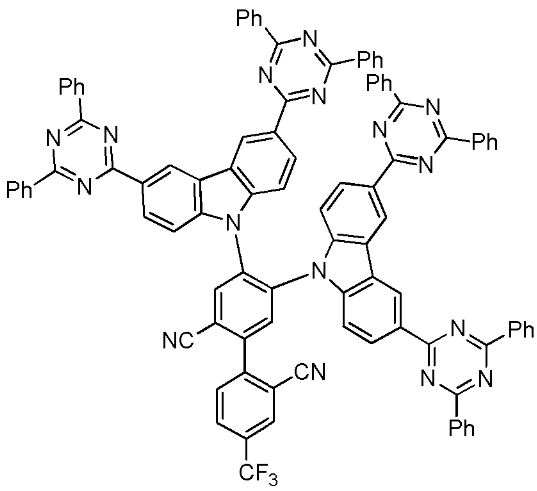

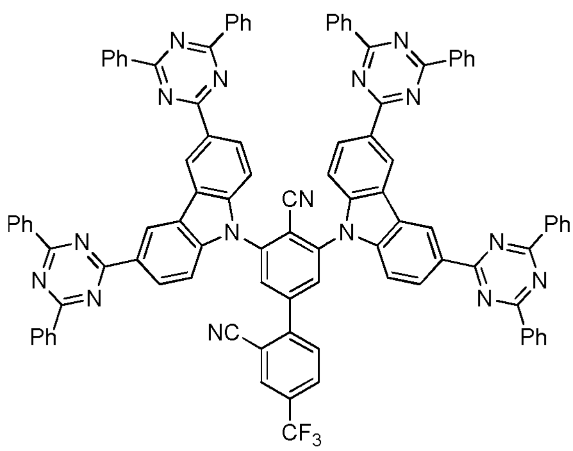

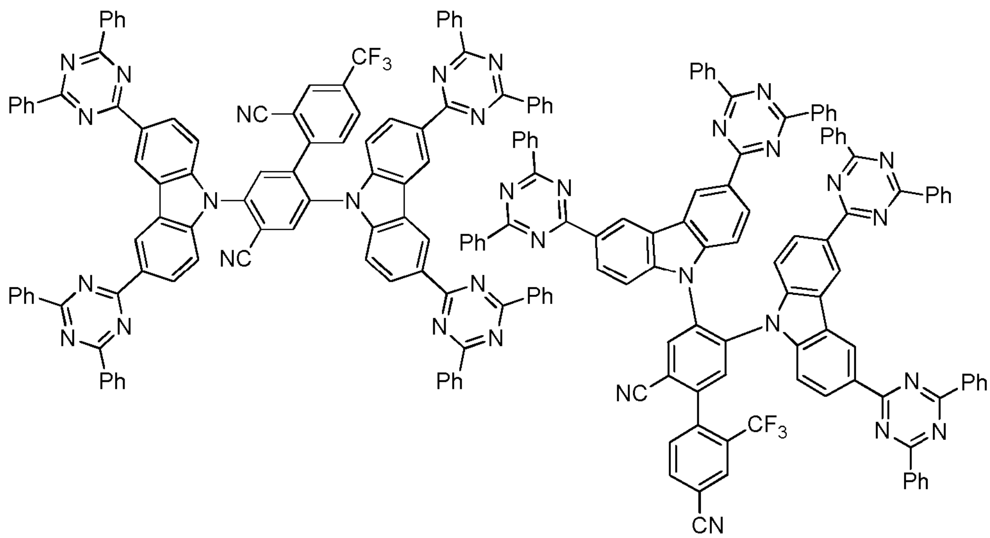

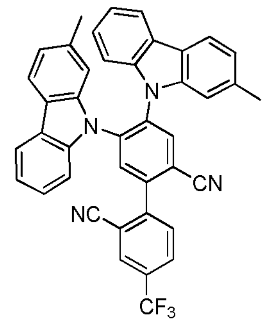
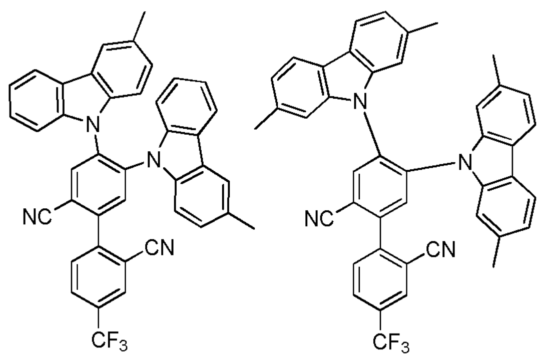
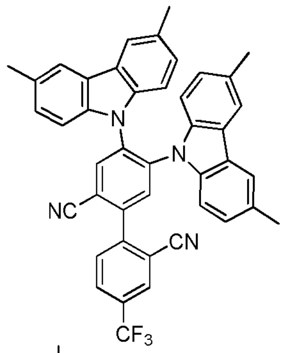

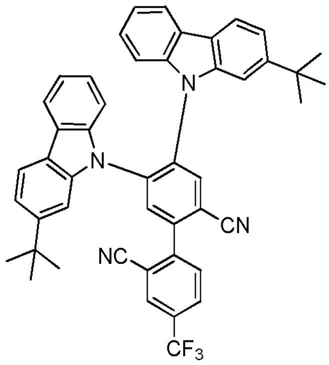
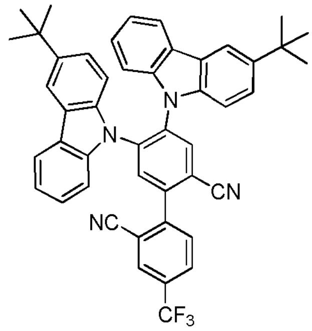
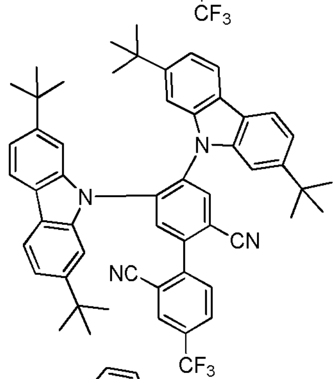

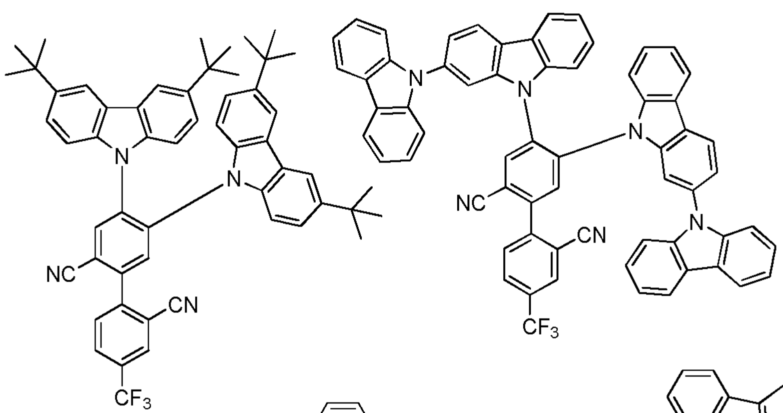
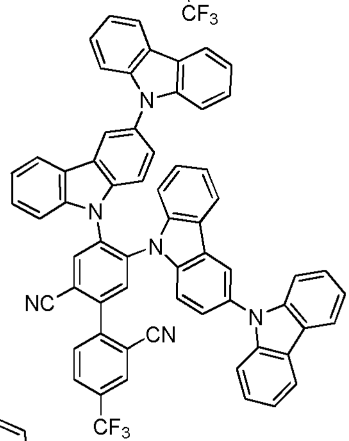

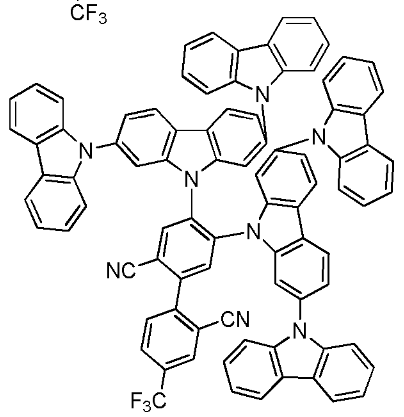
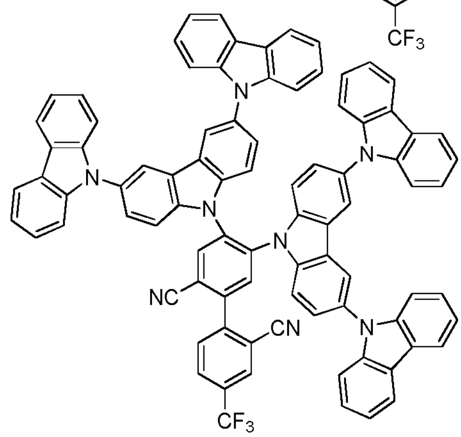

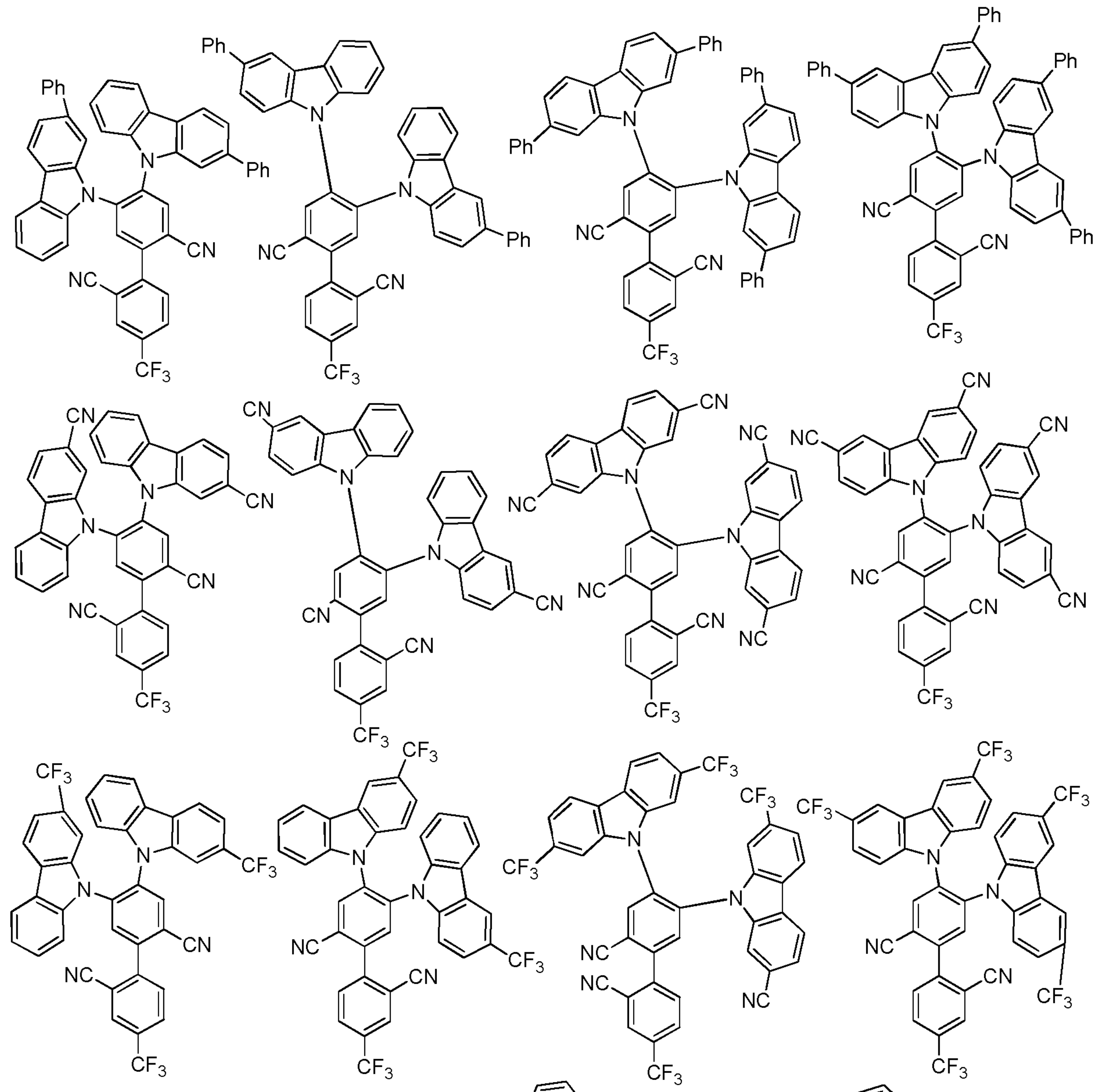

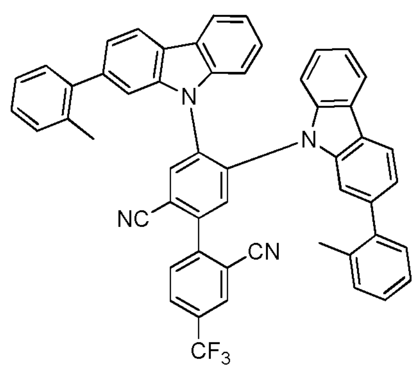

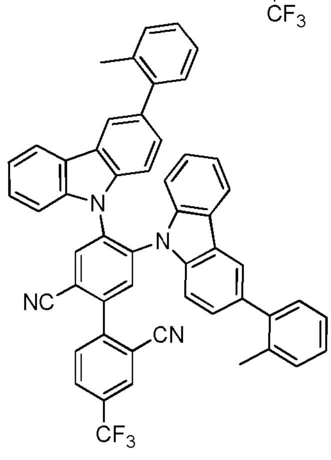

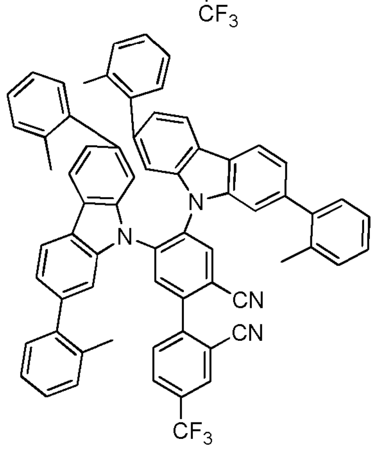

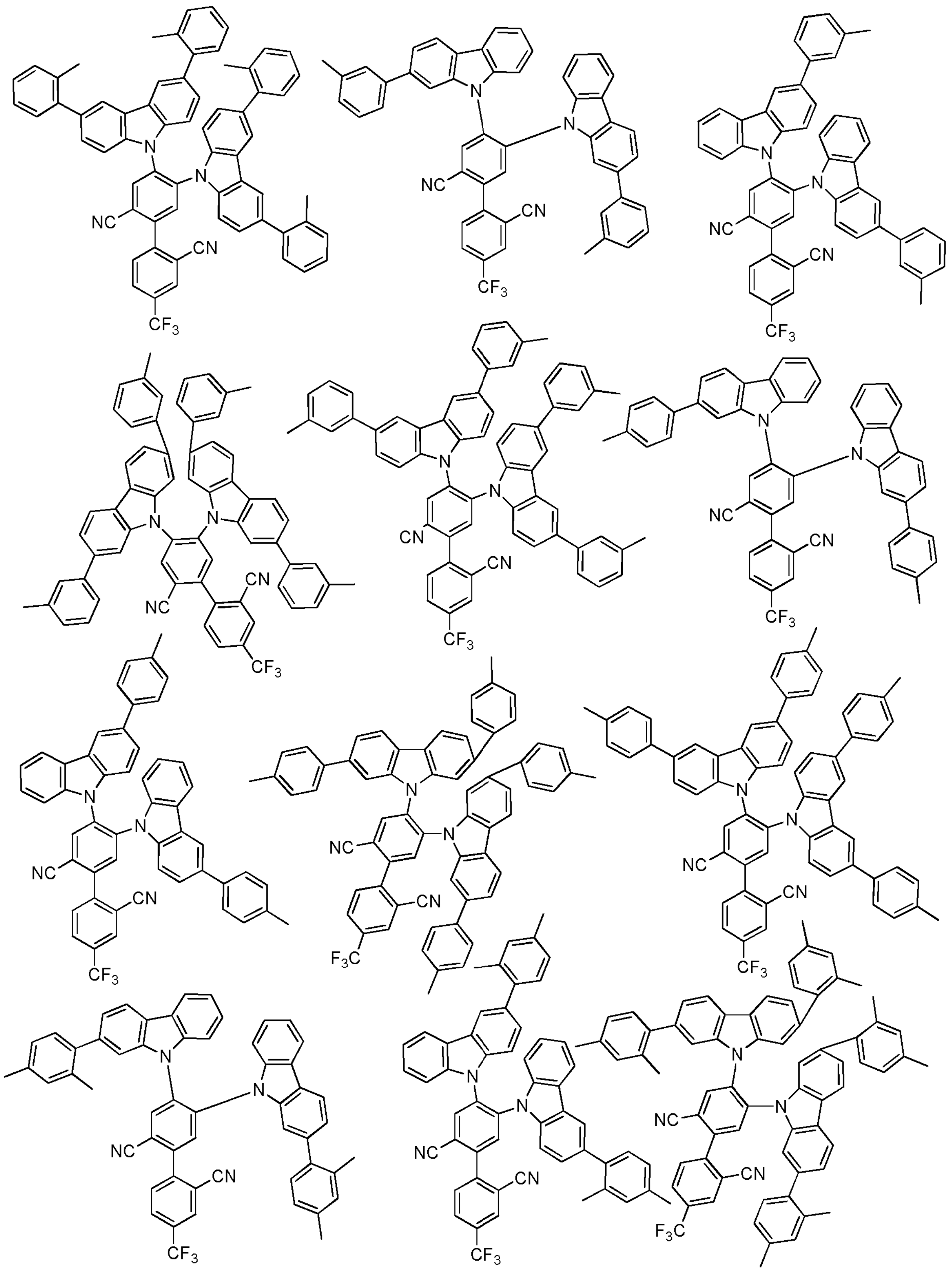

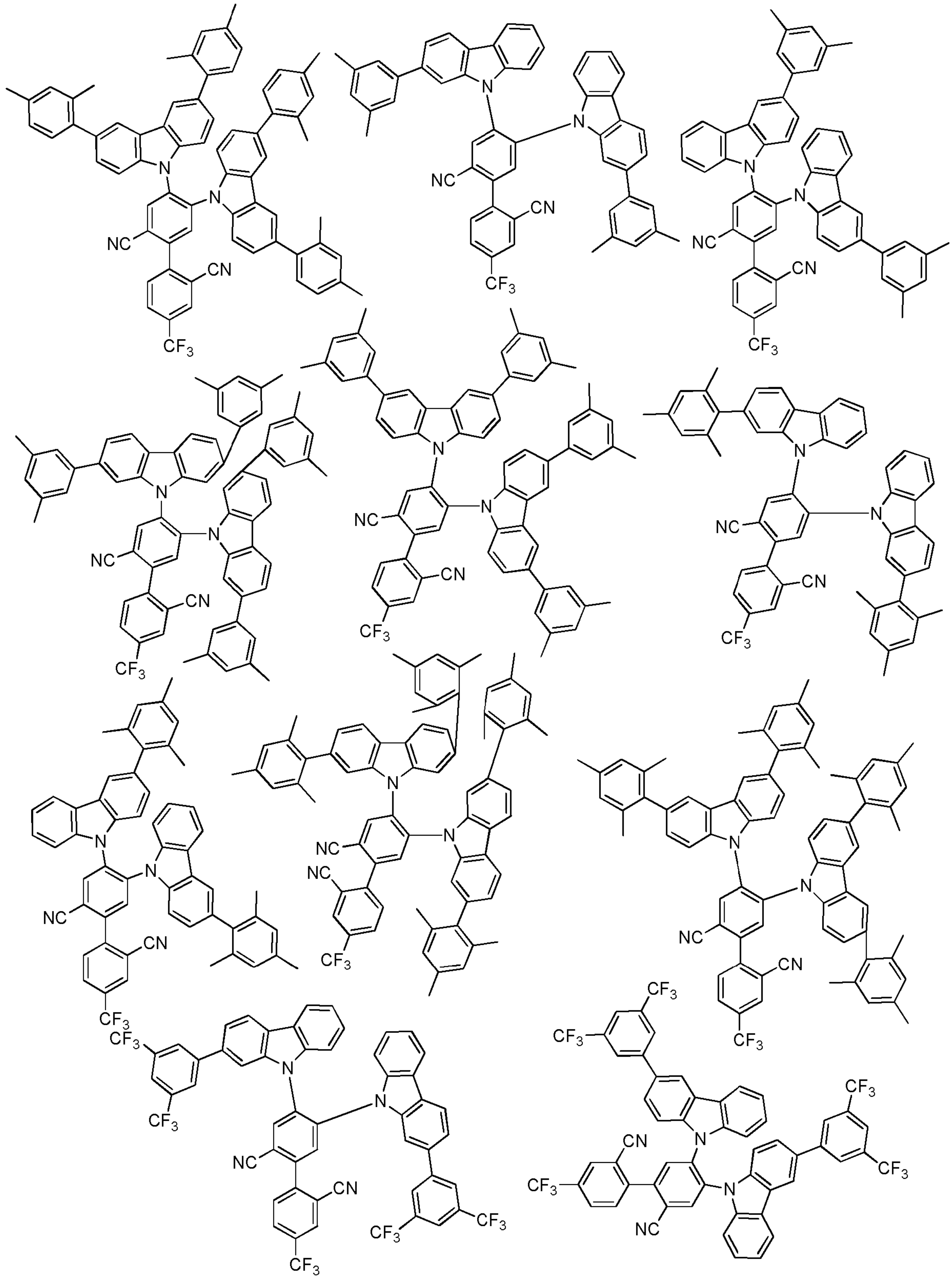

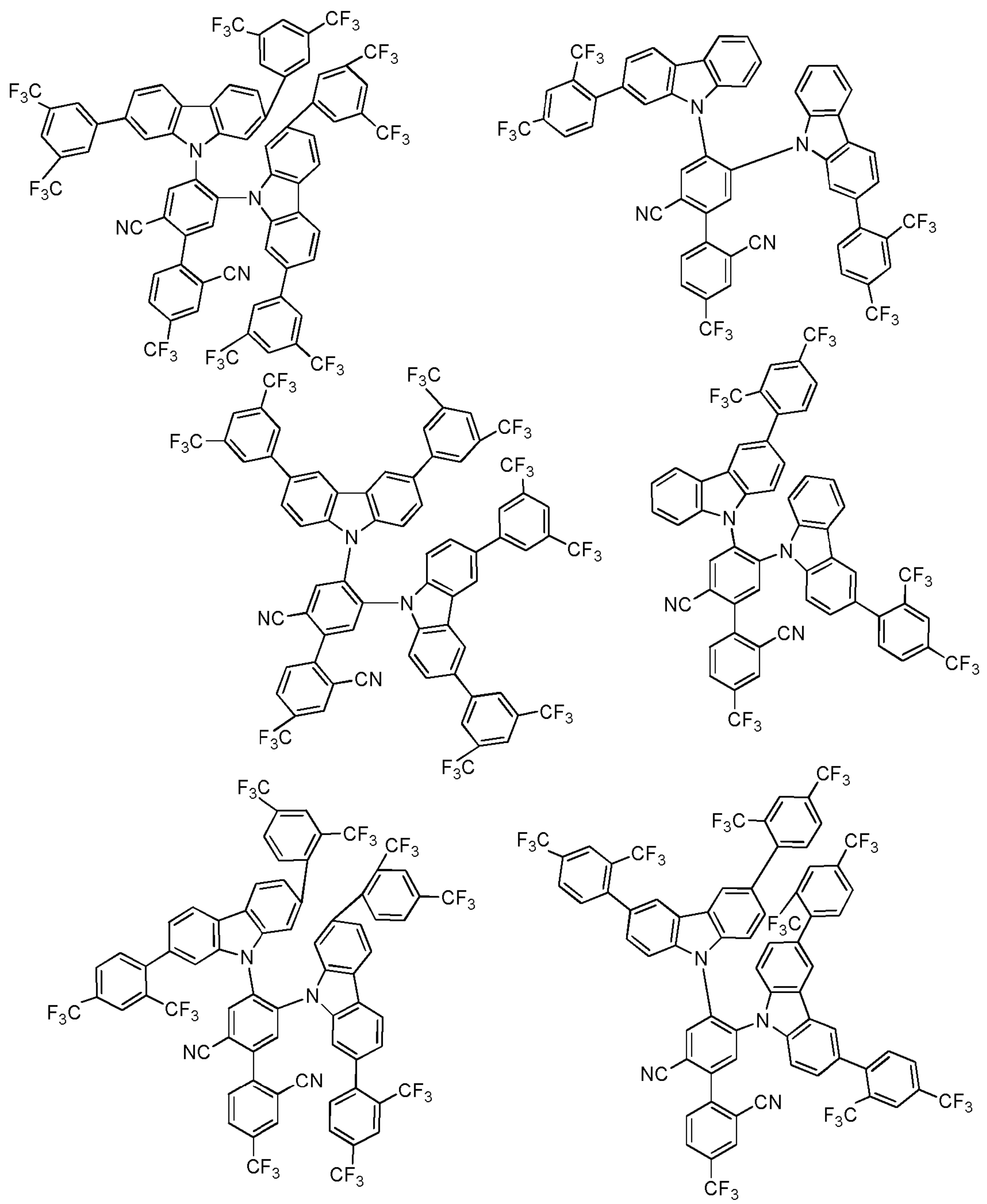

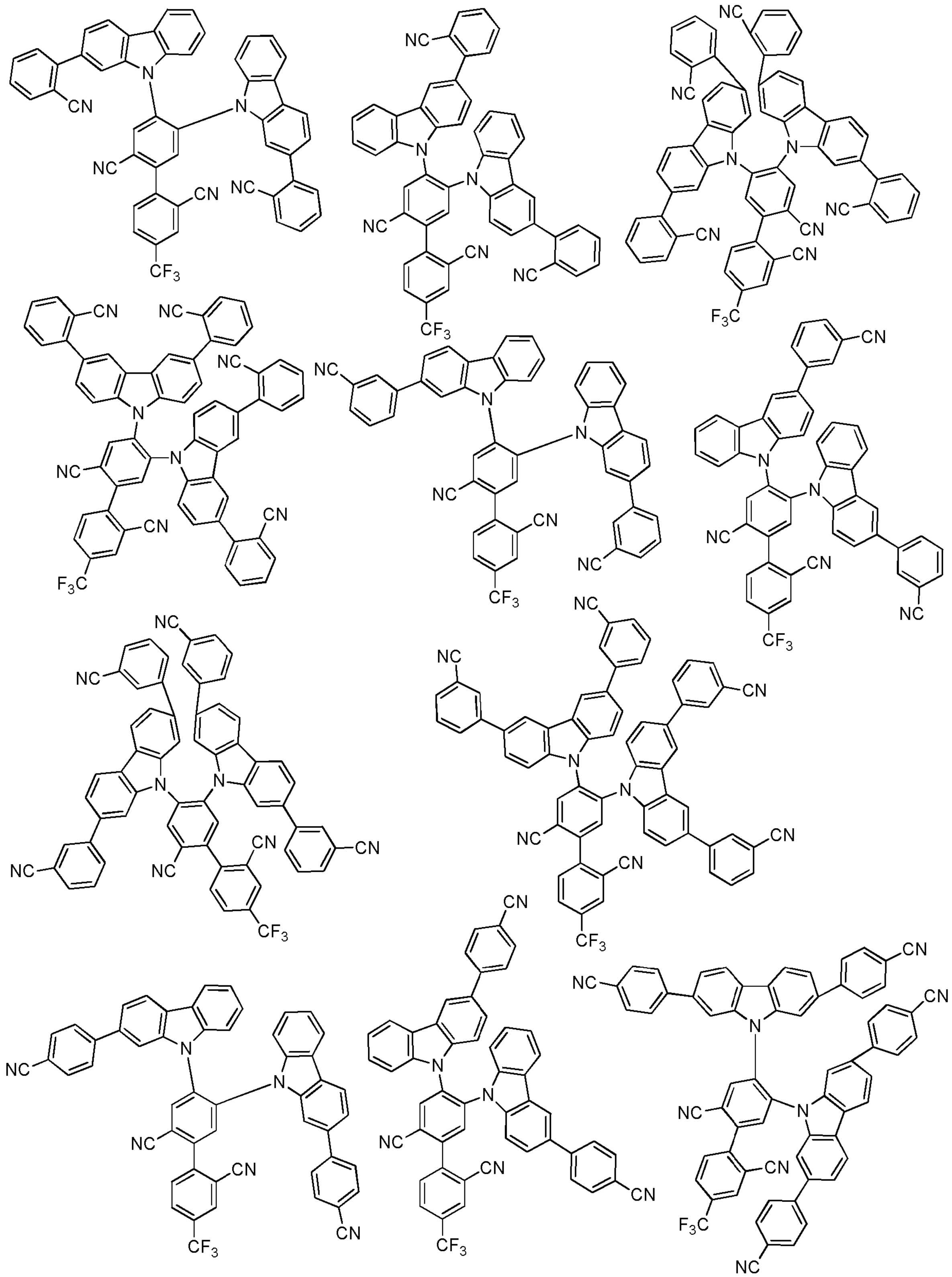

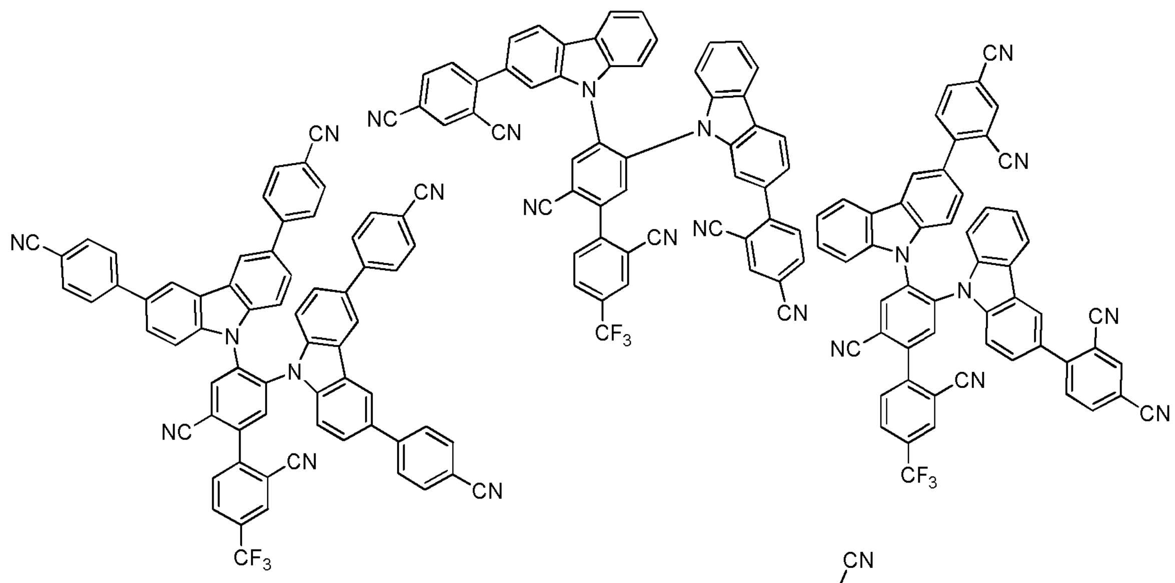

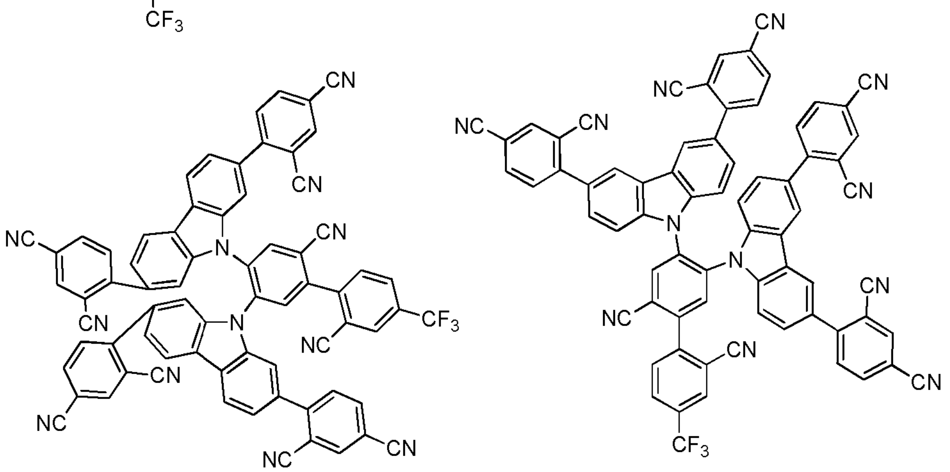

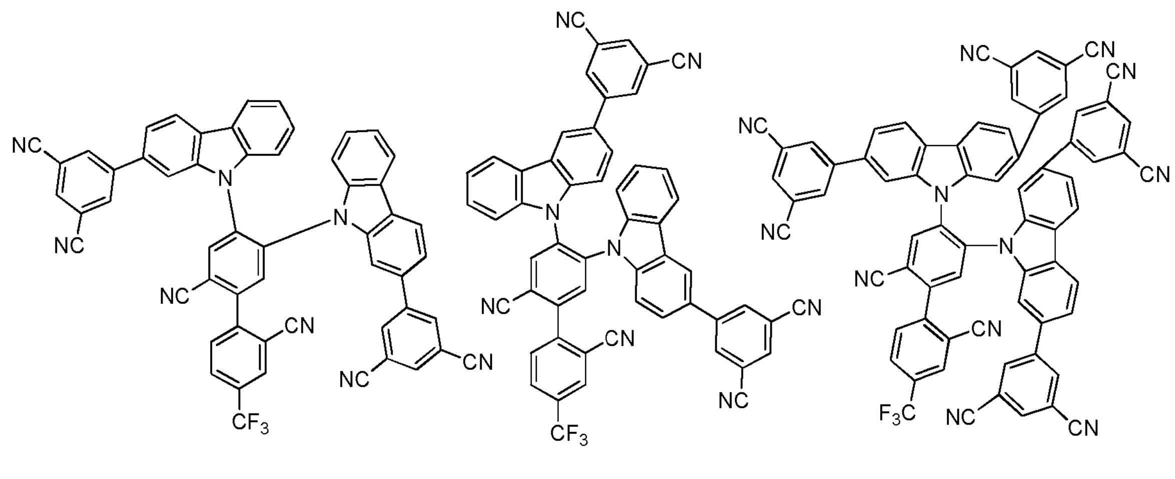

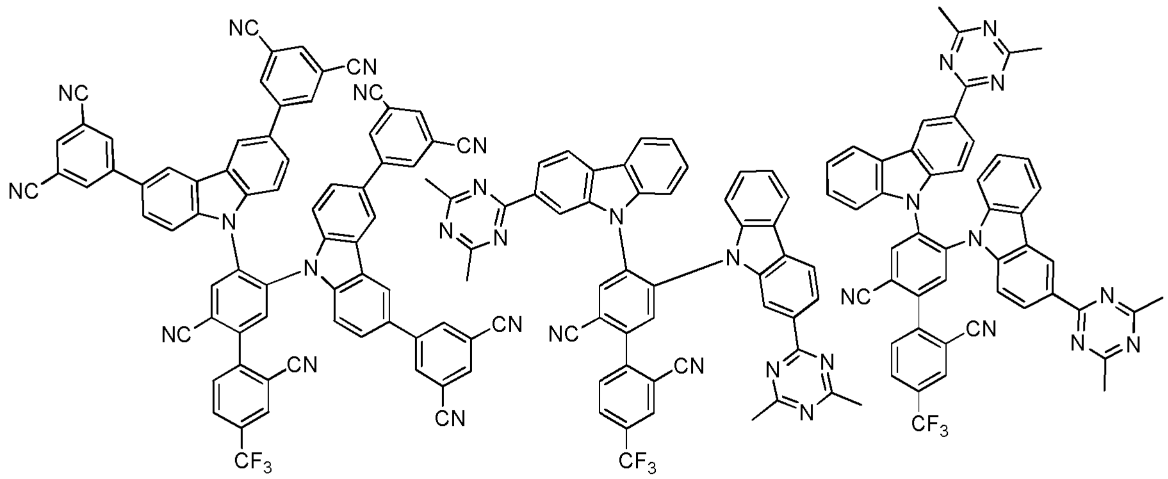

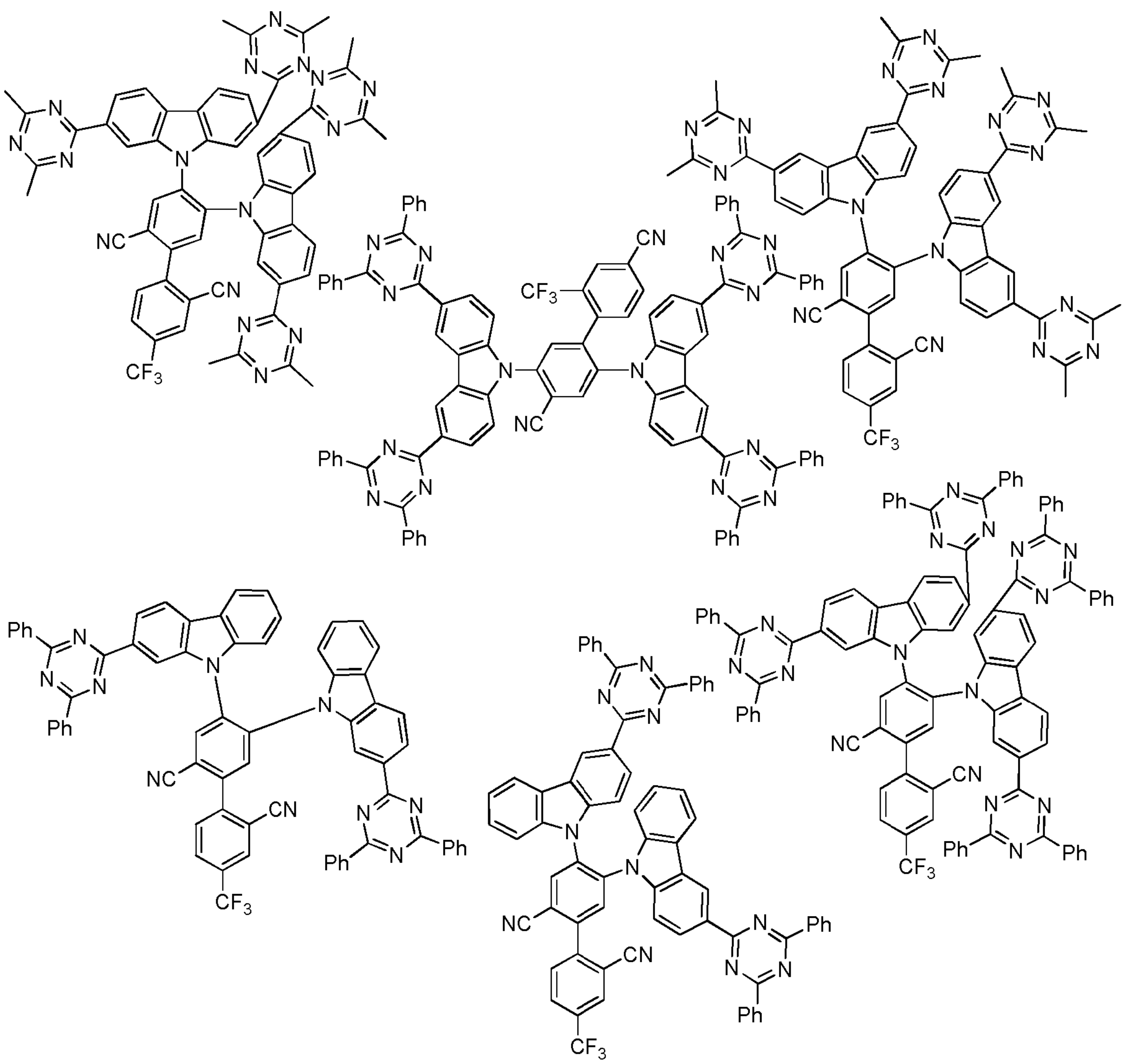

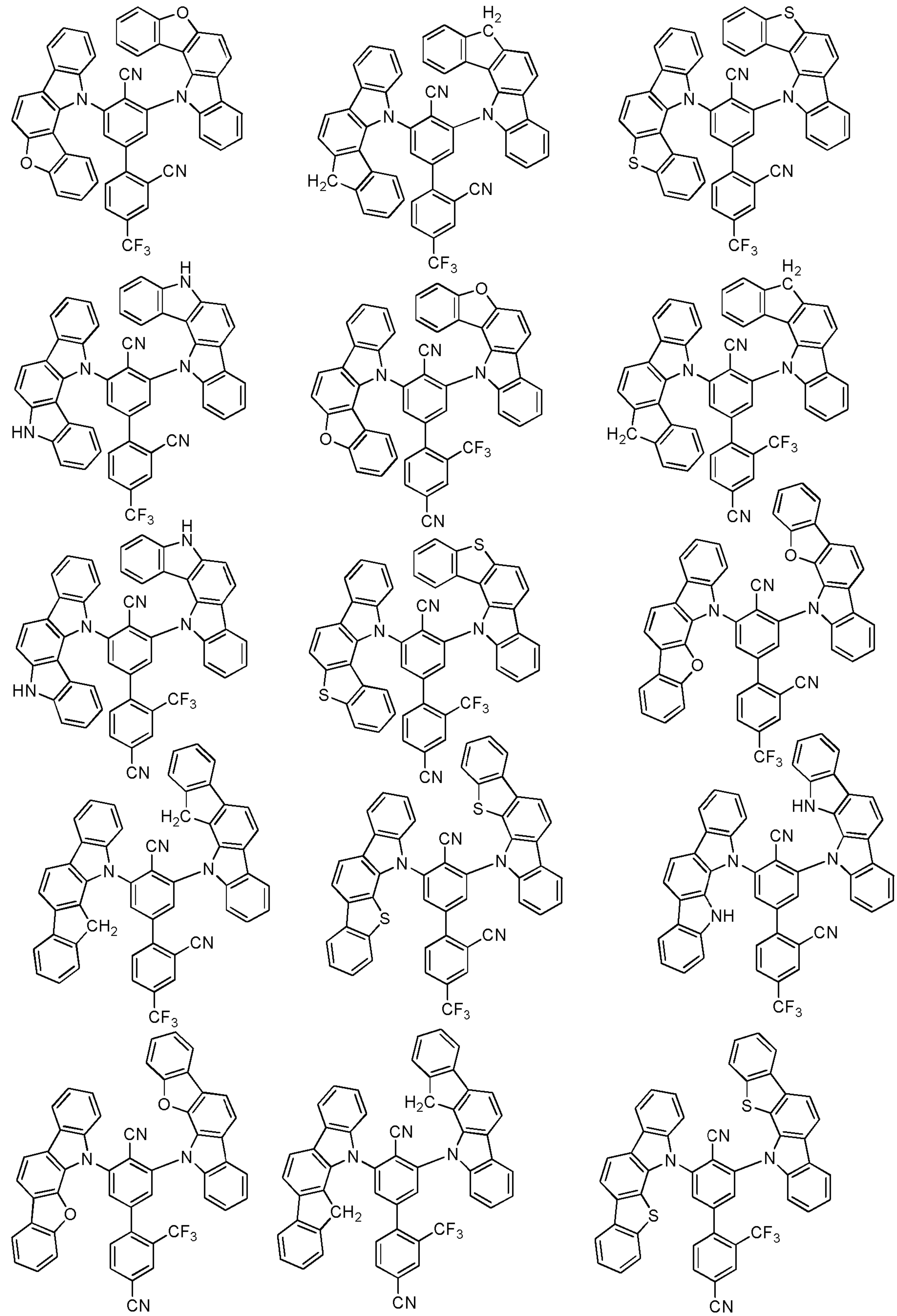

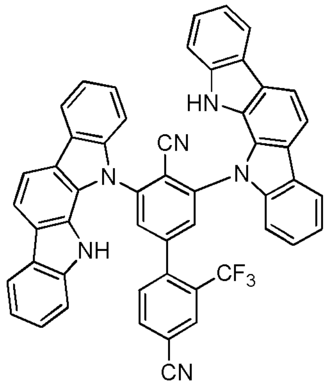
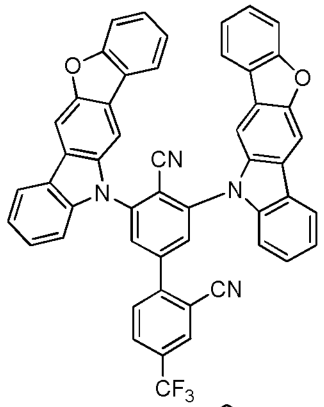
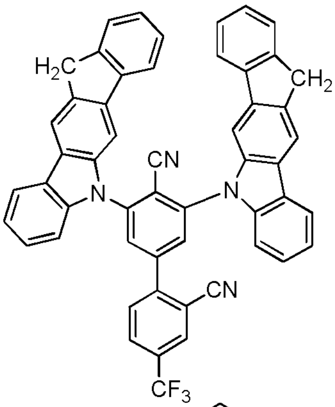
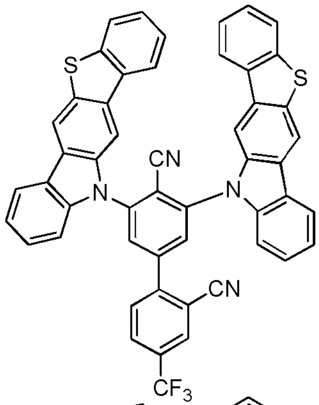
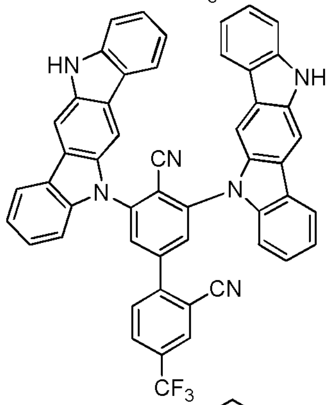
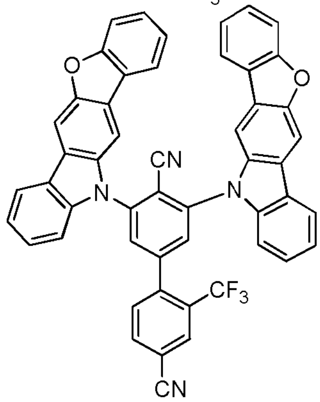
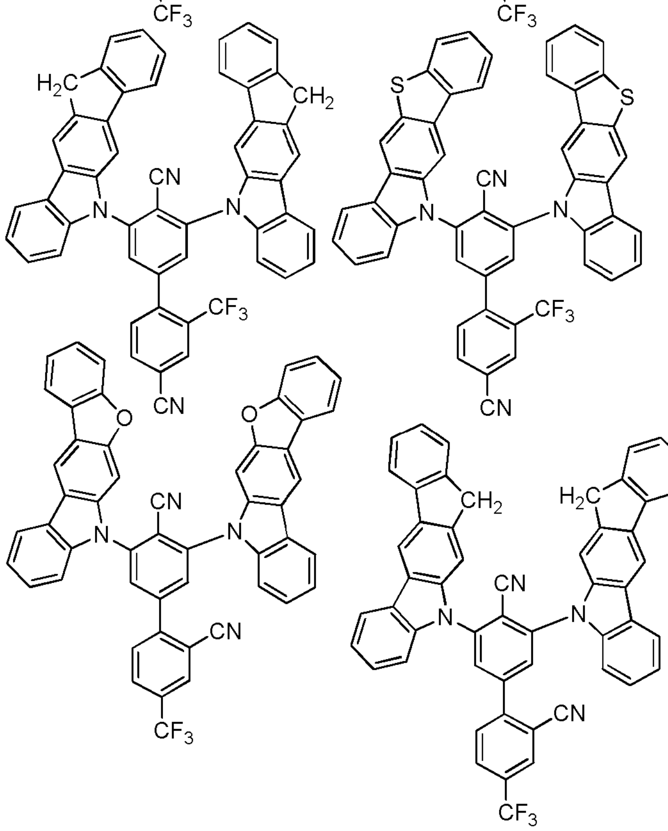
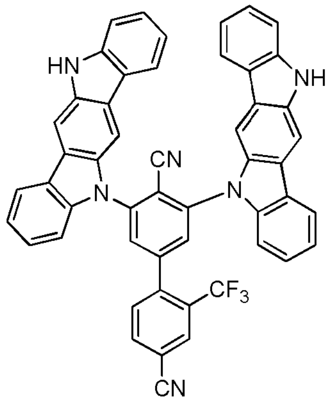
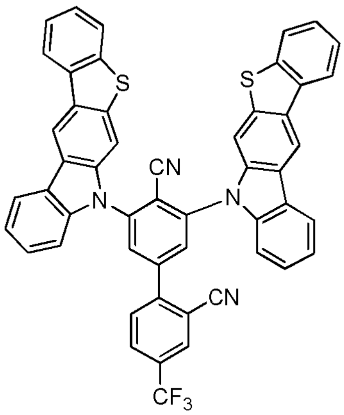

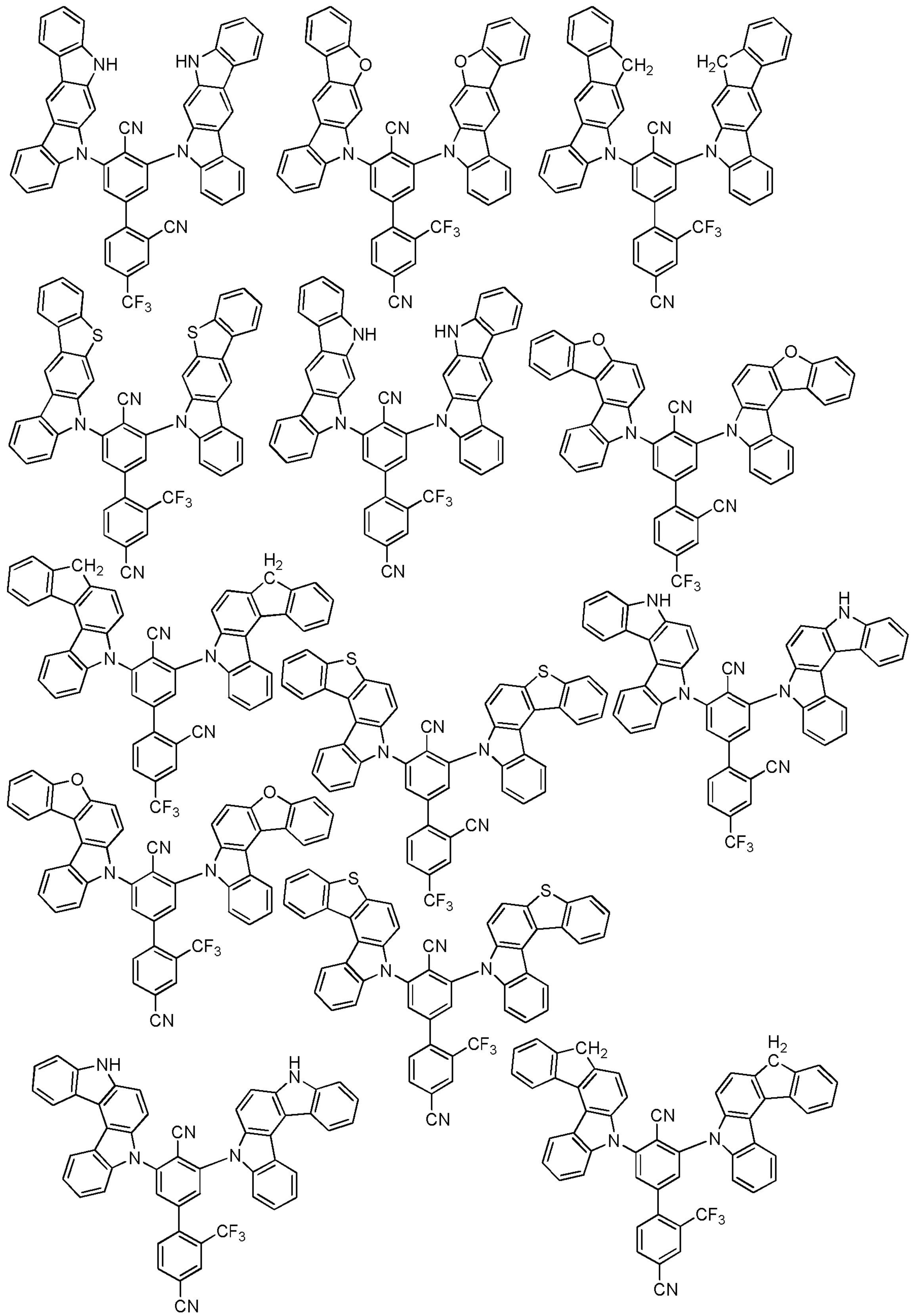

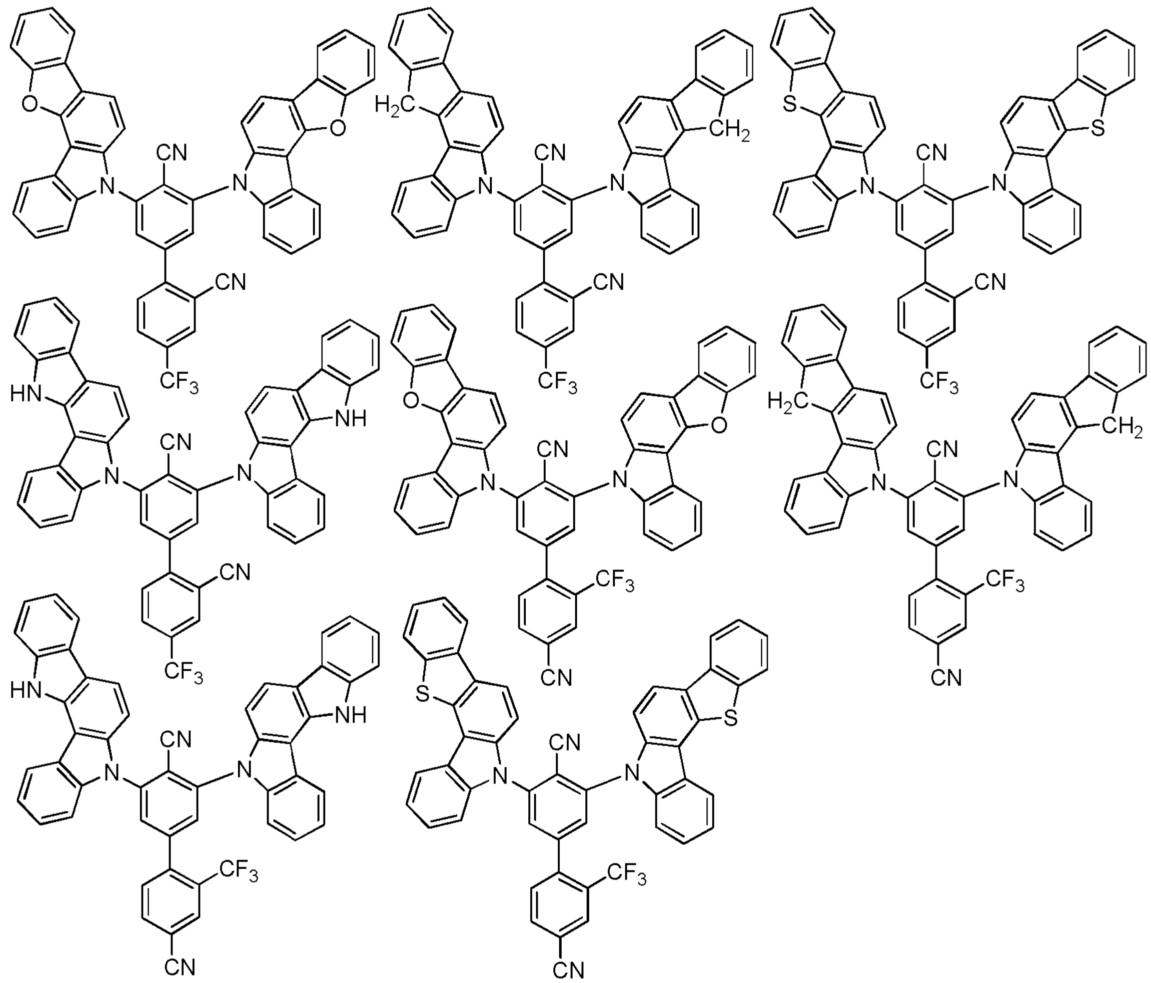

## Figuren

Es zeigen:

| | |
|---|---|
| Figur 1 | Emissionsspektrum von Beispiel **1** (10 % in PMMA). |
| Figur 2 | Emissionsspektrum von Beispiel **2** (10 % in PMMA). |
| Figur 3 | Emissionsspektrum von Beispiel **3** (10 % in PMMA). |
| Figur 4 | Emissionsspektrum von Beispiel **4** (10 % in PMMA). |
| Figur 5 | Emissionsspektrum von Beispiel **5** (10 % in PMMA). |
| Figur 6 | Emissionsspektrum von Beispiel **6** (10 % in PMMA). |
| Figur 7 | Emissionsspektrum von Beispiel **7** (10 % in PMMA). |
| Figur 8 | Emissionsspektrum von Beispiel **8** (10 % in PMMA). |
| Figur 9 | Emissionsspektrum von Beispiel **9** (10 % in PMMA). |
| Figur 10 | Emissionsspektrum von Beispiel **10** (10 % in PMMA). |
| Figur 11 | Emissionsspektrum von Beispiel **11** (10 % in PMMA). |
| Figur 12 | Emissionsspektrum von Beispiel **12** (10 % in PMMA). |

## Patentansprüche

**1.** Organisches Molekül, aufweisend

- eine erste chemische Einheit aufweisend eine oder bestehend aus einer Struktur gemäß Formel I

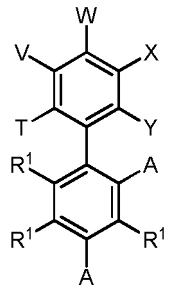

Formel I

und

- zwei zweite chemische Einheiten D, die jeweils bei jedem Auftreten gleich oder verschieden sind, aufweisend eine oder bestehend aus einer Struktur gemäß Formel II,

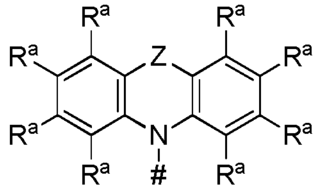

Formel II

wobei die erste chemische Einheit jeweils über eine Einfachbindung mit den zwei zweiten chemischen Einheiten D verknüpft ist;
mit

T ist Anknüpfungspunkt der Einfachbindung zwischen der ersten chemischen Einheit und einer zweiten chemischen Einheit D oder ist H;
V ist Anknüpfungspunkt der Einfachbindung zwischen der ersten chemischen Einheit und einer zweiten chemischen Einheit D oder ist H;
W ist Anknüpfungspunkt der Einfachbindung zwischen der chemischen Einheit und einer zweiten chemischen Einheit D oder ist ausgewählt aus der Gruppe bestehend aus H, CN und $CF_3$;
X ist Anknüpfungspunkt der Einfachbindung zwischen der ersten chemischen Einheit und einer zweiten chemischen Einheit D oder ist ausgewählt aus der Gruppe bestehend aus H, CN und $CF_3$;
Y ist Anknüpfungspunkt der Einfachbindung zwischen der ersten chemischen Einheit und einer zweiten chemischen Einheit D oder ist ausgewählt aus der Gruppe bestehend aus H, CN und $CF_3$;
# kennzeichnet den Anknüpfungspunkt der Einfachbindung zwischen einer zweiten chemischen Einheit D und der ersten chemischen Einheit;
Z ist bei jedem Auftreten gleich oder verschieden eine direkte Bindung oder ist ausgewählt aus der Gruppe bestehend aus $CR^3R^4$, $C=CR^3R^4$, C=O, $C=NR^3$, $NR^3$, O, $SiR^3R^4$, S, S(O) und $S(O)_2$;
A ist bei jedem Auftreten CN oder $CF_3$;
$R^1$ ist bei jedem Auftreten gleich oder verschieden H, Deuterium, eine lineare Alkylgruppe mit 1 bis 5 C-Atomen, wobei jeweils ein oder mehrere H-Atome durch Deuterium ersetzt sein können; eine lineare Alkenyl- oder Alkinylgruppe mit 2 bis 8 C-Atomen, wobei jeweils ein oder mehrere H-Atome durch Deuterium ersetzt sein können; eine verzweigte oder cyclische Alkyl- , Alkenyl- oder Alkinylgruppe mit 3 bis 10 C-Atomen, wobei jeweils ein oder mehrere H-Atome durch Deuterium ersetzt sein können; oder ein aromatisches oder heteroaromatisches Ringsystem mit 5 bis 15 aromatischen Ringatomen, das jeweils mit einem oder mehreren Resten $R^6$ substituiert sein kann;
$R^a$, $R^3$ und $R^4$ ist bei jedem Auftreten gleich oder verschieden H, Deuterium, $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder eine lineare Alkenyl- oder Alkinylgruppe mit 2 bis 40 C-Atomen, die jeweils mit einem oder mehreren Resten $R^5$ substituiert sein kann, wobei eine oder mehrere nicht

benachbarte $CH_2$-Gruppen durch $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^5$ substituiert sein kann, oder eine Aryloxy- oder Heteroaryloxygruppe mit 5 bis 60 aromatischen Ringatomen, die jeweils mit einem oder mehreren Resten $R^5$ substituiert sein kann, oder eine Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann;

$R^5$ ist bei jedem Auftreten gleich oder verschieden H, Deuterium, $N(R^6)_2$, OH, $Si(R^6)_3$, $B(OR^6)_2$, $OSO_2R^6$, $CF_3$, CN, F, Br, I, eine lineare Alkyl-, Alkoxy- oder Thioalkoxygruppe mit 1 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder eine lineare Alkenyl- oder Alkinylgruppe mit 2 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^6$ substituiert sein kann, wobei eine oder mehrere nicht benachbarte $CH_2$-Gruppen durch $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^6$ substituiert sein kann, oder eine Aryloxy- oder Heteroaryloxygruppe mit 5 bis 60 aromatischen Ringatomen, die jeweils mit einem oder mehreren Resten $R^6$ substituiert sein kann, oder eine Diarylaminogruppe, Diheteroarylaminogruppe oder Arylheteroarylaminogruppe mit 10 bis 40 aromatischen Ringatomen, welche jeweils mit einem oder mehreren Resten $R^6$ substituiert sein kann;

$R^6$ ist bei jedem Auftreten gleich oder verschieden H, Deuterium, OH, $CF_3$, CN, F, eine lineare Alkyl-, Alkoxy- oder Thioalkoxygruppe mit 1 bis 5 C-Atomen, wobei jeweils ein oder mehrere H-Atome durch Deuterium, CN, $CF_3$ oder $NO_2$ ersetzt sein können; oder eine lineare Alkenyl- oder Alkinylgruppe mit 2 bis 5 C-Atomen, wobei jeweils ein oder mehrere H-Atome durch Deuterium, CN, $CF_3$ oder $NO_2$ ersetzt sein können; oder eine verzweigte oder cyclische Alkyl- , Alkenyl-, Alkinyl-, Alkoxy- oder Thioalkoxygruppe mit 3 bis 5 C-Atomen, wobei jeweils ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen oder eine Aryloxy- oder Heteroaryloxygruppe mit 5 bis 60 aromatischen Ringatomen oder eine Diarylaminogruppe, Diheteroarylaminogruppe oder Arylheteroarylaminogruppe mit 10 bis 40 aromatischen Ringatomen;

wobei jeder der Reste $R^a$, $R^3$, $R^4$ oder $R^5$ auch mit einem oder mehreren weiteren Resten $R^a$, $R^3$, $R^4$ oder $R^5$ ein mono- oder polycyclisches, aliphatisches, aromatisches und/oder benzoanelliertes Ringsystem bilden kann;

wobei ein A gleich $CF_3$ ist und das andere A gleich CN ist;

wobei genau ein Rest ausgewählt aus der Gruppe bestehend aus W, X und Y gleich CN oder gleich $CF_3$ ist und genau zwei Reste ausgewählt aus der Gruppe bestehend aus T, V, W, X und Y gleich einem Anknüpfungspunkt der Einfachbindung zwischen der ersten chemischen Einheit und einer zweiten chemischen Einheit D sind.

**2.** Organisches Molekül nach Anspruch 1, wobei die erste chemische Einheit eine Struktur der Formel Ia aufweist oder aus einer derartigen Struktur besteht:

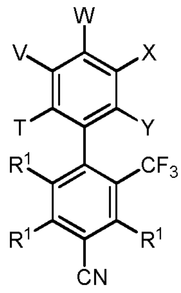

Formel Ia

wobei für $R^1$, T, V, W, X, und Y die in Anspruch 1 genannten Definitionen gelten.

3. Organisches Molekül nach Anspruch 1 oder 2, wobei $R^1$ bei jedem Auftreten gleich oder verschieden H, Methyl oder Phenyl ist.

4. Organisches Molekül nach Anspruch 1 bis 3, wobei W gleich CN ist.

5. Organisches Molekül nach Anspruch 1 bis 4, wobei die zweite chemische Einheit D bei jedem Auftreten gleich oder verschieden eine Struktur der Formel IIa aufweist oder aus einer derartigen Struktur besteht:

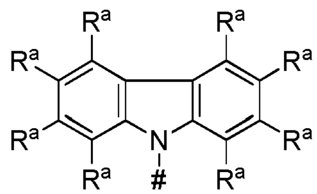

Formel IIa

wobei für # und $R^a$ die in Anspruch 1 genannten Definitionen gelten.

6. Organisches Molekül nach Anspruch 1 bis 5, wobei die zweite chemische Einheit D jeweils eine Struktur der Formel IIb aufweist oder aus einer derartigen Struktur besteht:

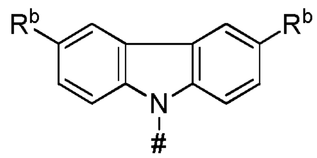

Formel IIb

wobei
$R^b$ bei jedem Auftreten gleich oder verschieden $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder eine lineare Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches

oder heteroaromatisches Ringsystem mit 5 bis 60 aromatischen Ringatomen, das jeweils mit einem oder mehreren Resten $R^5$ substituiert sein kann, oder eine Aryloxy- oder Heteroaryloxygruppe mit 5 bis 60 aromatischen Ringatomen, die jeweils mit einem oder mehreren Resten $R^5$ substituiert sein kann, oder eine Diarylaminogruppe, Diheteroarylaminogruppe oder Arylheteroarylaminogruppe mit 10 bis 40 aromatischen Ringatomen, welche jeweils mit einem oder mehreren Resten $R^5$ substituiert sein kann, ist;
und für # und $R^5$ die in Anspruch 1 genannten Definitionen gelten.

**7.** Organisches Molekül nach Anspruch 1 bis 5, wobei die zweite chemische Einheit D jeweils eine Struktur der Formel IIc aufweist oder aus einer derartigen Struktur besteht:

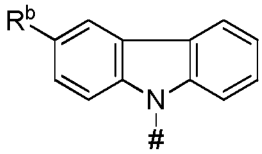

Formel IIc

wobei

$R^b$ bei jedem Auftreten gleich oder verschieden $N(R^5)_2$, OH, $Si(R^5)_3$, $B(OR^5)_2$, $OSO_2R^5$, $CF_3$, CN, F, Br, I, eine lineare Alkyl-, Alkoxy- oder Thioalkoxygruppe mit 1 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder eine lineare Alkenyl- oder Alkinylgruppe mit 2 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^5$ substituiert sein kann, wobei eine oder mehrere nicht benachbarte $CH_2$-Gruppen durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sein können und wobei ein oder mehrere H-Atome durch Deuterium, CN, $CF_3$ oder $NO_2$ ersetzt sein können; oder ein aromatisches oder heteroaromatisches Ringsystem mit 5 bis 60 aromatischen Ringatomen, das jeweils mit einem oder mehreren Resten $R^5$ substituiert sein kann, oder eine Aryloxy- oder Heteroaryloxygruppe mit 5 bis 60 aromatischen Ringatomen, die jeweils mit einem oder mehreren Resten $R^5$ substituiert sein kann, oder eine Diarylaminogruppe, Diheteroarylaminogruppe oder Arylheteroarylaminogruppe mit 10 bis 40 aromatischen Ringatomen, jeweils mit einem oder mehreren Resten $R^5$ substituiert sein kann, ist;
und im Übrigen die in Anspruch 1 genannte Definitionen gelten.

**8.** Organisches Molekül nach Anspruch 6 oder 7, wobei $R^b$ bei jedem Auftreten gleich oder verschieden Me, $^i$Pr, $^t$Bu, CN, $CF_3$ oder Ph, das jeweils mit einem oder mehreren Resten ausgewählt aus Me, $^i$Pr, $^t$Bu, CN, $CF_3$ oder Ph substituiert sein kann, Pyridinyl, das jeweils mit einem oder mehreren Resten ausgewählt aus Me, $^i$Pr, $^t$Bu, CN, $CF_3$ oder Ph substituiert sein kann, Pyrimidinyl, das jeweils mit einem oder mehreren Resten ausgewählt aus Me, $^i$Pr, $^t$Bu, CN, $CF_3$ oder Ph substituiert sein kann, Triazinyl, das jeweils mit einem oder mehreren Resten ausgewählt aus Me, $^i$Pr, $^t$Bu, CN, $CF_3$ oder Ph substituiert sein kann, oder Carbazolyl, das jeweils mit einem oder mehreren Resten ausgewählt aus Me, $^i$Pr, $^t$Bu, CN, $CF_3$ oder Ph substituiert sein kann, ist.

**9.** Verfahren zur Herstellung eines organischen Moleküls nach Anspruch 1 bis 8, wobei ein Halogen-substituiertes Trifluormethylbenzonitril als Edukt eingesetzt wird, wobei der Halogensubstituent in 4-Position ist und der Trifluormethylsubstituent in 3-Position ist oder der Halogensubstituent in 2-Position ist und der Trifluormethylsubstituent in 5-Position ist.

**10.** Verwendung eines organischen Moleküls nach Anspruch 1 bis 8 als lumineszierender Emitter und/oder als Hostmaterial und/oder als Elektronentransportmaterial und/oder als Lochinjektionsmaterial und/oder als Lochblockiermaterial in einer organischen optoelektronischen Vorrichtung.

**11.** Verwendung nach Anspruch 10, wobei die organische optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

**12.** Zusammensetzung, aufweisend oder bestehend aus:

(a) mindestens einem organischen Molekül nach einem der Ansprüche 1 bis 8, insbesondere in Form eines Emitters und/oder Hosts, und
(b) einem oder mehrerer Emitter- und/oder Hostmaterialien, die sich von dem organischen Molekül nach Anspruch 1 bis 8 unterscheiden und
(c) optional einem oder mehreren Farbstoffen und/oder einem oder mehreren Lösungsmitteln.

**13.** Organische optoelektronische Vorrichtung, aufweisend ein organisches Molekül nach Anspruch 1 bis 8 oder eine Zusammensetzung nach Anspruch 12, insbesondere ausgeformt als eine Vorrichtung ausgewählt aus der Gruppe bestehend aus organischer lichtemittierender Diode (OLED), lichtemittierender elektrochemischer Zelle, OLED-Sensor, insbesondere nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

**14.** Organische optoelektronische Vorrichtung nach Anspruch 13, aufweisend

- ein Substrat,
- eine Anode und
- eine Kathode, wobei die Anode oder die Kathode auf das Substrat aufgebracht sind, und
- mindestens eine lichtemittierende Schicht, die zwischen Anode und Kathode angeordnet ist und die ein organisches Molekül nach Anspruch 1 bis 8 oder eine Zusammensetzung nach Anspruch 12 aufweist.

**15.** Verfahren zur Herstellung eines optoelektronischen Bauelements, wobei ein organisches Molekül nach Anspruch 1 bis 8 verwendet wird, insbesondere umfassend die Verarbeitung des organischen Moleküls mittels eines Vakuumverdampfungsverfahrens oder aus einer Lösung.

## Claims

**1.** An organic molecule, comprising:

- a first chemical unit comprising a or consisting of a structure according to Formula I

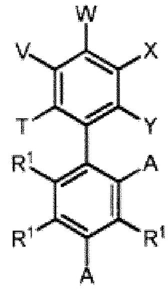

Formula I

and

- two second chemical units D, which are respectively the same or different in each occurrence, having or consisting of a structure according to Formula II,

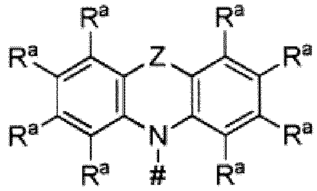

Formula II

wherein, in each case, the first chemical unit is connected to the two second chemical units D via a single bond; with

T is the point of attachment of the single bond between the first chemical unit and a second chemical unit D or is H;
V is the point of attachment of the single bond between the first chemical unit and a second chemical unit D or is H;
W is the point of attachment of the single bond between the chemical unit and a second chemical unit D or is selected from the group consisting of H, CN and $CF_3$;
X is the point of attachment of the single bond between the first chemical unit and a second chemical unit D or is selected from the group consisting of H, CN and $CF_3$;
Y is the point of attachment of the single bond between the first chemical unit and a second chemical unit D or is selected from the group consisting of H, CN and $CF_3$;
# identifies the point of attachment of the single bond between a second chemical unit D and the first chemical unit;
in each occurrence, Z is the same or different, is a direct bond or is selected from the group consisting of $CR^3R^4$, $C=CR^3R^4$, C=O, $C=NR^3$, $NR^3$, O, $SiR^3R^4$, S, S(O) and $S(O)_2$;
in each occurrence, A is CN or $CF_3$;
in each occurrence, $R^1$ is the same or different and is H, deuterium, a linear alkyl group having 1 to 5 C atoms, wherein in each case one or more H atoms can be replaced by deuterium; a linear alkenyl or alkynyl group having 2 to 8 C atoms, wherein in each case one or more H atoms can be replaced by deuterium; a branched or cyclic alkyl, alkenyl or alkynyl group having 3 to 10 C atoms, wherein in each case one or more H atoms can be replaced by deuterium; or an aromatic or heteroaromatic ring system having 5 to 15 aromatic ring atoms, which can in each case be substituted with one or more radicals $R^6$;
in each occurrence, $R^a$, $R^3$ and $R^4$ is the same or different and is H, deuterium, $N(R^5)_2$, OH, $Si(R^5)_3$, $B(OR^5)_2$, $OSO_2R^5$, $CF_3$, CN, F, Br, I, a linear alkyl, alkoxy or thioalkoxy group having 1 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or a linear alkenyl or alkynyl group having 2 to 40 C atoms, which can in each case be substituted with one or more radicals $R^5$, wherein one or more non-adjacent $CH_2$ groups can be replaced by $R^5C=CR^5$, C≡C,

$Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or a branched or cyclic alkyl, alkenyl, alkynyl, alkoxy or thioalkoxy group having 3 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or an aromatic or heteroaromatic ring system having 5 to 60 aromatic ring atoms, which can in each case be substituted with one or more radicals $R^5$, or an aryloxy or heteroaryloxy group having 5 to 60 aromatic ring atoms, which can in each case be substituted with one or more radicals $R^5$, or a diarylamino group, diheteroarylamino group or arylheteroarylamino group having 10 to 40 aromatic ring atoms, which can in each case be substituted with one or more radicals $R^5$;

in each occurrence, $R^5$ is the same or different and is H, deuterium, $N(R^6)_2$, OH, $Si(R^6)_3$, $B(OR^6)_2$, $OSO_2R^6$, $CF_3$, CN, F, Br, I, a linear alkyl, alkoxy or thioalkoxy group having 1 to 40 C atoms, which can in each case be substituted with one or more radicals $R^6$, wherein one or more non-adjacent $CH_2$ groups can be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms can be replaced by deuterium, CN, $CF_3$ or $NO_2$; or a linear alkenyl or alkynyl group having 2 to 40 C atoms, which can in each case be substituted with one or more radicals $R^6$, wherein one or more non-adjacent $CH_2$ groups can be replaced by $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms can be replaced by deuterium, CN, $CF_3$ or $NO_2$; or a branched or cyclic alkyl, alkenyl, alkynyl, alkoxy or thioalkoxy group having 3 to 40 C atoms, which can in each case be substituted with one or more radicals $R^6$, wherein one or more non-adjacent $CH_2$ groups can be replaced by $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$ and wherein one or more H atoms can be replaced by deuterium, CN, $CF_3$ or $NO_2$, or an aromatic or heteroaromatic ring system having 5 to 60 aromatic ring atoms, which can in each case be substituted with one or more radicals $R^6$, or an aryloxy or heteroaryloxy group having 5 to 60 aromatic ring atoms, which can in each case be substituted with one or more radicals $R^6$, or a diarylamino group, diheteroarylamino group or arylheteroarylamino group having 10 to 40 aromatic ring atoms, which can in each case be substituted with one or more radicals $R^6$;

in each occurrence, $R^6$ is the same or different and is H, deuterium, OH, $CF_3$, CN, F, a linear alkyl, alkoxy or thioalkoxy group having 1 to 5 C atoms, wherein one or more H atoms can respectively be replaced by deuterium, CN, $CF_3$ or $NO_2$; or a linear alkenyl or alkynyl group having 2 to 5 C atoms, wherein one or more H atoms can respectively be replaced by deuterium, CN, $CF_3$ or $NO_2$; or a branched or cyclic alkyl, alkenyl, alkynyl, alkoxy or thioalkoxy group having 3 to 5 C atoms, wherein one or more H atoms can respectively be replaced by deuterium, CN, $CF_3$ or $NO_2$; or an aromatic or heteroaromatic ring system having 5 to 60 aromatic ring atoms or an aryloxy or heteroaryloxy group having 5 to 60 aromatic ring atoms or a diarylamino group, diheteroarylamino group or arylheteroarylamino group having 10 to 40 aromatic ring atoms;

wherein each of the radicals $R^a$, $R^3$, $R^4$ or $R^5$ can also form a mono- or polycyclic, aliphatic, aromatic and/or benzoannelated ring system with one or more further radicals $R^a$, $R^3$, $R^4$ or $R^5$;

wherein one A is $CF_3$ and the other A is CN;

wherein exactly one radical selected from the group consisting of W, X and Y is CN or is $CF_3$ and exactly two radicals selected from the group consisting of T, V, W, X and Y are a point of attachment of the single bond between the first chemical unit and a second chemical unit D.

**2.** The organic molecule according to claim 1, wherein the first chemical unit has a structure of Formula Ia or consists of such a structure:

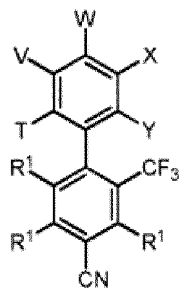

Formula Ia

wherein for $R^1$, T, V, W, X, and Y the meanings given in claim 1 apply.

**3.** The organic molecule according to claim 1 or 2, wherein, in each occurrence, $R^1$ is the same or different and is H, methyl or phenyl.

**4.** The organic molecule according to claim 1 to 3, wherein W is CN.

**5.** The organic molecule according to claim 1 to 4, wherein, in each occurrence, the second chemical unit D is the same or different and has a structure of Formula IIa or consists of such a structure:

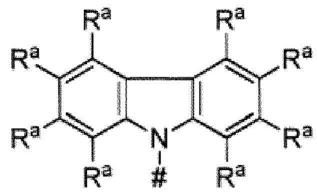

Formula IIa

wherein for # and $R^a$ the meanings given in claim 1 apply.

**6.** The organic molecule according to claim 1 to 5, wherein, in each case, the second chemical unit D has a structure of Formula Iib or consists of such a structure:

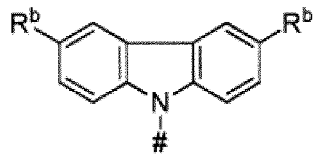

Formula IIb

wherein,

in each occurrence, $R^b$ is the same or different and is $N(R^5)_2$, OH, $Si(R^5)_3$, $B(OR^5)_2$, $OSO_2R^5$, $CF_3$, CN, F, Br, I, a linear alkyl, alkoxy or thioalkoxy group having 1 to 40 C atoms, which can in each case be substituted with one or more radicals $R^5$,

wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or a linear alkenyl or alkynyl group having 2 to 40 C atoms, which can in each case be substituted with one or more radicals $R^5$, wherein one or more non-adjacent $CH_2$ groups can be replaced by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or a branched or cyclic alkyl, alkenyl, alkynyl, alkoxy or thioalkoxy group having 3 to 40 C atoms, which can in each case be substituted with one or more radicals $R^5$, wherein one or more non-adjacent $CH_2$ groups can be replaced

by $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or an aromatic or heteroaromatic ring system having 5 to 60 aromatic ring atoms, which can in each case be substituted with one or more radicals $R^5$, or an aryloxy or heteroaryloxy group having 5 to 60 aromatic ring atoms, which can in each case be substituted with one or more radicals $R^5$, or a diarylamino group, diheteroarylamino group or arylheteroarylamino group having 10 to 40 aromatic ring atoms, which can in each case be substituted with one or more radicals $R^5$;
and for # and $R^5$ the meanings given in claim 1 apply.

**7.** The organic molecule according to claim 1 to 5, wherein, in each case, the second chemical unit D has a structure of Formula IIc or consists of such a structure:

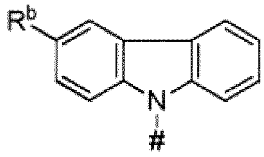

Formula IIc

wherein,

in each occurrence, $R^b$ is the same or different and is $N(R^5)_2$, OH, $Si(R^5)_3$, $B(OR^5)_2$, $OSO_2R^5$, $CF_3$, CN, F, Br, I, a linear alkyl, alkoxy or thioalkoxy group having 1 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or a linear alkenyl or alkynyl group having 2 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or a branched or cyclic alkyl, alkenyl, alkynyl, alkoxy or thioalkoxy group having 3 to 40 C atoms, which can in each case be substituted with one or more radicals $R^5$, wherein one or more non-adjacent $CH_2$ groups can be replaced by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$ and wherein one or more H atoms can be replaced by deuterium, CN, $CF_3$ or $NO_2$; or an aromatic or heteroaromatic ring system having 5 to 60 aromatic ring atoms, which can in each case be substituted with one or more radicals $R^5$, or an aryloxy or heteroaryloxy group having 5 to 60 aromatic ring atoms, which can in each case be substituted with one or more radicals $R^5$, or a diarylamino group, diheteroarylamino group or arylheteroarylamino group having 10 to 40 aromatic ring atoms, which can in each case be substituted with one or more radicals $R^5$;
and in all other respects the meanings given in claim 1 apply.

**8.** The organic molecule according to claim 6 or 7, wherein, in each occurrence, $R^b$ is the same or different and is Me, $^i$Pr, $^t$Bu, CN, $CF_3$ or Ph, which can in each case be substituted with one or more radicals selected from Me, $^i$Pr, $^t$Bu, CN, $CF_3$ or Ph, pyridinyl, which can in each case be substituted with one or more radicals selected from Me, $^i$Pr, $^t$Bu, CN, $CF_3$ or Ph, pyrimidinyl, which can in each case be substituted with one or more radicals selected from Me, $^i$Pr, $^t$Bu, CN, $CF_3$ or Ph, triazinyl, which can in each case be substituted with one or more radicals selected from Me, $^i$Pr, $^t$Bu, CN, $CF_3$ or Ph, or carbazolyl, which can in each case be substituted with one or more radicals selected from Me, $^i$Pr, $^t$Bu, CN, $CF_3$ or Ph.

**9.** A method for producing an organic molecule according to claim 1 to 8, wherein a halogen-substituted trifluoromethylbenzonitrile is used as starting material, wherein the halogen substituent is in 4-position and the trifluoromethyl substituent is in 3-position or the halogen substituent is in 2-position and the trifluoromethyl substituent is in 5-position.

**10.** Use of an organic molecule according to claim 1 to 8 as luminescent emitter and/or as host material and/or as electron transport material and/or as hole injection material and/or as hole blocking material in an organic optoelectronic device.

**11.** Use according to claim 10, wherein the organic optoelectronic device is selected from the group consisting of:

• organic light emitting diodes (OLEDs),
• light emitting electrochemical cells,
• OLED sensors, particularly in gas and vapor sensors that are not hermetically shielded from the outside,
• organic diodes
• organic solar cells,
• organic transistors,
• organic field-effect transistors
• organic lasers and
• down-conversion elements.

**12.** A composition, comprising or consisting of:

a) at least one organic molecule according to one of claims 1 to 8, in particular in form of an emitter and/or host;
b) one or more emitter and/or host materials different from the at least one organic molecule according to claim 1 to 8, and
c) optionally one or more dyes and/or one or more solvents.

**13.** An organic optoelectronic device, comprising an organic molecule according to claim 1 to 8 or a composition according to claim 12, in particular formed as a device selected from the group consisting of organic light emitting diode (OLED), light emitting electrochemical cell, OLED sensor, in particular non-hermetically outwardly shielded gas and vapor sensors, organic diode, organic solar cell, organic transistor, organic field-effect transistor, organic laser and down-conversion element.

**14.** The organic optoelectronic device according to claim 13, comprising:

- a substrate,
- an anode, and
- a cathode, wherein the anode or the cathode is applied to the substrate, and
- at least one light emitting layer disposed between anode and cathode and which comprises an organic molecule according to claim 1 to 8 or a composition according to claim 12.

**15.** The method for producing an optoelectronic device, wherein an organic molecule according to claim 1 to 8 is used, in particular comprising processing the organic molecule by means of a vacuum evaporation method or from a solution.

## Revendications

**1.** Molécule organique présentant

- une première entité chimique présentant ou consistant en une structure selon la formule I

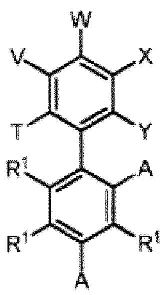

Formule I

et
- deux deuxièmes unités chimiques D, qui sont respectivement identiques ou différentes à chaque occurrence, présentant une structure ou consistant en une structure selon la formule II,

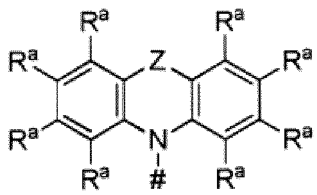

Formule II

la première unité chimique étant liée à chacune des deux deuxièmes unités chimiques D par une liaison simple ; avec

T est le point de fixation de la liaison simple entre la première unité chimique et une deuxième unité chimique D ou est H ;
V est le point de fixation de la liaison simple entre la première unité chimique et une deuxième unité chimique D ou est H ;
W est le point de fixation de la liaison simple entre l'unité chimique et une deuxième unité chimique D ou est choisi dans le groupe constitué par H, CN et $CF_3$ ;
X est le point de fixation de la liaison simple entre la première unité chimique et une deuxième unité chimique D ou est choisi dans le groupe constitué par H, CN et $CF_3$ ;
Y est le point de fixation de la liaison simple entre la première unité chimique et une deuxième unité chimique D ou est choisi dans le groupe constitué par H, CN et $CF_3$ ;
# indique le point de fixation de la liaison simple entre une deuxième unité chimique D et la première unité chimique ;
Z est, à chaque occurrence identique ou différent, une liaison directe ou est choisi dans le groupe constitué par $CR^3$ $R^4$, C=$CR^3$ $R^4$, C=0, C=$NR^3$, $NR^3$, O, $SiR^3R^4$, S, S(O) et $S(O)_2$ ;
A est CN ou $CF_3$ à chaque occurrence ;
$R^1$ est, à chaque occurrence, identique ou différent de H, du deutérium, un groupe alkyle linéaire ayant de 1 à 5 atomes de carbone, un ou plusieurs atomes de H pouvant à chaque fois être remplacés par du deutérium ; un groupe alcényle ou alcynyle linéaire ayant de 2 à 8 atomes de carbone, un ou plusieurs atomes de H pouvant à chaque fois être remplacés par du deutérium ; un groupe alkyle, alcényle ou alcynyle ramifié ou cyclique ayant de 3 à 10 atomes de carbone, un ou plusieurs atomes d'hydrogène pouvant être remplacés par du deutérium dans chaque cas ; ou un système cyclique aromatique ou hétéroaromatique ayant de 5 à 15 atomes cycliques aromatiques, dont chacun peut être substitué par un ou plusieurs radicaux $R^6$ ;
$R^a$, $R^3$ et $R^4$, identiques ou différents à chaque occurrence, représentent H, le deutérium, $N(R^5)_2$, OH, $Si(R^5)_3$, $B(OR^5)_2$, $OSO_2R^5$, $CF_3$, CN, F, Br, I, un groupe alkyle, alcoxy ou thioalcoxy linéaire ayant de 1 à 40 atomes de carbone, dont chacun peut être substitué par un ou plusieurs radicaux $R^5$, où un ou plusieurs groupes $CH_2$ non adjacents peuvent être remplacés par $R^5C=CR^5$, C=C, $Si(R^5)_2$, $Ge(R^5)_2$, $Sn(R^5)_2$, C=0, C=S, C=Se, C=$NR^5$, P(=O)( $R^5$), SO, $SO_2$, $NR^5$, O, S ou $CONR^5$ et où un ou plusieurs atomes H peuvent être remplacés par du deutérium, CN, $CF_3$ ou $NO_2$ ; ou un groupe alcényle ou alcynyle linéaire ayant de 2 à 40 atomes de carbone, dont chacun peut être substitué par un ou plusieurs radicaux $R^5$ ; un ou plusieurs groupes $CH_2$ non adjacents pouvant être remplacés par $R^5$ C=$CR^5$, C≡C, $Si(R^5)_2$, $Ge(R^5)_2$, $Sn(R^5)_2$, C=0, C=S, C=Se, C=$NR^5$, P(=O)( $R^5$), SO, $SO_2$, $NR^5$, O, S ou $CONR^5$ et où un ou plusieurs atomes H peuvent être remplacés par du deutérium, CN, $CF_3$ ou $NO_2$ ; ou un groupe alkyle, alcényle, alcynyle, alcoxy ou thioalcoxy ramifié ou cyclique ayant de 3 à 40 atomes de carbone, dont chacun peut être substitué par un ou plusieurs radicaux $R^5$, un ou plusieurs groupes $CH_2$ non adjacents pouvant être remplacés par $R^5$ C=$CR^5$, C=C, $Si(R^5)_2$, $Ge(R^5)_2$, $Sn(R^5)_2$, C=O, C=S, C=Se, C=$NR^5$, P(=O)( $R^5$), SO, $SO_2$, $NR^5$, O, S ou $CONR^5$ et où un ou plusieurs atomes H peuvent être remplacés par du deutérium, CN, $CF_3$ ou $NO_2$ ; ou un système cyclique aromatique ou hétéroaromatique ayant de 5 à 60 atomes cycliques aromatiques, dont chacun peut être substitué par un ou plusieurs radicaux $R^5$, ou un groupe aryloxy ou hétéroaryloxy ayant de 5 à 60 atomes cycliques aromatiques, dont chacun peut être substitué par un ou plusieurs radicaux $R^5$, ou un groupe diarylamino, un groupe dihétéroarylamino ou un groupe arylhétéroarylamino ayant de 10 à 40 atomes cycliques aromatiques, dont chacun peut être substitué par un ou plusieurs radicaux $R^5$ ;
$R^5$ est, à chaque occurrence, identique ou différent, H, deutérium, $N(R^6)_2$, OH, $Si(R^6)_3$, $B(OR^6)_2$, $OSO_2R^6$, $CF_3$, CN, F, Br, I, un groupe alkyle, alcoxy ou thioalcoxy linéaire ayant de 1 à 40 atomes de carbone, dont chacun peut être substitué par un ou plusieurs radicaux $R^6$, où un ou plusieurs groupes $CH_2$ non adjacents

peuvent être remplacés par $R^6C=CR^6$, C=C, $Si(R^6)_2$, $Ge(R^6)_2$, $Sn(R^6)_2$, C=0, C=S, C=Se, $C=NR^6$, $P(=O)(R^6)$, SO, $SO_2$, $NR^6$, O, S ou $CONR^6$ et où un ou plusieurs atomes H peuvent être remplacés par du deutérium, CN, $CF_3$ ou $NO_2$ ; ou un groupe alcényle ou alcynyle linéaire ayant de 2 à 40 atomes de carbone, dont chacun peut être substitué par un ou plusieurs radicaux $R^6$, un ou plusieurs groupes $CH_2$ non adjacents étant remplacés par $R^6C=CR^6$, C=C, $Si(R^6)_2$, $Ge(R^6)_2$, $Sn(R^6)_2$, CO, C=S, C=Se, $C=NR^6$, $P(=O)(R^6)$, SO, $SO_2$, $NR^6$, O, S ou $CONR^6$ et où un ou plusieurs atomes H peuvent être remplacés par du deutérium, CN, $CF_3$ ou $NO_2$ ; ou un groupe alkyle, alcényle, alcynyle, alcoxy ou thioalcoxy ramifié ou cyclique ayant de 3 à 40 atomes de carbone, dont chacun peut être substitué par un ou plusieurs radicaux $R^6$, un ou plusieurs groupes $CH_2$ non adjacents pouvant être remplacés par $R^6C=CR^6$, C=C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ et où un ou plusieurs atomes H peuvent être remplacés par du deutérium, CN, $CF_3$ ou $NO_2$ ; ou un système cyclique aromatique ou hétéroaromatique ayant de 5 à 60 atomes cycliques aromatiques, dont chacun peut être substitué par un ou plusieurs radicaux $R^6$, ou un groupe aryloxy ou hétéroaryloxy ayant de 5 à 60 atomes cycliques aromatiques, dont chacun peut être substitué par un ou plusieurs radicaux $R^6$, ou un groupe diarylamino, un groupe dihétéroarylamino ou un groupe arylhétéroarylamino ayant de 10 à 40 atomes cycliques aromatiques, dont chacun peut être substitué par un ou plusieurs radicaux $R^6$ ;

$R^6$ est, à chaque occurrence, identique ou différent, H, deutérium, OH, $CF_3$, CN, F, un groupe alkyle, alcoxy ou thioalcoxy linéaire ayant de 1 à 5 atomes de carbone, un ou plusieurs atomes de H pouvant à chaque fois être remplacés par du deutérium, CN, $CF_3$ ou $NO_2$ ; ou un groupe alcényle ou alcynyle linéaire ayant de 2 à 5 atomes de carbone, un ou plusieurs atomes de H pouvant à chaque fois être remplacés par du deutérium, CN, $CF_3$ ou $NO_2$ ; ou un groupe alkyle, alcényle, alcynyle, alcoxy ou thioalcoxy ramifié ou cyclique ayant de 3 à 5 atomes de carbone, dans lequel un ou plusieurs atomes d'hydrogène peuvent être remplacés par du deutérium, CN, $CF_3$ ou $NO_2$ ; ou un système cyclique aromatique ou hétéroaromatique comportant 5 à 60 atomes aromatiques dans le cycle ou un groupe aryloxy ou hétéroaryloxy comportant 5 à 60 atomes aromatiques dans le cycle ou un groupe diarylamino, un groupe dihétéroarylamino ou un groupe arylhétéroarylamino comportant 10 à 40 atomes aromatiques dans le cycle ;

où chacun des radicaux $R^a$, $R^3$, $R^4$ ou $R^5$ peut également former avec un ou plusieurs autres radicaux $R^a$, $R^3$, $R^4$ ou $R^5$ un système cyclique mono- ou polycyclique, aliphatique, aromatique et/ou benzocondensé ;

où un A est $CF_3$ et l'autre A est CN ;

où exactement un radical choisi dans le groupe constitué par W, X et Y est CN ou $CF_3$ et exactement deux radicaux choisis dans le groupe constitué par T, V, W, X et Y sont égaux à un point de fixation de la liaison simple entre la première unité chimique et une deuxième unité D.

2. Molécule organique selon la revendication 1, dans laquelle la première unité chimique a une structure de formule Ia ou est constituée d'une telle structure :

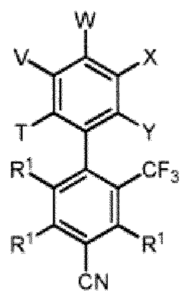

Formule Ia

où $R^1$, T, V, W, X et Y sont tels que définis dans la revendication 1.

3. Molécule organique selon la revendication 1 ou 2, dans laquelle $R^1$ est, à chaque occurrence, identique ou différent de H, méthyle ou phényle.

4. Molécule organique selon les revendications 1 à 3, dans laquelle W est CN.

5. Molécule organique selon les revendications 1 à 4, dans laquelle la deuxième unité chimique D présente, à chaque occurrence, de manière identique ou différente, une structure de formule IIa ou est constituée d'une telle structure :

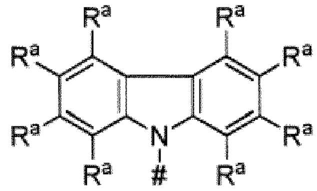

Formule IIa

où # et $R^a$ sont tels que définis dans la revendication 1.

6. Molécule organique selon les revendications 1 à 5, dans laquelle chaque deuxième unité chimique D a une structure de formule IIb ou est constituée d'une telle structure :

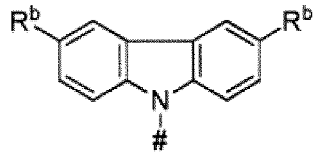

Formule IIb

où

$R^b$, identique ou différent à chaque occurrence, représente $N(R^5)_2$, OH, $Si(R^5)_3$, $B(OR^5)_2$, $OSO_2R^5$, $CF_3$, CN, F, Br, I, un groupe alkyle, alcoxy ou thioalcoxy linéaire ayant de 1 à 40 atomes de carbone, qui peut être substitué à chaque fois par un ou plusieurs radicaux $R^5$, où un ou plusieurs groupes $CH_2$ non adjacents peuvent être remplacés par $R^5C=CR^5$, C=C, $Si(R^5)_2$, $Ge(R^5)_2$, $Sn(R^5)_2$, C=0, C=S, C=Se, $C=NR^5$, $P(=O)(R^5)$, SO, $SO_2$, $NR^5$, O, S ou $CONR^5$ et où un ou plusieurs atomes H peuvent être remplacés par du deutérium, CN, $CF_3$ ou $NO_2$ ; ou un groupe alcényle ou alcynyle linéaire ayant de 2 à 40 atomes de carbone, dont chacun peut être substitué par un ou plusieurs radicaux $R^5$, un ou plusieurs groupes $CH_2$ non adjacents étant remplacés par $R^5C=CR^5$, C=C, $Si(R^5)_2$, $Ge(R^5)_2$, $Sn(R^5)_2$, C=0, C=S, C=Se, $C=NR^5$, $P(=0)(R^5)$, SO, $SO_2$, $NR^5$, O, S ou $CONR^5$ et où un ou plusieurs atomes H peuvent être remplacés par du deutérium, CN, $CF_3$ ou $NO_2$ ; ou un groupe alkyle, alcényle, alcynyle, alcoxy ou thioalcoxy ramifié ou cyclique ayant de 3 à 40 atomes de carbone, dont chacun peut être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=O, C=S, C=Se, $C=NR^5$, $P(=0)(R^5)$, SO, $SO_2$, $NR^5$, O, S ou $CONR^5$ et où un ou plusieurs atomes H peuvent être remplacés par du deutérium, CN, $CF_3$ ou $NO_2$ ; ou un système cyclique aromatique ou hétéroaromatique comportant 5 à 60 atomes cycliques aromatiques, dont chacun peut être substitué par un ou plusieurs radicaux $R^5$, ou un groupe aryloxy ou hétéroaryloxy comportant 5 à 60 atomes cycliques aromatiques, qui peut être substitué dans chaque cas par un ou plusieurs radicaux $R^5$, ou un groupe diarylamino, un groupe dihétéroarylamino ou un groupe arylhétéroarylamino comportant 10 à 40 atomes cycliques aromatiques, qui peut être substitué dans chaque cas par un ou plusieurs radicaux $R^5$ ;
et pour # et $R^5$, les définitions mentionnées dans la revendication 1 s'appliquent.

7. Molécule organique selon les revendications 1 à 5, dans laquelle la deuxième unité chimique D présente respectivement une structure de formule IIc ou est constituée d'une telle structure :

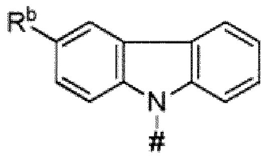

Formule IIc

où

$R^b$, identique ou différent à chaque occurrence, représente $N(R^5)_2$, OH, $Si(R^5)_3$, $B(OR^5)_2$, $OSO_2R^5$, $CF_3$, CN, F, Br, I, un groupe alkyle, alcoxy ou thioalcoxy linéaire ayant de 1 à 40 atomes de carbone, qui peut être substitué à chaque fois par un ou plusieurs radicaux $R^5$, où un ou plusieurs groupes $CH_2$ non adjacents peuvent être remplacés par $R^5C=CR^5$, C=C, $Si(R^5)_2$, $Ge(R^5)_2$, $Sn(R^5)_2$, C=0, C=S, C=Se, $C=NR^5$, $P(=O)(R^5)$, SO, SO 2, $NR^5$, O, S ou $CONR^5$ et où un ou plusieurs atomes H peuvent être remplacés par du deutérium, CN, $CF_3$ ou $NO_2$ ; ou un groupe alcényle ou alcynyle linéaire ayant de 2 à 40 atomes de carbone, dont chacun peut être substitué par un ou plusieurs radicaux $R^5$, un ou plusieurs groupes $CH_2$ non adjacents étant remplacés par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ et où un ou plusieurs atomes H peuvent être remplacés par du deutérium, CN, CF3 ou $NO_2$ ; ou un groupe alkyle, alcényle, alcynyle, alcoxy ou thioalcoxy ramifié ou cyclique ayant de 3 à 40 atomes de carbone, dont chacun peut être substitué par un ou plusieurs radicaux $R^5$, un ou plusieurs groupes $CH_2$ non adjacents pouvant être remplacés par $R^5C=CR^5$, C=C, $Si(R^5)_2$, $Ge(R^5)_2$, $Sn(R^5)_2$, C=0, C=S, C=Se, $C=NR^5$, $P(=O)(R^5)$, SO, $SO_2$, $NR^5$, O, S ou $CONR^5$ et où un ou plusieurs atomes H peuvent être remplacés par du deutérium, CN, $CF_3$ ou $NO_2$ ; ou un système cyclique aromatique ou hétéroaromatique comportant 5 à 60 atomes cycliques aromatiques, dont chacun peut être substitué par un ou plusieurs radicaux $R^5$, ou un groupe aryloxy ou hétéroaryloxy comportant 5 à 60 atomes cycliques aromatiques, qui peut être substitué dans chaque cas par un ou plusieurs radicaux $R^5$, ou un groupe diarylamino, un groupe dihétéroarylamino ou un groupe arylhétéroarylamino comportant 10 à 40 atomes cycliques aromatiques, qui peut être substitué dans chaque cas par un ou plusieurs radicaux $R^5$ ;

et, pour le reste, les définitions mentionnées dans la revendication 1 s'appliquent.

**8.** Molécule organique selon la revendication 6 ou 7, dans laquelle $R^b$, identique ou différent à chaque occurrence, est Me, $^iPr$, $^tBu$, CN, $CF_3$ ou Ph, dont chacun peut être substitué par un ou plusieurs radicaux choisis parmi Me, $^iPr$, $^tBu$, CN, $CF_3$ ou Ph, pyridinyle, dont chacun peut être substitué par un ou plusieurs radicaux choisis parmi Me, $^iPr$, $^tBu$, CN, $CF_3$ ou Ph, pyrimidinyle, dont chacun peut être substitué par un ou plusieurs radicaux choisis parmi Me, $^iPr$, $^tBu$, CN, $CF_3$ ou Ph, un triazinyle dont chacun peut être substitué par un ou plusieurs radicaux choisis parmi Me, $^iPr$, $^tBu$, CN, $CF_3$ ou Ph, ou un carbazolyle dont chacun peut être substitué par un ou plusieurs radicaux choisis parmi Me, $^iPr$, $^tBu$, CN, $CF_3$ ou Ph.

**9.** Procédé de préparation d'une molécule organique selon les revendications 1 à 8, dans lequel un trifluorométhylbenzonitrile substitué par un halogène est utilisé comme réactif, dans lequel le substituant halogène est en position 4 et le substituant trifluorométhyle est en position 3 ou le substituant halogène est en position 2 et le substituant trifluorométhyle est en position 5.

**10.** Utilisation d'une molécule organique selon les revendications 1 à 8 comme émetteur luminescent et/ou comme matériau hôte et/ou comme matériau de transport d'électrons et/ou comme matériau d'injection de trous et/ou comme matériau de blocage de trous dans un dispositif optoélectronique organique.

**11.** Utilisation selon la revendication 10, dans laquelle le dispositif optoélectronique organique est choisi dans le groupe constitué par :

- diodes électroluminescentes organiques (DELO),
- cellules électrochimiques émettant de la lumière,
- capteurs DELO, en particulier dans les capteurs de gaz et de vapeur qui ne sont pas hermétiquement protégés de l'extérieur,
- diodes organiques,
- cellules solaires organiques,
- transistors organiques,
- transistors organiques à effet de champ,
- lasers organiques et
- éléments de conversion vers le bas.

**12.** Composition présentant ou constituée par :

(a) au moins une molécule organique selon l'une quelconque des revendications 1 à 8, en particulier sous la forme d'un émetteur et/ou d'un hôte, et

(b) un ou plusieurs matériaux émetteurs et/ou hôtes qui sont différents de la molécule organique selon les revendications 1 à 8 ; et

(c) éventuellement, un ou plusieurs colorants et/ou un ou plusieurs solvants.

**13.** Dispositif optoélectronique organique présentant une molécule organique selon les revendications 1 à 8 ou une composition selon la revendication 12, en particulier formé comme un dispositif choisi dans le groupe constitué par une diode électroluminescente organique (DELO), une cellule électrochimique émettant de la lumière, un capteur DELO, en particulier des capteurs de gaz et de vapeur non hermétiquement protégés de l'extérieur, une diode organique, une cellule solaire organique, un transistor organique, un transistor à effet de champ organique, un laser organique et un élément de conversion vers le bas.

**14.** Dispositif optoélectronique organique selon la revendication 13, présentant

- un substrat,
- une anode et
- une cathode, l'anode ou la cathode étant appliquée sur le substrat, et
- au moins une couche émettrice de lumière, qui est disposée entre l'anode et la cathode et qui présente une molécule organique selon les revendications 1 à 8 ou une composition selon la revendication 12.

**15.** Procédé de fabrication d'un dispositif optoélectronique, dans lequel on utilise une molécule organique selon les revendications 1 à 8, comprenant notamment le traitement de la molécule organique par un procédé d'évaporation sous vide ou à partir d'une solution.

**Figur 1**

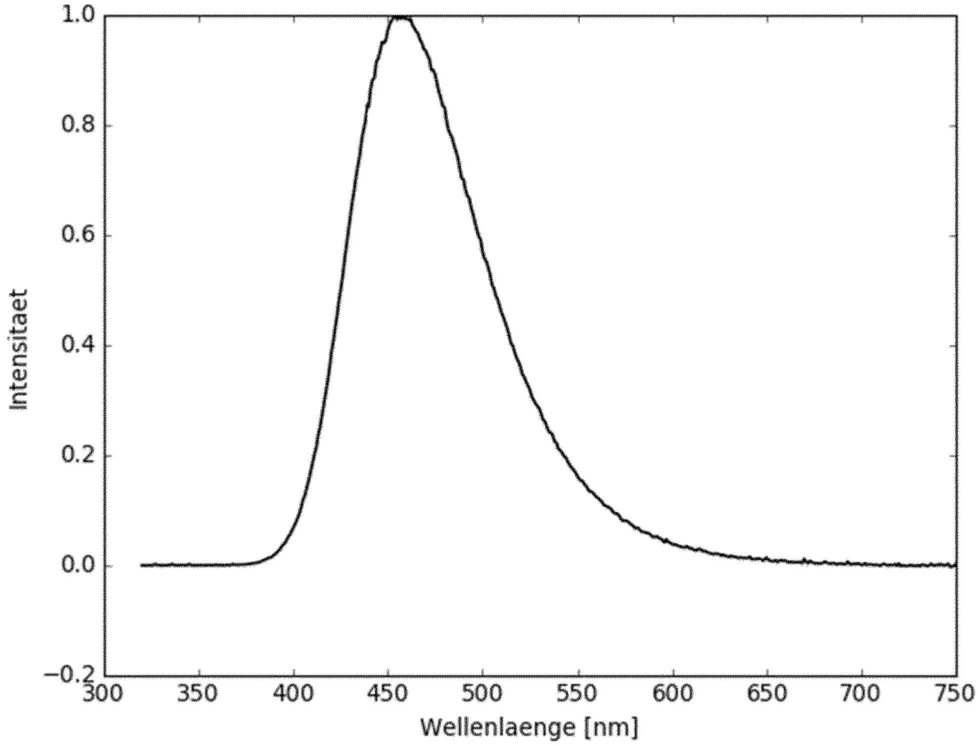


**Figur 2**

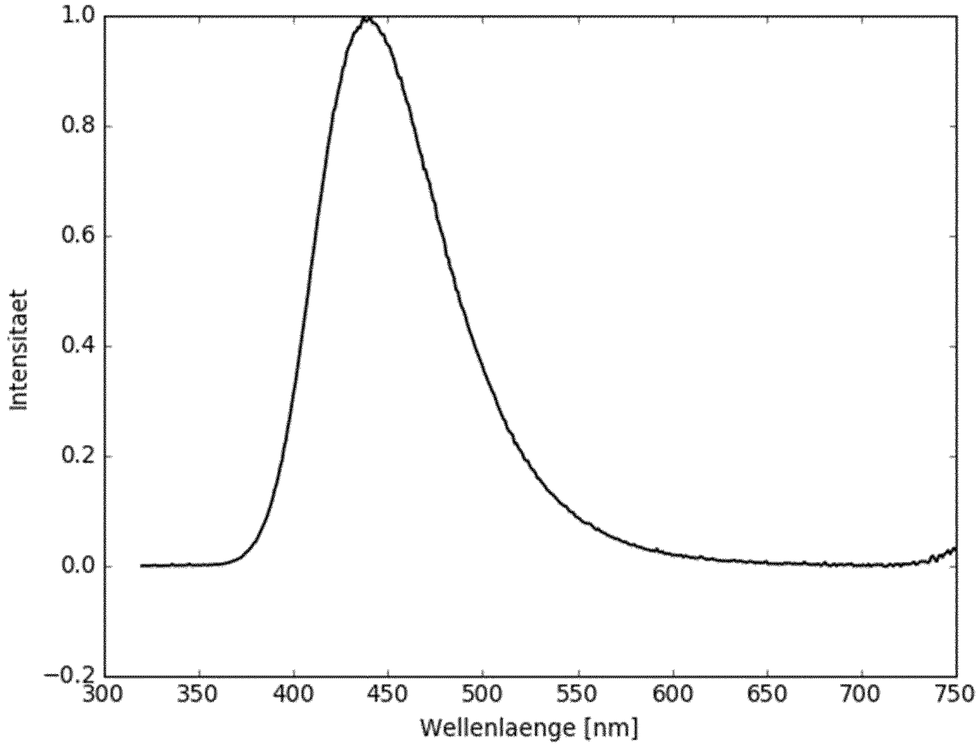

Figur 3

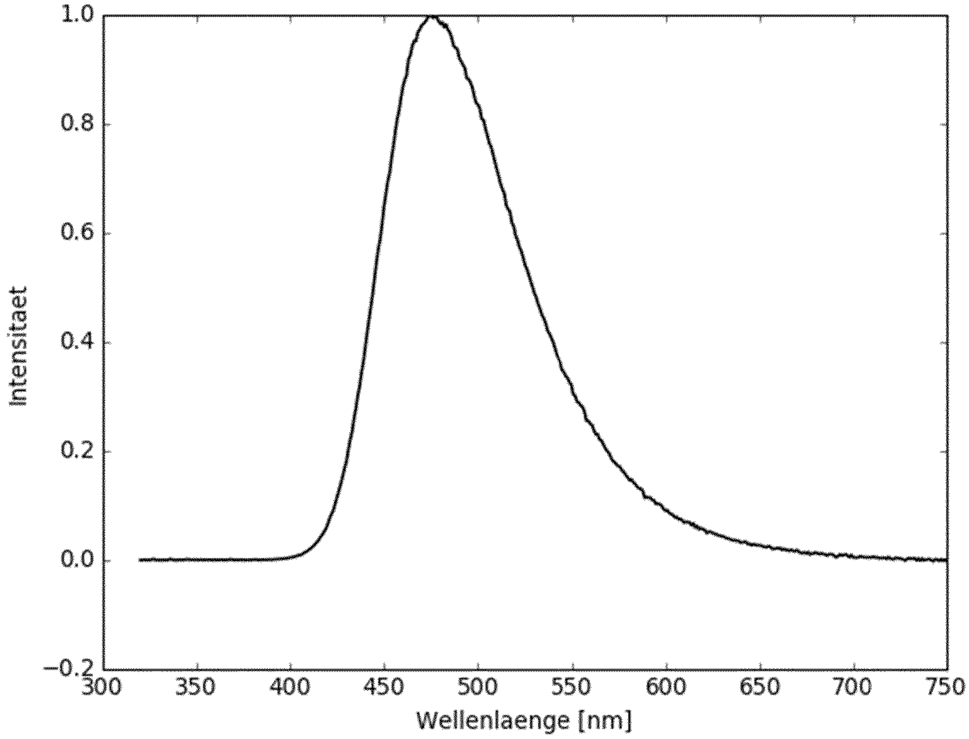


Figur 4

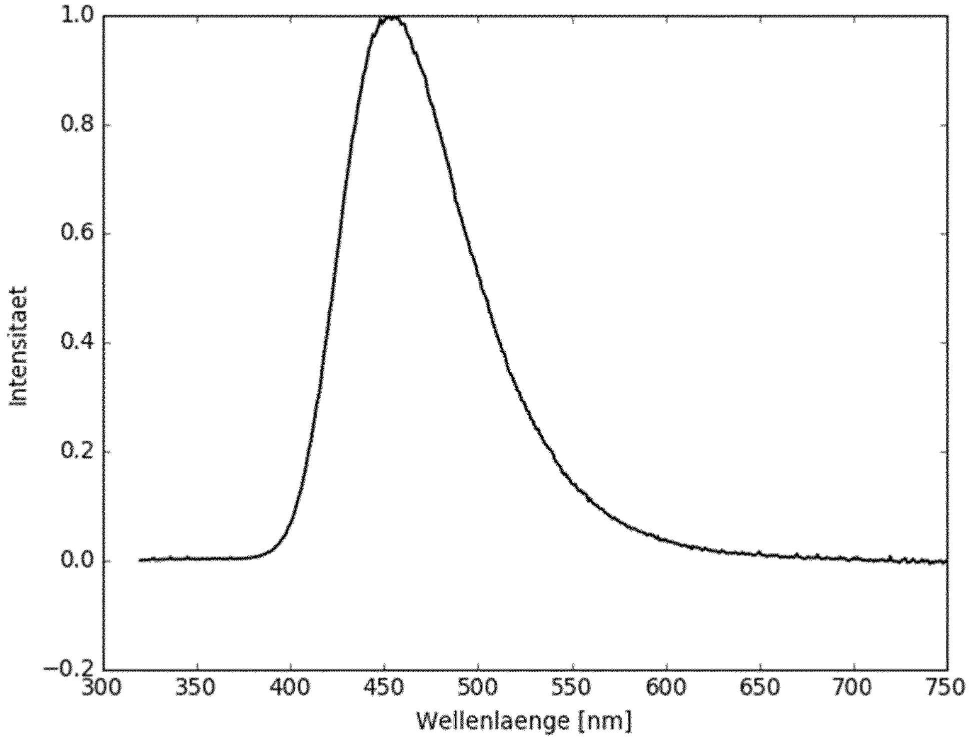

Figur 5

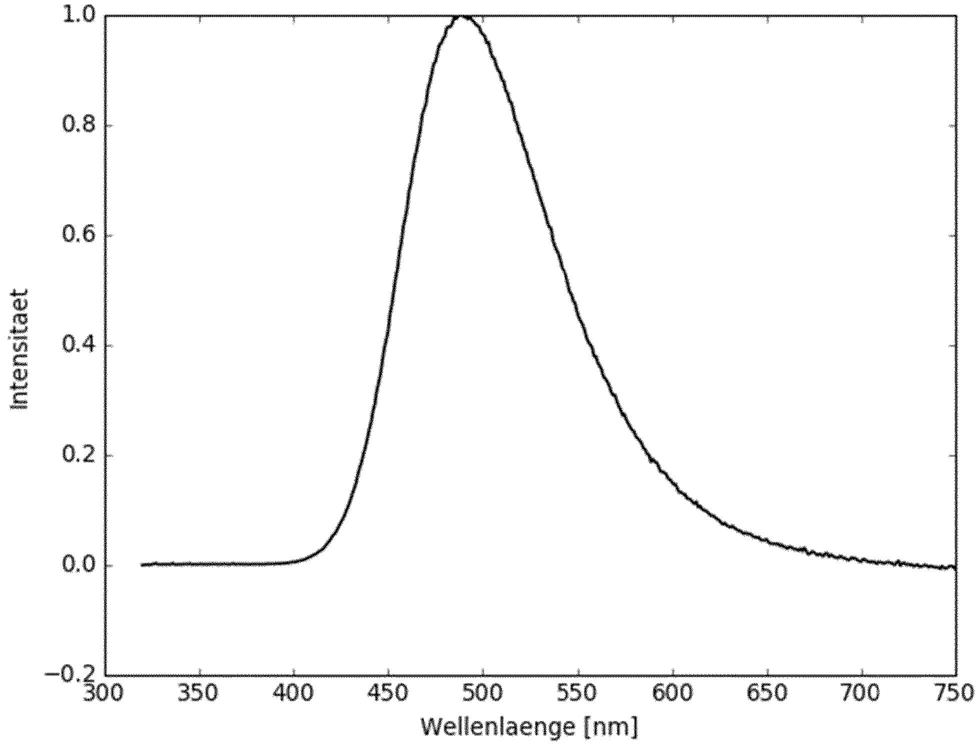


Figur 6

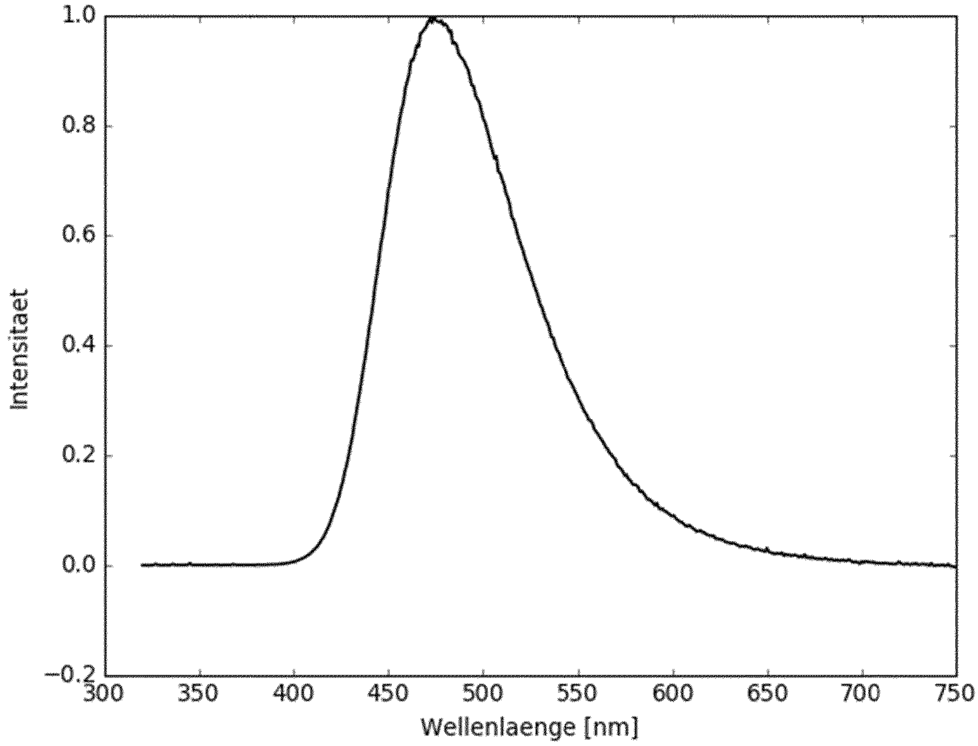

Figur 7

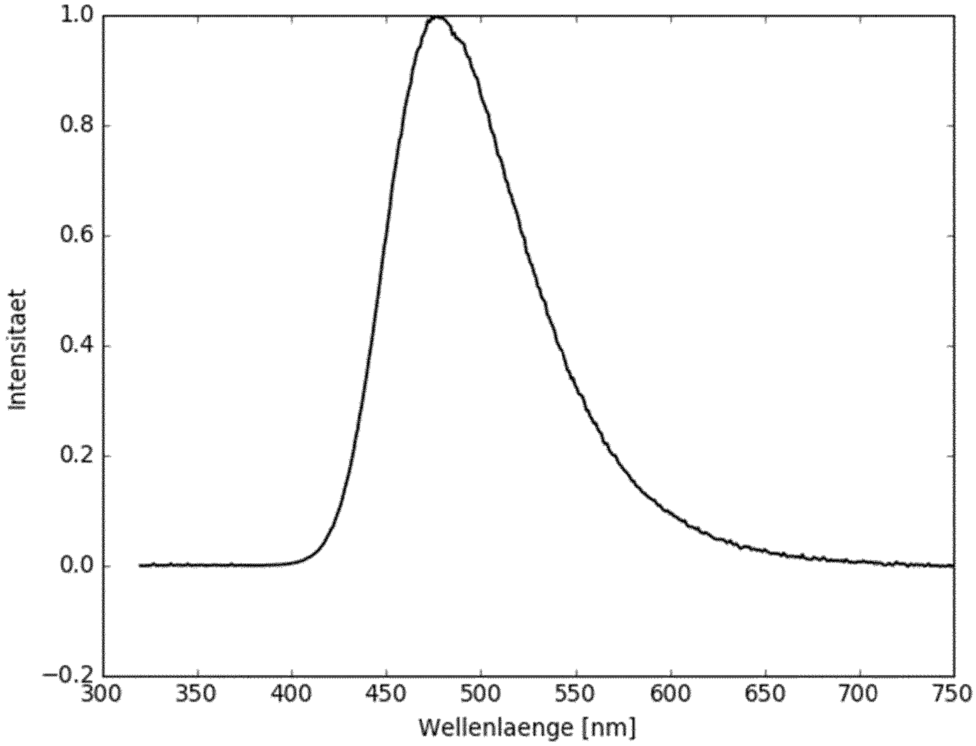


Figur 8

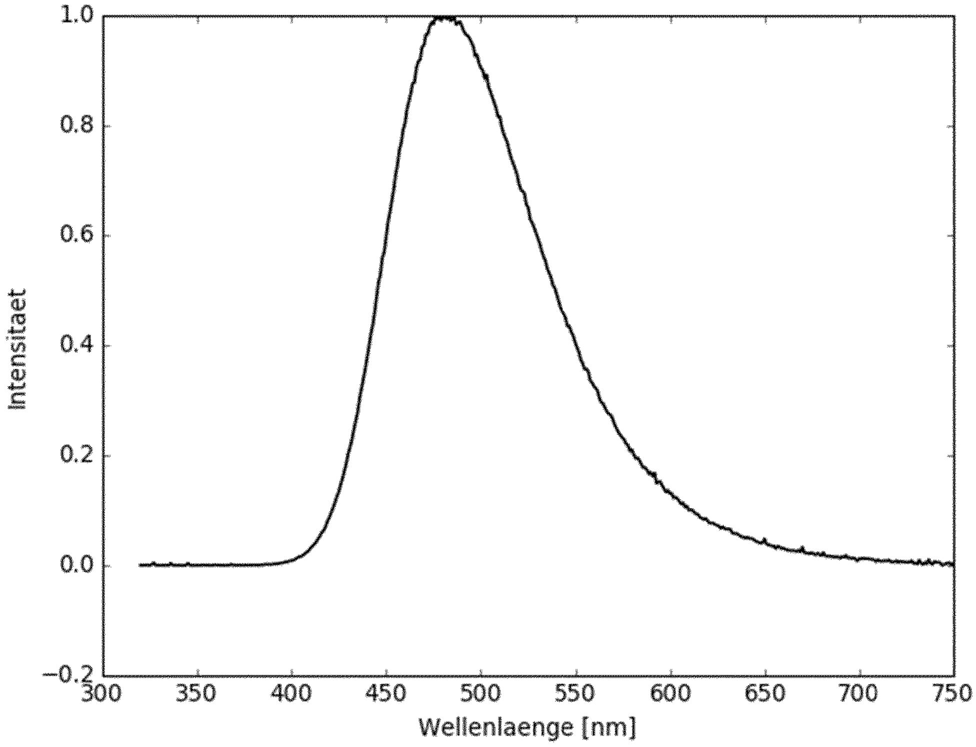

Figur 9

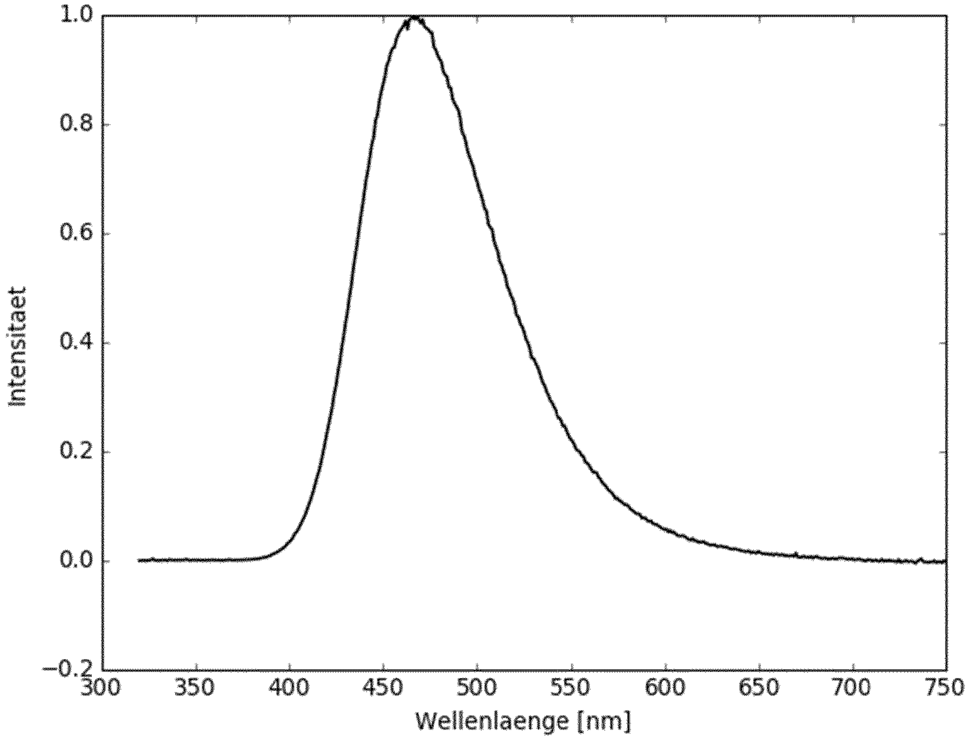


Figur 10

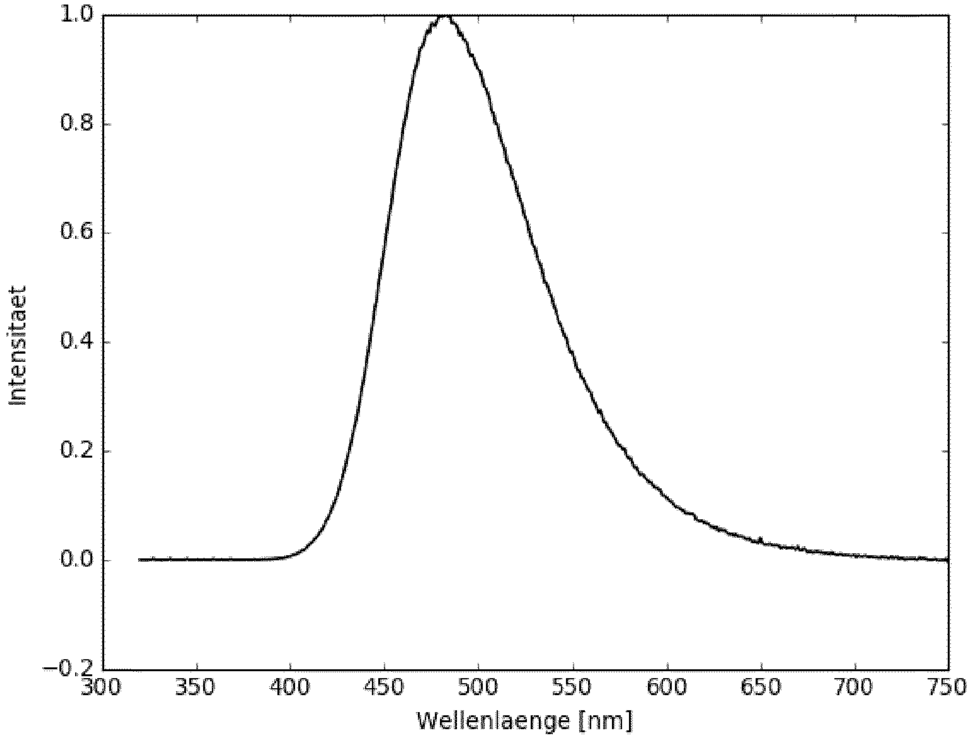

Figur 11

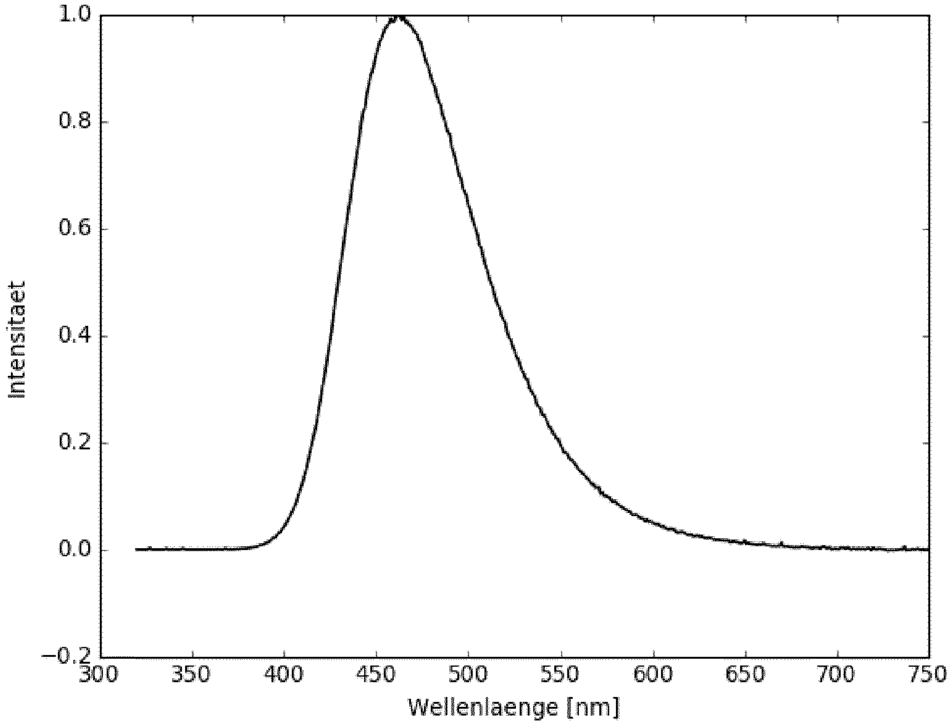


Figur 12

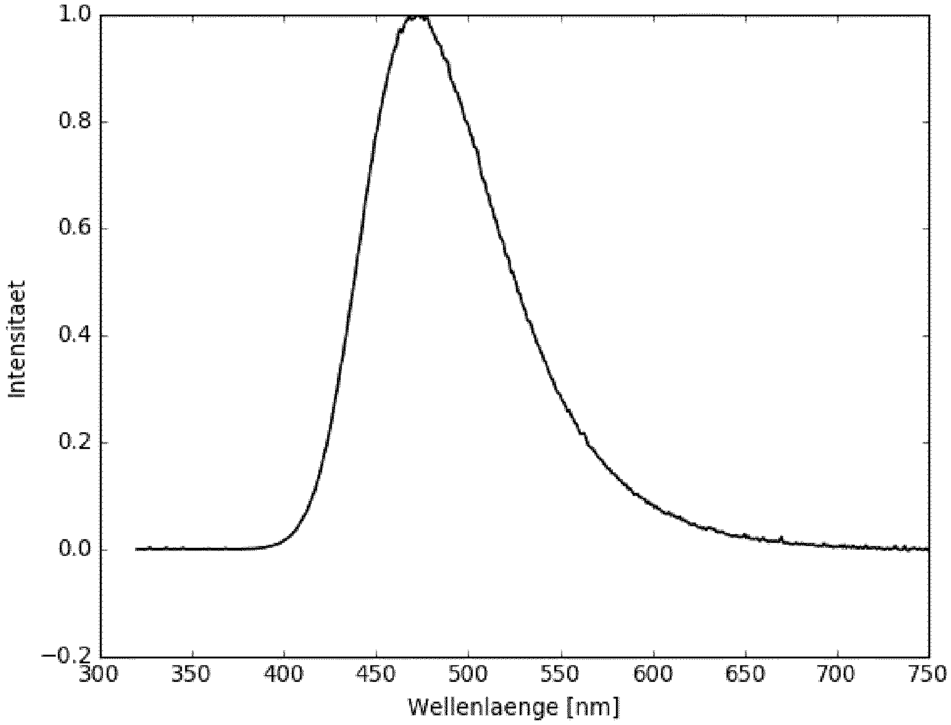

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2016126474 A1